(19) Europäisches Patentamt
European Patent Office
Office européen des brevets

(11) **EP 3 039 044 B1**

(12) **EUROPEAN PATENT SPECIFICATION**

(45) Date of publication and mention
of the grant of the patent:
**12.02.2020 Bulletin 2020/07**

(21) Application number: **14711267.6**

(22) Date of filing: **20.03.2014**

(51) Int Cl.:
**C08F 220/06** (2006.01) **A61L 15/22** (2006.01)

(86) International application number:
**PCT/EP2014/055611**

(87) International publication number:
**WO 2015/028158 (05.03.2015 Gazette 2015/09)**

(54) **FLUID-ABSORBENT ARTICLE**

FLÜSSIGKEITSAUFNEHMENDER ARTIKEL

ARTICLE ABSORBANT LES FLUIDES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **26.08.2013 EP 13181703**
**30.10.2013 EP 13190781**
**29.01.2014 EP 14152979**

(43) Date of publication of application:
**06.07.2016 Bulletin 2016/27**

(73) Proprietor: **BASF SE**
**67056 Ludwigshafen am Rhein (DE)**

(72) Inventors:
• **SCHRÖDER, Ulrich**
**67227 Frankenthal (DE)**
• **FUNK, Rüdiger**
**65527 Niedernhausen (DE)**
• **LOUDEN, John Joseph**
**Manchester M25 1NJ (GB)**

(74) Representative: **BASF IP Association**
**BASF SE**
**G-FLP-C006**
**67056 Ludwigshafen (DE)**

(56) References cited:
**EP-A1- 2 524 679     WO-A1-2014/079694**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

The present invention relates to a fluid-absorbent article, comprising a water-absorbent core with a basis weight of the fluid-absorbent core at the insult zone of at least 500 gsm, comprising 10 to 100% by weight water-absorbent polymer particles,
and the water-absorbent polymer particles having a mean sphericity (SPHT) from 0.8 to 0.95 and satisfying the following conditions:

(a) the sum of centrifuge retention capacity (CRC) and absorbency under high load (AUHL) being at least 60 g/g, and/or
(b) the $\log_{10}$(SFC) > 5.7 - 0.138 x CRC, with SFC being not less than 5,

wherein the CRC is given in g/g and the SFC in $10^{-7} cm^3 \cdot S/g$.

The production of fluid-absorbent articles is described in the monograph "Modern Superabsorbent Polymer Technology", F.L. Buchholz and A.T. Graham, Wiley-VCH, 1998, pages 252 to 258.

Fluid-absorbent articles consist typically of an upper liquid-pervious top-sheet, a lower liquid-impervious layer, an absorption and distribution layer and fluid-absorbing composite between the top-sheet and the liquid-impervious layer. The composite consists of water-absorbing polymers and fibers. Further layers are, for example tissue layers.

The preparation of water-absorbing polymer particles is likewise described in the monograph "Modern Superabsorbent Polymer Technology", F.L. Buchholz and A.T. Graham, Wiley-VCH, 1998, pages 71 to 103. The water-absorbing polymer particles are also referred to as "fluid-absorbing polymer particles", "superabsorbent polymers" or "superabsorbents".

The preparation of water-absorbent polymer particles by polymerizing droplets of a monomer solution is described, for example, in EP 0 348 180 A1, WO 96/40427 A1, US 5,269,980, WO 2008/009580 A1, WO 2008/052971 A1, WO2011/026876 A1, and WO 2011/117263 A1.

Polymerization of monomer solution droplets in a gas phase surrounding the droplets ("dropletization polymerization") affords round water-absorbent polymer particles of high mean sphericity (mSPHT). The mean sphericity is a measure of the roundness of the polymer particles and can be determined, for example, with the Camsizer® image analysis system (Retsch Technology GmbH; Haan; Germany).

Usually the several layers of fluid-absorbent articles fulfill definite functions such as dryness for the upper liquid-pervious layer, vapor permeability without wetting through for the lower liquid-impervious layer, a flexible, vapor permeable and thin fluid-absorbent core, showing fast absorption rates and being able to retain highest quantities of body fluids, and an acquisition-distribution layer between the upper layer and the core, acting as transport and distribution layer of the discharged body fluids.

These individual elements are combined such that the resultant fluid-absorbent article meets overall criteria such as flexibility, water vapour breathability, dryness, wearing comfort, protection and also performance criteria such as high liquid retention, low rewet and prevention of wet through. The specific combination of these layers provides a fluid-absorbent article delivering both high protection levels as well as high comfort to the consumer.

Besides improved fluid acquisition and distribution behavior in optimized fluid-absorbent articles resulting in better "dry feeling", higher comfort also results from lower noise generation during the wearing of fluid-absorbent articles. As movement of the wearer and mechanical stress in general causes noises, it is a problem especially for adults indicating persons to wear a fluid-absorbent article. Psychological discomfort results, to solve this problem additional layers or films rendering weakness to the fluid-absorbent article are added, as e.g. written in EP 1562747 B1, where a breathable multi-microlayerfilm having a reduced noisiness and improved softness is integrated.

Higher comfort is further connected to improved softness of the fluid-absorbent article, concerning the skin-related feeling by wearing it. This problem is often solved by adding additional construction layers, webs or films. Other attempts are made disclosed in DE 10107709 A1, offering absorbent articles containing cellulose fibres which are at least partly in the form of granules. Another attempt towards absorbent structures with improved softness is made by BKI Holding in US 7176149 B, claiming high-performance absorbent structures having softness of higher than 8.0/J and a pliability higher than about 70/N. Again, this absorbent article is a construction of several layers of different fibrous material to form the core.

But all attempts to reduce noise or increase softness are resulting in increased thickness of the absorbent core and the absorbent article respectively, as several layers are added. As the thickness is also a great issue in respect to absorbent articles especially in respect to noticeability for adult articles and also hindrance, especially for baby diapers and pants.

One additional problem especially in diapers is the pinholing. To prevent this effect, special multilayer elastomeric films are developed, including olefin based and non-olefin based polymers (WO 2009094506 A1).
Several layers require more material and therefore are cost intensive. Simple constructions without any such additional layers require less material, are easier to produce and cheaper.

Thus, there is a need for Fluid-absorbent articles, showing the best possible liquid acquisition and retention behavior and furthermore also exhibit improved softness and reduced noise.

It is therefore an object of the present invention to provide fluid-absorbent articles with improved possible liquid acquisition and retention behavior.

It is also an object of the present invention to provide ultrathin-fluid-absorbent articles with improved rewet performance.

Furthermore it is an object of the present invention to provide fluid-aborbent articles with improved softness and reduced noise.

It is also an object of the present invention to provide fluid-aborbent articles with less pinholing.

The object is achieved by a fluid-absorbent article, comprising

(A) an upper liquid-pervious layer,
(B) a lower liquid-impervious layer,
(C) a fluid-absorbent core between the layer (A) and the layer (B), comprising from 0 to 90% by weight fibrous material and from 10 to 100% by weight water-absorbent polymer particles, based on the sum of water-absorbent polymer particles and fibrous material; wherein the basis weight of the fluid-absorbent core at the insult zone is of at least 500 gsm,
(D) an optional acquisition-distribution layer between (A) and (C),
(E) an optional tissue layer disposed immediately above and/or below (C); and
(F) other optional components,
wherein the water-absorbent polymer particles having a mean sphericity (SPHT) from 0.8 to 0.95 and satisfying the following conditions:

(c) the sum of centrifuge retention capacity (CRC) and absorbency under high load (AUHL) being at least 60 g/g, and/or
(d) the $\log_{10}(SFG) > 5.7 - 0.138 \times CRC$, with SFC being not less than 5,
wherein the CRC is given in g/g and the SFC in $10^{-7} cm^3 \cdot s/g$

According to the invention it is preferred that the insult zone is a zone of a high absorption rate and high retention capacity. Therefore it is preferred that the basis weight of the fluid-absorbent core at the insult zone is of a special amount. Usually of at least 500 gsm, preferably of at least 600 gsm, more preferred of at least 700 gsm, particularly preferred of at least 800 gsm, more particularly preferred of at least 900 gsm, most preferred of at least 1000 gsm.

The fluid-absorbent articles provide improved haptic properties. The articles further show high softness, less noise and negligible pinholing. The water-absorbing particles used in the inventive fluid-absorbent article provide a mean sphericity of at least 0.8. The particles itself feel soft and the coarse feeling even in high loaded water-absorbent articles is reduced. Furthermore the almost missing rough edges lead to reduced noise in case of friction between the particles, especially in water-absorbent articles with a high amount, e.g. more than 80%, of water-absorbent particles within the absorbent core.

The sum of CRC and AUHL for water-absorbent particles useful for the fluid-absorbent article according to the present invention being at least 60 g/g, and the amount of the basis weight of the acquisition distribution layer (D) in gsm being not less than the amount of water-absorbent polymer particles contained in the fluid absorbent core (C) in % by weight, based on the sum of water-absorbent polymer particles and fibrous material.

It is preferred that the the water-absorbent polymer particles have a centrifuge retention capacity of at least 25 g/g and an absorbency under high load of at least 20 g/g. The water-absorbent polymer particles therefore possess a high centrifuge retention capacity which impart good liquid distribution when used in hygiene articles. Furthermore fluid-absorbent articles according to one embodiment of the present invention comprise low absolute amounts of water-absorbent particles while maintaining excellent dryness, e.g. for a maxi diaper (size L) 10 g, preferably 8 g, water-absorbent particles are sufficient.

The fluid-absorbent articles according to one embodiment of the present invention are comprising water-absorbent polymer particles and less than 15% by weight fibrous material and/or adhesives in the absorbent core.

The water-absorbent polymer particles suitable for the present invention having a high centrifuge retention capacity (CRC) and a high absorption under a load of 49.2 g/cm$^2$ (AUHL).

Suitable water-absorbent polymers are produced by a process, comprising the steps forming water-absorbent polymer particles by polymerizing a monomer solution, coating of water-absorbent polymer particles with at least one surface-postcrosslinker and thermal surface-postcrosslinking of the coated water-absorbent polymer particles, wherein the content of residual monomers in the water-absorbent polymer particles prior to the coating with the surface-postcrosslinker is in the range from 0.03 to 15% by weight, and the temperature during the thermal surface-postcrosslinking is in the range from 100 to 180°C.

Suitable water-absorbent polymers can be also produced by a process, comprising the steps forming water-absorbent polymer particles by polymerizing a monomer solution, coating of water-absorbent polymer particles with at least one surface-postcrosslinker and thermal surface-postcrosslinking of the coated water-absorbent polymer particles, wherein the content of residual monomers in the water-absorbent polymer particles prior to the coating with the surface-postcrosslinker is in the range from 0.1 to 10% by weight, the surface-postcrosslinker is an alkylene carbonate, and the temperature during the thermal surface-postcrosslinking is in the range from 100 to 180°C.

The level of residual monomers in the water-absorbent polymer particles prior to the thermal surface-postcrosslinking, the temperature of the thermal surface-postcrosslinking, and the surface-postcrosslinker itself have an important impact on the properties of the formed surface-postcrosslinked water-absorbent polymer particles.

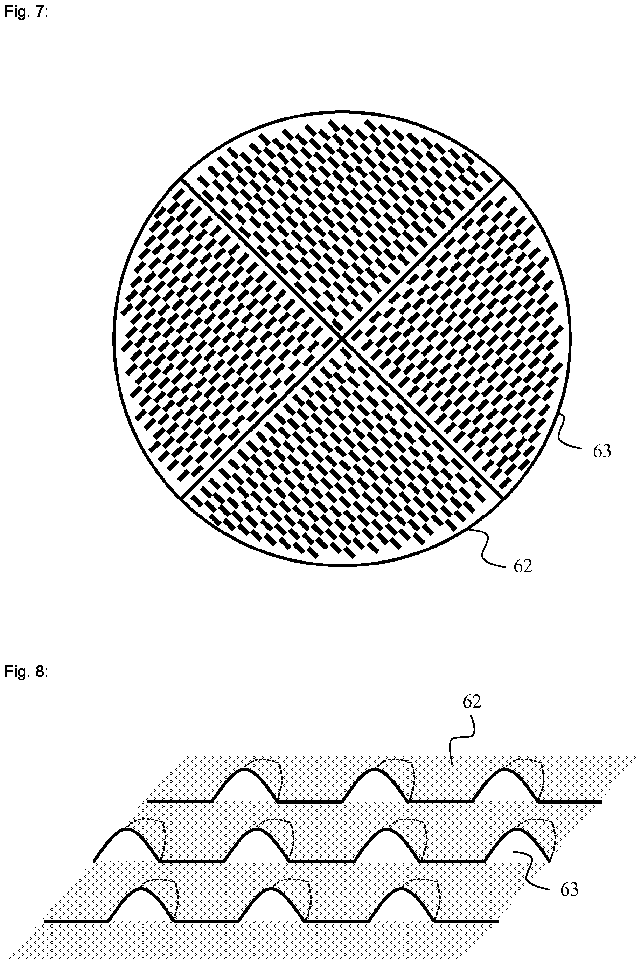

The specific conditions according to the production process resulting in water-absorbent polymer particles having a high centrifuge retention capacity (CRC) and a high absorption under a load of 49.2 g/cm$^2$ (AUHL). That is a surprising result. It is known that the centrifuge retention capacity (CRC) significantly decreases during thermal surface-postcrosslinking as proven by Ullmann's Encyclopedia of Industrial Chemistry, 6th Ed., Vol. 35, page 84, Figure 7. Further surprising is that the less reactive alkylene carbonate reacts under the inventive conditions at unusual low temperatures. Other cyclic surface-postcrosslinkers, for example 2-oxazoliidinone, show a very similar behaviour. According to the monograph "Modern Superabsorbent Polymer Technology", F.L. Buchholz and A.T. Graham, Wiley-VCH, 1998, page 98, the recommended reaction temperatures for alkylene carbonates are in the range from 180 to 215°C.

The combination of having a high centrifuge retention capacity (CRC) and a high absorption under a load of 49.2 g/cm$^2$ (AUHL) results in water-absorbent polymer particles having a high total liquid uptake in the wicking absorption test.

The water-absorbent polymer particles further having a reduced pressure dependency of the characteristic swelling time in the VAUL test at high centrifuge retention capacities (CRC).

The water-absorbent polymer particles further having a level of extractable constituents of less than 10% by weight

Furthermore it is preferred that the surface-postcrosslinked water-absorbent polymer particles having a centrifuge retention capacity (CRC) from 35 to 75 g/g, an absorption under high load (AUHL) from 20 to 50 g/g, a level of extractable constituents of less than 10% by weight, and a porosity from 20 to 40%.

It is preferred that the water-absorbent polymer particles having a total liquid uptake of

$$Y > -500 \times \ln(X) + 1880$$

wherein Y [g] is the total liquid uptake and X [g/g] is the centrifuge retention capacity, wherein the centrifuge retention capacity is at least 25 g/g and the liquid uptake is at least 30 g.

Further suitable water-absorbent polymer particles having a change of characteristic swelling time of less than 0.6 and a centrifuge retention capacity of at least 35 g/g, wherein the change of characteristic swelling time is

$$Z < (\tau_{0.5} - \tau_{0.1}) / \tau_{0.5}$$

wherein Z is the change of characteristic swelling time, $\tau_{0.1}$ is the characteristic swelling time under a pressure of 0.1 psi (6.9 g/cm$^2$) and $\tau_{0.5}$ is the characteristic swelling time under a pressure of 0.5 psi (35.0 g/cm$^2$).

Detailed description of the invention

A. Definitions

As used herein, the term "fluid-absorbent article" refers to any three-dimensional solid material being able to acquire and store fluids discharged from the body. Preferred fluid-absorbent articles are disposable fluid-absorbent articles that are designed to be worn in contact with the body of a user such as disposable fluid-absorbent pantyliners, sanitary napkins, catamenials, incontinence inserts/pads, diapers, training pant diapers, breast pads, interlabial inserts/pads or other articles useful for absorbing body fluids.

As used herein, the term "fluid-absorbent composition" refers to a component of the fluid-absorbent article which is primarily responsible for the fluid handling of the fluid-absorbent article including acquisition, transport, distribution and storage of body fluids.

As used herein, the term "fluid-absorbent core" refers to a fluid-absorbent composition comprising water-absorbent polymer particles and a fibrous material. The fluid-absorbent core is primarily responsible for the fluid handling of the fluid-absorbent article including acquisition, transport, distribution and storage of body fluids.

As used herein, the term "layer" refers to a fluid-absorbent composition whose primary dimension is along its length and width. It should be known that the term "layer" is not necessarily limited to single layers or sheets of the fluid-absorbent composition. Thus a layer can comprise laminates, composites, combinations of several sheets or webs of different materials.

As used herein the term "x-dimension" refers to the length, and the term "y-dimension" refers to the width of the fluid-absorbent composition, layer, core or article. Generally, the term "x-y-dimension" refers to the plane, orthogonal to the height or thickness of the fluid-absorbent composition, layer, core or article.

As used herein the term "z-dimension" refers to the dimension orthogonal to the length and width of the fluid absorbent composition, layer, core or article. Generally, the term "z-dimension" refers to the height of the fluid-absorbent composition, layer, core or article.

As used herein, the term "basis weight" indicates the weight of the fluid-absorbent core per square meter and it includes the chassis of the fluid-absorbent article. The basis weight is determined at discrete regions of the fluid-absorbent core: the front overall average is the basis weight of the fluid-absorbent core 5.5 cm forward of the center of the core to the front distal edge of the core; the insult zone is the basis weight of the fluid-absorbent core 5.5 cm forward and 0.5cm backwards of the center of the core; the back overall average is the basis weight of the fluid-absorbent core 0.5 cm backward of the center of the core to the rear distal edge of the core.

As used herein, the term "density" indicates the weight of the fluid-absorbent core per volume and it includes the chassis of the fluid-absorbent article. The density is determined at discrete regions of the fluid-absorbent core: the front overall average is the density of the fluid-absorbent core 5.5 cm forward of the center of the core to the front distal edge of the core; the insult zone is the density of the fluid-absorbent core 5.5 cm forward and 0.5cm backwards of the center of the core; the back overall average is the density of the fluid-absorbent core 0.5 cm backward of the center of the core to the rear distal edge of the core.

Further, it should be understood, that the term "upper" refers to fluid-absorbent composition which are nearer to the wearer of the fluid-absorbent article. Generally, the topsheet is the nearest composition to the wearer of the fluid-absorbent article, hereinafter described as "upper liquid-pervious layer". Contrarily, the term "lower" refers to fluid-absorbent compositions which are away from the wearer of the fluid-absorbent article. Generally, the backsheet is the component which is furthermost away from the wearer of the fluid-absorbent article, hereinafter described as "lower liquid-impervious layer".

As used herein, the term "liquid-pervious" refers to a substrate, layer or a laminate thus permitting liquids, i.e. body fluids such as urine, menses and/or vaginal fluids to readily penetrate through its thickness.

As used herein, the term "liquid-impervious" refers to a substrate, layer or a laminate that does not allow body fluids to pass through in a direction generally perpendicular to the plane of the layer at the point of liquid contact under ordinary use conditions.

As used herein, the term "chassis" refers to fluid-absorbent material comprising the upper liquid-pervious layer and the lower liquid-impervious layer, elastication and closure systems for the absorbent article.

As used herein, the term "hydrophilic" refers to the wettability of fibers by water deposited on these fibers. The term "hydrophilic" is defined by the contact angle and surface tension of the body fluids. According to the definition of Robert F. Gould in the 1964 American Chemical Society publication "Contact angle, wettability and adhesion", a fiber is referred to as hydrophilic, when the contact angle between the liquid and the fiber, especially the fiber surface, is less than 90° or when the liquid tends to spread spontaneously on the same surface.

Contrarily, term "hydrophobic" refers to fibers showing a contact angle of greater than 90° or no spontaneously spreading of the liquid across the surface of the fiber.

As used herein, the term "body fluids" refers to any fluid produced and discharged by human or animal body, such as urine, menstrual fluids, faeces, vaginal secretions and the like.

As used herein, the term "breathable" refers to a substrate, layer, film or a laminate that allows vapour to escape from the fluid-absorbent article, while still preventing fluids from leakage. Breathable substrates, layers, films or laminates may be porous polymeric films, nonwoven laminates from spunbond and melt- blown layers, laminates from porous polymeric films and nonwovens.

As used herein, the term "longitudinal" refers to a direction running perpendicular from a waist edge to an opposing waist edge of the fluid-absorbent article.

B. Water-absorbent polymer Particles

The water-absorbent polymer particles are prepared by a process, comprising the steps forming water-absorbent polymer particles by polymerizing a monomer solution, comprising

    a) at least one ethylenically unsaturated monomer which bears acid groups and may be at least partly neutralized,
    b) optionally one or more crosslinker,

c) at least one initiator,

d) optionally one or more ethylenically unsaturated monomers copolymerizable with the monomers mentioned under a),

e) optionally one or more water-soluble polymers, and

f) water,

coating of water-absorbent polymer particles with at least one surface-postcrosslinker and thermal surface-postcrosslinking of the coated water-absorbent polymer particles, wherein the content of residual monomers in the water-absorbent polymer particles prior to the coating with the surface-postcrosslinker is in the range from 0.03 to 15% by weight, the surface-postcrosslinker is an alkylene carbonate, and the temperature during the thermal surface-postcrosslinking is in the range from 100 to 180°C.

The water-absorbent polymer particles are typically insoluble but swellable in water.

The monomers a) are preferably water-soluble, i.e. the solubility in water at 23°C is typically at least 1 g/100 g of water, preferably at least 5 g/100 g of water, more preferably at least 25 g/100 g of water, most preferably at least 35 g/100 g of water.

Suitable monomers a) are, for example, ethylenically unsaturated carboxylic acids such as acrylic acid, methacrylic acid, maleic acid, and itaconic acid. Particularly preferred monomers are acrylic acid and methacrylic acid. Very particular preference is given to acrylic acid.

Further suitable monomers a) are, for example, ethylenically unsaturated sulfonic acids such as vinylsulfonic acid, styrenesulfonic acid and 2-acrylamido-2-methylpropanesulfonic acid (AMPS).

Impurities may have a strong impact on the polymerization. Preference is given to especially purified monomers a). Useful purification methods are disclosed in WO 2002/055469 A1, WO 2003/078378 A1 and WO 2004/035514 A1. A suitable monomer a) is according to WO 2004/035514 A1 purified acrylic acid having 99.8460% by weight of acrylic acid, 0.0950% by weight of acetic acid, 0.0332% by weight of water, 0.0203 by weight of propionic acid, 0.0001 % by weight of furfurals, 0.0001 % by weight of maleic anhydride, 0.0003% by weight of diacrylic acid and 0.0050 by weight of hydroquinone monomethyl ether.

Polymerized diacrylic acid is a source for residual monomers due to thermal decomposition. If the temperatures during the process are low, the concentration of diacrylic acid is no more critical and acrylic acids having higher concentrations of diacrylic acid, i.e. 500 to 10,000 ppm, can be used for the inventive process.

The content of acrylic acid and/or salts thereof in the total amount of monomers a) is preferably at least 50 mol%, more preferably at least 90 mol%, most preferably at least 95 mol%.

The acid groups of the monomers a) are typically partly neutralized in the range of 0 to 100 mol%, preferably to an extent of from 25 to 85 mol%, preferentially to an extent of from 50 to 80 mol%, more preferably from 60 to 75 mol%, for which the customary neutralizing agents can be used, preferably alkali metal hydroxides, alkali metal oxides, alkali metal carbonates or alkali metal hydrogen carbonates, and mixtures thereof. Instead of alkali metal salts, it is also possible to use ammonia or organic amines, for example, triethanolamine. It is also possible to use oxides, carbonates, hydrogencarbonates and hydroxides of magnesium, calcium, strontium, zinc or aluminum as powders, slurries or solutions and mixtures of any of the above neutralization agents. Example for a mixture is a solution of sodiumaluminate. Sodium and potassium are particularly preferred as alkali metals, but very particular preference is given to sodium hydroxide, sodium carbonate or sodium hydrogen carbonate, and mixtures thereof. Typically, the neutralization is achieved by mixing in the neutralizing agent as an aqueous solution, as a melt or preferably also as a solid. For example, sodium hydroxide with water content significantly below 50% by weight may be present as a waxy material having a melting point above 23°C. In this case, metered addition as piece material or melt at elevated temperature is possible.

Optionally, it is possible to add to the monomer solution, or to starting materials thereof, one or more chelating agents for masking metal ions, for example iron, for the purpose of stabilization. Suitable chelating agents are, for example, alkali metal citrates, citric acid, alkali metal tartrates, alkali metal lactates and glycolates, pentasodium triphosphate, ethylenediamine tetraacetate, nitrilotriacetic acid, and all chelating agents known under the Trilon® name, for example Trilon® C (pentasodium diethylenetriaminepentaacetate), Trilon® D (trisodium (hydroxyethyl)-ethylenediaminetriacetate), and Trilon® M (methylglycinediacetic acid).

The monomers a) comprise typically polymerization inhibitors, preferably hydroquinone monoethers, as inhibitor for storage.

The monomer solution comprises preferably up to 250 ppm by weight, more preferably not more than 130 ppm by weight, most preferably not more than 70 ppm by weight, preferably not less than 10 ppm by weight, more preferably not less than 30 ppm by weight and especially about 50 ppm by weight of hydroquinone monoether, based in each case on acrylic acid, with acrylic acid salts being counted as acrylic acid. For example, the monomer solution can be prepared using acrylic acid having appropriate hydroquinone monoether content. The hydroquinone monoethers may, however, also be removed from the monomer solution by absorption, for example on activated carbon.

Preferred hydroquinone monoethers are hydroquinone monomethyl ether (MEHQ) and/or alpha-tocopherol (vitamin E).

Suitable crosslinkers b) are compounds having at least two groups suitable for crosslinking. Such groups are, for example, ethylenically unsaturated groups which can be polymerized by a free-radical mechanism into the polymer chain and functional groups which can form covalent bonds with the acid groups of monomer a). In addition, polyvalent metal ions which can form coordinate bond with at least two acid groups of monomer a) are also suitable crosslinkers b).

The crosslinkers b) are preferably compounds having at least two free-radically polymerizable groups which can be polymerized by a free-radical mechanism into the polymer network. Suitable crosslinkers b) are, for example, ethylene glycol dimethacrylate, diethylene glycol diacrylate, polyethylene glycol diacrylate, allyl methacrylate, trimethylolpropane triacrylate, triallylamine, tetraallylammonium chloride, tetraallyloxyethane, as described in EP 0 530 438 A1, di- and triacrylates, as described in EP 0 547 847 A1, EP 0 559 476 A1, EP 0 632 068 A1, WO 93/21237 A1, WO 2003/104299 A1, WO 2003/104300 A1, WO 2003/104301 A1 and in DE 103 31 450 A1, mixed acrylates which, as well as acrylate groups, comprise further ethylenically unsaturated groups, as described in DE 103 314 56 A1 and DE 103 55 401 A1, or crosslinker mixtures, as described, for example, in DE 195 43 368 A1, DE 196 46 484 A1, WO 90/15830 A1 and WO 2002/32962 A2.

Suitable crosslinkers b) are in particular pentaerythritol triallyl ether, tetraallyloxyethane, polyethyleneglycole diallylethers (based on polyethylene glycole having a molecular weight between 400 and 20000 g/mol), N,N'-methylenebisacrylamide, 15-tuply ethoxylated trimethylolpropane, polyethylene glycol diacrylate, trimethylolpropane triacrylate and triallylamine.

Very particularly preferred crosslinkers b) are the polyethoxylated and/or -propoxylated glycerols which have been esterified with acrylic acid or methacrylic acid to give di- or triacrylates, as described, for example in WO 2003/104301 A1. Di- and/or triacrylates of 3- to 18-tuply ethoxylated glycerol are particularly advantageous. Very particular preference is given to di- or triacrylates of 1- to 5-tuply ethoxylated and/or propoxylated glycerol. Most preferred are the triacrylates of 3- to 5-tuply ethoxylated and/or propoxylated glycerol and especially the triacrylate of 3-tuply ethoxylated glycerol.

The amount of crosslinker b) is preferably from 0.0001 to 0.6% by weight, more preferably from 0.001 to 0.2% by weight, most preferably from 0.01 to 0.06% by weight, based in each case on monomer a). On increasing the amount of crosslinker b) the centrifuge retention capacity (CRC) decreases and the absorption under a pressure of 21.0 g/cm$^2$ (AUL) passes through a maximum.

The surface-postcrosslinked polymer particles of the present invention surprisingly require very little or even no cross-linker during the polymerization step. So, in one particularly preferred embodiment of the present invention no crosslinker b) is used.

The initiators c) used may be all compounds which disintegrate into free radicals under the polymerization conditions, for example peroxides, hydroperoxides, hydrogen peroxide, persulfates, azo compounds and redox initiators. Preference is given to the use of water-soluble initiators. In some cases, it is advantageous to use mixtures of various initiators, for example mixtures of hydrogen peroxide and sodium or potassium peroxodisulfate. Mixtures of hydrogen peroxide and sodium peroxodisulfate can be used in any proportion.

Particularly preferred initiators c) are azo initiators such as 2,2'-azobis[2-(2-imidazolin-2-yl)propane] dihydrochloride and 2,2'-azobis[2-(5-methyl-2-imidazolin-2-yl)propane] dihydrochloride, 2,2'-azobis(2-amidinopropane) dihydrochloride, 4,4'-azobis(4-cyanopentanoic acid), 4,4'-azobis(4-cyanopentanoic acid) sodium salt, 2,2'-azobis[2-methyl-N-(2-hydroxyethyl)propionamide], and photoinitiators such as 2-hydroxy-2-methylpropiophenone and 1-[4-(2-hydroxyethoxy)phenyl]-2-hydroxy-2-methyl-1-propan-1-one, redox initiators such as sodium persulfate/hydroxymethylsulfinic acid, ammonium peroxodisulfate/hydroxymethylsulfinic acid, hydrogen peroxide/hydroxymethylsulfinic acid, sodium persulfate/ascorbic acid, ammonium peroxodisulfate/ascorbic acid and hydrogen peroxide/ascorbic acid, photoinitiators such as 1-[4-(2-hydroxyethoxy)phenyl]-2-hydroxy-2-methyl-1-propan-1-one, and mixtures thereof. The reducing component used is, however, preferably a mixture of the sodium salt of 2-hydroxy-2-sulfinatoacetic acid, the disodium salt of 2-hydroxy-2-sulfonatoacetic acid and sodium bisulfite. Such mixtures are obtainable as Brüggolite® FF6 and Brüggolite® FF7 (Brüggemann Chemicals; Heilbronn; Germany). Of course it is also possible within the scope of the present invention to use the purified salts or acids of 2-hydroxy-2-sulfinatoacetic acid and 2-hydroxy-2-sulfonatoacetic acid - the latter being available as sodium salt under the trade name Blancolen® (Brüggemann Chemicals; Heilbronn; Germany).

The initiators are used in customary amounts, for example in amounts of from 0.001 to 5% by weight, preferably from 0.01 to 2% by weight, most preferably from 0.05 to 0.5% by weight, based on the monomers a).

Examples of ethylenically unsaturated monomers d) which are copolymerizable with the monomers a) are acrylamide, methacrylamide, hydroxyethyl acrylate, hydroxyethyl methacrylate, dimethylaminoethyl acrylate, dimethylaminoethyl methacrylate, dimethylaminopropyl acrylate and diethylaminopropyl methacrylate.

Useful water-soluble polymers e) include polyvinyl alcohol, modified polyvinyl alcohol comprising acidic side groups for example Poval® K (Kuraray Europe GmbH; Frankfurt; Germany), polyvinylpyrrolidone, starch, starch derivatives, modified cellulose such as methylcellulose, carboxymethylcellulose or hydroxyethylcellulose, gelatin, polyglycols or polyacrylic acids, polyesters and polyamides, polylactic acid, polyglycolic acid, co-polylactic-polyglycolic acid, polyvinylamine, polyallylamine, water soluble copolymers of acrylic acid and maleic acid available as Sokalan® (BASF SE;

Ludwigshafen; Germany), preferably starch, starch derivatives and modified cellulose.

For optimal action, the preferred polymerization inhibitors require dissolved oxygen. Therefore, the monomer solution can be freed of dissolved oxygen before the polymerization by inertization, i.e. flowing through with an inert gas, preferably nitrogen. It is also possible to reduce the concentration of dissolved oxygen by adding a reducing agent. The oxygen content of the monomer solution is preferably lowered before the polymerization to less than 1 ppm by weight, more preferably to less than 0.5 ppm by weight.

The water content of the monomer solution is preferably less than 65% by weight, preferentially less than 62% by weight, more preferably less than 60% by weight, most preferably less than 58% by weight.

The monomer solution has, at 20°C, a dynamic viscosity of preferably from 0.002 to 0.02 Pa·s, more preferably from 0.004 to 0.015 Pa·s, most preferably from 0.005 to 0.01 Pa·s. The mean droplet diameter in the droplet generation rises with rising dynamic viscosity.

The monomer solution has, at 20°C, a density of preferably from 1 to 1.3 g/cm$^3$, more preferably from 1.05 to 1.25 g/cm$^3$, most preferably from 1.1 to 1.2 g/cm$^3$.

The monomer solution has, at 20°C, a surface tension of from 0.02 to 0.06 N/m, more preferably from 0.03 to 0.05 N/m, most preferably from 0.035 to 0.045 N/m. The mean droplet diameter in the droplet generation rises with rising surface tension.

Polymerization

The monomer solution is polymerized. Suitable reactors are, for example, kneading reactors or belt reactors. In the kneader, the polymer gel formed in the polymerization of an aqueous monomer solution or suspension is comminuted continuously by, for example, contrarotatory stirrer shafts, as described in WO 2001/038402 A1. Polymerization on the belt is described, for example, in DE 38 25 366 A1 and US 6,241,928. Polymerization in a belt reactor forms a polymer gel which has to be comminuted in a further process step, for example in an extruder or kneader.

To improve the drying properties, the comminuted polymer gel obtained by means of a kneader can additionally be extruded.

It is preferred to produce the water-absorbent polymer particles polymerizing droplets of the monomer in a surrounding heated gas phase, for example using a system described in WO 2008/040715 A2, WO 2008/052971 A1, WO 2008/069639 A1 and WO 2008/086976 A1.

The droplets are preferably generated by means of a droplet plate. A droplet plate is a plate having a multitude of bores, the liquid entering the bores from the top. The droplet plate or the liquid can be oscillated, which generates a chain of ideally monodisperse droplets at each bore on the underside of the droplet plate. In a preferred embodiment, the droplet plate is not agitated.

It is also possible to use two or more droplet plates with different bore diameters so that a range of desired particle sizes can be produced. It is preferable that each droplet plate carries only one bore diameter, however mixed bore diameters in one plate are also possible.

The number and size of the bores are selected according to the desired capacity and droplet size. The droplet diameter is typically 1.9 times the diameter of the bore. What is important here is that the liquid to be dropletized does not pass through the bore too rapidly and the pressure drop over the bore is not too big. Otherwise, the liquid is not dropletized, but rather the liquid jet is broken up (sprayed) owing to the high kinetic energy. The Reynolds number based on the throughput per bore and the bore diameter is preferably less than 2000, preferentially less than 1600, more preferably less than 1400 and most preferably less than 1200.

The underside of the droplet plate has at least in part a contact angle preferably of at least 60°, more preferably at least 75° and most preferably at least 90° with regard to water.

The contact angle is a measure of the wetting behavior of a liquid, in particular water, with regard to a surface, and can be determined using conventional methods, for example in accordance with ASTM D 5725. A low contact angle denotes good wetting, and a high contact angle denotes poor wetting.

It is also possible for the droplet plate to consist of a material having a lower contact angle with regard to water, for example a steel having the German construction material code number of 1.4571, and be coated with a material having a larger contact angle with regard to water.

Useful coatings include for example fluorous polymers, such as perfluoroalkoxyethylene, polytetrafluoroethylene, ethylene-chlorotrifluoroethylene copolymers, ethylene-tetrafluoroethylene copolymers and fluorinated polyethylene.

The coatings can be applied to the substrate as a dispersion, in which case the solvent is subsequently evaporated off and the coating is heat treated. For polytetrafluoroethylene this is described for example in US-3,243,321.

Further coating processes are to be found under the headword "Thin Films" in the electronic version of "Ullmann's Encyclopedia of Industrial Chemistry" (Updated Sixth Edition, 2000 Electronic Release).

The coatings can further be incorporated in a nickel layer in the course of a chemical nickelization.

It is the poor wettability of the droplet plate that leads to the production of monodisperse droplets of narrow droplet size distribution.

The droplet plate has preferably at least 5, more preferably at least 25, most preferably at least 50 and preferably up to 750, more preferably up to 500 bores, most preferably up to 250. The number of bores is determined mainly by geometrical and manufacturing constraints and can be adjusted to practical use conditions even outside the above given range. The diameter of the bores is adjusted to the desired droplet size.

The separation of the bores is usually from 5 to 50 mm, preferably from 6 to 40 mm, more preferably from 7 to 35 mm, most preferably from 8 to 30 mm. Smaller separations of the bores may cause agglomeration of the polymerizing droplets.

The diameter of the bores is preferably from 50 to 500 $\mu$m, more preferably from 100 to 300 $\mu$m, most preferably from 150 to 250 $\mu$m.

For optimizing the average particle diameter, droplet plates with different bore diameters can be used. The variation can be done by different bores on one plate or by using different plates, where each plate has a different bore diameter. The average particle size distribution can be monomodal, bimodal or multimodal. Most preferably it is monomodal or bimodal.

The temperature of the monomer solution as it passes through the bore is preferably from 5 to 80°C, more preferably from 10 to 70°C, most preferably from 30 to 60°C.

A gas flows through the reaction chamber. The carrier gas is conducted through the reaction chamber in cocurrent to the free-falling droplets of the monomer solution, i.e. from the top downward. After one pass, the gas is preferably recycled at least partly, preferably to an extent of at least 50%, more preferably to an extent of at least 75%, into the reaction chamber as cycle gas. Typically, a portion of the carrier gas is discharged after each pass, preferably up to 10%, more preferably up to 3% and most preferably up to 1%.

The carrier gas may be composed of air. The oxygen content of the carrier gas is preferably from 0.1 to 15% by volume, more preferably from 1 to 10% by volume, most preferably from 2 to 7% by weight. In the scope of the present invention it is also possible to use a carrier gas which is free of oxygen.

As well as oxygen, the carrier gas preferably comprises nitrogen. The nitrogen content of the gas is preferably at least 80% by volume, more preferably at least 90% by volume, most preferably at least 95% by volume. Other possible carrier gases may be selected from carbon dioxide, argon, xenon, krypton, neon, helium, sulfurhexafluoride. Any mixture of carrier gases may be used. The carrier gas may also become loaded with water and/or acrylic acid vapors.

The gas velocity is preferably adjusted such that the flow in the reaction zone is directed, for example no convection currents opposed to the general flow direction are present, and is preferably from 0.1 to 2.5 m/s, more preferably from 0.3 to 1.5 m/s, even more preferably from 0.5 to 1.2 m/s, most preferably from 0.7 to 0.9 m/s.

The gas entrance temperature, i.e. the temperature with which the gas enters the reaction zone, is preferably from 160 to 200°C, more preferably from 165 to 195°C, even more preferably from 170 to 190°C, most preferably from 175 to 185°C.

The steam content of the gas that enters the reaction zone is preferably from 0.01 to 0.15 kg per kg dry gas, more preferably from 0.02 to 0.12 kg per kg dry gas, most preferably from 0.03 to 0.10 kg per kg dry gas.

The gas entrance temperature is controlled in such a way that the gas exit temperature, i.e. the temperature with which the gas leaves the reaction zone, is less than 150°C, preferably from 90 to 140°C, more preferably from 100 to 130°C, even more preferably from 105 to 125°C, most preferably from 110 to 120°C.

The steam content of the gas that leaves the reaction zone is preferably from 0.02 to 0.30 kg per kg dry gas, more from 0.04 to 0.28 kg per kg dry gas, most from 0.05 to 0.25 kg per kg dry gas.

The water-absorbent polymer particles can be divided into three categories: water-absorbent polymer particles of Type 1 are particles with one cavity, water-absorbent polymer particles of Type 2 are particles with more than one cavity, and water-absorbent polymer particles of Type 3 are solid particles with no visible cavity. Type 1 particles are represented by hollow-spheres, Type 2 particles are represented by spherical closed cell sponges, and Type 3 particles are represented by solid spheres. Type 2 or Type 3 particles or mixtures thereof with little or no Type 1 particles are preferred.

The morphology of the water-absorbent polymer particles can be controlled by the reaction conditions during polymerization. Water-absorbent polymer particles having a high amount of particles with one cavity (Type 1) can be prepared by using low gas velocities and high gas exit temperatures. Water-absorbent polymer particles having a high amount of particles with more than one cavity (Type 2) can be prepared by using high gas velocities and low gas exit temperatures.

Water-absorbent polymer particles having no cavity (Type 3) and water-absorbent polymer particles having more than one cavity (Type 2) show an improved mechanical stability compared with water-absorbent polymer particles having only one cavity (Type 1).

As a particular advantage round shaped particles have no edges that can easily be broken by processing stress in diaper production and during swelling in aqueous liquid there are no breakpoints on the surface that could lead to loss of mechanical strength.

The reaction can be carried out under elevated pressure or under reduced pressure, preferably from 1 to 100mbar below ambient pressure, more preferably from 1.5 to 50mbar below ambient pressure, most preferably from 2 to 10mbar below ambient pressure.

The reaction off-gas, i.e. the gas leaving the reaction chamber, may be cooled in a heat exchanger. This condenses water and unconverted monomer a). The reaction off-gas can then be reheated at least partly and recycled into the reaction chamber as cycle gas. A portion of the reaction off-gas can be discharged and replaced by fresh gas, in which case water and unconverted monomers a) present in the reaction off-gas can be removed and recycled.

Particular preference is given to a thermally integrated system, i.e. a portion of the waste heat in the cooling of the off-gas is used to heat the cycle gas.

The reactors can be trace-heated. In this case, the trace heating is adjusted such that the wall temperature is at least 5°C above the internal reactor temperature and condensation on the reactor walls is reliably prevented.

Thermal posttreatment

The water-absorbent polymer particles obtained by dropletization may be thermal posttreated for adjusting the content of residual monomers to the desired value.

Generally the level of residual monomers can be influenced by process parameter settings, for example; the temperature of posttreatment of the water-absorbent particles. The residual monomers can be removed better at relatively high temperatures and relatively long residence times. What is important here is that the water-absorbent polymer particles are not too dry. In the case of excessively dry particles, the residual monomers decrease only insignificantly. Too high a water content increases the caking tendency of the water-absorbent polymer particles.

The thermal posttreatment can be done in a fluidized bed. In a preferred embodiment of the present invention an internal fluidized bed is used. An internal fluidized bed means that the product of the dropletization polymerization is accumulated in a fluidized bed below the reaction zone.

In the fluidized state, the kinetic energy of the polymer particles is greater than the cohesion or adhesion potential between the polymer particles.

The fluidized state can be achieved by a fluidized bed. In this bed, there is upward flow toward the water-absorbing polymer particles, so that the particles form a fluidized bed. The height of the fluidized bed is adjusted by gas rate and gas velocity, i.e. via the pressure drop of the fluidized bed (kinetic energy of the gas).

The velocity of the gas stream in the fluidized bed is preferably from 0.3 to 2.5 m/s, more preferably from 0.4 to 2.0 m/s, most preferably from 0.5 to 1.5 m/s.

The pressure drop over the bottom of the internal fluidized bed is preferably from 1 to 100mbar, more preferably from 3 to 50mbar, most preferably from 5 to 25mbar.

The moisture content of the water-absorbent polymer particles at the end of the thermal posttreatment is preferably from 1 to 20% by weight, more preferably from 2 to 15% by weight, even more preferably from 3 to 12% by weight, most preferably 5 to 8% by weight.

The temperature of the water-absorbent polymer particles during the thermal posttreatment is from 20 to 120°C, preferably from 40 to 100°C, more preferably from 50 to 95°C, even more preferably from 55 to 90°C, most preferably from 60 to 80°C.

The average residence time in the internal fluidized bed is from 10 to 300 minutes, preferably from 60 to 270 minutes, more preferably from 40 to 250 minutes, most preferably from 120 to 240 minutes.

The condition of the fluidized bed can be adjusted for reducing the amount of residual monomers of the water-absorbent polymers leaving the fluidized bed. The amount of residual monomers can be reduced to levels below 0.1% by weight by a thermal posttreatment using additional steam.

The steam content of the gas is preferably from 0.005 to 0.25 kg per kg of dry gas, more preferably from 0.01 to 0.2 kg per kg of dry gas, most preferably from 0.02 to 0.15 kg per kg of dry gas.

By using additional steam the condition of the fluidized bed can be adjusted that the amount of residual monomers of the water-absorbent polymers leaving the fluidized bed is from 0.03 to 15% by weight, preferably from 0.05 to 12% by weight, more preferably from 0.1 to 10% by weight, even more preferably from 0.15 to 7.5% by weight most preferably from 0.2 to 5% by weight, even most preferably from 0.25 to 2.5% by weight.

The level of residual monomers in the water-absorbent polymer has in important impact on the properties of the later formed surface-postcrosslinked water-absorbent polymer particles. That means that very low levels of residual monomers must be avoided.

It is preferred that the thermal posttreatment is completely or at least partially done in an external fluidized bed. The operating conditions of the external fluidized bed are within the scope for the internal fluidized bed as described above.

It is alternatively preferred that the thermal posttreatment is done in an external mixer with moving mixing tools, preferably horizontal mixers, such as screw mixers, disk mixers, screw belt mixers and paddle mixers. Suitable mixers are, for example, Becker shovel mixers (Gebr. Lödige Maschinenbau GmbH; Paderborn; Germany), Nara paddle mixers (NARA Machinery Europe; Frechen; Germany), Pflugschar® plowshare mixers (Gebr. Lödige Maschinenbau GmbH; Paderborn; Germany), Vrieco-Nauta Continuous Mixers (Hosokawa Micron BV; Doetinchem; the Netherlands), Processall Mixmill Mixers (Processall Incorporated; Cincinnati; U.S.A.) and Ruberg continuous flow mixers (Gebrüder Ruberg GmbH & Co KG, Nieheim, Germany). Ruberg continuous flow mixers, Becker shovel mixers and Pflugschar® plowshare mixers are preferred.

The thermal posttreatment can be done in a discontinuous external mixer or a continuous external mixer.

The amount of gas to be used in the discontinuous external mixer is preferably from 0.01 to 5 $Nm^3/h$, more preferably from 0.05 to 2 $Nm^3/h$, most preferably from 0.1 to 0.5 $Nm^3/h$, based in each case on kg water-absorbent polymer particles.

The amount of gas to be used in the continuous external mixer is preferably from 0.01 to 5 $Nm^3/h$, more preferably from 0.05 to 2 $Nm^3/h$, most preferably from 0.1 to 0.5 $Nm^3/h$, based in each case on kg/h throughput of water-absorbent polymer particles.

The other constituents of the gas are preferably nitrogen, carbon dioxide, argon, xenon, krypton, neon, helium, air or air/nitrogen mixtures, more preferably nitrogen or air/nitrogen mixtures comprising less than 10% by volume of oxygen. Oxygen may cause discoloration.

The morphology of the water-absorbent polymer particles can also be controlled by the reaction conditions during thermal posttreatment. Water-absorbent polymer particles having a high amount of particles with one cavity (Type 1) can be prepared by using high product temperatures and short residence times. Water-absorbent polymer particles having a high amount of particles with more than one cavity (Type 2) can be prepared by using low product temperatures and long residence times.

Surface-postcrosslinking

The polymer particles can be surface-postcrosslinked for further improvement of the properties.

Surface-postcrosslinkers are compounds which comprise groups which can form at least two covalent bonds with the carboxylate groups of the polymer particles. Suitable compounds are, for example, polyfunctional amines, polyfunctional amidoamines, polyfunctional epoxides, as described in EP 0 083 022 A2, EP 0 543 303 A1 and EP 0 937 736 A2, di- or polyfunctional alcohols as described in DE 33 14 019 A1, DE 35 23 617 A1 and EP 0 450 922 A2, or β-hydroxyalkylamides, as described in DE 102 04 938 A1 and US 6,239,230. Also ethyleneoxide, aziridine, glycidol, oxetane and its derivatives may be used.

Polyvinylamine, polyamidoamines and polyvinylalcohole are examples of multifunctional polymeric surface-postcrosslinkers.

In addition, DE 40 20 780 C1 describes alkylene carbonates, DE 198 07 502 A1 describes 1,3-oxazolidin-2-one and its derivatives such as 2-hydroxyethyl-1,3-oxazolidin-2-one, DE 198 07 992 C1 describes bis- and poly-1,3-oxazolidin-2-ones, EP 0 999 238 A1 describes bis- and poly-1,3-oxazolidines, DE 198 54 573 A1 describes 2-oxotetrahydro-1,3-oxazine and its derivatives, DE 198 54 574 A1 describes N-acyl-1,3-oxazolidin-2-ones, DE 102 04 937 A1 describes cyclic ureas, DE 103 34 584 A1 describes bicyclic amide acetals, EP 1 199 327 A2 describes oxetanes and cyclic ureas, and WO 2003/31482 A1 describes morpholine-2,3-dione and its derivatives, as suitable surface-postcrosslinkers.

In addition, it is also possible to use surface-postcrosslinkers which comprise additional polymerizable ethylenically unsaturated groups, as described in DE 37 13 601 A1.

The at least one surface-postcrosslinker is selected from alkylene carbonates, 1,3-oxazolidin-2-ones, bis- and poly-1,3-oxazolidin-2-ones, bis- and poly-1,3-oxazolidines, 2-oxotetrahydro-1,3-oxazines, N-acyl-1,3-oxazolidin-2-ones, cyclic ureas, bicyclic amide acetals, oxetanes, and morpholine-2,3-diones. Suitable surface-postcrosslinkers are ethylene carbonate, 3-methyl-1,3-oxazolidin-2-one, 3-methyl-3-oxethanmethanol, 1,3-oxazolidin-2-one, 3-(2-hydroxyethyl)-1,3-oxazolidin-2-one, 1,3-dioxan-2-one or a mixture thereof.

It is also possible to use any suitable mixture of surface-postcrosslinkers. It is particularly favorable to use mixtures of 1,3-dioxolan-2-on (ethylene carbonate) and 1,3-oxazolidin-2-ones. Such mixtures are obtainable by mixing and partly reacting of 1,3-dioxolan-2-on (ethylene carbonate) with the corresponding 2-amino-alcohol (e.g. 2-aminoethanol) and may comprise ethylene glycol from the reaction.

It is preferred that at least one alkylene carbonate is used as surface-postcrosslinker. Suitable alkylene carbonates are 1,3-dioxolan-2-on (ethylene carbonate), 4-methyl-1,3-dioxolan-2-on (propylene carbonate), 4,5-dimethyl-1,3-dioxolan-2-on, 4,4-dimethyl-1,3-dioxolan-2-on, 4-ethyl-1,3-dioxolan-2-on, 4-hydroxymethyl-1,3-dioxolan-2-on (glycerine carbonate), 1,3-dioxane-2-on (trimethylene carbonate), 4-methyl-1,3-dioxane-2-on, 4,6-dimethyl-1,3-dioxane-2-on and 1,3-dioxepan-2-on, preferably 1,3-dioxolan-2-on (ethylene carbonate) and 1,3-dioxane-2-on (trimethylene carbonate), most preferably 1,3-dioxolan-2-on (ethylene carbonate).

The amount of surface-postcrosslinker is preferably from 0.1 to 10% by weight, more preferably from 0.5 to 7.5% by weight, most preferably from 1 to 5% by weight, based in each case on the polymer.

The content of residual monomers in the water-absorbent polymer particles prior to the coating with the surface-postcrosslinker is in the range from 0.03 to 15% by weight, preferably from 0.05 to 12%by weight, more preferably from 0.1 to 10% by weight, even more preferably from 0.15 to 7.5% by weight, most preferably from 0.2 to 5% by weight, even most preferably from 0.25 to 2.5% by weight.

The moisture content of the water-absorbent polymer particles prior to the thermal surface-postcrosslinking is preferably from 1 to 20% by weight, more preferably from 2 to 15% by weight, most preferably from 3 to 10% by weight.

Polyvalent cations can be applied to the particle surface in addition to the surface-postcrosslinkers before, during or after the thermal surface-postcrosslinking.

The polyvalent cations usable in the process according to the invention are, for example, divalent cations such as the cations of zinc, magnesium, calcium, iron and strontium, trivalent cations such as the cations of aluminum, iron, chromium, rare earths and manganese, tetravalent cations such as the cations of titanium and zirconium, and mixtures thereof. Possible counterions are chloride, bromide, sulfate, hydrogensulfate, methanesulfate, carbonate, hydrogencarbonate, nitrate, hydroxide, phosphate, hydrogenphosphate, dihydrogenphosphate, glycophosphate and carboxylate, such as acetate, glycolate, tartrate, formiate, propionate, 3-hydroxypropionate, lactamide and lactate, and mixtures thereof. Aluminum sulfate, aluminum acetate, and aluminum lactate are preferred. Aluminum lactate is more preferred. Using the inventive process in combination with the use of aluminum lactate, water-absorbent polymer particles having an extremely high total liquid uptake at lower centrifuge retention capacities (CRC) can be prepared.

Apart from metal salts, it is also possible to use polyamines and/or polymeric amines as polyvalent cations. A single metal salt can be used as well as any mixture of the metal salts and/or the polyamines above.

Preferred polyvalent cations and corresponding anions are disclosed in WO 2012/045705 A1 and are expressly incorporated herein by reference. Preferred polyvinylamines are disclosed in WO 2004/024816 A1 and are expressly incorporated herein by reference.

The amount of polyvalent cation used is, for example, from 0.001 to 1.5% by weight, preferably from 0.005 to 1% by weight, more preferably from 0.02 to 0.8% by weight, based in each case on the polymer.

The addition of the polyvalent metal cation can take place prior, after, or cocurrently with the surface-postcrosslinking. Depending on the formulation and operating conditions employed it is possible to obtain a homogeneous surface coating and distribution of the polyvalent cation or an inhomogeneous typically spotty coating. Both types of coatings and any mixes between them are useful within the scope of the present invention.

The surface-postcrosslinking is typically performed in such a way that a solution of the surface-postcrosslinker is sprayed onto the hydrogel or the dry polymer particles. After the spraying, the polymer particles coated with the surface-postcrosslinker are dried thermally and cooled.

The spraying of a solution of the surface-postcrosslinker is preferably performed in mixers with moving mixing tools, such as screw mixers, disk mixers and paddle mixers. Suitable mixers are, for example, vertical Schugi Flexomix® mixers (Hosokawa Micron BV; Doetinchem; the Netherlands), Turbolizers® mixers (Hosokawa Micron BV; Doetinchem; the Netherlands), horizontal Pflugschar® plowshare mixers (Gebr. Lödige Maschinenbau GmbH; Paderborn; Germany), Vrieco-Nauta Continuous Mixers (Hosokawa Micron BV; Doetinchem; the Netherlands), Processall Mixmill Mixers (Processall Incorporated; Cincinnati; US) and Ruberg continuous flow mixers (Gebrüder Ruberg GmbH & Co KG, Nieheim, Germany). Ruberg continuous flow mixers and horizontal Pflugschar® plowshare mixers are preferred. The surface-postcrosslinker solution can also be sprayed into a fluidized bed.

The solution of the surface-postcrosslinker can also be sprayed on the water-absorbent polymer particles during the thermal posttreatment. In such case the surface-postcrosslinker can be added as one portion or in several portions along the axis of thermal posttreatment mixer. In one embodiment it is preferred to add the surface-postcrosslinker at the end of the thermal posttreatment step. As a particular advantage of adding the solution of the surface-postcrosslinker during the thermal posttreatment step it may be possible to eliminate or reduce the technical effort for a separate surface-postcrosslinker addition mixer.

The surface-postcrosslinkers are typically used as an aqueous solution. The addition of nonaqueous solvent can be used to improve surface wetting and to adjust the penetration depth of the surface-postcrosslinker into the polymer particles.

The thermal surface-postcrosslinking is preferably carried out in contact dryers, more preferably paddle dryers, most preferably disk dryers. Suitable driers are, for example, Hosokawa Bepex® horizontal paddle driers (Hosokawa Micron GmbH; Leingarten; Germany), Hosokawa Bepex® disk driers (Hosokawa Micron GmbH; Leingarten; Germany), Holo-Flite® dryers (Metso Minerals Industries Inc.; Danville; U.S.A.) and Nara paddle driers (NA-RA Machinery Europe; Frechen; Germany). Moreover, it is also possible to use fluidized bed dryers. In the latter case the reaction times may be shorter compared to other embodiments.

When a horizontal dryer is used then it is often advantageous to set the dryer up with an inclined angle of a few degrees vs. the earth surface in order to impart proper product flow through the dryer. The angle can be fixed or may be adjustable and is typically between 0 to 10 degrees, preferably 1 to 6 degrees, most preferably 2 to 4 degrees.

A contact dryer can be used that has two different heating zones in one apparatus. For example Nara paddle driers are available with just one heated zone or alternatively with two heated zones. The advantage of using a two or more heated zone dryer is that different phases of the thermal post-treatment and/or of the post-surface-crosslinking can be combined.

It is possible to use a contact dryer with a hot first heating zone which is followed by a temperature holding zone in the same dryer. This set up allows a quick rise of the product temperature and evaporation of surplus liquid in the first heating zone, whereas the rest of the dryer is just holding the product temperature stable to complete the reaction.

It is also possible to use a contact dryer with a warm first heating zone which is then followed by a hot heating zone. In the first warm zone the thermal post-treatment is affected or completed whereas the surface-postcrosslinking takes place in the subsequential hot zone.

Typically a paddle heater with just one temperature zone is employed.

A person skilled in the art will depending on the desired finished product properties and the available base polymer qualities from the polymerization step choose any one of these set ups.

The thermal surface-postcrosslinking can be effected in the mixer itself, by heating the jacket, blowing in warm air or steam. Equally suitable is a downstream dryer, for example a shelf dryer, a rotary tube oven or a heatable screw. It is particularly advantageous to mix and dry in a fluidized bed dryer.

Preferred thermal surface-postcrosslinking temperatures are ususally in the range of 100-195°C, mostly in the range of 100 to 180°C, preferably from 120 to 170°C, more preferably from 130 to 165°C, most preferably from 140 to 160°C. The preferred residence time at this temperature in the reaction mixer or dryer is preferably at least 5 minutes, more preferably at least 20 minutes, most preferably at least 40 minutes, and typically at most 120 minutes.

It is preferable to cool the polymer particles after thermal surface-postcrosslinking. The cooling is preferably carried out in contact coolers, more preferably paddle coolers, most preferably disk coolers. Suitable coolers are, for example, Hosokawa Bepex® horizontal paddle coolers (Hosokawa Micron GmbH; Leingarten; Germany), Hosokawa Bepex® disk coolers (Hosokawa Micron GmbH; Leingarten; Germany), Holo-Flite® coolers (Metso Minerals Industries Inc.; Danville; U.S.A.) and Nara paddle coolers (NARA Machinery Europe; Frechen; Germany). Moreover, it is also possible to use fluidized bed coolers.

In the cooler the polymer particles are cooled to temperatures in the range from 20 to 150°C, preferably from 40 to 120°C, more preferably from 60 to 100°C, most preferably from 70 to 90°C. Cooling using warm water is preferred, especially when contact coolers are used.

Coating

To improve the properties, the water-absorbent polymer particles can be coated and/or optionally moistened. The internal fluidized bed, the external fluidized bed and/or the external mixer used for the thermal posttreatment and/or a separate coater (mixer) can be used for coating of the water-absorbent polymer particles. Further, the cooler and/or a separate coater (mixer) can be used for coating/moistening of the surface-postcrosslinked water-absorbent polymer particles. Suitable coatings for controlling the acquisition behavior and improving the permeability (SFC or GBP) are, for example, inorganic inert substances, such as water-insoluble metal salts, organic polymers, cationic polymers, anionic polymers and polyvalent metal cations. Suitable coatings for improving the color stability are, for example reducing agents, chelating agents and anti-oxidants. Suitable coatings for dust binding are, for example, polyols. Suitable coatings against the undesired caking tendency of the polymer particles are, for example, fumed silica, such as Aerosil® 200, and surfactants, such as Span® 20 and Plantacare® 818 UP. Preferred coatings are aluminium dihydroxy monoacetate, aluminium sulfate, aluminium lactate, aluminium 3-hydroxypropionate, zirconium acetate, citric acid or its water soluble salts, di- and mono-phosphoric acid or their water soluble salts, Blancolen®, Brüggolite® FF7, Cublen®, Span® 20 and Plantacare® 818 UP.

If salts of the above acids are used instead of the free acids then the preferred salts are alkali-metal, earth alkali metal, aluminum, zirconium, titanium, zinc and ammonium salts.

Under the trade name Cublen® (Zschimmer & Schwarz Mohsdorf GmbH & Co KG; Burgstädt; Germany)the following acids and/or their alkali metal salts (preferably Na and K-salts) are available and may be used within the scope of the present invention for example to impart color-stability to the finished product:

1-Hydroxyethane-1,1-diphosphonic acid, Amino-tris(methylene phosphonic acid), Ethylenediamine-tetra(methylene phosphonic acid), Diethylenetriamine-penta(methylene phosphonic acid), Hexamethylene diamine-tetra(methylenephosphonic acid), Hydroxyethyl-amino-di(methylene phosphonic acid), 2-Phosphonobutane-1,2,4-tricarboxylic acid, Bis(hexamethylenetriamine penta(methylene phosphonic acid).

Most preferably 1-Hydroxyethane-1,1-diphosphonic acid or its salts with sodium, potassium, or ammonium are employed. Any mixture of the above Cublenes® can be used.

Alternatively, any of the chelating agents described before for use in the polymerization can be coated onto the finished product.

Suitable inorganic inert substances are silicates such as montmorillonite, kaolinite and talc, zeolites, activated carbons, polysilicic acids, magnesium carbonate, calcium carbonate, calcium phosphate, aluminum phosphate, barium sulfate, aluminum oxide, titanium dioxide and iron(II) oxide. Preference is given to using polysilicic acids, which are divided between precipitated silicas and fumed silicas according to their mode of preparation. The two variants are commercially available under the names Silica FK, Sipernat®, Wessalon® (precipitated silicas) and Aerosil® (fumed silicas) respectively. The inorganic inert substances may be used as dispersion in an aqueous or water-miscible dispersant or in substance.

When the water-absorbent polymer particles are coated with inorganic inert substances, the amount of inorganic inert substances used, based on the water-absorbent polymer particles, is preferably from 0.05 to 5% by weight, more preferably from 0.1 to 1.5% by weight, most preferably from 0.3 to 1% by weight.

Suitable organic polymers are polyalkyl methacrylates or thermoplastics such as polyvinyl chloride, waxes based on polyethylene, polypropylene, polyamides or polytetrafluoroethylene. Other examples are styrene-isoprene-styrene block-copolymers or styrenebutadiene-styrene block-copolymers. Another example are silanole-group bearing polyvinylalcoholes available under the trade name Poval® R (Kuraray Europe GmbH; Frankfurt; Germany).

Suitable cationic polymers are polyalkylenepolyamines, cationic derivatives of polyacrylamides, polyethyleneimines and polyquaternary amines.

Polyquaternary amines are, for example, condensation products of hexamethylenediamine, dimethylamine and epichlorohydrin, condensation products of dimethylamine and epichlorohydrin, copolymers of hydroxyethylcellulose and diallyldimethylammonium chloride, copolymers of acrylamide and $\alpha$-methacryloyloxyethyltrimethylammonium chloride, condensation products of hydroxyethylcellulose, epichlorohydrin and trimethylamine, homopolymers of diallyldimethylammonium chloride and addition products of epichlorohydrin to amidoamines. In addition, polyquaternary amines can be obtained by reacting dimethyl sulfate with polymers such as polyethyleneimines, copolymers of vinylpyrrolidone and dimethylaminoethyl methacrylate or copolymers of ethyl methacrylate and diethylaminoethyl methacrylate. The polyquaternary amines are available within a wide molecular weight range.

However, it is also possible to generate the cationic polymers on the particle surface, either through reagents which can form a network with themselves, such as addition products of epichlorohydrin to polyamidoamines, or through the application of cationic polymers which can react with an added crosslinker, such as polyamines or polyimines in combination with polyepoxides, polyfunctional esters, polyfunctional acids or polyfunctional (meth)acrylates.

It is possible to use all polyfunctional amines having primary or secondary amino groups, such as polyethyleneimine, polyallylamine and polylysine. The liquid sprayed by the process according to the invention preferably comprises at least one polyamine, for example polyvinylamine or a partially hydrolyzed polyvinylformamide.
The cationic polymers may be used as a solution in an aqueous or water-miscible solvent, as dispersion in an aqueous or water-miscible dispersant or in substance.

When the water-absorbent polymer particles are coated with a cationic polymer, the use amount of cationic polymer based on the water-absorbent polymer particles is usually not less than 0.001% by weight, typically not less than 0.01% by weight, preferably from 0.1 to 15% by weight, more preferably from 0.5 to 10% by weight, most preferably from 1 to 5% by weight.

Suitable anionic polymers are polyacrylates (in acidic form or partially neutralized as salt), copolymers of acrylic acid and maleic acid available under the trade name Sokalan® (BASF SE; Ludwigshafen; Germany), and polyvinylalcohols with built in ionic charges available under the trade name Poval® K (Kuraray Europe GmbH; Frankfurt; Germany).

Suitable polyvalent metal cations are $Mg^{2+}$, $Ca^{2+}$, $Al^{3+}$, $Sc^{3+}$, $Ti^{4+}$, $Mn^{2+}$, $Fe^{2+/3+}$, $CO^{2+}$, $Ni^{2+}$, $Cu^{+/2+}$, $Zn^{2+}$, $Y^{3+}$, $Zr^{4+}$, $Ag^+$, $La^{3+}$, $Ce^{4+}$, $Hf^{4+}$ and $Au^{+/3+}$; preferred metal cations are $Mg^{2+}$, $Ca^{2+}$, $Al^{3+}$, $Ti^{4+}$, $Zr^{4+}$ and $La^{3+}$; particularly preferred metal cations are $Al^{3+}$, $Ti^{4+}$ and $Zr^{4+}$. The metal cations may be used either alone or in a mixture with one another. Suitable metal salts of the metal cations mentioned are all of those which have a sufficient solubility in the solvent to be used. Particularly suitable metal salts have weakly complexing anions, such as chloride, hydroxide, carbonate, acetate, formiate, propionate, nitrate, sulfate and methanesulfate. The metal salts are preferably used as a solution or as a stable aqueous colloidal dispersion. The solvents used for the metal salts may be water, alcohols, ethylenecarbonate, propylenecarbonate, dimethylformamide, dimethyl sulfoxide and mixtures thereof. Particular preference is given to water and water/alcohol mixtures, such as water/methanol, water/isopropanol, water/1,3-propanediole, water/1,2-propandiole/1,4-butanediole or water/propylene glycol.

When the water-absorbent polymer particles are coated with a polyvalent metal cation, the amount of polyvalent metal cation used, based on the water-absorbent polymer particles, is preferably from 0.05 to 5% by weight, more preferably from 0.1 to 1.5% by weight, most preferably from 0.3 to 1% by weight.

Suitable reducing agents are, for example, sodium sulfite, sodium hydrogensulfite (sodium bisulfite), sodium dithionite, sulfinic acids and salts thereof, ascorbic acid, sodium hypophosphite, sodium phosphite, and phosphinic acids and salts thereof. Preference is given, however, to salts of hypophosphorous acid, for example sodium hypophosphite, salts of sulfinic acids, for example the disodium salt of 2-hydroxy-2-sulfinatoacetic acid, and addition products of aldehydes, for example the disodium salt of 2-hydroxy-2-sulfonatoacetic acid. The reducing agent used can be, however, a mixture of the sodium salt of 2-hydroxy-2-sulfinatoacetic acid, the disodium salt of 2-hydroxy-2-sulfonatoacetic acid and sodium bisulfite. Such mixtures are obtainable as Brüggolite® FF6 and Brüggolite® FF7 (Brüggemann Chemicals; Heilbronn; Germany). Also useful is the purified 2-hydroxy-2-sulfonatoacetic acid and its sodium salts, available under the trade name Blancolen® from the same company.

The reducing agents are typically used in the form of a solution in a suitable solvent, preferably water. The reducing agent may be used as a pure substance or any mixture of the above reducing agents may be used.

When the water-absorbent polymer particles are coated with a reducing agent, the amount of reducing agent used, based on the water-absorbent polymer particles, is preferably from 0.01 to 5% by weight, more preferably from 0.05 to 2% by weight, most preferably from 0.1 to 1% by weight.

Suitable polyols are polyethylene glycols having a molecular weight of from 400 to 20000 g/mol, polyglycerol, 3- to 100-tuply ethoxylated polyols, such as trimethylolpropane, glycerol, sorbitol, mannitol, inositol, pentaerythritol and neopentyl glycol. Particularly suitable polyols are 7- to 20-tuply ethoxylated glycerol or trimethylolpropane, for example Polyol TP 70® (Perstorp AB, Perstorp, Sweden). The latter have the advantage in particular that they lower the surface tension of an aqueous extract of the water-absorbent polymer particles only insignificantly. The polyols are preferably used as a solution in aqueous or water-miscible solvents.

The polyol can be added before, during, or after surface-crosslinking. Preferably it is added after surface-cross linking. Any mixture of the above listed poyols may be used.

When the water-absorbent polymer particles are coated with a polyol, the use amount of polyol, based on the water-absorbent polymer particles, is preferably from 0.005 to 2% by weight, more preferably from 0.01 to 1% by weight, most preferably from 0.05 to 0.5% by weight.

The coating is preferably performed in mixers with moving mixing tools, such as screw mixers, disk mixers, paddle mixers and drum coater. Suitable mixers are, for example, horizontal Pflugschar® plowshare mixers (Gebr. Lödige Maschinenbau GmbH; Paderborn; Germany), Vrieco-Nauta Continuous Mixers (Hosokawa Micron BV; Doetinchem; the Netherlands), Processall Mixmill Mixers (Processall Incorporated; Cincinnati; US) and Ruberg continuous flow mixers (Gebrüder Ruberg GmbH & Co KG, Nieheim, Germany). Moreover, it is also possible to use a fluidized bed for mixing.

Agglomeration

The water-absorbent polymer particles can further selectivily be agglomerated. The agglomeration can take place after the polymerization, the thermal postreatment, the thermal surface-postcrosslinking or the coating.

Useful agglomeration assistants include water and water-miscible organic solvents, such as alcohols, tetrahydrofuran and acetone; water-soluble polymers can be used in addition.

For agglomeration a solution comprising the agglomeration assistant is sprayed onto the water-absorbing polymeric particles. The spraying with the solution can, for example, be carried out in mixers having moving mixing implements, such as screw mixers, paddle mixers, disk mixers, plowshare mixers and shovel mixers. Useful mixers include for example Lödige® mixers, Bepex® mixers, Nauta® mixers, Processall® mixers and Schugi® mixers. Vertical mixers are preferred. Fluidized bed apparatuses are particularly preferred.

Combination of thermal posttreatment, surface-postcrosslinking and optionally coating

It is preferred that the steps of thermal posttreatment and thermal surface-postcrosslinking are combined in one process step. Such combination allows the use of low cost equipment and moreover the process can be run at low temperatures, that is cost-efficient and avoids discoloration and loss of performance properties of the finished product by thermal degradation.

The mixer may be selected from any of the equipment options cited in the thermal post-treatment section. Ruberg continuous flow mixers, Becker shovel mixers and Pflugschar® plowshare mixers are preferred.

It is particular preferred that the surface-postcrosslinking solution is sprayed onto the water-absorbent polymer particles under agitation.

Following the thermal posttreatment/surface-postcrosslinking the water-absorbent polymer particles are dried to the desired moisture level and for this step any dryer cited in the surface-postcrosslinking section may be selected. However, as only drying needs to be accomplished in this particular preferred embodiment it is possible to use simple and low cost heated contact dryers like a heated screw dryer, for example a Holo-Flite® dryer (Metso Minerals Industries Inc.; Danville; U.S.A.). Alternatively a fluidized bed may be used. In cases where the product needs to be dried with a predetermined and narrow residence time it is possible to use torus disc dryers or paddle dryers, for example a Nara paddle dryer (NARA Machinery Europe; Frechen; Germany).

In a preferred embodiment of the present invention, polyvalent cations cited in the surface-postcrosslinking section are applied to the particle surface before, during or after addition of the surface-postcrosslinker by using different addition points along the axis of a horizontal mixer.

It is very particular preferred that the steps of thermal post-treatment, surface-postcrosslinking, and coating are combined in one process step. Suitable coatings are cationic polymers, surfactants, and inorganic inert substances that are cited in the coating section. The coating agent can be applied to the particle surface before, during or after addition of the surface-postcrosslinker also by using different addition points along the axis of a horizontal mixer.

The polyvalent cations and/or the cationic polymers can act as additional scavengers for residual surface-postcrosslinkers. It is preferred that the surface-postcrosslinkers are added prior to the polyvalent cations and/or the cationic polymers to allow the surface-postcrosslinker to react first.

The surfactants and/or the inorganic inert substances can be used to avoid sticking or caking during this process step under humid atmospheric conditions. Preferred surfactants are non-ionic and amphoteric surfactants. Preferred inorganic inert substances are precipitated silicas and fumed silcas in form of powder or dispersion.

The amount of total liquid used for preparing the solutions/dispersions is typically from 0.01% to 25% by weight, preferably from 0.5% to 12% by weight, more preferably from 2% to 7% by weight, most preferably from 3% to 6% by weight, in respect to the weight amount of water-absorbent polymer particles to be processed.

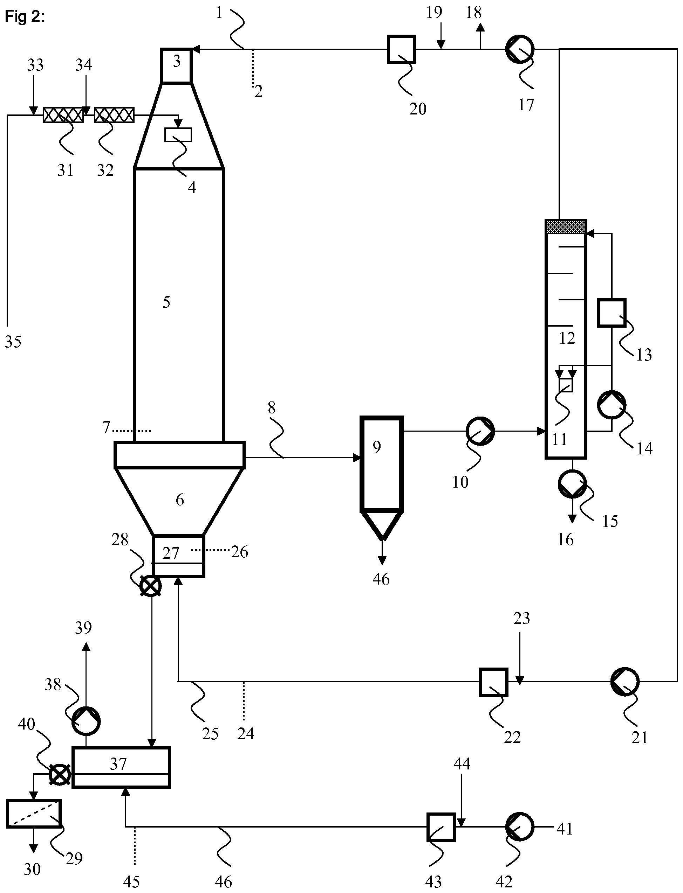

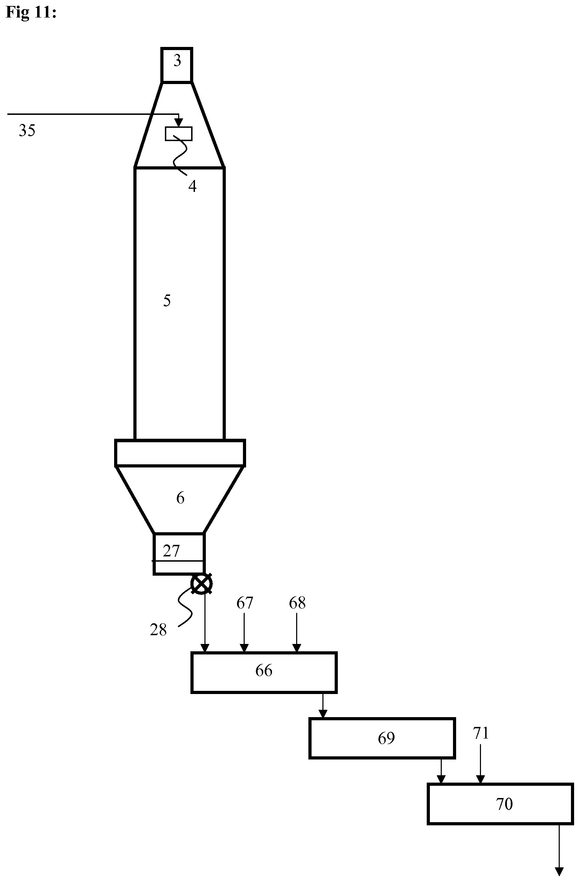

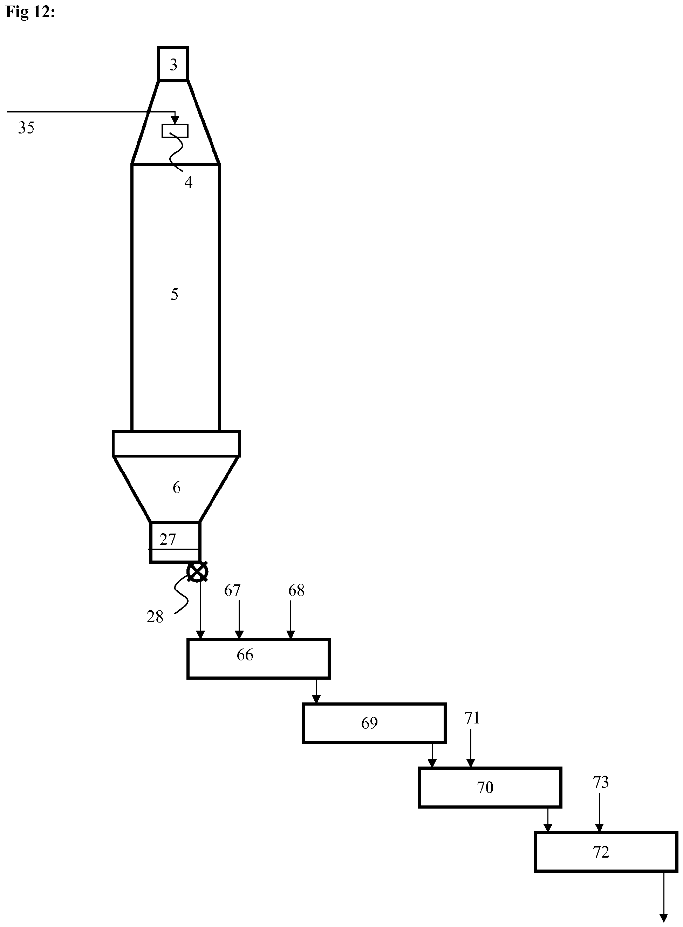

Preferred embodiments are depicted in figures 1 to 12.

Fig. 1: Process scheme (without external fluidized bed)
Fig. 2: Process scheme (with external fluidized bed)
Fig. 3: Arrangement of the T_outlet measurement
Fig. 4: Arrangement of the dropletizer units
Fig. 5: Dropletizer unit (longitudinal cut)
Fig. 6: Dropletizer unit (cross sectional view)
Fig. 7: Bottom of the internal fluidized bed (top view)
Fig. 8: openings in the bottom of the internal fluidized bed
Fig. 9: Rake stirrer for the intern fluidized bed (top view)
Fig. 10: Rake stirrer for the intern fluidized bed (cross sectional view)
Fig. 11: Process scheme (surface-postcrosslinking)
Fig. 12: Process scheme (surface-postcrosslinking and coating)
Fig. 13: Contact dryer for surface-postcrosslnking

The reference numerals have the following meanings:

1    Drying gas inlet pipe
2    Drying gas amount measurement
3    Gas distributor
4    Dropletizer units
5    Cocurrent spray dryer, cylindrical part
6    Cone
7    T_outlet measurement
8    Tower offgas pipe
9    Baghouse filter
10   Ventilator
11   Quench nozzles
12   Condenser column, counter current cooling
13   Heat exchanger
14   Pump
15   Pump
16   Water outlet
17   Ventilator
18   Offgas outlet
19   Nitrogen inlet
20   Heat exchanger
21   Ventilator
22   Heat exchanger
23   Steam injection via nozzles
24   Water loading measurement
25   Conditioned internal fluidized bed gas
26   Internal fluidized bed product temperature measurement
27   Internal fluidized bed

| 28 | Rotary valve |
|----|---|
| 29 | Sieve |
| 30 | End product |
| 31 | Static mixer |
| 32 | Static mixer |
| 33 | Initiator feed |
| 34 | Initiator feed |
| 35 | Monomer feed |
| 36 | Fine particle fraction outlet to rework |
| 37 | External fluidized bed |
| 38 | Ventilator |
| 39 | External fluidized bed offgas outlet to baghouse filter |
| 40 | Rotary valve |
| 41 | Filtered air inlet |
| 42 | Ventilator |
| 43 | Heat exchanger |
| 44 | Steam injection via nozzle |
| 45 | Water loading measurement |
| 46 | Conditioned external fluidized bed gas |
| 47 | T_outlet measurement (average temperature out of 3 measurements around tower circumference) |
| 48 | Dropletizer unit |
| 49 | Monomer premixed with initiator feed |
| 50 | Spray dryer tower wall |
| 51 | Dropletizer unit outer pipe |
| 52 | Dropletizer unit inner pipe |
| 53 | Dropletizer cassette |
| 54 | Teflon block |
| 55 | Valve |
| 56 | Monomer premixed with initiator feed inlet pipe connector |
| 57 | Droplet plate |
| 58 | Counter plate |
| 59 | Flow channels for temperature control water |
| 60 | Dead volume free flow channel for monomer solution |
| 61 | Dropletizer cassette stainless steel block |
| 62 | Bottom of the internal fluidized bed with four segments |
| 63 | Split openings of the segments |
| 64 | Rake stirrer |
| 65 | Prongs of the rake stirrer |
| 66 | Mixer |
| 67 | Optional coating feed |
| 68 | Postcrosslinker feed |
| 69 | Thermal dryer (surface-postcrosslinking) |
| 70 | Cooler |
| 71 | Optional coating/water feed |
| 72 | Coater |
| 73 | Coating/water feed |
| 74 | Base polymer feed |
| 75 | Discharge zone |
| 76 | Weir opening |
| 77 | weir plate |
| 78 | Weir height 100% |
| 79 | Weir height 50% |

80      Shaft
81      Discharge cone
82      Inclination angle $\alpha$
83      Temperature sensors ($T_1$ to $T_6$)
84      Paddle (shaft offset 90°)

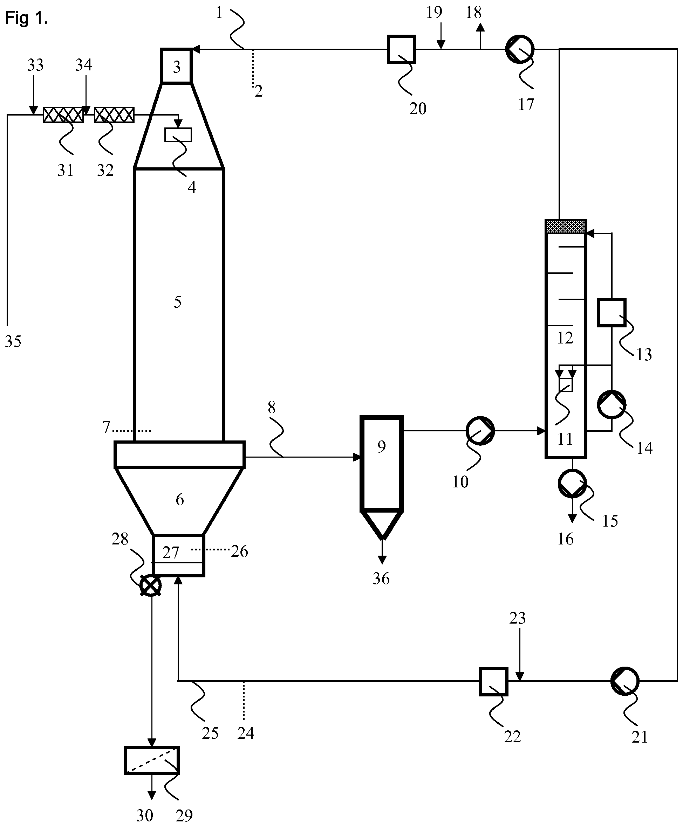

The drying gas is fed via a gas distributor (3) at the top of the spray dryer as shown in Fig. 1. The drying gas is partly recycled (drying gas loop) via a baghouse filter (9) and a condenser column (12). The pressure inside the spray dryer is below ambient pressure.

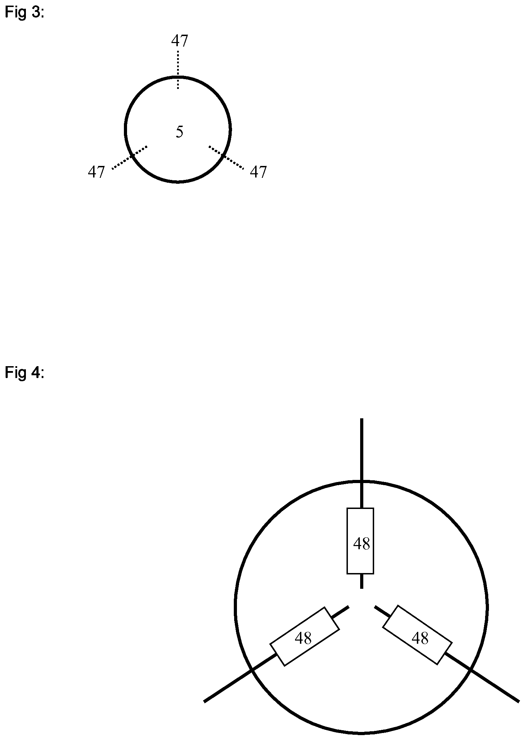

The spray dryer outlet temperature is preferably measured at three points around the circumference at the end of the cylindrical part as shown in Fig. 3. The single measurements (47) are used to calculate the average cylindrical spray dryer outlet temperature.

The product accumulated in the internal fluidized bed (27). Conditioned internal fluidized bed gas is fed to the internal fluidized bed (27) via line (25). The relative humidity of the internal fluidized bed gas is preferably controlled by adding steam via line (23).

The spray dryer offgas is filtered in baghouse filter (9) and sent to a condenser column (12) for quenching/cooling. After the baghouse filter (9) a recuperation heat exchanger system for preheating the gas after the condenser column (12) can be used. The baghouse filter (9) may be trace-heated on a temperature of preferably from 80 to 180°C, more preferably from 90 to 150°C, most preferably from 100 to 140°C. Excess water is pumped out of the condenser column (12) by controlling the (constant) filling level inside the condenser column (12). The water inside the condenser column (12) is cooled by a heat exchanger (13) and pumped counter-current to the gas via quench nozzles (11) so that the temperature inside the condenser column (12) is preferably from 20 to 100°C, more preferably from 25 to 80°C, most preferably from 30 to 60°C. The water inside the condenser column (12) is set to an alkaline pH by dosing a neutralizing agent to wash out vapors of monomer a). Aqueous solution from the condenser column (12) can be sent back for preparation of the monomer solution.

The condenser column offgas is split to the drying gas inlet pipe (1) and the conditioned internal fluidized bed gas (25). The gas temperatures are controlled via heat exchangers (20) and (22). The hot drying gas is fed to the cocurrent spray dryer via gas distributor (3). The gas distributor (3) consists preferably of a set of plates providing a pressure drop of preferably 1 to 100mbar, more preferably 2 to 30mbar, most preferably 4 to 20mbar, depending on the drying gas amount. Turbulences and/or a centrifugal velocity can also be introduced into the drying gas if desired by using gas nozzles or baffle plates. Conditioned internal fluidized bed gas is fed to the internal fluidized bed (27) via line (25). The relative humidity of the external fluidized bed gas is preferably controlled by adding steam via line (23). To prevent any condensation the steam is added together with the internal fluidized bed into the heat exchanger (22). The product holdup in the internal fluidized bed (27) can be controlled via rotational speed of the rotary valve (28).

The product is discharged from the internal fluidized bed (27) via rotary valve (28). The product holdup in the internal fluidized bed (27) can be controlled via rotational speed of the rotary valve (28). The sieve (29) is used for sieving off overs/lumps.

The monomer solution is preferably prepared by mixing first monomer a) with a neutralization agent and optionally secondly with crosslinker b). The temperature during neutralization is controlled to preferably from 5 to 60°C, more preferably from 8 to 40°C, most preferably from 10 to 30°C, by using a heat exchanger and pumping in a loop. A filter unit is preferably used in the loop after the pump. The initiators are metered into the monomer solution upstream of the dropletizer by means of static mixers (31) and (32) via lines (33) and (34) as shown in Fig. 1. Preferably a peroxide solution having a temperature of preferably from 5 to 60°C, more preferably from 10 to 50°C, most preferably from 15 to 40°C, is added via line (33) and preferably an azo initiator solution having a temperature of preferably from 2 to 30°C, more preferably from 3 to 15°C, most preferably from 4 to 8°C, is added via line (34). Each initiator is preferably pumped in a loop and dosed via control valves to each dropletizer unit. A second filter unit is preferably used after the static mixer (32). The mean residence time of the monomer solution admixed with the full initiator package in the piping before the droplet plates (57) is preferably less than 60s, more preferably less than 30s, most preferably less than 10s.

For dosing the monomer solution into the top of the spray dryer preferably three dropletizer units are used as shown in Fig. 4. However, any number of dropletizers can be used that is required to optimize the throughput of the process and the quality of the product. Hence, in the present invention at least one dropletizer is employed, and as many dropletizers as geometrically allowed may be used.

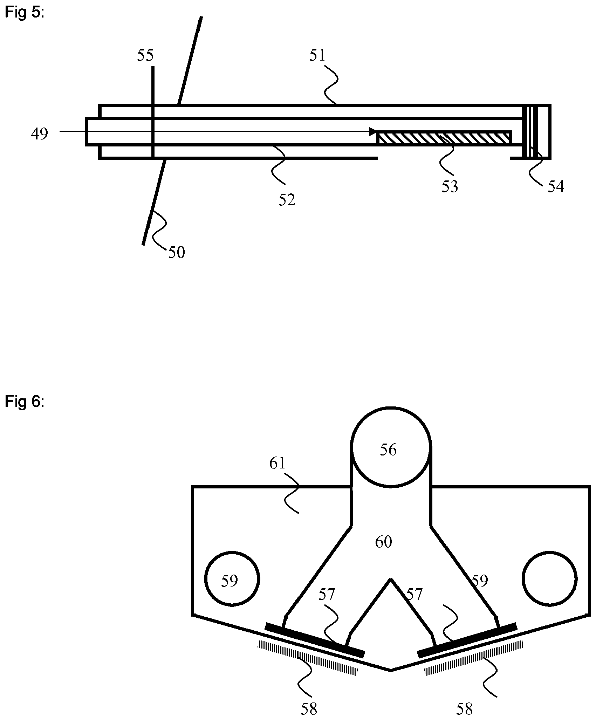

A dropletizer unit consists of an outer pipe (51) having an opening for the dropletizer cassette (53) as shown in Fig. 5. The dropletizer cassette (53) is connected with an inner pipe (52). The inner pipe (53) having a PTFE block (54) at the end as sealing can be pushed in and out of the outer pipe (51) during operation of the process for maintenance purposes.

The temperature of the dropletizer cassette (61) is controlled to preferably 5 to 80°C, more preferably 10 to 70°C, most preferably 30 to 60°C, by water in flow channels (59) as shown in Fig. 6.

The dropletizer cassette has preferably from 10 to 1500, more preferably from 50 to 1000, most preferably from 100 to 500, bores having a diameter of preferably from 50 to 500μm, more preferably from 100 to 300μm, most preferably from 150 to 250μm. The bores can be of circular, rectangular, triangular or any other shape. Circular bores are preferred. The ratio of bore length to bore diameter is preferably from 0.5 to 10, more preferably from 0.8 to 5, most preferably from 1 to 3. The droplet plate (57) can have a greater thickness than the bore length when using an inlet bore channel. The droplet plate (57) is preferably long and narrow as disclosed in WO 2008/086976 A1. Multiple rows of bores per droplet plate can be used, preferably from 1 to 20 rows, more preferably from 2 to 5 rows.

The dropletizer cassette (61) consists of a flow channel (60) having essential no stagnant volume for homogeneous distribution of the premixed monomer and initiator solutions and two droplet plates (57). The droplet plates (57) have an angled configuration with an angle of preferably from 1 to 90°, more preferably from 3 to 45°, most preferably from 5 to 20°. Each droplet plate (57) is preferably made of a heat and/or chemically resistant material, such as stainless steel, polyether ether ketone, polycarbonate, polyarylsulfone, such as polysulfone, or polyphenylsulfone, or fluorous polymers, such as perfluoroalkoxyethylene, polytetrafluoroethylene, polyvinylidenfluorid, ethylene-chlorotrifluoroethylene copolymers, ethylene-tetrafluoroethylene copolymers and fluorinated polyethylene. Coated droplet plates as disclosed in WO 2007/031441 A1 can also be used. The choice of material for the droplet plate is not limited except that droplet formation must work and it is preferable to use materials which do not catalyze the start of polymerization on its surface.

The throughput of monomer including initiator solutions per dropletizer unit is preferably from 150 to 2500kg/h, more preferably from 200 to 1000kg/h, most preferably from 300 to 600kg/h. The throughput per bore is preferably from 0.1 to 10kg/h, more preferably from 0.5 to 5kg/h, most preferably from 0.7 to 2kg/h.

The start-up of the cocurrent spray dryer (5) can be done in the following sequence:

- starting the condenser column (12),
- starting the ventilators (10) and (17),
- starting the heat exchanger (20),
- heating up the drying gas loop up to 95°C,
- starting the nitrogen feed via the nitrogen inlet (19),
- waiting until the residual oxygen is below 4% by weight,
- heating up the drying gas loop,
- at a temperature of 105°C starting the water feed (not shown) and
- at target temperature stopping the water feed and starting the monomer feed via dropletizer unit (4)

The shut-down of the cocurrent spray dryer (5) can be done in the following sequence:

- stopping the monomer feed and starting the water feed (not shown),
- shut-down of the heat exchanger (20),
- cooling the drying gas loop via heat exchanger (13),
- at a temperature of 105°C stopping the water feed,
- at a temperature of 60°C stopping the nitrogen feed via the nitrogen inlet (19) and
- feeding air into the drying gas loop (not shown)

To prevent damages the cocurrent spray dryer (5) must be heated up and cooled down very carefully. Any quick temperature change must be avoided.

The openings in the bottom of the internal fluidized bed may be arranged in a way that the water-absorbent polymer particles flow in a cycle as shown in Fig. 7. The bottom shown in Fig. 7 comprises of four segments (62). The openings (63) in the segments (62) are in the shape of slits that guides the passing gas stream into the direction of the next segment (62). Fig. 8 shows an enlarged view of the openings (63).

The opening may have the shape of holes or slits. The diameter of the holes is preferred from 0.1 to 10mm, more preferred from 0.2 to 5mm, most preferred from 0.5 to 2mm. The slits have a length of preferred from 1 to 100mm, more preferred from 2 to 20mm, most preferred from 5 to 10mm, and a width of preferred from 0.5 to 20mm, more preferred from 1 to 10mm, most preferred from 2 to 5mm.

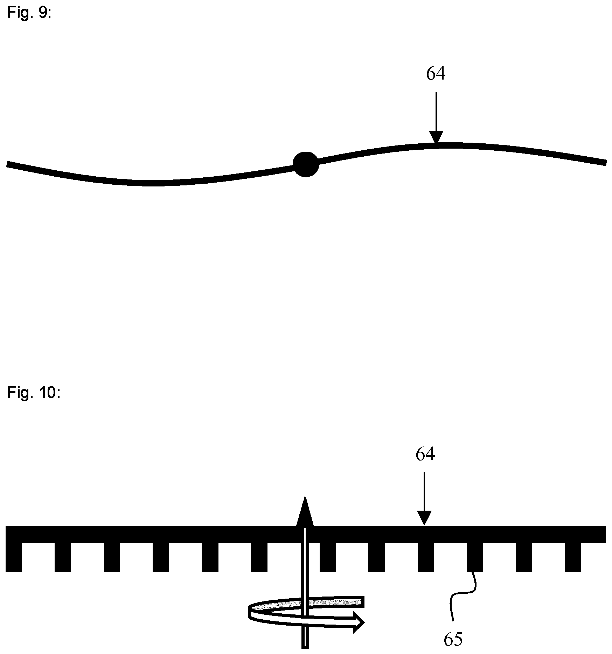

Fig. 9 and Fig. 10 show a rake stirrer (64) that may be used in the internal fluidized bed. The prongs (65) of the rake have a staggered arrangement. The speed of rake stirrer is preferably from 0.5 to 20 rpm, more preferably from 1 to 10 rpm most preferably from 2 to 5 rpm.

For start-up the internal fluidized bed may be filled with a layer of water-absorbent polymer particles, preferably 5 to 50 cm, more preferably from 10 to 40 cm, most preferably from 15 to 30 cm.

The surface-postcrosslinked water-absorbent polymer particles having a centrifuge retention capacity from 35 to 75 g/g, an absorption under high load from 20 to 50 g/g, a level of extractable constituents of less than 10% by weight, and a porosity from 20 to 40%.

It is particular advantageous that the surface-postcrosslinked water-absorbent polymer particles exhibit a very high centrifuge retention capacity (CRC) and a high absorption under high load (AUHL), and that the sum of these parameters (= CRC + AUHL) is at least 60 g/g, preferably at least 65 g/g, most preferably at least 70 g/g, and not more than 120 g/g, preferably less than 100 g/g, more preferably less than 90 g/g, and most preferably less than 80 g/g. The surface-postcrosslinked water-absorbent polymer particles further preferably exhibit an absorption under high load (AUHL) of at least 15 g/g, preferably at least 18 g/g, more preferably at least 21 g/g, most preferably at least 25 g/g, and not more than 50 g/g.

As the centrifuge retention capacity (CRC) is the maximum water retention capacity of the surface-postcrosslinked water-absorbent polymer particles it is of interest to maximize this parameter. However the absorption under high load (AUHL) is important to allow the fiber-matrix in a hygiene article to open up pores during swelling to allow further liquid to pass easily through the article structure to enable rapid uptake of this liquid. Hence there is a need to maximize both parameters.

The water-absorbent polymer particles have a centrifuge retention capacity (CRC) from 35 to 75 g/g, preferably from 37 to 65 g/g, more preferably from 39 to 60 g/g, most preferably from 40 to 55 g/g.

The water-absorbent polymer particles have an absorbency under a load of 49.2 $g/cm^2$ (AUHL) from 20 to 50 g/g, preferably from 22 to 45 g/g, more preferably from 24 to 40 g/g, most preferably from 25 to 35 g/g.

The water-absorbent polymer particles have a level of extractable constituents of less than 10% by weight, preferably less than 8% by weight, more preferably less than 6% by weight, most preferably less than 5% by weight.

The water-absorbent polymer particles have a porosity from 20 to 40%, preferably from 22 to 38%, more preferably from 24 to 36%, most preferably from 25 to 35%.

Preferred water-absorbent polymer particles are polymer particles having a centrifuge retention capacity (CRC) from 37 to 65 g/g, an absorption under high load (AUHL) from 22 to 45 g/g, a level of extractable constituents of less than 8% by weight and a porosity from 22 to 45%.

More preferred water-absorbent polymer particles are polymer particles having a centrifuge retention capacity (CRC) from 39 to 60 g/g, an absorption under high load (AUHL) from 24 to 40 g/g, a level of extractable constituents of less than 6% by weight and a porosity from 24 to 40%.

Most preferred water-absorbent polymer particles are polymer particles having a centrifuge retention capacity (CRC) from 40 to 55 g/g, an absorption under high load (AUHL) from 25 to 35 g/g, a level of extractable constituents of less than 5% by weight and a porosity from 25 to 35%.

Also preferred are surface-postcrosslinked water-absorbent polymer particles having a total liquid uptake of

$$Y > -500 \times \ln(X) + 1880,$$

$$\text{preferably } Y > -495 \times \ln(X) + 1875,$$

$$\text{more preferably } Y > -490 \times \ln(X) + 1870,$$

$$\text{most preferably } Y > -485 \times \ln(X) + 1865,$$

wherein Y [g] is the total liquid uptake and X [g/g] is the centrifuge retention capacity (CRC), wherein the centrifuge retention capacity (CRC) is at least 25 g/g, preferably at least 30 g/g, more preferably at least 35 g/g, most preferably at least 40 g/g, and the liquid uptake is at least 30 g, preferably at least 35 g/g, more preferably at least 40 g/g, most preferably at least 45 g/g.

Further suited are surface-postcrosslinked water-absorbent polymer particles having a change of characteristic swelling time of less than 0.6, preferably less than 0.5, more preferably less than 0.45, most preferably less than 0.4, and a centrifuge retention capacity (CRC) of at least 35 g/g, preferably at least 37 g/g, more preferably at least 38.5 g/g, most preferably at least 40 g/g, wherein the change of characteristic swelling time is

$$Z < (\tau_{0.5} - \tau_{0.1}) / \tau_{0.5}$$

wherein Z is the change of characteristic swelling time, $\tau_{0.1}$ is the characteristic swelling time under a pressure of 0.1 psi (6.9 $g/cm^2$) and $\tau_{0.5}$ is the characteristic swelling time under a pressure of 0.5 psi (35.0 $g/cm^2$).

The water-absorbent polymer particles suited for the present invention have a mean sphericity from 0.80 to 0.95, preferably from 0.82 to 0.93, more preferably from 0.84 to 0.91, most preferably from 0.85 to 0.90. The sphericity (SPHT) is defined as

$$ SPHT = \frac{4\pi A}{U^2}, $$

where A is the cross-sectional area and U is the cross-sectional circumference of the polymer particles. The mean sphericity is the volume-average sphericity.

The mean sphericity can be determined, for example, with the Camsizer® image analysis system (Retsch Technolgy GmbH; Haan; Germany):

For the measurement, the product is introduced through a funnel and conveyed to the falling shaft with a metering channel. While the particles fall past a light wall, they are recorded selectively by a camera. The images recorded are evaluated by the software in accordance with the parameters selected.

To characterize the roundness, the parameters designated as sphericity in the program are employed. The parameters reported are the mean volume-weighted sphericities, the volume of the particles being determined via the equivalent diameter $xc_{min}$. To determine the equivalent diameter $xc_{min}$, the longest chord diameter for a total of 32 different spatial directions is measured in each case. The equivalent diameter $xc_{min}$ is the shortest of these 32 chord diameters. To record the particles, the so-called CCD-zoom camera (CAM-Z) is used. To control the metering channel, a surface coverage fraction in the detection window of the camera (transmission) of 0.5% is predefined.

Water-absorbent polymer particles with relatively low sphericity are obtained by reverse suspension polymerization when the polymer beads are agglomerated during or after the polymerization.

The water-absorbent polymer particles prepared by customary solution polymerization (gel polymerization) are ground and classified after drying to obtain irregular polymer particles. The mean sphericity of these polymer particles is between approx. 0.72 and approx. 0.78.

Water-absorbent polymer particles suited for the present invention have a content of hydrophobic solvent of preferably less than 0.005% by weight, more preferably less than 0.002% by weight and most preferably less than 0.001% by weight. The content of hydrophobic solvent can be determined by gas chromatography, for example by means of the headspace technique. A hydrophobic solvent within the scope of the present invention is either immiscible in water or only sparingly miscible. Typical examples of hydrophobic solvents are pentane, hexane, cyclohexane, toluene.

Water-absorbent polymer particles which have been obtained by reverse suspension polymerization still comprise typically approx. 0.01% by weight of the hydrophobic solvent used as the reaction medium.

The water-absorbent polymer particles useful for the present invention have a dispersant content of typically less than 1% by weight, preferably less than 0.5% by weight, more preferably less than 0.1% by weight and most preferably less than 0.05% by weight.

Water-absorbent polymer particles which have been obtained by reverse suspension polymerization still comprise typically at least 1% by weight of the dispersant, i.e. ethylcellulose, used to stabilize the suspension.

Suitable water-absorbent polymer particles have a bulk density preferably from 0.6 to 1 g/cm³, more preferably from 0.65 to 0.9 g/cm³, most preferably from 0.68 to 0.8 g/cm³.

The average particle diameter (APD) of the water-absorbent particles useful for the present invention is preferably from 200 to 550 $\mu$m, more preferably from 250 to 500 $\mu$m, most preferably from 350 to 450 $\mu$m.

The particle diameter distribution (PDD) of the useful water-absorbent particles is preferably less than 0.7, more preferably less than 0.65, more preferably less than 0.6.

One kind of water-absorbent polymer particles can be mixed with other water-absorbent polymer particles prepared by other processes, i.e. solution polymerization.

C. Fluid-absorbent articles

The fluid-absorbent article comprises of

(A) an upper liquid-pervious layer
(B) a lower liquid-impervious layer
(C) a fluid-absorbent core between (A) and (B) comprising
from 0 to 90% by weight a fibrous material and from 10 to 100% by weight water-absorbent polymer particles;
preferably from 20 to 80% by weight a fibrous material and from 20 to 80% by weight water-absorbent polymer particles;
more preferably from 30 to 75% by weight a fibrous material and from 25 to 70% by weight water-absorbent polymer particles;

most preferably from 40 to 70% by weight a fibrous material and from 30 to 60% by weight water-absorbent polymer particles;

(D) an optional acquisition-distribution layer between (A) and (C), comprising from 80 to 100% by weight a fibrous material and from 0 to 20% by weight water-absorbent polymer particles;

preferably from 85 to 99.9% by weight a fibrous material and from 0.01 to 15% by weight water-absorbent polymer particles;

morepreferably from 90 to 99.5% by weight a fibrous material and from 0.5 to 10% by weight water-absorbent polymer particles;

most preferably from 95 to 99% by weight a fibrous material and from 1 to 5% by weight water-absorbent polymer particles;

(E) an optional tissue layer disposed immediately above and/or below (C) or wrapped fully or partially around (C) ; and

(F) other optional components.

Fluid-absorbent articles are understood to mean, for example, incontinence pads and incontinence briefs for adults or diapers and training pants for babies. Suitable fluid-absorbent articles including fluid-absorbent compositions comprising fibrous materials and optionally water-absorbent polymer particles to form fibrous webs or matrices for the substrates, layers, sheets and/or the fluid-absorbent core.

The acquisition-distribution layer acts as transport and distribution layer of the discharged body fluids and is typically optimized to affect efficient liquid distribution with the underlying fluid-absorbent core. Hence, for quick temporary liquid retention it provides the necessary void space while its area coverage of the underlying fluid-absorbent core must affect the necessary liquid distribution and is adopted to the ability of the fluid-absorbent core to quickly dewater the acquisition-distribution layer.

For fluid-absorbent articles that possess a very good dewatering that has excellent wicking capability it is advantageous to use acquisition-distribution layers. For fluid-absorbent articles that possess a fluid-absorbent core comprising very permeable water-absorbent polymer particles a small and thin acquisition-distribution layer can be used.

Suitable fluid-absorbent articles are composed of several layers whose individual elements must show preferably definite functional parameter such as dryness for the upper liquid-pervious layer, vapor permeability without wetting through for the lower liquid-impervious layer, a flexible, vapor permeable and thin fluid-absorbent core, showing fast absorption rates and being able to retain highest quantities of body fluids, and an acquisition-distribution layer between the upper layer and the core, acting as transport and distribution layer of the discharged body fluids. These individual elements are combined such that the resultant fluid-absorbent article meets overall criteria such as flexibility, water vapour breathability, dryness, wearing comfort and protection on the user facing side, and concerning liquid retention, rewet and prevention of wet through on the garment side. The specific combination of these layers provides a fluid-absorbent article delivering both high protection levels as well as high comfort to the consumer.

Designs for fluid-absorbent articles and methods to make them are for example described in the following publications and literature cited therein and are expressly incorporated into the present invention: EP 2 301 499 A1, EP 2 314 264 A1, EP 2 387 981 A1, EP 2 486 901 A1, EP 2 524 679 A1, EP 2 524 679 A1, EP 2 524 680 A1, EP 2 565 031 A1, US 6,972,011, US 2011/0162989, US 2011/0270204, WO 2010/004894 A1, WO 2010/004895 A1, WO 2010/076857 A1, WO 2010/082373 A1, WO 2010/118409 A1, WO 2010/133529 A2, WO 2010/143635 A1, WO 2011/084981 A1, WO 2011/086841 A1, WO 2011/086842 A1, WO 2011/086843 A1, WO 2011/086844 A1, WO 2011/117997 A1, WO 2011/136087 A1, WO 2012/048879 A1, WO 2012/052173 A1 und WO 2012/052172 A1.

Liquid-pervious Layer (A)

The liquid-pervious layer (A) is the layer which is in direct contact with the skin. Thus, the liquid-pervious layer is preferably compliant, soft feeling and non-irritating to the consumer's skin. Generally, the term "liquid-pervious" is understood thus permitting liquids, i.e. body fluids such as urine, menses and/or vaginal fluids to readily penetrate through its thickness. The principle function of the liquid-pervious layer is the acquisition and transport of body fluids from the wearer towards the fluid-absorbent core. Typically liquid-pervious layers are formed from any materials known in the art such as nonwoven material, films or combinations thereof. Suitable liquid-pervious layers (A) consist of customary synthetic or semisynthetic fibers or bicomponent fibers or films of polyester, polyolefins, rayon or natural fibers or any combinations thereof. In the case of nonwoven materials, the fibers should generally be bound by binders such as polyacrylates. Additionally the liquid-pervious layer may contain elastic compositions thus showing elastic characteristics allowing to be stretched in one or two directions.

Suitable synthetic fibers are made from polyvinyl chloride, polyvinyl fluoride, polytetrafluorethylene, polyvinylidene chloride, polyacrylics, polyvinyl acetate, polyethylvinyl acetate, non-soluble or soluble polyvinyl alcohol, polyolefins such as polyethylene, polypropylene, polyamides, polyesters, polyurethanes, polystyrenes and the like.

Examples for films are apertured formed thermoplastic films, apertured plastic films, hydro-formed thermoplastic films, reticulated thermoplastic films, porous foams, reticulated foams, and thermoplastic scrims.

Examples of suitable modified or unmodified natural fibers include cotton, bagasse, kemp, flax, silk, wool, wood pulp, chemically modified wood pulp, jute, rayon, ethyl cellulose, and cellulose acetate.

Suitable wood pulp fibers can be obtained by chemical processes such as the Kraft and sulfite processes, as well as from mechanical processes, such as ground wood, refiner mechanical, thermo-mechanical, chemi-mechanical and chemi-thermo-mechanical pulp processes. Further, recycled wood pulp fibers, bleached, unbleached, elementally chlorine free (ECF) or total chlorine free (TCF) wood pulp fibers can be used.

The fibrous material may comprise only natural fibers or synthetic fibers or any combination thereof. Preferred materials are polyester, rayon and blends thereof, polyethylene, and polypropylene.

The fibrous material as a component of the fluid-absorbent compositions may be hydrophilic, hydrophobic or can be a combination of both hydrophilic and hydrophobic fibers. The definition of hydrophilic is given in the section "definitions" in the chapter above. The selection of the ratio hydrophilic/hydrophobic and accordingly the amount of hydrophilic and hydrophobic fibers within fluid-absorbent composition will depend upon fluid handling properties and the amount of water-absorbent polymer particles of the resulting fluid-absorbent composition. Such, the use of hydrophobic fibers is preferred if the fluid-absorbent composition is adjacent to the wearer of the fluid-absorbent article, that is to be used to replace partially or completely the upper liquid-pervious layer, preferably formed from hydrophobic nonwoven materials. Hydrophobic fibers can also be member of the lower breathable, but fluid-impervious layer, acting there as a fluid-impervious barrier.

Examples for hydrophilic fibers are cellulosic fibers, modified cellulosic fibers, rayon, polyester fibers such as polyethylen terephthalate, hydrophilic nylon and the like. Hydrophilic fibers can also be obtained from hydrophobic fibers which are hydrophilized by e. g. surfactant-treating or silica-treating. Thus, hydrophilic thermoplastic fibers derived from polyolefins such as polypropylene, polyamides, polystyrenes or the like by surfactant-treating or silica-treating.

To increase the strength and the integrity of the upper-layer, the fibers should generally show bonding sites, which act as crosslinks between the fibers within the layer.

Technologies for consolidating fibers in a web are mechanical bonding, thermal bonding and chemical bonding. In the process of mechanical bonding the fibers are entangled mechanically, e.g., by water jets (spunlace) to give integrity to the web. Thermal bonding is carried out by means of raising the temperature in the presence of low-melting polymers. Examples for thermal bonding processes are spunbonding, through-air bonding and resin bonding.

Preferred means of increasing the integrity are thermal bonding, spunbonding, resin bonding, through-air bonding and/or spunlace.

In the case of thermal bonding, thermoplastic material is added to the fibers. Upon thermal treatment at least a portion of this thermoplastic material is melting and migrates to intersections of the fibers caused by capillary effects. These intersections solidify to bond sites after cooling and increase the integrity of the fibrous matrix. Moreover, in the case of chemically stiffened cellulosic fibers, melting and migration of the thermoplastic material has the effect of increasing the pore size of the resultant fibrous layer while maintaining its density and basis weight. Upon wetting, the structure and integrity of the layer remains stable. In summary, the addition of thermoplastic material leads to improved fluid permeability of discharged body fluids and thus to improved acquisition properties.

Suitable thermoplastic materials including polyolefins such as polyethylene and polypropylene, polyesters, copolyesters, polyvinyl acetate, polyethylvinyl acetate, polyvinyl chloride, polyvinylidene chloride, polyacrylics, polyamides, copolyamides, polystyrenes, polyurethanes and copolymers of any of the mentioned polymers.

Suitable thermoplastic fibers can be made from a single polymer that is a monocomponent fiber. Alternatively, they can be made from more than one polymer, e.g., bi-component or multicomponent fibers. The term "bicomponent fibers" refers to thermoplastic fibers that comprise a core fiber made from a different fiber material than the shell. Typically, both fiber materials have different melting points, wherein generally the sheath melts at lower temperatures. Bi-component fibers can be concentric or eccentric depending whether the sheath has a thickness that is even or uneven through the cross-sectional area of the bi-component fiber. Advantage is given for eccentric bi-component fibers showing a higher compressive strength at lower fiber thickness. Further bi-component fibers can show the feature "uncrimped" (unbent) or "crimped" (bent), further bi-component fibers can demonstrate differing aspects of surface lubricity.

Examples of bi-component fibers include the following polymer combinations: polyethylene/polypropylene, polyethylvinyl acetate/polypropylene, polyethylene/polyester, polypropylene/polyester, copolyester/polyester and the like.

Suitable thermoplastic materials have a melting point of lower temperatures that will damage the fibers of the layer; but not lower than temperatures, where usually the fluid-absorbent articles are stored. Preferably the melting point is between about 75°C and 175°C. The typical length of thermoplastic fibers is from about 0.4 to 6 cm, preferably from about 0.5 to 1 cm. The diameter of thermoplastic fibers is defined in terms of either denier (grams per 9000 meters) or dtex (grams per 10 000 meters). Typical thermoplastic fibers have a dtex in the range from about 1.2 to 20, preferably from about 1.4 to 10.

A further mean of increasing the integrity of the fluid-absorbent composition is the spunbonding technology. The nature of the production of fibrous layers by means of spunbonding is based on the direct spinning of polymeric granulates into continuous filaments and subsequently manufacturing the fibrous layer.

Spunbond fabrics are produced by depositing extruded, spun fibers onto a moving belt in a uniform random manner followed by thermal bonding the fibers. The fibers are separated during the web laying process by air jets. Fiber bonds are generated by applying heated rolls or hot needles to partially melt the polymer and fuse the fibers together. Since molecular orientation increases the melting point, fibers that are not highly drawn can be used as thermal binding fibers. Polyethylene or random ethylene/propylene copolymers are used as low melting bonding sites.

Besides spunbonding, the technology of resin bonding also belongs to thermal bonding subjects. Using this technology to generate bonding sites, specific adhesives, based on e.g. epoxy, polyurethane and acrylic are added to the fibrous material and the resulting matrix is thermically treated. Thus the web is bonded with resin and/or thermal plastic resins dispersed within the fibrous material.

As a further thermal bonding technology through-air bonding involves the application of hot air to the surface of the fibrous fabric. The hot air is circulated just above the fibrous fabric, but does not push through the fibrous fabric. Bonding sites are generated by the addition of binders. Suitable binders used in through-air thermal bonding include crystalline binder fibers, bi-component binder fibers, and powders. When using crystalline binder fibers or powders, the binder melts entirely and forms molten droplets throughout the nonwoven's cross-section. Bonding occurs at these points upon cooling. In the case of sheath/core binder fibers, the sheath is the binder and the core is the carrier fiber. Products manufactured using through-air ovens tend to be bulky, open, soft, strong, extensible, breathable and absorbent. Through-air bonding followed by immediate cold calendering results in a thickness between a hot roll calendered product and one that has been though-air bonded without compression. Even after cold calendering, this product is softer, more flexible and more extensible than area-bond hot-calendered material.

Spunlacing ("hydroentanglement") is a further method of increasing the integrity of a web. The formed web of loose fibers (usually air-laid or wet-laid) is first compacted and prewetted to eliminate air pockets. The technology of spunlacing uses multiple rows of fine high-speed jets of water to strike the web on a porous belt or moving perforated or patterned screen so that the fibers knot about one another. The water pressure generally increases from the first to the last injectors. Pressures as high as 150 bar are used to direct the water jets onto the web. This pressure is sufficient for most of the nonwoven fibers, although higher pressures are used in specialized applications.

The spunlace process is a nonwovens manufacturing system that employs jets of water to entangle fibers and thereby provide fabric integrity. Softness, drape, conformability, and relatively high strength are the major characteristics of spunlace nonwoven.

In newest researches benefits are found in some structural features of the resulting liquid-pervious layers. For example, the thickness of the layer is very important and influences together with its x-y dimension the acquisition-distribution behaviour of the layer. If there is further some profiled structure integrated, the acquisition-distribution behaviour can be directed depending on the three-dimensional structure of the layer. Thus 3D-polyethylene in the function of liquid-pervious layer is preferred.

Thus, suitable liquid-pervious layers (A) are nonwoven layers formed from the fibers above by thermal bonding, spunbonding, resin bonding or through-air bonding. Further suitable liquid-pervious layers are 3D-polyethylene layers and spunlace.

Preferably the 3D-polyethylene layers and spunlace show basis weights from 12 to 22 gsm.

Typically liquid- pervious layers (A) extend partially or wholly across the fluid-absorbent structure and can extend into and/or form part of all the preferred sideflaps, side wrapping elements, wings and ears.

Liquid-impervious Layer (B)

The liquid-impervious layer (B) prevents the exudates absorbed and retained by the fluid-absorbent core from wetting articles which are in contact with the fluid-absorbent article, as for example bedsheets, pants, pyjamas and undergarments. The liquid-impervious layer (B) may thus comprise a woven or a nonwoven material, polymeric films such as thermoplastic film of polyethylene or polypropylene, or composite materials such as film-coated nonwoven material.

Suitable liquid-impervious layers include nonwoven, plastics and/or laminates of plastic and nonwoven. Both, the plastics and/or laminates of plastic and nonwoven may appropriately be breathable, that is, the liquid-impervious layer (B) can permit vapors to escape from the fluid-absorbent material. Thus the liquid-impervious layer has to have a definite water vapor transmission rate and at the same time the level of impermeability. To combine these features, suitable liquid-impervious layers including at least two layers, e.g. laminates from fibrous nonwoven having a specified basis weight and pore size, and a continuous three-dimensional film of e.g. polyvinylalcohol as the second layer having a specified thickness and optionally having pore structure. Such laminates acting as a barrier and showing no liquid transport or wet through. Thus, suitable liquid-impervious layers comprising at least a first breathable layer of a porous web which is a fibrous nonwoven, e.g. a composite web of a meltblown nonwoven layer or of a spunbonded nonwoven layer made from synthetic fibers and at least a second layer of a resilient three dimensional web consisting of a liquid-impervious polymeric film, e.g. plastics optionally having pores acting as capillaries, which are preferably not perpendicular to the plane of the film but are disposed at an angle of less than 90° relative to the plane of the film.

Suitable liquid-impervious layers are permeable for vapor. Preferably the liquid-impervious layer is constructed from vapor permeable material showing a water vapor transmission rate (WVTR) of at least about 100 gsm per 24 hours, preferably at least about 250 gsm per 24 hours and most preferred at least about 500 gsm per 24 hours.

Preferably the liquid-impervious layer (B) is made of nonwoven comprising hydrophobic materials, e.g. synthetic fibers or a liquid-impervious polymeric film comprising plastics e.g. polyethylene. The thickness of the liquid-impervious layer is preferably 15 to 30 $\mu$m.

Further, the liquid-impervious layer (B) is preferably made of a laminate of nonwoven and plastics comprising a nonwoven having a density of 12 to 15 gsm and a polyethylene layer having a thickness of about 10 to 20 $\mu$m.

The typically liquid-impervious layer (B) extends partially or wholly across the fluid-absorbent structure and can extend into and/or form part of all the preferred sideflaps, side wrapping elements, wings and ears.

Fluid-absorbent Core (C)

The fluid-absorbent core (C) is disposed between the upper liquid-pervious layer (A) and the lower liquid-impervious layer (B). Suitable fluid-absorbent cores (C) may be selected from any of the fluid-absorbent core-systems known in the art provided that requirements such as vapor permeability, flexibility and thickness are met. Suitable fluid-absorbent cores refer to any fluid-absorbent composition whose primary function is to acquire, transport, distribute, absorb, store and retain discharged body fluids.

The top view area of the fluid-absorbent core (C) is preferably at least 200cm$^2$, more preferably at least 250 cm$^2$, most preferably at least 300cm$^2$. The top view area is the part of the core that is face-to-face to the upper liquid-pervious layer.

According to the present invention the fluid-absorbent core can include the following components:

1. an optional core cover
2. a fluid storage layer
3. an optional dusting layer

1. Optional Core Cover

In order to increase the integrity of the fluid-absorbent core, the core is provided with a cover. This cover may be at the top and/or at the bottom of the fluid-absorbent core with bonding at lateral juncture and/or bonding at the distal juncture by hot-melt, ultrasonic bonding, thermal bonding or combination of bonding techniques know to persons skilled in the art. Further, this cover may include the whole fluid-absorbent core with a unitary sheet of material and thus function as a wrap. Wrapping is possible as a full wrap, a partial wrap or as a C-Wrap.

The material of the core cover may comprise any known type of substrate, including nonwovens, webs, garments, textiles, films, tissues and laminates of two or more substrates or webs. The core cover material may comprise natural fibers, such as cellulose, cotton, flax, linen, hemp, wool, silk, fur, hair and naturally occurring mineral fibers. The core cover material may also comprise synthetic fibers such as rayon and lyocell (derived from cellulose), polysaccharides (starch), polyolefin fibers (polypropylene, polyethylene), polyamides, polyester, butadiene-styrene block copolymers, polyurethane and combinations thereof. Preferably, the core cover comprises synthetic fibers or tissue.

The fibers may be mono- or multicomponent. Multicomponent fibers may comprise a homopolymer, a copolymer or blends thereof.

2. Fluid-storage Layer

The fluid-absorbent compositions included in the fluid-absorbent core comprise fibrous materials and water-absorbent polymer particles.

Fibers useful in the present invention include natural fibers and synthetic fibers. Examples of suitable modified or unmodified natural fibers are given in the chapter "Liquid-pervious Layer (A)" above. From those, wood pulp fibers are preferred.

Examples of suitable synthetic fibers are given in the chapter "Liquid-pervious Layer (A)" above. The fibrous material may comprise only natural fibers or synthetic fibers or any combination thereof.

The fibrous material as a component of the fluid-absorbent compositions may be hydrophilic, hydrophobic or can be a combination of both hydrophilic and hydrophobic fibers.

Generally for the use in a fluid-absorbent core, which is the embedded between the upper layer (A) and the lower layer (B), hydrophilic fibers are preferred. This is especially the case for fluid-absorbent compositions that are desired to quickly acquire, transfer and distribute discharged body fluids to other regions of the fluid-absorbent composition or fluid-absorbent core. The use of hydrophilic fibers is especially preferred for fluid-absorbent compositions comprising water-absorbent polymer particles.

Examples for hydrophilic fibers are given in the chapter "Liquid-pervious Layer (A)" above. Preferably, the fluid-absorbent core is made from viscose acetate, polyester and/or polypropylene.

The fibrous material of the fluid-absorbent core may be uniformly mixed to generate a homogeneous or in-homogeneous fluid-absorbent core. Alternatively the fibrous material may be concentrated or laid in separate layers optionally comprising water-absorbent polymer material. Suitable storage layers of the fluid-absorbent core comprising homogeneous mixtures of fibrous materials comprising water-absorbent polymer material. Suitable storage layers of the fluid-absorbent core including a layered core-system comprise homogeneous mixtures of fibrous materials and comprise water-absorbent polymer material, whereby each of the layers may be built from any fibrous material by means known in the art. The sequence of the layers may be directed such that a desired fluid acquisition, distribution and transfer results, depending on the amount and distribution of the inserted fluid-absorbent material, e.g. water-absorbent polymer particles. Preferably there are discrete zones of highest absorption rate or retention within the storage layer of the fluid-absorbent core, formed of layers or in-homogeneous mixtures of the fibrous material, acting as a matrix for the incorporation of water-absorbent polymer particles. The zones may extend over the full area or may form only parts of the fluid-absorbent core.

Suitable fluid-absorbent cores comprise fibrous material and fluid-absorbent material. Suitable is any fluid-absorbent material that is capable of absorbing and retaining body fluids or body exudates such as cellulose wadding, modified and unmodified cellulose, crosslinked cellulose, laminates, composites, fluid-absorbent foams, materials described as in the chapter "Liquid-pervious Layer (A)" above, water-absorbent polymer particles and combinations thereof.

Typically the fluid-absorbent cores may contain a single type of water-absorbent polymer particles or may contain water-absorbent polymer particles derived from different kinds of water-absorbent polymer material. Thus, it is possible to add water-absorbent polymer particles from a single kind of polymer material or a mixture of water-absorbent polymer particles from different kinds of polymer materials, e.g. a mixture of regular water-absorbent polymer particles, derived from gel polymerization with water-absorbent polymer particles, derived from dropletization polymerization. Alternatively it is possible to add water-absorbent polymer particles derived from inverse suspension polymerization.

Alternatively it is possible to mix water-absorbent polymer particles showing different feature profiles. Thus, the fluid-absorbent core may contain water-absorbent polymer particles with uniform pH value, or it may contain water-absorbent polymer particles with different pH values, e.g. two- or more component mixtures from water-absorbent polymer particles with a pH in the range from about 4.0 to about 7.0. Preferably, applied mixtures deriving from mixtures of water-absorbent polymer particles got from gel polymerization or inverse suspension polymerization with a pH in the range from about 4.0 to about 7.0 and water-absorbent polymer particles got from drop polymerization.

Suitable fluid-absorbent cores are also manufactured from loose fibrous materials by adding water-absorbent particles and/or water-absorbent polymer fibers or mixtures thereof. The water-absorbent polymer fibers may be formed from a single type of water-absorbent polymer fiber or may contain water-absorbent polymer fibers from different polymeric materials. The addition of water-absorbent polymer fibers may be preferred for being distributed and incorporated easily into the fibrous structure and remaining better in place than water-absorbent polymer particles. Thus, the tendency of gel blocking caused by contacting each other is reduced. Further, water-absorbent polymer fibers are softer and more flexible.

In the process of manufacturing the fluid-absorbent core, water-absorbent polymer particles and/or fluid-absorbent fibers are brought together with structure forming compounds such as fibrous matrices. Thus, the water-absorbent polymer particles and/or fluid-absorbent fibers may be added during the process of forming the fluid-absorbent core from loose fibers. The fluid-absorbent core may be formed by mixing water-absorbent polymer particles and/or fluid-absorbent fibers with fibrous materials of the matrix at the same time or adding one component to the mixture of two or more other components either at the same time or by continuously adding.

Suitable fluid-absorbent cores including mixtures of water-absorbent polymer particles and/or fluid-absorbent fibers and fibrous material building matrices for the incorporation of the fluid-absorbent material. Such mixtures can be formed homogeneously, that is all components are mixed together to get a homogeneous structure. The amount of the fluid-absorbent materials may be uniform throughout the fluid-absorbent core, or may vary, e.g. between the central region and the distal region to give a profiled core concerning the concentration of fluid-absorbent material.

Techniques of application of the water-absorbent polymer materials into the absorbent core are known to persons skilled in the art and may be volumetric, loss-in-weight or gravimetric. Known techniques include the application by vibrating systems, single and multiple auger systems, dosing roll, weigh belt, fluid bed volumetric systems and gravitational sprinkle and/or spray systems. Further techniques of insertion are falling dosage systems consensus and contradictory pneumatic application or vacuum printing method of applying the fluid absorbent polymer materials.

Preferably drum-forming techniques are used where the fluid-absorbent core is formed in cavities of a drum rotating about a horizontal axis and being fed at a point on its periphery with a flow of water-absorbent polymer particles and/or fluid-absorbent fibers and fibrous material. The cylindrical surface of the drum on which the fluid-absorbent core is formed is surmounted by a hood, into which said flow is fed pneumatically from the top, bottom or tangentially. The inside of the hood may also contain the outlet of a feed duct, from which discrete quantities of additional water-absorbent polymer particles are dispensed by intermittently operating valve means under pressure. However, using prior art drum-forming techniques it is not possible to obtain uniform distribution of discrete quantities of water-absorbent polymer particles. Thus, in order to get profiled structures having different concentrations of water-absorbent polymer particles in discrete areas, it is preferred to use the technique written in WO 2010103453 in detail. Using this unit for the production of absorbent cores, by which a defined proportion of water-absorbent polymer particles are dispensed intermittently and controllably by adjustable elements controlling position and speed of application, it is possible to apply discrete quantities of water-absorbent polymer particles to a circumscribed area of precise geometrical shape.

Suitable fluid-absorbent cores may also include layers, which are formed by the process of manufacturing the fluid-absorbent article. The layered structure may be formed by subsequently generating the different layers in z-direction.

Alternatively a core-structure can be formed from two or more preformed layers to get a layered fluid-absorbent core. The layers may have different concentrations of water-absorbent polymer material showing concentrations in the range from about 10 to 95%. These uniform or different layers can be fixed to each other at their adjacent plane surfaces. Alternatively, the layers may be combined in a way that a plurality of chambers are formed, in which separately water-absorbent polymer material is incorporated.

Furthermore it can be preferred that the water-absorbent polymer particles are placed within the core in discrete regions even without chambers, e.g. supportet by at least an adhesive.

According to the invention is it preferred that the water-absorbent polymer particles are profiled in the fluid-absorbent core according to the equation (I):

$$(A_{total} - A_{profiled}) \times \text{Basisweight} + \text{Loading-Factor} \times A_{profiled} \times \text{Basisweight} = W_{SAP} \quad (I)$$

With $A_{total}$ = Area of the whole fluid-absorbent core (C) in $m^2$,
$A_{profiled}$ = Area with different loading $m^2$,
Basisweight = the basis weight of the fluid-absorbent core in $g/m^2$
L = Loading-Factor (e.g. 2 means double loading, 3 means threefold loading) and
$W_{SAP}$ = total content of water-absorbent polymer particles within the fluid-absorbent core.

The equation allows the distribution of a given amount of water-absorbent particles, to be distributed in the fluid-absorbent article according to special patterns, especially adapt the amount applied to the function of an area of the fluid-absorbent article. Therefore a given amount of water-absorbent particles can be applied more systematically. This results in a more use dependent distribution of the water-absorbent particles and overall reducing noise and increasing softness, as there is a use dependent load of the fluid-absorbent articles with water-absorbent particles.

Suitable preformed layers are processed as e.g. air-laid, wet-laid, laminate or composite structure.

Alternatively layers of other materials can be added, e.g. layers of opened or closed celled foams or perforated films. Included are also laminates of at least two layers comprising said water-absorbent polymer material.

Alternatively a core-structure can be formed from two or more layers, formed of e.g. nonwoven and/or thermoplastic materials containing water-absorbent polymer particles discretely contained in closed pockets. Such structures are preferably used for forming ultrathin absorbent products. The pockets are free of cellulose pulp. The bonds to define pockets are formed e.g. by intersection of ultrasonic contact areas between two thermoplastic containment layers. Further methods of immobilization of particulate fluid-absorbent material as well as the joining of layers in a layered structure are explained later on in more detail.

Alternatively a core-structure for ultrathin fluid-absorbent products can be formed from absorbent paper, e.g. a thin and flexible single layer of any suitable absorbent material known in the art including, but not limited to, short-fiber air-laid nonwoven materials; nonwoven of materials such as polyethylene, polypropylene, nylon, polyester, and the like; cellulosic fibrous materials such as paper tissue or towels known in the art, wax-coated papers, corrugated paper materials, and the like; or fluff pulp. The layer is macroscopically two-dimensional and planar and of very low thickness compared to the other dimensions. Said single layer may also incorporate superabsorbent material throughout the layer. Said single layer may further incorporate bi-component binding fibers. It may be also preferred to combine at least two of such layers in a core structure.

Water-absorbent polymer material may be incorporated as e.g. water-absorbent polymer fibers and/or water-absorbent polymer particles. Water-absorbent polymer particles may be bond to said single layer on one or both sides by attachment means known in the art.

Alternatively said absorbent paper may by formed from more layers, e.g. a layered absorbent sheet comprising a first layer on the wearer side, a second layer on the non-absorbing side and water-absorbent polymer particles in between or coated on one or both sides of the sheet layers.

The absorbent paper layer has a total basis weight ranging from about 100 gsm to about 2000 gsm, preferably from about 200 gsm to about 750 gsm, and more preferrably from about 400 gsm to about 600 gsm.

Further a composite structure can be formed from a carrier layer (e.g. a polymer film), onto which the water-absorbent polymer material is affixed. The fixation can be done at one side or at both sides. The carrier layer may be pervious or impervious for body-fluids.

Alternatively, it is possible to add monomer solution after the formation of a layer or onto a carrier layer and polymerize the coating solution by means of UV-induced polymerization technologies. Thus, "in situ"-polymerization is a further method for the application of water-absorbent polymers.

Thus, suitable fluid-absorbent cores comprising from 0 to 90% by weight a fibrous material and from 10 to 100% by weight water-absorbent polymer particles; preferably from 20 to 80% by weight a fibrous material and from 20 to 80% by weight water-absorbent polymer particles; more preferably from 30 to 75% by weight a fibrous material and from 25 to 70% by weight water-absorbent polymer particles and most preferably from 40 to 70% by weight a fibrous material and from 30 to 60% by weight water-absorbent polymer particles.

It is particularly preferred according to the present invention that the fluid-absorbent core of the inventive fluid-absorbent article comprises at least 12 % by weight of water-absorbent polymer particles, more preferred at least 50% by weight of water-absorbent polymer particles, most preferred at least 85% by weight of water-absorbent polymer particles.

It is preferred that the fluid-absorbent core comprises less than 50% by weight fibrous material, more preferred less than 15%, most preferred less than 5% by weight fibrous material.

According to the invention it is preferred that the fluid-absorbent core comprises not more than 10% by weight of an adhesive.

The quantity of water-absorbent polymer particles and/or fluid-absorbent fibers within the fluid-absorbent core is from 3 to 20 g, preferably from 6 to 14 g, and from 8 to 12 g in the case of maxi-diapers, and in the case of incontinence products up to about 50 g.

Typically fluid-absorbent articles comprising at least an upper liquid-pervious layer (A), at least a lower liquid-impervious layer (B) and at least one fluid-absorbent core between the layer (A) and the layer (B) besides other optional layers. In order to increase the control of body fluid absorption and/or to increase the flexibility in the ratio weight percentages of water-absorbent polymer particles to fibrous matrix it may be advantageous to add one or more further fluid-absorbent cores. The addition of a second fluid-absorbent core to the first fluid-absorbent core offers more possibilities in body fluid transfer and distribution. Moreover higher quantities of discharged body fluids can be retained. Having the opportunity of combining several layers showing different water-absorbent polymer concentration and content, it is possible to reduce the thickness of the fluid-absorbent article to a minimum even if there are several fluid-absorbent cores included.

Suitable fluid-absorbent cores may be formed from any material known in the art which is designed to acquire, transfer, and retain discharged body fluids. The technology of manufacturing may also be anyone known in the art. Preferred technologies include the application of monomer-solution to a transported fibrous matrix and thereby polymerizing, known as in-situ technology, or the manufacturing of air-laid composites.

Suitable fluid-absorbent articles are including single or multi-core systems in any combination with other layers which are typically found in fluid-absorbent articles. Preferred fluid-absorbent articles include single- or double-core systems; most preferably fluid-absorbent articles include a single fluid-absorbent core.

The fluid-absorbent core typically has a uniform size or profile. Suitable fluid-absorbent cores can also have profiled structures, concerning the shape of the core and/or the content of water-absorbent polymer particles and/or the distribution of the water-absorbent polymer particles and/or the dimensions of the different layers if a layered fluid-absorbent core is present.

The shape of the core in view from above (x-y dimension) can be rectangular, anatomical shaped with a narrower crotch area or any other shapes.

It is known that absorbent cores providing a good wet immobilization by combining several layers, e.g. a substrate layer, layers of water-absorbent polymer and layers of thermoplastic material. Suitable absorbent cores may also comprise tissue or tissue laminates. Known in the art are single or double layer tissue laminates formed by folding the tissue or the tissue laminate onto itself.

These layers or foldings are preferably joined to each e.g. by addition of adhesives or by mechanical, thermal or ultrasonic bonding or combinations thereof. Water-absorbent polymer particles may be comprised within or between the individual layers, e.g. by forming separate water-absorbent polymer-layers.

Thus, according to the number of layers or the height of a voluminous core, the resulting thickness of the fluid-absorbent core will be determined. Thus, fluid-absorbent cores may be flat as one layer (plateau) or have three-dimensional profile.

Generally the upper liquid-pervious layer (A) and the lower liquid-impervious layer (B) may be shaped and sized according to the requirements of the various types of fluid-absorbent articles and to accommodate various wearer's sizes. Thus, the combination of the upper liquid-pervious layer and the lower liquid-impervious layer may have all dimensions or shapes known in the art. Suitable combinations have an hourglass shape, rectangular shape, trapezoidal shape, t- or double t-shape or showing anatomical dimensions.

The fluid-absorbent core may comprise additional additives typically present in fluid-absorbent articles known in the art. Exemplary additives are fibers for reinforcing and stabilizing the fluid-absorbent core. Preferably polyethylene is used for reinforcing the fluid-absorbent core.

Further suitable stabilizer for reinforcing the fluid-absorbent core are materials acting as binder.

In varying the kind of binder material or the amount of binder used in different regions of the fluid-absorbent core it is possible to get a profiled stabilization. For example, different binder materials exhibiting different melting temperatures may be used in regions of the fluid-absorbent core, e.g. the lower melting one in the central region of the core, and the higher melting in the distal regions. Suitable binder materials may be adhesive or non-adhesive fibers, continuously or discontinuously extruded fibers, bi-component staple fibers, non-elastomeric fibers and sprayed liquid binder or any combination of these binder materials.

Further, thermoplastic compositions usually are added to increase the integrity of the core layer. Thermoplastic compositions may comprise a single type of thermoplastic polymers or a blend of thermoplastic polymers. Alternatively, the thermoplastic composition may comprise hot melt adhesives comprising at least one thermoplastic polymer together with thermoplastic diluents such as tackifiers, plasticizers or other additives, e.g. antioxidants. The thermoplastic composition may further comprise pressure sensitive hot melt adhesives comprising e.g. crystalline polypropylene and an amorphous polyalphaolefin or styrene block copolymer and mixture of waxes.

Suitable thermoplastic polymers are styrenic block copolymers including A-B-A triblock segments, A-B diblock segments and (A-B)$_n$ radial block copolymer segments. The letter A designs non-elastomeric polymer segments, e.g. polystyrene, and B stands for unsaturated conjugated diene or their (partly) hydrogenated form. Preferably B comprises isoprene, butadiene, ethylene/butylene (hydrogenated butadiene), ethylene/propylene (hydrogenated isoprene) and mixtures thereof.

Other suitable thermoplastic polymers are amorphous polyolefins, amorphous polyalphaolefins and metallocene polyolefins.

Concerning odor control, perfumes and/or odor control additives are optionally added. Suitable odor control additives are all substances of reducing odor developed in carrying fluid-absorbent articles over time known in the art. Thus, suitable odor control additives are inorganic materials, such as zeolites, activated carbon, bentonite, silica, aerosile, kieselguhr, clay; chelants such as ethylenediamine tetraacetic acid (EDTA), cyclodextrins, aminopolycarbonic acids, ethylenediamine tetramethylene phosphonic acid, aminophosphate, polyfunctional aromates, N,N-disuccinic acid.

Suitable odor control additives are further antimicrobial agents such as quaternary ammonium, phenolic, amide and nitro compounds and mixtures thereof; bactericides such as silver salts, zinc salts, cetylpyridinium chloride and/or triclosan as well as surfactants having an HLB value of less than 12.

Suitable odor control additives are further compounds with anhydride groups such as maleic-, itaconic-, polymaleic- or polyitaconic anhydride, copolymers of maleic acid with $C_2$-$C_8$ olefins or styrene, polymaleic anhydride or copolymers of maleic anhydride with isobutene, di-isobutene or styrene, compounds with acid groups such as ascorbic, benzoic, citric, salicylic or sorbic acid and fluid-soluble polymers of monomers with acid groups, homo- or copolymers of $C_3$-$C_5$ mono-unsaturated carboxylic acids.

Suitable odor control additives are further perfumes such as allyl caproate, allyl cyclohex-aneacetate, allyl cyclohexanepropionate, allyl heptanoate, amyl acetate, amyl propionate, anethol, anixic aldehyde, anisole, benzaldehyde, benzyl acetete, benzyl acetone, benzyl alcohole, benzyl butyrate, benzyl formate, camphene, camphor gum, laevo-carveol, cinnamyl formate, cis-jasmone, citral, citronellol and its derivatives, cuminic alcohol and its derivatives, cyclal C, dimethyl benzyl carbinol and its derivatives, dimethyl octanol and its derivatives, eucalyptol, geranyl derivatives, lavandulyl acetete, ligustral, d-limonene, linalo-ol, linalyl derivatives, menthone and its derivatives, myrcene and its derivatives, neral, nerol, p-cresol, p-cymene, orange terpenes, alpha-ponene, 4-terpineol, thymol etc.

Masking agents are also used as odor control additives. Masking agents are in solid wall material encapsulated perfumes. Preferably, the wall material comprises a fluid-soluble cellular matrix which is used for time-delay release of the perfume ingredient.

Further suitable odor control additives are transition metals such as Cu, Ag, Zn; enzymes such as urease-inhibitors, starch, pH buffering material, chitin, green tea plant extracts, ion exchange resin, carbonate, bicarbonate, phosphate, sulfate or mixtures thereof.

Preferred odor control additives are green tea plant extracts, tannin, silica, zeolite, carbon, starch, chelating agent, pH buffering material, chitin, kieselguhr, clay, ion exchange resin, carbonate, bicarbonate, phosphate, sulfate, masking agent or mixtures thereof. Suitable concentrations of odor control additives are from about 0.5 to about 300 gsm.

Newest developments propose the addition of wetness indication additives. Besides electrical monitoring the wetness in the fluid-absorbent article, wetness indication additives comprising a hot melt adhesive with a wetness indicator are known. The wetness indication additive changes the colour from yellow to a relatively dark and deep blue. This colour change is readily perceivable through the liquid-impervious outer material of the fluid-absorbent article. Existing wetness indication is also achieved via application of water soluble ink patterned on the backsheet which disappears when wet.

Suitable wetness indication additives comprising a mixture of sorbitan monooleate and polyethoxylated hydrogenated castor oil. Preferably, the amount of the wetness indication additive is in the range of about 0.0001 to 2 % by weight related to the weight of the fluid-absorbent core.

The basis weight of the fluid-absorbent core is in the range of 600 to 1200 gsm, preferably 500 to 1200 gsm. The density of the fluid-absorbent core is in the range of 0.1 to 0.25 $g/cm^3$, preferably 0.1 to 0.28 $g/cm^3$. The thickness of the fluid-absorbent core is in the case of diapers in the range of 1 to 8 mm, preferably 1 to 5 mm, more preferably 1.5 to 3 mm, in the case of incontinence products in the range of 3 to 15 mm.

3. Optional Dusting Layer

An optional component for inclusion into the absorbent core is a dusting layer adjacent to the liquid-impervious backsheet. The dusting layer is a fibrous layer and may be placed on the top and/or the bottom of the absorbent core. Typically, the dusting layer is underlying the storage layer. This underlying layer is referred to as a dusting layer, since it serves as carrier for deposited water-absorbent polymer particles during the manufacturing process of the fluid-absorbent core. If the water-absorbent polymer material is in the form of macro-structures, films or flakes, the insertion of a dusting layer is not necessary. In the case of water-absorbent polymer particles derived from dropletization polymerization, the particles have a smooth surface with no edges. Also in this case, the addition of a dusting layer to the fluid-absorbent core is not necessary. On the other side, as a great advantage the dusting layer provides some additional fluid-handling properties such as wicking performance and may offer reduced incidence of pin-holing and or pock marking of the liquid impervious layer (B).

Preferably, the dusting layer is a fibrous layer comprising fluff (cellulose fibers).

Optional Acquisition-distribution Layer (D)

An optional acquisition-distribution layer (D) is located between the upper layer (A) and the fluid-absorbent core (C) and is preferably constructed to efficiently acquire discharged body fluids and to transfer and distribute them to other regions of the fluid-absorbent composition or to other layers, where the body fluids are immobilized and stored. Thus, the upper layer transfers the discharged liquid to the acquisition-distribution layer (D) for distributing it to the fluid-absorbent core.

The acquisition-distribution layer comprises fibrous material and optionally water-absorbent polymer particles.

The fibrous material may be hydrophilic, hydrophobic or can be a combination of both hydrophilic and hydrophobic fibers. It may be derived from natural fibers, synthetic fibers or a combination of both.

Suitable acquisition-distribution layers are formed from cellulosic fibers and/or modified cellulosic fibers and/or synthetics or combinations thereof. Thus, suitable acquisition-distribution layers may contain cellulosic fibers, in particular wood pulp fluff. Examples of further suitable hydrophilic, hydrophobic fibers, as well as modified or unmodified natural fibers are given in the chapter "Liquid-pervious Layer (A)" above.

Especially for providing both fluid acquisition and distribution properties, the use of modified cellulosic fibers is preferred. Examples for modified cellulosic fibers are chemically treated cellulosic fibers, especially chemically stiffened cellulosic fibers. The term "chemically stiffened cellulosic fibers" means cellulosic fibers that have been stiffened by chemical means to increase the stiffness of the fibers. Such means include the addition of chemical stiffening agent in the form of surface coatings, surface cross-linking and impregnates. Suitable polymeric stiffening agents can include: cationic modified starches having nitrogen-containing groups, latexes, wet strength resins such as polyamide-epichlorohydrin resin, polyacrylamide, urea formaldehyde and melamine formaldehyde resins and polyethylenimine resins.

Stiffening may also include altering the chemical structure, e.g. by crosslinking polymer chains. Thus crosslinking agents can be applied to the fibers that are caused to chemically form intrafiber crosslink bonds. Further cellulosic fibers may be stiffened by crosslink bonds in individualized form. Suitable chemical stiffening agents are typically monomeric crosslinking agents including $C_2$-$C_8$ dialdehyde, $C_2$-$C_8$ monoaldehyde having an acid functionality, and especially $C_2$-$C_9$ polycarboxylic acids.

Preferably the modified cellulosic fibers are chemically treated cellulosic fibers. Especially preferred are curly fibers which can be obtained by treating cellulosic fibers with citric acid. Preferebly the basis weight of cellulosic fibers and modified cellulosic fibers is from 50 to 200 gsm.

Suitable acquisition-distribution layers further include synthetic fibers. Known examples of synthetic fibers are found in the Chapter "Liquid-pervious Layer (A)" above. Another possibility available is 3D-polyethylene film with dual function as a liquid-pervious layer (A) and acquisition-distribution layer.

Further, as in the case of cellulosic fibers, hydrophilic synthetic fibers are preferred. Hydrophilic synthetic fibers may be obtained by chemical modification of hydrophobic fibers. Preferably, hydrophilization is carried out by surfactant treatment of hydrophobic fibers. Thus the surface of the hydrophobic fiber can be rendered hydrophilic by treatment with a nonionic or ionic surfactant, e.g., by spraying the fiber with a surfactant or by dipping the fiber into a surfactant. Further preferred are permanent hydrophilic synthetic fibers.

The fibrous material of the acquisition-distribution layer may be fixed to increase the strength and the integrity of the layer. Technologies for consolidating fibers in a web are mechanical bonding, thermal bonding and chemical bonding. Detailed description of the different methods of increasing the integrity of the web is given in the Chapter "Liquid-pervious Layer (A)" above.

Preferred acquisition-distribution layers comprise fibrous material and water-absorbent polymer particles distributed within. The water-absorbent polymer particles may be added during the process of forming the layer from loose fibers, or, alternatively, it is possible to add monomer solution after the formation of the layer and polymerize the coating solution by means of UV-induced polymerisation technologies. Thus, "in situ"-polymerisation is a further method for the application of water-absorbent polymers.

Thus, suitable acquisition-distribution layers comprising from 80 to 100% by weight a fibrous material and from 0 to 20% by weight water-absorbent polymer particles; preferably from 85 to 99.9% by weight a fibrous material and from 0.1 to 15% by weight water-absorbent polymer particles; more preferably from 90 to 99.5% by weight a fibrous material and from 0.5 to 10% by weight water-absorbent polymer particles; and most preferably from 95 to 99% by weight a fibrous material and from 1 to 5% by weight water-absorbent polymer particles.

Preferred acquisition-distribution layers show basis weights in the range from 20 to 200 gsm, most preferred in the range from 40 to 60 gsm, depending on the concentration of water-absorbent polymer particles.

Alternatively a liquid-impervious layer (D) comprising a synthetic resin film between (A) and (C) acting as an distribution layer and quickly transporting the supplied urine along the surface to the upper lateral portion of the fluid-absorbent core (C). Preferably, the upper liquid-impervious layer (D) is smaller than the under-laying fluid-absorbent core (C). There is no limit in particular to the material of the liquid-impervious layer (D). Such a film made of a resin such as polyethylene, polypropylene, polyethylene therephthalate, polyurethane, or crosslinked polyvinyl alcohol and an air-permeable, but liquid-impervious, so-called: "breathable" film made of above described resin, may be used.

Preferably, the upper liquid-impervious layer (D) comprises a porous polyethylene film for both quickl acquisition and distribution of fluid.

Alternatively a bundle of synthetic fibers acting as acquisition-distribution layer loosely distributed on top of the fluid-absorbent core may be used. Suitable synthetic fibers are of copolyester, polyamide, copolyamide, polylactic acid, polypropylene or polyethylene, viscose or blends thereof. Further bicomponent fibers can be used. The synthetic fiber component may be composed of either a single fiber type with a circular cross-section or a blend of two fibre types with different cross- sectional shapes. Synthetic fibers arranged in that way ensuring a very fast liquid transport and canalisation. Preferrably bundles of polyethylene fibers are used.

Optional Tissue Layer (E)

An optional tissue layer is disposed immediately above and/or below (C).

The term tissue is not restricted to tissue material such as paper it also refers to nonwovens. The material of the layer may comprise any known type of substrate, including webs, garments, textiles and films. The tissuelayer may comprise natural fibers, such as cellulose, cotton, flax, linen, hemp, wool, silk, fur, hair and naturally occurring mineral fibers. The tissue layer may also comprise synthetic fibers such as rayon and lyocell (derived from cellulose), polysaccharides (starch), polyolefin fibers (polypropylene, polyethylene), polyamides, polyester, butadiene-styrene block copolymers, polyurethane and combinations thereof. Preferably, the tissue layer comprises cellulose fibers.

Other Optional Components (F)

1. Leg Cuff

Typical leg cuffs comprising nonwoven materials which can be formed by direct extrusion processes during which the fibers and the nonwoven materials are formed at the same time, or by laying processes of preformed fibers which can be laid into nonwoven materials at a later point of time. Examples for direct extrusion processes include spunbonding, melt-blowing, solvent spinning, electrospinning and combinations thereof. Examples of laying processes include wet-laying and dry-laying (e.g. air-laying, carding) methods. Combinations of the processes above include spunbond-meltblown-spunbond (sms), spunbond-meltblow-meltblown-spunbond (smms), spunbond-carded (sc), spunbond-airlaid (sa), meltblown-airlaid (ma) and combinations thereof. The combinations including direct extrusion can be combined at the same point in time or at a subsequent point in time. In the examples above, one or more individual layers can be produced by each process. Thus, "sms" means a three layer nonwoven material, "smsms" or "ssmms" means a five layer nonwoven material. Usually, small type letters (sms) designate individual layers, whereas capital letters (SMS) designate the compilation of similar adjacent layers.

Further, suitable leg cuffs are provided with elastic strands.

Preferred are leg cuffs from synthetic fibers showing the layer combinations sms, smms or smsms. Preferred are nonwovens with the density of 13 to 17 gsm. Preferably leg cuffs are provided with two elastic strands.

2. Elastics

The elastics are used for securely holding and flexibly closing the fluid-absorbent article around the wearers' body, e.g. the waist and the legs to improve containment and fit. Leg elastics are placed between the outer and inner layers or the fluid-absorbent article, or between the outer garment facing cover and the user facing bodyside liner. Suitable elastics comprising sheets, ribbons or strands of thermoplastic polyurethane, elastomeric materials, poly(ether-amide) block copolymers, thermoplastic rubbers, styrene-butadiene copolymers, silicon rubbers, natural rubbers, synthetic rubbers, styrene isoprene copolymers, styrene ethylene butylene copolymers, nylon copolymers, spandex fibers comprising segmented polyurethane and/or ethylene-vinyl acetate copolymer. The elastics may be secured to a substrate after being stretched, or secured to a stretched substrate. Otherwise, the elastics may be secured to a substrate and then elastisized or shrunk, e.g. by the application of heat.

3. Closing System

The closing system can include tape tabs, landing zone, elastomerics, pull ups and the belt system or combinations thereof

At least a part of the first waist region is attached to a part of the second waist region by the closing system to hold the fluid-absorbent article in place and to form leg openings and the waist of the fluid-absorbent article. Preferably the fluid-absorbent article is provided with a re-closable closing system.

The closing system is either re-sealable or permanent, including any material suitable for such a use, e.g. plastics, elastics, films, foams, nonwoven substrates, woven substrates, paper, tissue, laminates, fiber reinforced plastics and the like, or combinations therof. Preferably the closing system includes flexible materials and works smooth and softly without irritating the wearer's skin.

One part of the closing elements is an adhesive tape, or comprises a pair of laterally extending tabs disposed on the lateral edges of the first waist region. Tape tabs are typically attached to the front body panel and extend laterally from each corner of the first waistband. These tape tabs include an adhesive inwardly facing surface which is typically protected prior to use by a thin, removable cover sheet.

Suitable tape tabs may be formed of thermoplastic polymers such as polyethylene, polyurethane, polystyrene, polycarbonate, polyester, ethylene vinyl acetate, ethylene vinyl alcohol, ethylene vinyl acetate acrylate or ethylene acrylic acid copolymers.

Suitable closing systems comprise further a hook portion of a hook and loop fastener and the target devices comprise the loop portion of a hook and loop fastener.

Suitable mechanical closing systems including a landing zone. Mechanical closing systems may fasten directly into the outer cover. The landing zone may act as an area of the fluid-absorbent article into which it is desirable to engage the tape tabs. The landing zone may include a base material and a plurality of tape tabs. The tape tabs may be embedded in the base material of the landing zone. The base material may include a loop material. The loop material may include a backing material and a layer of a nonwoven spunbond web attached to the backing material.

Thus suitable landing zones can be made by spunbonding. Spunbonded nonwoven are made from melt-spun fibers formed by extruding molten thermoplastic material. Preferred is bi-oriented polypropylene (BOPP), or brushed/closed loop in the case of mechanical closing systems.

Further, suitable mechanical closing systems including elasticomeric units serving as a flexible abdominal and/or dorsal discrete waist band, flexible abdomen and/or dorsal zones located at distal edge for fluid-absorbents articles, such as pants or pull-ups. The elastico-meric units enable the fluid-absorbent article to be pulled down by the wearer as e.g. a training pant.

Suitable pants-shaped fluid-absorbent article has front abdominal section, rear dorsal section, crotch section, side sections for connecting the front and rear sections in lateral direction, hip section, elastic waist region and liquid-tight outer layer. The hip section is arranged around the waist of the user. The disposable pants-shaped fluid-absorbent article (pull-up) has favorable flexibility, stretchability, leak-proof property and fit property, hence imparts excellent comfort to the wearer and offers improved mobility and discretion.

Suitable pull-ups comprising thermoplastic films, sheets and laminates having a low modulus, good tear strength and high elastic recovery.

Suitable closing systems may further comprise elastomerics for the production of elastic areas within the fastening devices of the fluid-absorbent article. Elastomerics provide a conformable fit of the fluid-absorbent article to the wearer at the waist and leg openings, while maintaining adequate performance against leakage.

Suitable elastomerics are elastomeric polymers or elastic adhesive materials showing vapor permeability and liquid barrier properties. Preferred elastomerics are retractable after elongation to a length equivalent to its original length.

Suitable closing systems further comprise a belt system, comprising waist-belt and leg-belts for flexibly securing the fluid-absorbent article on the body of the wearer and to provide an improved fit on the wearer. Suitable waist-belts comprising two elastic belts, a left elastic belt, and a right elastic belt. The left elastic belt is associated with each of the left angular edges. The right elastic belt associated with each of the right angular edges. The left and right side belts are elastically extended when the absorbent garment is laid flat. Each belt is connected to and extends between the front and rear of the fluid-absorbent article to form a waist hole and leg holes.

Preferably the belt system is made of elastomerics, thus providing a conformable fit of the fluid-absorbent article and maintaining adequate performance against leakage.

Preferred closing systems are so-called "elastic ears" attached with one side of the ear to the longitudinal side edges located at the rear dorsal longitudinal edge of the chassis of the fluid-absorbent article. Commercially available fluid-absorbent articles include stretchable ears or side panels which are made from a stretchable laminate e.g. nonwoven webs made of mono- or bi-component fibers. Especially preferred closing systems are stretchable laminates comprising a core of several layers each of different fibrous materials, e.g. meltblown fibers, spunbond fibers, containing multicomponent fibers having a core comprising a first polymer having a first melt temperature and a sheath comprising a second polymer having a second melt temperature; and a web of an elastomeric material as top and bottom surfaces to form said laminate.

D. Fluid-absorbent Article Construction

The present invention further relates to the joining of the components and layers, films, sheets, tissues or substrates mentioned above to provide the fluid-absorbent article. At least two, preferably all layers, films, sheets, tissues or substrates are joined.

Suitable fluid-absorbent articles include a single- or multiple fluid-absorbent core-system. Preferably fluid-absorbent articles include a single- or double fluid-absorbent core-system.

Suitable fluid-storage layers of the fluid-absorbent core comprising homogeneous or inhomogeneous mixtures of fibrous materials comprising water-absorbent polymer particles homogeneously or in-homogeneously dispersed in it. Suitable fluid-storage layers of the fluid-absorbent core including a layered fluid-absorbent core-system comprising homogeneous mixtures of fibrous materials and optionally comprising water-absorbent polymer particles, whereby each of the layers may be prepared from any fibrous material by means known in the art.

In order to immobilize the water-absorbent polymer particles, the adjacent layers are fixed by the means of thermoplastic materials, thereby building connections throughout the whole surface or alternatively in discrete areas of junction. For the latter case, cavities or pockets are built carrying the water-absorbent particles. The areas of junction may have a regular or irregular pattern, e.g. aligned with the longitudinal axis of the fluid-absorbent core or in a pattern of polygons, e.g. pentagons or hexagons. The areas of junction itself may be of rectangular, circular or squared shape with diameters between about 0.5 mm and 2 mm. Fluid-absorbent articles comprising areas of junction show a better wet strength.

The construction of the products chassis and the components contained therein is made and controlled by the discrete application of hotmelt adhesives as known to people skilled in the art. Examples would be e.g. Dispomelt 505B, Dispomelt Cool 1101, as well as other specific function adhesives manufactured by Bostik, Henkel or Fuller.

In order to ensure wicking of applied body fluids, preferred fluid-absorbent article show channels for better transport. Channels are formed by compressional forces of e.g. the top sheet against the fluid-absorbent core. Compressive forces may be applied e.g. by heat-treatment between two heated calendar rollers. As an effect of compression both on top sheet and fluid-absorbent core deform such that a channel is created. Body fluids are flowing along this channel to places where they are absorbed and leakage is prevented. Otherwise, compression leads to higher density; this is the second effect of the channel to canalize insulted fluids. Additionally, compressive forces on diaper construction improve the structural integrity of the fluid-absorbent article.

In order to describe the present invention in detail, embodiments are generated which are described hereinafter.

Thus, preferred fluid-absorbent articles are subsequently described in detail.

Embodiment 1

One preferred embodiment of the present invention is described in Embodiment 1 hereinafter. Thus, a preferred fluid-absorbent article comprising

(A) an upper liquid-pervious layer comprising a spunbond layer (three piece coverstock);
(B) a lower liquid-impervious layer comprising a composite of breathable polyethylene film and spunbond nonwoven;
(C) a single fluid-absorbent core between (A) and (B) comprising between 10 to 50 % by weight water-absorbent polymer particles based on the total absorbent core weight and including a multi-layered fluid-storage section comprising the following sequence:

1. a homogeneous upper layer of hydrophilic fibrous matrix of wood pulp fibers (cellulose fibers) containing about 50% of the total fluff amount;
2. a fluid-absorbent layer comprising water-absorbent polymer particles; suitable water-absorbent polymer particles for such construction having a centrifuge retention capacity (CRC) from about 32 to 60 g/g;
3. a homogeneous lower layer of hydrophilic fibrous matrix of wood pulp fibers (cellulose fibers) containing about 50% of the total fluff amount and acting as a dusting layer; and

(D) an air-through bonded acquisition-distribution layer between (A) and (C) having a basis weight of 30 to 80 gsm; the acquisition-distribution layer is rectangular shaped and smaller than the fluid-absorbent core having a size of about 150 to about 250 cm$^2$.

The construction of the products chassis and the components contained therein is made and controlled by the discrete application of hotmelt adhesives as known to people skilled in the art. Examples would be e.g. Dispomelt 505B, Dispomelt Cool 1101, as well as other specific function adhesives manufactured by Bostik, Henkel or Fuller.

Ultra-thin high-loaded fluid-absorbent layers can be formed by immobilization of water-absorbent polymer particles on a non-woven sheet using hotmelt adhesives. Preferably the water-absorbent polymer particles form longitudinal strips or discrete spots. Other patterns of the water-absorbent polymer particles are also possible.

In a preferred embodiment the ultra-thin high-loaded fluid-absorbent layers comprise at least two sheets comprising immobilized water-absorbent polymer particles.

Examples of ultra-thin high-loaded fluid-absorbent layers are described in EP 1 293 187 A1, US 6,972,011, EP 1 447 066 A1, EP 1 447 067 A1, EP 1 609 448 A1, JP 2004/313580, US 2005/0137085, US 2006/0004336, US 2007/0135785 WO 2008/155699 A1, WO 2008/155701 A2, WO 2008/155702 A1, WO 2008/155710 A1, WO 2008/155711 A1, WO 2004/071363 A1, US 2003/0181115, WO 2005/097025, US 2007/156108, US 2008/0125735, and WO 2008/155722 A2, which explicitly forms part of the present disclosure.

First Construction Example of Embodiment 1

The fluid-absorbent core consists of a multi-layered single core system each layer having a uniform rectangular size. The layered fluid-absorbent core between (A) and (B) comprises a multi-layered system of hydrophilic fibers (cellulose fibers, fluff pulp fibers). The total fluff pulp weight is 20.45 g divided equally between upper core (1) and lower core (3). The density of the fluid-absorbent core is for the front overall average 0.18 g/cm$^3$, for the insult zone 0.17 g/cm$^3$, for the back overall average 0.15 g/cm$^3$. The basis weight of the fluid-absorbent core is for the front overall average 802.75 gsm, for the insult zone 825.94 gsm, for the back overall average 766.14 gsm.

Fluid-absorbent layer (2) holds 31.38 % by weight distributed water-absorbent polymer particles, the quantity of water-absorbent polymer particles within the fluid-absorbent core is 9.34 g.

The water-absorbent polymer particles, for example derived from dropletization polymerization as described in production example 11, exhibiting the following features and absorption profile:

CRC of 41 g/g
SFC of less than 5 x 10$^{-7}$cm$^3$s/g
AUHL of 21 g/g
AUL of 33 g/g
Extractables of 4 wt.%
Residual monomers of 282 ppm
Moisture content of 5,1 wt.%
FSR of 0.39 g/gs
PSD of 200 to 600 $\mu$m

Anticaking of 3

The water-absorbent polymer particles can be re-moisturized to a moisture content of 13% by weight.

Dimension of the fluid-absorbent core: length: 37.5 cm; width: 10.0 cm.

An air through bonded acquisition-distribution layer between (A) and (C) having a basis weight of 50 gsm is rectangular shaped with dimensions of 20 cm x 10 cm and smaller than the fluid-absorbent core.

The fluid-absorbent article further comprises:

- flat rubber elastics; elastics from spandex type fibers: 3 leg elastics and 1 cuff elastics
- leg cuffs from synthetic fibers showing the layer combination SMS and having a basis weight of between 13 to 17 gsm and a height of 4.6 cm
- mechanical closure system with landing zone of dimension 18.3 cm x 4.0 cm and flexiband closure tapes of 3.4 cm x 1.0 cm; attached to hook fastening tape of 3.4 cm x 1.4 cm
- Also incorporated is elasticated waistband located to the rear of the product with dimensions of 14.6 cm x 4.5 cm

Dimension of the fluid-absorbent article: length: 49.6 cm; front width: 34.0 cm; crotch width: 24.0 cm; rear width: 34.3 cm.

Second Construction Example of Embodiment 1

The fluid-absorbent core consists of a multi-layered single core system each layer having a uniform rectangular size. The layered fluid-absorbent core between (A) and (B) comprises a multi-layered system of hydrophilic fibers (cellulose fibers, fluff pulp fibers). The total fluff pulp weight is 20.45 g divided equally between upper core (1) and lower core (3). The density of the fluid-absorbent core is for the front overall average 0.18 g/cm$^3$, for the insult zone 0.17 g/cm$^3$, for the back overall average 0.15 g/cm$^3$. The basis weight of the fluid-absorbent core is for the front overall average 802.75 gsm, for the insult zone 825.94 gsm, for the back overall average 766.14 gsm.

Fluid-absorbent layer (2) holds 31.38 % by weight distributed water-absorbent polymer particles, the quantity of water-absorbent polymer particles within the fluid-absorbent core is 9.34 g.

The water-absorbent polymer particles, for example derived from dropletization polymerization as described herein, exhibiting the following features and absorption profile:

CRC of 47.0 g/g
SFC of less than 10 x 10$^{-7}$cm$^3$s/g
AUHL of 26 g/g
AUL of 35 g/g
Extractables of 4 wt.%
Residual monomers of 350 ppm
Moisture content of 1 wt.%
FSR of 0.52 g/gs
Mean Sphericity of 0.89
PSD of 200 to 600 $\mu$m
Anticaking of 3 -

The water-absorbent polymer particles can be re-moisturized to a moisture content of 13% by weight.

Dimension of the fluid-absorbent core: length: 37.5 cm; width: 10.0 cm.

An air through bonded acquisition-distribution layer between (A) and (C) having a basis weight of 50 gsm is rectangular shaped with dimensions of 20 cm x 10 cm and smaller than the fluid-absorbent core.

The fluid-absorbent article further comprises:

- flat rubber elastics; elastics from spandex type fibers: 3 leg elastics and 1 cuff elastics
- leg cuffs from synthetic fibers showing the layer combination SMS and having a basis weight of between 13 to 17 gsm and a height of 4.6 cm
- mechanical closure system with landing zone of dimension 18.3 cm x 4.0 cm and flexiband closure tapes of 3.4 cm x 1.0 cm; attached to hook fastening tape of 3.4 cm x 1.4 cm
- Also incorporated is elasticated waistband located to the rear of the product with dimensions of 14.6 cm x 4.5 cm

Dimension of the fluid-absorbent article: length: 49.6 cm; front width: 34.0 cm; crotch width: 24.0 cm; rear width: 34.3 cm.

Embodiment 2

A further preferred embodiment of the present invention is described in Embodiment 2 hereinafter. Thus, a preferred fluid-absorbent article comprising

(A) an upper liquid-pervious layer comprising a thermalbond layer (three piece coverstock);
(B) a lower liquid-impervious layer comprising a composite of breathable polyethylene film and spunbond nonwoven;
(C) a single fluid-absorbent core between (A) and (B) comprising between 40 to 80 % by weight water-absorbent polymer particles based on the total absorbent core weight and including a multi-layered fluid-storage section comprising the following sequence:

1. a homogeneous upper layer of hydrophilic fibrous matrix of wood pulp fibers (cellulose fibers) containing about 50% of the total fluff amount;
2. a fluid-absorbent layer comprising water-absorbent polymer particles; suitable water-absorbent polymer particles for such construction having a saline flow conductivity (SFC) from about 35 to 100 x $10^{-7}cm^3s/g$;
3. a homogeneous lower layer of hydrophilic fibrous matrix of wood pulp fibers (cellulose fibers) containing about 50% of the total fluff amount and acting as a dusting layer; and

(D) an air-through bonded acquisition-distribution layer between (A) and (C) having a basis weight of 40 to 80 gsm; the acquisition-distribution layer is rectangular shaped and smaller than the fluid-absorbent core having a size of about 150 to about 250 cm$^2$.

The construction of the products chassis and the components contained therein is made and controlled by the discrete application of hotmelt adhesives as known to people skilled in the art. Examples would be e.g. Dispomelt 505B, Dispomelt Cool 1101, as well as other specific function adhesives manufactured by Bostik, Henkel or Fuller.

Ultra-thin high-loaded fluid-absorbent layers can be formed by immobilization of water-absorbent polymer particles on a non-woven sheet using hotmelt adhesives. Preferably the water-absorbent polymer particles form longitudinal strips or discrete spots. Other patterns of the water-absorbent polymer particles are also possible.

In a preferred embodiment the ultra-thin high-loaded fluid-absorbent layers comprise at least two sheets comprising immobilized water-absorbent polymer particles.

Examples of ultra-thin high-loaded fluid-absorbent layers are described in EP 1 293 187 A1, US 6,972,011, EP 1 447 066 A1, EP 1 447 067 A1, EP 1 609 448 A1, JP 2004/313580, US 2005/0137085, US 2006/0004336, US 2007/0135785 WO 2008/155699 A1, WO 2008/155701 A2, WO 2008/155702 A1, WO 2008/155710 A1, WO 2008/155711 A1, WO 2004/071363 A1, US 2003/0181115, WO 2005/097025, US 2007/156108, US 2008/0125735, and WO 2008/155722 A2, which explicitly forms part of the present disclosure.

Construction Example of Embodiment 2

The fluid-absorbent core consists of a multi-layered single core system each layer having a uniform rectangular size. The layered fluid-absorbent core between (A) and (B) comprises a multi-layered system of hydrophilic fibers (cellulose fibers, fluff pulp fibers). The total fluff pulp weight is 12 g divided equally between upper core (1) and lower core (3). The density of the fluid-absorbent core is for the front overall average 0.19 g/cm$^3$, for the insult zone 0.20 g/cm$^3$, for the back overall average 0.18 g/cm$^3$. The basis weight of the fluid-absorbent core is for the front overall average 989 gsm, for the insult zone 1101 gsm, for the back overall average 664 gsm. The thickness of the fluid-absorbent core has an average of 4.5 mm.

The fluid-absorbent layer (2) holds 56.5% by weight distributed water-absorbent polymer particles, the quantity of water-absorbent polymer particles within the fluid-absorbent core is 12 g.

The water-absorbent polymer particles derived from dropletization polymerization as described in production example 24, exhibiting the following features and absorption profile:

CRC of 32,4 g/g
SFC of 65 x $10^{-7}cm^3s/g$
AUHL of 24 g/g
AUL of 30 g/g
Extractables of 5 wt.%
Residual monomers of 594 ppm
Moisture content of 0.6 wt.%
FSR of 0.29 g/gs

Mean Sphericity of 0.89
PSD of 200 to 600 $\mu$m
Anticaking of 3

The water-absorbent polymer particles can be remoisturized to a moisture content of 13% by weight.

Dimension of the fluid-absorbent core: length: 38 cm; width: 10 cm.

An air through bonded acquisition-distribution layer between (A) and (C) having a basis weight of 50 gsm is rectangular shaped with dimensions of 24 cm x 8 cm and smaller than the fluid-absorbent core.

The fluid-absorbent article further comprises:

- flat rubber elastics; elastics from spandex type fibers: 3 leg elastics and 1 cuff elastics
- leg cuffs from synthetic fibers showing the layer combination SMS and having a basis weight of between 13 to 17 gsm and a height of 4.6 cm
- mechanical closure system with landing zone of dimension 18.3 cm x 4.0 cm and flexiband closure tapes of 3.4 cm x 1.0 cm; attached to hook fastening tape of 3.4 cm x 1.4 cm
- Also incorporated is elasticated waistband located to the rear of the product with dimensions of 14.6 x 4.5 cm

Dimension of the fluid-absorbent article: length: 49.6 cm; front width: 34.0 cm; crotch width: 24.0 cm; rear width: 34.3 cm.

Embodiment 3

A further preferred embodiment of the present invention is described in Embodiment 3 hereinafter. Thus, a preferred fluid-absorbent article comprising

(A) an upper liquid-pervious layer comprising a spunbond web (three piece coverstock);
(B) a lower liquid-impervious layer comprising a composite of breathable polyethylene film and spunbond nonwoven;
(C) a single fluid-absorbent core between (A) and (B) comprising a mixture of wood pulp fibers (cellulose fibers) and between 10 to 50 % by weight homogeneously distributed water-absorbent polymer particles within the fluid-absorbent core (C); suitable water-absorbent polymer particles for such construction having a centrifuge retention capacity (CRC) from about 32 to 60 g/g; the fluid-absorbent core is further comprising a dusting layer adjacent to the liquid-impervious layer (B) and underlying the fluid-absorbent core above; the dusting layer is a fibrous layer comprising fluff only (cellulose fibers); and
(D) an air-through bonded acquisition-distribution layer between (A) and (C) having a basis weight of 30 to 80 gsm; the acquisition-distribution layer is rectangular shaped and smaller than the fluid-absorbent core having a size of about 150 to about 250 cm$^2$.

The construction of the products chassis and the components contained therein is made and controlled by the discrete application of hotmelt adhesives as known to people skilled in the art. Examples would be e.g. Dispomelt 505B, Dispomelt Cool 1101, as well as other specific function adhesives manufactured by Bostik, Henkel or Fuller.

Ultra-thin high-loaded fluid-absorbent layers can be formed by immobilization of water-absorbent polymer particles on a non-woven sheet using hotmelt adhesives. Preferably the water-absorbent polymer particles form longitudinal strips, discrete spots or discrete target zones. Other patterns of the water-absorbent polymer particles are also possible.

In a preferred embodiment the ultra-thin high-loaded fluid-absorbent layers comprise at least two sheets comprising immobilized water-absorbent polymer particles.

Examples of ultra-thin high-loaded fluid-absorbent layers are described in EP 1 293 187 A1, US 6,972,011, EP 1 447 066 A1, EP 1 447 067 A1, EP 1 609 448 A1, JP 2004/313580, US 2005/0137085, US 2006/0004336, US 2007/0135785 WO 2008/155699 A1, WO 2008/155701 A2, WO 2008/155702 A1, WO 2008/155710 A1, WO 2008/155711 A1, WO 2004/071363 A1, US 2003/0181115, WO 2005/097025, US 2007/156108, US 2008/0125735, and WO 2008/155722 A2, which explicitly forms part of the present disclosure.

Construction Example of Embodiment 3

The fluid-absorbent core consists of a single fluid-absorbent Core between (A) and (B) comprising a mixture of wood pulp fibers (cellulose fibers) and 37.11% by weight homogeneously distributed water-absorbent polymer particles within the fluid-absorbent core (C) having a uniform rectangular size. The quantity of water-absorbent polymer particles within the fluid-absorbent core is 11.38 g. The total fluff pulp weight is 19.25 g. The density of the fluid-absorbent core is for the front overall average 0.22 g/cm$^3$, for the insult zone 0.18 g/cm$^3$, for the back overall average 0.18 g/cm$^3$. The

basis weight of the fluid-absorbent core is for the front overall average 914.18 gsm, for the insult zone 925.47 gsm, for the back overall average 886.32 gsm.

The water-absorbent polymer particles derived from dropletization polymerization as described inproduction example 21, exhibiting the following features and absorption profile:

CRC of 37.7 g/g
SFC of 23 x $10^{-7}$cm$^3$s/g
AUHL of 25 g/g
AUL of 33 g/g
Extractables of 6 wt.%
Residual monomers of 373 ppm
Moisture content of 1.1 wt.%
FSR of 0.31 g/gs
Mean Sphericity of 0.89
PSD of 200 to 600 $\mu$m
Anticaking of 3

The water-absorbent polymer particles can be remoisturized to a moisture content of 13% by weight.

Dimension of the fluid-absorbent core: length: 39.2 cm; width: 10.0 cm.

The thickness of the fluid-absorbent core has an average of 4.7 mm.

An air through bonded acquisition-distribution layer between (A) and (C) having a basis weight of 40 gsm is rectangular shaped with dimensions of 24.4 cm x 8.6 cm and smaller than the fluid-absorbent core.

The fluid-absorbent article further comprises:

- flat rubber elastics; elastics from spandex type fibers: 3 leg elastics and 1 cuff elastics
- leg cuffs from synthetic fibers showing the layer combination SMS and having a basis weight of between 13 to 17 gsm and a height of 3.0 cm
- mechanical closure system with landing zone of dimension 18.9 cm x 3.8 cm and flexiband closure tapes of 1.6 cm x 3.4 cm; attached to hook fastening tape of 1.3 cm x 3.4 cm
- also incorporated is elasticated waistband located to the rear of the product with dimensions of 10.8 cm x 2.8 cm

Dimension of the fluid-absorbent article: length: 47.8 cm; front width: 31.5 cm; crotch width: 20.6 cm; rear width: 31.1 cm.

Embodiment 4

A further preferred embodiment of the present invention is described in Embodiment 4 hereinafter. Thus, a preferred fluid-absorbent article comprising

(A) an upper liquid-pervious layer comprising a spunbond web (three piece coverstock);
(B) a lower liquid-impervious layer comprising a composite of breathable polyethylene film and spunbond nonwoven;
(C) a single fluid-absorbent core between (A) and (B) comprising a mixture of wood pulp fibers (cellulose fibers) and between 40 to 80 % by weight homogeneously distributed water-absorbent polymer particles within the fluid-absorbent core; suitable water-absorbent polymer particles for such construction having a saline flow conductivity (SFC) from about 35 to 100 x $10^{-7}$cm$^3$s/g; and
(D) a system of two acquisition-distribution layers between (A) and (C), comprising an upper resinbonded layer having a basis weight of 40 to 80 gsm; the upper acquisition-distribution layer is rectangular shaped having a size of about 150 to about 250 cm$^2$; the lower acquisition-distribution layer comprising of modified cellulosic fibers (e.g. from Buckeye Technologies Inc.) having a basis weight of 40 to 80 gsm and a size of about 100 to about 300 cm$^2$; both acquisition-distribution layers are smaller than the fluid-absorbent core.

The construction of the products chassis and the components contained therein is made and controlled by the discrete application of hotmelt adhesives as known to people skilled in the art. Examples would be e.g. Dispomelt 505B, Dispomelt Cool 1101, as well as other specific function adhesives manufactured by Bostik, Henkel or Fuller.

Ultra-thin high-loaded fluid-absorbent layers can be formed by immobilization of water-absorbent polymer particles on a non-woven sheet using hotmelt adhesives. Preferably the water-absorbent polymer particles form longitudinal strips, discrete spots or target zones. Other patterns of the water-absorbent polymer particles are also possible.

In a preferred embodiment the ultra-thin high-loaded fluid-absorbent layers comprise at least two sheets comprising immobilized water-absorbent polymer particles.

Examples of ultra-thin high-loaded fluid-absorbent layers are described in EP 1 293 187 A1, US 6,972,011, EP 1 447 066 A1, EP 1 447 067 A1, EP 1 609 448 A1, JP 2004/313580, US 2005/0137085, US 2006/0004336, US 2007/0135785 WO 2008/155699 A1, WO 2008/155701 A2, WO 2008/155702 A1, WO 2008/155710 A1, WO 2008/155711 A1, WO 2004/071363 A1, US 2003/0181115, WO 2005/097025, US 2007/156108, US 2008/0125735, and WO 2008/155722 A2, which explicitly forms part of the present disclosure.

Construction Example of Embodiment 4

The fluid-absorbent core consists of a single fluid-absorbent core between (A) and (B) comprising a mixture of wood pulp fibers (cellulose fibers) and 67.12 % by weight homogeneously distributed water-absorbent polymer particles within the fluid-absorbent core (C) having a uniform rectangular size. The fluid-absorbent core is encapsulated by wrapping with a spunbond material having a basis weight of 10 gsm. The quantity of water-absorbent polymer particles within the fluid-absorbent core is 12.18 g. The total fluff pulp weight is 5.95 g. The density of the fluid-absorbent core is for the front overall average 0.19 g/cm$^3$, for the insult zone 0.18 g/cm$^3$, for the back overall average 0.18 g/cm$^3$. The basis weight of the fluid-absorbent core is for the front overall average 965.79 gsm, for the insult zone 913.38 gsm, for the back overall average 658.85 gsm.

The water-absorbent polymer particles derived from dropletization polymerization as described in production example 18, exhibiting the following features and absorption profile:

CRC of 27.6 g/g
SFC of 125 x 10$^{-7}$cm$^3$s/g
AUHL of 24 g/g
AUL of 28 g/g
Extractables of 3 wt.%
Residual monomers of 565 ppm
Moisture content of 0.8 wt.%
FSR of 0.22 g/gs
Mean Sphericity of 0.89
PSD of 200 to 600 $\mu$m
Anticaking of 3

Dimension of the fluid-absorbent core: length: 40.0 cm; width: 10.0 cm.
The thickness of the fluid-absorbent core has an average of 4.4 mm.
An air through bonded acquisition-distribution layer between (A) and (C) having a basis weight of 60 gsm is rectangular shaped with dimensions of 24.0 cm x 7.5 cm and smaller than the fluid-absorbent core.
The fluid-absorbent article further comprises:

- flat rubber elastics; elastics from spandex type fibers: 3 leg elastics and 2 cuff elastics
- leg cuffs from synthetic fibers showing the layer combination SMS and having a basis weight of between 13 to 17 gsm and a height of 3.3 cm
- mechanical closure system with landing zone of dimension 19.8 cm x 5.0 cm and flexiband closure tapes of 3.5 cm x 2.7 cm consisting of pressure sensitive adhesive zone 3.5 cm x 1.5 cm and mechanical hook of 3.5 cm x 1.2 cm

For improving the fit of the fluid-absorbent article, the product of embodiment 4 provides a stretchable side panel and a reduced width chassis.
Dimension of the fluid-absorbent article: length: 48.0 cm; front width: 32.3 cm; crotch width: 20.3 cm; rear width: 31.0 cm.

Embodiment 5

A further preferred embodiment of the present invention is described in Embodiment 5 hereinafter. Thus, a preferred fluid-absorbent article comprising

(A) an upper liquid-pervious layer comprising a spunbond layer (three piece coverstock);
(B) a lower liquid-impervious layer comprising a composite of breathable polyethylene film and spunbond nonwoven;
(C) a double fluid-absorbent core between (A) and (B) comprising a homogeneous mixture of wood pulp fibers (cellulose fibers) and water-absorbent polymer particles as primary core and a layered secondary fluid-absorbent core; the total double fluid-absorbent core comprising the following sequence:

1. a homogeneous primary core of hydrophilic fibrous matrix of wood pulp fibers (cellulose fibers) comprising between 10 to 50% by weight water-absorbent polymer particles based on the primary absorbent core weight; the primary core contains about 30% of the total fluff amount; suitable water-absorbent polymer particles for such construction having a centrifuge retention capacity (CRC) from about 32 to 60 g/g;

2. a secondary core upper fluff layer of hydrophilic fibrous matrix of wood pulp fibers (cellulose fibers); the secondary core upper layer contains about 35% of the total fluff amount;

3. a fluid-absorbent layer comprising between 10 to 50 % by weight water-absorbent polymer particles based on the secondary absorbent core weight; suitable water-absorbent polymer particles for such construction having a centrifuge retention capacity CRC from about 32 to 60 g/g;

4. a secondary core lower fluff layer of hydrophilic fibrous matrix of wood pulp fibers (cellulose fibers) acting as a dusting layer; the lower core contains about 35% of the total fluff amount; and

(D) an air through bonded acquisition-distribution layer between (A) and (C) having a basis weight of 30 to 80 gsm; the acquisition-distribution layer is rectangular shaped and smaller than the primary fluid-absorbent core having a size of about 150 to about 250cm$^2$.

The construction of the products chassis and the components contained therein is made and controlled by the discrete application of hotmelt adhesives as known to people skilled in the art. Examples would be e.g. Dispomelt 505B, Dispomelt Cool 1101, as well as other specific function adhesives manufactured by Bostik, Henkel or Fuller.

Ultra-thin high-loaded fluid-absorbent layers can be formed by immobilization of water-absorbent polymer particles on a non-woven sheet using hotmelt adhesives. Preferably the water-absorbent polymer particles form longitudinal strips, discrete spots or target zones. Other patterns of the water-absorbent polymer particles are also possible.

In a preferred embodiment the ultra-thin high-loaded fluid-absorbent layers comprise at least two sheets comprising immobilized water-absorbent polymer particles.

Examples of ultra-thin high-loaded fluid-absorbent layers are described in EP 1 293 187 A1, US 6,972,011, EP 1 447 066 A1, EP 1 447 067 A1, EP 1 609 448 A1, JP 2004/313580, US 2005/0137085, US 2006/0004336, US 2007/0135785 WO 2008/155699 A1, WO 2008/155701 A2, WO 2008/155702 A1, WO 2008/155710 A1, WO 2008/155711 A1, WO 2004/071363 A1, US 2003/0181115, WO 2005/097025, US 2007/156108, US 2008/0125735, and WO 2008/155722 A2, which explicitly forms part of the present disclosure.

Construction Example of Embodiment 5

The total fluid-absorbent core includes a double-core system, the primary and secondary cores each having an almost uniform rectangular size. The primary core is smaller than the secondary core and is positioned 6 cm from the front distal edge of the secondary core and 10 cm from the rear distal edge of the secondary core and is 9 cm in width. The primary fluid-absorbent core between (A) and (B) comprising a homogeneous mixture of hydrophilic fibrous matrix of wood pulp fibers and 25 % by weight of water absorbent polymer particles. The primary core has a total weight of 8 g. The secondary core is a multi-layered system of hydrophilic fibrous matrix of wood pulp fibers (cellulose fibers) and 30% by weight water-absorbent polymer particles. The quantity of water-absorbent polymer particles within the secondary fluid-absorbent core is 6.0 g. The density of the fluid-absorbent core is for the front overall average 0.15 g/cm$^3$, for the insult zone 0.19 g/cm$^3$, for the back overall average 0.18 g/cm$^3$. The basis weight of the fluid-absorbent core is for the front overall average 790.63 gsm, for the insult zone 1121.38 gsm, for the back overall average 976.83 gsm.

The water-absorbent polymer particles derived from dropletization polymerization as described in production example 9, exhibiting the following features and absorption profile:

CRC of 41,7g/g
SFC of 3 x 10$^{-7}$cm$^3$s/g
AUHL of 26 g/g
AUL of 36 g/g
Extractables of 6 wt.%
Residual monomers of 557 ppm
Moisture content of 1.2 wt.%
FSR of 0.27 g/gs
Mean Sphericity of 0.89
PSD of 200 to 600 $\mu$m
Anticaking of 3

The water-absorbent polymer particles can be remoisturized to a moisture content of 13% by weight.

Dimension of the secondary fluid-absorbent core: length: 40.8 cm; front width: 10.5 cm; crotch width: 9.3 cm; rear width: 10.3 cm.

The total thickness of both fluid-absorbent cores has an average of 5.4 mm.

An air through bonded acquisition-distribution layer between (A) and (C) having a basis weight of 40 gsm is rectangular shaped and smaller than the primary fluid- absorbent core having a size of 19.7 cm x 7.6 cm.

The fluid-absorbent article further comprises:

- flat rubber elastics; elastics from spandex type fibers: 3 leg elastics and 2 cuff elastics
- leg cuffs from synthetic fibers showing the layer combination SMS and having a basis weight of between 13 to 17 gsm and a height of 3.8 cm
- mechanical closure system with landing zone of dimension 22.0 cm x 4.0 cm and flexiband closure tapes of 3.4 cm x 1.5 cm; attached to hook fastening tape of 3.4 cm x 1.4 cm

Dimension of the fluid-absorbent article: length: 48.0 cm; front width: 29.7 cm; crotch width: 22.0 cm; rear width: 31.6 cm.

Embodiment 6

A further preferred embodiment of the present invention is described in Embodiment 6 hereinafter. Thus, a preferred fluid-absorbent article comprising

(A) an upper liquid-pervious layer comprising a spunbond layer (three piece coverstock);
(B) a lower liquid-impervious layer comprising a composite of breathable polyethylene film and spunbond nonwoven;
(C) a double fluid-absorbent core between (A) and (B) comprising a homogeneous mixture of wood pulp fibers (cellulose fibers) and water-absorbent polymer particles as primary core and a layered secondary fluid-absorbent core; the total double fluid-absorbent core comprising the following sequence:

1. a homogeneous primary core of hydrophilic fibrous matrix of wood pulp fibers (cellulose fibers) comprising between 40 to 80% by weight water-absorbent polymer particles based on the primary absorbent core weight; the primary core contains about 50% of the total fluff amount; suitable water-absorbent polymer particles for such construction having a saline flow conductivity (SFC) from about 35 to 100 x $10^{-7}$cm$^3$s/g;
2. a secondary core upper fluff layer of hydrophilic fibrous matrix of wood pulp fibers (cellulose fibers); the secondary core upper layer contains about 25% of the total fluff amount;
3. a fluid-absorbent layer comprising between 40 to 80% by weight water-absorbent polymer particles based on the secondary absorbent core weight; suitable water-absorbent polymer particles for such construction having a saline flow conductivity (SFC) from about 35 to 100 x $10^{-7}$cm$^3$s/g;
4. a secondary core lower fluff layer of hydrophilic fibrous matrix of wood pulp fibers (cellulose fibers) acting as a dusting layer; the lower core contains about 25% of the total fluff amount; and

(D) an air through bonded acquisition-distribution layer between (A) and (C) having a basis weight of 40 to 80 gsm; the acquisition-distribution layer is rectangular shaped and smaller than the primary fluid-absorbent core having a size of about 150 to about 250 cm$^2$.

The construction of the products chassis and the components contained therein is made and controlled by the discrete application of hotmelt adhesives as known to people skilled in the art. Examples would be e.g. Dispomelt 505B, Dispomelt Cool 1101, as well as other specific function adhesives manufactured by Bostik, Henkel or Fuller.

Ultra-thin high-loaded fluid-absorbent layers can be formed by immobilization of water-absorbent polymer particles on a non-woven sheet using hotmelt adhesives. Preferably the water-absorbent polymer particles form longitudinal strips, discrete spots or target zones. Other patterns of the water-absorbent polymer particles are also possible.

In a preferred embodiment the ultra-thin high-loaded fluid-absorbent layers comprise at least two sheets comprising immobilized water-absorbent polymer particles.

Examples of ultra-thin high-loaded fluid-absorbent layers are described in EP 1 293 187 A1, US 6,972,011, EP 1 447 066 A1, EP 1 447 067 A1, EP 1 609 448 A1, JP 2004/313580, US 2005/0137085, US 2006/0004336, US 2007/0135785 WO 2008/155699 A1, WO 2008/155701 A2, WO 2008/155702 A1, WO 2008/155710 A1, WO 2008/155711 A1, WO 2004/071363 A1, US 2003/0181115, WO 2005/097025, US 2007/156108, US 2008/0125735, and WO 2008/155722 A2, which explicitly forms part of the present disclosure.

Construction Example of Embodiment 6

The total fluid-absorbent core includes a double-core system, the primary and secondary cores each having an almost uniform rectangular size. The primary core is smaller than the secondary core and is positioned 6 cm from the front distal edge of the secondary core and 10 cm from the rear distal edge of the secondary core and is 9 cm in width. The primary fluid-absorbent core between (A) and (B) comprising a homogeneous mixture of hydrophilic fibrous matrix of wood pulp fibers and 50 % by weight of water absorbent polymer particles. The primary core has a total weight of 8 g. The secondary core is a multi-layered system of hydrophilic fibrous matrix of wood pulp fibers (cellulose fibers) and 50% by weight water-absorbent polymer particles. The quantity of water-absorbent polymer particles within the secondary fluid-absorbent core is 10.0 g. The density of the fluid-absorbent core is for the front overall average 0.19 $g/cm^3$, for the insult zone 0.19 $g/cm^3$, for the back overall average 0.18 $g/cm^3$. The basis weight of the fluid-absorbent core is for the front overall average 813.46 gsm, for the insult zone 1209.15 gsm, for the back overall average 986.27 gsm.
The water-absorbent polymer particles derived from dropletization polymerization as described in production example 24, exhibiting the following features and absorption profile:

CRC of 32,4 g/g
SFC of 65 x $10^{-7}cm^3s/g$
AUHL of 24 g/g
AUL of 30 g/g
Extractables of 5 wt.%
Residual monomers of 594 ppm
Moisture content of 0.6- wt.%
FSR of 0.29 g/gs
Mean Sphericity of 0.89
PSD of 200 to 600 $\mu$m
Anticaking of 3

The water-absorbent polymer particles can be remoisturized to a moisture content of 13% by weight.
Dimension of the secondary fluid-absorbent core: length: 40.8 cm; front width: 10.0 cm; crotch width: 9.0 cm; rear width: 10.0 cm.
The total thickness of both fluid-absorbent cores has an average of 3.9 mm.
An air through bonded acquisition-distribution layer between (A) and (C) having a basis weight of 60 gsm is rectangular shaped and smaller than the primary fluid-absorbent core having a size of 19.0 cm x 7.6 cm.
The fluid-absorbent article further comprises:

- flat rubber elastics; elastics from spandex type fibers: 3 leg elastics and 2 cuff elastics
- leg cuffs from synthetic fibers showing the layer combination SMS and having a basis weight of between 13 to 17 gsm and a height of 3.8 cm
- mechanical closure system with landing zone of dimension 22.0 cm x 4.0 cm and flexiband closure tapes of 3.4 cm x 1.5 cm; attached to hook fastening tape of 3.4 cm x 1.4 cm

Dimension of the fluid-absorbent article: length: 48.0 cm; front width: 29.7 cm; crotch width: 20.0 cm; rear width: 31.6 cm.

Embodiment 7

A further preferred embodiment of the present invention is described in Embodiment 7 hereinafter. Thus, a preferred fluid-absorbent article comprising

(A) an upper liquid-pervious layer comprising a spunbond layer (three piece coverstock);
(B) a lower liquid-impervious layer comprising a composite of breathable polyethylene film and spunbond nonwoven;
(C) a double fluid-absorbent core between (A) and (B) comprising a homogeneous mixture of wood pulp fibers (cellulose fibers) and polymer particles for each the primary core and the secondary fluid-absorbent core; the total double fluid-absorbent core comprising:

1. a homogeneous primary core of hydrophilic fibrous matrix of wood pulp fibers (cellulose fibers) comprising between 10 to 50% by weight water-absorbent polymer particles based on the primary absorbent core weight; the primary core contains about 30% of the total fluff amount; suitable water-absorbent polymer particles for such construction having a centrifuge retention capacity (CRC) from about 32 to 60 g/g;

2. a homogeneous secondary core of hydrophilic fibrous matrix of wood pulp fibers (cellulose fibers) comprising between 10 to 50% by weight water-absorbent polymer particles based on the secondary absorbent core weight; the secondary core contains about 70% of the total fluff amount; suitable water-absorbent polymer particles for such construction having a centrifuge retention capacity (CRC) from about 32 to 60 g/g; and

(D) an air through bonded acquisition-distribution layer between (A) and (C) having a basis weight of 30 to 80 gsm; the acquisition-distribution layer is rectangular shaped and smaller than the primary fluid-absorbent core having a size of about 150 to about 250 cm$^2$.

The construction of the products chassis and the components contained therein is made and controlled by the discrete application of hotmelt adhesives as known to people skilled in the art. Examples would be e.g. Dispomelt 505B, Dispomelt Cool 1101, as well as other specific function adhesives manufactured by Bostik, Henkel or Fuller.

Ultra-thin high-loaded fluid-absorbent layers can be formed by immobilization of water-absorbent polymer particles on a non-woven sheet using hotmelt adhesives. Preferably the water-absorbent polymer particles form longitudinal strips, discrete spots or target zones. Other patterns of the water-absorbent polymer particles are also possible.

In a preferred embodiment the ultra-thin high-loaded fluid-absorbent layers comprise at least two sheets comprising immobilized water-absorbent polymer particles.

Examples of ultra-thin high-loaded fluid-absorbent layers are described in EP 1 293 187 A1, US 6,972,011, EP 1 447 066 A1, EP 1 447 067 A1, EP 1 609 448 A1, JP 2004/313580, US 2005/0137085, US 2006/0004336, US 2007/0135785 WO 2008/155699 A1, WO 2008/155701 A2, WO 2008/155702 A1, WO 2008/155710 A1, WO 2008/155711 A1, WO 2004/071363 A1, US 2003/0181115, WO 2005/097025, US 2007/156108, US 2008/0125735, and WO 2008/155722 A2, which explicitly forms part of the present disclosure.

Construction Example of Embodiment 7

The total fluid-absorbent core includes a double-core system, the primary and secondary cores each having an almost uniform rectangular size. The primary core is smaller than the secondary core and is positioned 6 cm from the front distal edge of the secondary core and 10 cm from the rear distal edge of the secondary core and is 9 cm in width. The primary fluid-absorbent core between (A) and the secondary fluid-absorbent core comprising a homogeneous mixture of hydrophilic fibrous matrix of wood pulp fibers and 25 % by weight of water absorbent polymer particles. The primary core has a total weight of 8 g. The secondary core between the primary core and (B) comprising a homogeneous mixture of hydrophilic fibrous matrix of wood pulp fibers and 30 % by weight of water absorbent polymer particles. The quantity of water-absorbent polymer particles within the secondary fluid-absorbent core is 6.0 g. The secondary core has a total weight of 20 g. The density of the fluid-absorbent core is for the front overall average 0.15 g/cm$^3$, for the insult zone 0.19 g/cm$^3$, for the back overall average 0.18 g/cm$^3$. The basis weight of the fluid-absorbent core is for the front overall average 790.63 gsm, for the insult zone 1121.38 gsm, for the back overall average 976.83 gsm.

The water-absorbent polymer particles derived from dropletization polymerization as described in production example 9, exhibiting the following features and absorption profile:

CRC of 41.7g/g
SFC of 3 x 10$^{-7}$cm$^3$s/g
AUHL of 26 g/g
AUL of 36 g/g
Extractables of 6 wt.%
Residual monomers of 557 ppm
Moisture content of 1.2 wt.%
FSR of 0.27 g/gs
Mean Sphericity of 0.89
PSD of 200 to 600 $\mu$m
Anticaking of 3

The water-absorbent polymer particles can be remoisturized to a moisture content of 13% by weight.

Dimension of the secondary fluid-absorbent core: length: 40.8 cm; front width: 10.5 cm; crotch width: 9.3 cm; rear width: 10.3 cm

The total thickness of both fluid-absorbent cores has an average of 5.4 mm.

An air through bonded acquisition-distribution layer between (A) and (C) having a basis weight of 40 gsm is rectangular shaped and smaller than the primary fluid-absorbent core having a size of 19.7 cm x 7.6cm.

The fluid-absorbent article further comprises:

- flat rubber elastics; elastics from spandex type fibers: 3 leg elastics and 2 cuff elastics
- leg cuffs from synthetic fibers showing the layer combination SMS and having a basis weight of between 13 to 17 gsm and a height of 3.8 cm
- mechanical closure system with landing zone of dimension 22.0 cm x 4.0 cm and flexiband closure tapes of 3.4 cm x 1.5 cm; attached to hook fastening tape of 3.4 cm x 1.4 cm

Dimension of the fluid-absorbent article: length: 48.0 cm; front width: 29.7 cm; crotch width: 22.0 cm; rear width: 31.6 cm.

Embodiment 8

A further preferred embodiment of the present invention is described in Embodiment 8 hereinafter. Thus, a preferred fluid-absorbent article comprising

(A) an upper liquid-pervious layer comprising a spunbond layer (three piece coverstock);
(B) a lower liquid-impervious layer comprising a composite of breathable polyethylene film and spunbond nonwoven;
(C) a double fluid-absorbent core between (A) and (B) comprising a homogeneous mixture of wood pulp fibers (cellulose fibers) and polymer particles for each the primary core and the secondary fluid-absorbent core; the total double fluid-absorbent core comprising:

1. a homogeneous primary core of hydrophilic fibrous matrix of wood pulp fibers (cellulose fibers) comprising between 40 to 80 % by weight water-absorbent polymer particles based on the primary absorbent core weight; the primary core contains about 50% of the total fluff amount; suitable water-absorbent polymer particles for such construction having a saline flow conductivity (SFC) from about 35 to 100 x $10^{-7}$cm$^3$s/g;
2. a homogeneous secondary core of hydrophilic fibrous matrix of wood pulp fibers (cellulose fibers) comprising between 40 to 70 % by weight water-absorbent polymer particles based on the secondary absorbent core weight; the secondary core contains about 50% of the total fluff amount; suitable water-absorbent polymer particles for such construction having a saline flow conductivity (SFC) from about 35 to 100 x $10^{-7}$cm$^3$s/g; and

(D) an air through bonded acquisition-distribution layer between (A) and (C) having a basis weight of 40 to 80 gsm; the acquisition-distribution layer is rectangular shaped and smaller than the primary fluid-absorbent core having a size of about 150 to about 250 cm$^2$.

The construction of the products chassis and the components contained therein is made and controlled by the discrete application of hotmelt adhesives as known to people skilled in the art. Examples would be e.g. Dispomelt 505B, Dispomelt Cool 1101, as well as other specific function adhesives manufactured by Bostik, Henkel or Fuller.

Ultra-thin high-loaded fluid-absorbent layers can be formed by immobilization of water-absorbent polymer particles on a non-woven sheet using hotmelt adhesives. Preferably the water-absorbent polymer particles form longitudinal strips, discrete spots or target zones. Other patterns of the water-absorbent polymer particles are also possible. In a preferred embodiment the ultra-thin high-loaded fluid-absorbent layers comprise at least two sheets comprising immobilized water-absorbent polymer particles.

Examples of ultra-thin high-loaded fluid-absorbent layers are described in EP 1 293 187 A1, US 6,972,011, EP 1 447 066 A1, EP 1 447 067 A1, EP 1 609 448 A1, JP 2004/313580, US 2005/0137085, US 2006/0004336, US 2007/0135785 WO 2008/155699 A1, WO 2008/155701 A2, WO 2008/155702 A1, WO 2008/155710 A1, WO 2008/155711 A1, WO 2004/071363 A1, US 2003/0181115, WO 2005/097025, US 2007/156108, US 2008/0125735, and WO 2008/155722 A2, which explicitly forms part of the present disclosure.

Construction Example of Embodiment 8

The total fluid-absorbent core includes a double-core system, the primary and secondary cores each having an almost uniform rectangular size. The primary core is smaller than the secondary core and is positioned 6 cm from the front distal edge of the secondary core and 10 cm from the rear distal edge of the secondary core and is 9 cm in width. The primary fluid-absorbent core between (A) and the secondary fluid-absorbent core comprising a homogeneous mixture of hydrophilic fibrous matrix of wood pulp fibers and 28.6 % by weight of water absorbent polymer particles. The primary core has a total weight of 8 g. The secondary core between the primary core and (B) comprising a homogeneous mixture of hydrophilic fibrous matrix of wood pulp fibers and 71.4 % by weight of water absorbent polymer particles. The quantity of water-absorbent polymer particles within the secondary fluid-absorbent core is 10.0 g. The secondary core has a total weight of 14 g. The density of the fluid-absorbent core is for the front overall average 0.15 g/cm$^3$, for the insult zone 0.19 g/cm$^3$, for the back overall average 0.18 g/cm$^3$. The basis weight of the fluid-absorbent core is for the front overall average 790.63 gsm, for the insult zone 1121.38 gsm, for the back overall average 976.83 gsm.

The water-absorbent polymer particles derived from dropletization polymerization as described inproduction example 24, exhibiting the following features and absorption profile:

CRC of 32.4g/g
SFC of 65 x 10$^{-7}$cm$^3$s/g
AUHL of 24 g/g
AUL of 30g/g
Extractables of 5 wt.%
Residual monomers of 594 ppm
Moisture content of 0.6 wt.%
FSR of 0.29 g/gs
Mean Sphericity of 0.89
PSD of 200 to 600 μm
Anticaking of 3

The water-absorbent polymer particles can be remoisturized to a moisture content of 13% by weight.
Dimension of the secondary fluid-absorbent core: length: 40.8 cm; front width: 10.5 cm; crotch width: 9.3 cm; rear width: 10.3 cm.
The total thickness of both fluid-absorbent cores has an average of 5.4 mm.
An air through bonded acquisition-distribution layer between (A) and (C) having a basis weight of 60 gsm is rectangular shaped and smaller than the primary fluid-absorbent core having a size of 19.7 cm x 7.6 cm.
The fluid-absorbent article further comprises:

- flat rubber elastics; elastics from spandex type fibers: 3 leg elastics and 2 cuff elastics
- leg cuffs from synthetic fibers showing the layer combination SMS and having a basis weight of between 13 to 17 gsm and a height of 3.8 cm
- mechanical closure system with landing zone of dimension 22.0 cm x 4.0 cm and flexiband closure tapes of 3.4 cm x 1.5 cm; attached to hook fastening tape of 3.4 cm x 1.4 cm

Dimension of the fluid-absorbent article: length: 48.0 cm; front width: 29.7 cm; crotch width: 20.0 cm; rear width: 31.6 cm.

Embodiment 9

A further preferred embodiment of the present invention is described in Embodiment 9 hereinafter. Thus, a preferred fluid-absorbent article comprising

(A) an upper liquid-pervious layer comprising a spunbond layer (three piece coverstock);
(B) a lower liquid-impervious layer comprising a composite of breathable polyethylene film and spunbond nonwoven;
(C) a single fluid-absorbent core between (A) and (B) comprising between 10 to 50 % by weight water-absorbent polymer particles based on the total absorbent core weight and including a multi-layered fluid-storage section comprising the following sequence:

1. a homogeneous upper core fluff layer of hydrophilic fibrous matrix of wood pulp fibers (cellulose fibers) containing about 50% of the total fluff amount;
2. a fluid-absorbent layer comprising water-absorbent polymer particles; suitable water-absorbent polymer particles for such construction having a centrifuge retention capacity (CRC) from about 32 to 60 g/g; and

(D) an air through bonded acquisition-distribution layer between (A) and (C) having a basis weight of 40 to 80 gsm; the acquisition-distribution layer is rectangular shaped and smaller than the primary fluid-absorbent core having a size of about 150 to about 250 cm$^2$.

The construction of the products chassis and the components contained therein is made and controlled by the discrete application of hotmelt adhesives as known to people skilled in the art. Examples would be e.g. Dispomelt 505B, Dispomelt Cool 1101, as well as other specific function adhesives manufactured by Bostik, Henkel or Fuller.

Ultra-thin high-loaded fluid-absorbent layers can be formed by immobilization of water-absorbent polymer particles on a non-woven sheet using hotmelt adhesives. Preferably the water-absorbent polymer particles form longitudinal strips, discrete spots or target zones. Other patterns of the water-absorbent polymer particles are also possible.

In a preferred embodiment the ultra-thin high-loaded fluid-absorbent layers comprise at least two sheets comprising immobilized water-absorbent polymer particles.

Examples of ultra-thin high-loaded fluid-absorbent layers are described in EP 1 293 187 A1, US 6,972,011, EP 1 447 066 A1, EP 1 447 067 A1, EP 1 609 448 A1, JP 2004/313580, US 2005/0137085, US 2006/0004336, US 2007/0135785 WO 2008/155699 A1, WO 2008/155701 A2, WO 2008/155702 A1, WO 2008/155710 A1, WO 2008/155711 A1, WO 2004/071363 A1, US 2003/0181115, WO 2005/097025, US 2007/156108, US 2008/0125735, and WO 2008/155722 A2, which explicitly forms part of the present disclosure.

Construction Example of Embodiment 9

The fluid-absorbent core consists of a double-layered single core system each layer having a uniform rectangular size. The layered fluid-absorbent core between (A) and (B) comprises a double-layered system of hydrophilic fibers (cellulose fibers, fluff pulp fibers), each layer having an almost uniform rectangular size. The fluid-absorbent core is encapsulated by wrapping with a spunbond material having a basis weight of 10 gsm. The density of the fluid-absorbent core is for the front overall average 0.16 g/cm$^3$, for the insult zone 0.14 g/cm$^3$, for the back overall average 0.16 g/cm$^3$. The basis weight of the fluid-absorbent core is for the front overall average 598.16 gsm, for the insult zone 596.94 gsm, for the back overall average 626.23 gsm. The thickness of the fluid-absorbent core has an average of 3.8 mm.

The fluid-absorbent core holds 31.38 % by weight distributed water-absorbent polymer particles, the quantity of water-absorbent polymer particles within the fluid-absorbent core is 9.34 g.

The water-absorbent polymer particles derived from dropletization polymerization as described in production example 9, exhibiting the following features and absorption profile:

CRC of 41.7g/g
SFC of 3 x 10$^{-7}$cm$^3$s/g
AUHL of 26 g/g
AUL of 36 g/g
Extractables of 6 wt.%
Residual monomers of 557 ppm
Moisture content of 1.2 wt.%
FSR of 0.27 g/gs
Mean Sphericity of 0.89
PSD of 200 to 600 $\mu$m
Anticaking of 3

The water-absorbent polymer particles can be remoisturized to a moisture content of 13% by weight.

Dimension of the fluid-absorbent core: length: 40.8 cm; front width: 14.2 cm; crotch width: 14.5 cm; rear width: 14.1 cm.

An air through bonded acquisition-distribution layer between (A) and (C) having a basis weight of 35.7 gsm is rectangular shaped and smaller than the fluid-absorbent core having a size of 24.0 cm x 9.2 cm.

The fluid-absorbent article further comprises:

• flat rubber elastics; elastics from spandex type fibers: 5 cuff elastics
• leg cuffs from synthetic fibers showing the layer combination SMS and having a basis weight of between 13 to 17 gsm and a height of 4.7 cm

For improving the fit of the fluid-absorbent article, the pantiliner of embodiment 10 provides stretchable bands.

Dimension of the fluid-absorbent article: length: 47.9 cm; front width: 31.3 cm; crotch width: 15.4 cm; rear width: 31.3 cm.

Embodiment 10

A further preferred embodiment of the present invention is described in Embodiment 10 hereinafter. Thus, a preferred fluid-absorbent article comprising

(B) an upper liquid-pervious layer comprising a spunbond layer (three piece coverstock);

(C) a lower liquid-impervious layer comprising a composite of breathable polyethylene film and spunbond nonwoven;

(D) a single fluid-absorbent core between (A) and (B) comprising between 40 to 80 % by weight water-absorbent polymer particles based on the total absorbent core weight and including a multi-layered fluid-storage section comprising the following sequence:

1. a homogeneous upper core fluff layer of hydrophilic fibrous matrix of wood pulp fibers (cellulose fibers) containing about 50% of the total fluff amount;

2. a fluid-absorbent layer comprising water-absorbent polymer particles; suitable water-absorbent polymer particles for such construction having a saline flow conductivity (SFC) from about 35 to 100 x $10^{-7} cm^3 s/g$; and

(E) an air through bonded acquisition-distribution layer between (A) and (C) having a basis weight of 40 to 80 gsm; the acquisition-distribution layer is rectangular shaped and smaller than the primary fluid-absorbent core having a size of about 150 to about 250 $cm^2$.

The construction of the products chassis and the components contained therein is made and controlled by the discrete application of hotmelt adhesives as known to people skilled in the art. Examples would be e.g. Dispomelt 505B, Dispomelt Cool 1101, as well as other specific function adhesives manufactured by Bostik, Henkel or Fuller.

Ultra-thin high-loaded fluid-absorbent layers can be formed by immobilization of water-absorbent polymer particles on a non-woven sheet using hotmelt adhesives. Preferably the water-absorbent polymer particles form longitudinal strips, discrete spots or target zones. Other patterns of the water-absorbent polymer particles are also possible.

In a preferred embodiment the ultra-thin high-loaded fluid-absorbent layers comprise at least two sheets comprising immobilized water-absorbent polymer particles.

Examples of ultra-thin high-loaded fluid-absorbent layers are described in EP 1 293 187 A1, US 6,972,011, EP 1 447 066 A1, EP 1 447 067 A1, EP 1 609 448 A1, JP 2004/313580, US 2005/0137085, US 2006/0004336, US 2007/0135785 WO 2008/155699 A1, WO 2008/155701 A2, WO 2008/155702 A1, WO 2008/155710 A1, WO 2008/155711 A1, WO 2004/071363 A1, US 2003/0181115, WO 2005/097025, US 2007/156108, US 2008/0125735, and WO 2008/155722 A2, which explicitly forms part of the present disclosure.

Construction Example of Embodiment 10

The fluid-absorbent core consists of a double-layered single core system each layer having a uniform rectangular size. The layered fluid-absorbent core between (A) and (B) comprises a double-layered system of hydrophilic fibers (cellulose fibers, fluff pulp fibers), each layer having an almost uniform rectangular size. The fluid-absorbent core is encapsulated by wrapping with a spunbond material having a basis weight of 10 gsm. The density of the fluid-absorbent core is for the front overall average 0.16 $g/cm^3$, for the insult zone 0.14 $g/cm^3$, for the back overall average 0.16 $g/cm^3$. The basis weight of the fluid-absorbent core is for the front overall average 598.16 gsm, for the insult zone 596.94 gsm, for the back overall average 626.23 gsm. The thickness of the fluid-absorbent core has an average of 3.8 mm.

The fluid-absorbent core holds 59.05 % by weight distributed water-absorbent polymer particles, the quantity of water-absorbent polymer particles within the fluid-absorbent core is 11.9 g.

The water-absorbent polymer particles derived from dropletization polymerization as described in production example 20, exhibiting the following features and absorption profile:

CRC of 31.7 g/g
SFC of 41 x $10^{-7} cm^3 s/g$
AUHL of 22 g/g
AUL of 29 g/g
Extractables of 4 wt.%
Residual monomers of 421 ppm
Moisture content of 1,2 wt.%
FSR 0.30 g/gs
Mean Sphericity of 0.89
PSD of 200 to 600 $\mu$m
Anticaking of 3

Dimension of the fluid-absorbent core: length: 40.8 cm; front width: 14.2 cm; crotch width: 14.5 cm; rear width: 14.1 cm.

An air through bonded acquisition-distribution layer between (A) and (C) having a basis weight of 35.7 gsm is rectangular shaped and smaller than the fluid-absorbent core having a size of 24.0 cm x 9.2 cm.

The fluid-absorbent article further comprises:

- flat rubber elastics; elastics from spandex type fibers: 5 cuff elastics
- leg cuffs from synthetic fibers showing the layer combination SMS and having a basis weight of between 13 to 17 gsm and a height of 4.7 cm

For improving the fit of the fluid-absorbent article, the pantiliner of embodiment 10 provides stretchable bands.

Dimension of the fluid-absorbent article: length: 47.9 cm; front width: 31.3 cm; crotch width: 15.4 cm; rear width: 31.3 cm.

Embodiment 11

A further preferred embodiment of the present invention is described in Embodiment 11 hereinafter. Thus, a preferred fluid-absorbent article comprising

(A) an upper liquid-pervious layer comprising a spunbond layer (three piece coverstock);

(B) a lower liquid-impervious layer comprising a composite of breathable polyethylene film and spunbond nonwoven;

(C) a high-loaded single fluid-absorbent core between (A) and (B) comprising between 55 to 95 % by weight water-absorbent polymer particles based on the total absorbent core weight and including a multi-layered fluid-storage section comprising the following sequence:

1. a homogeneous upper core layer of hydrophilic synthetic fibers (fibrous matrix) containing about 95 % of the total fluff amount;

2. a high-loaded fluid-absorbent layer comprising water-absorbent polymer particles; suitable water-absorbent polymer particles for such construction having a saline flow conductivity (SFC) from about 50 to 150 x $10^{-7}$ cm$^3$s/g; and

(D) an air through bonded acquisition-distribution layer between (A) and (C) having a basis weight of 40 to 80 gsm; the acquisition-distribution layer is rectangular shaped and smaller than the primary fluid-absorbent core having a size of about 150 to about 250 cm$^2$.

The construction of the products chassis and the components contained therein is made and controlled by the discrete application of hotmelt adhesives as known to people skilled in the art. Examples would be e.g. Dispomelt 505B, Dispomelt Cool 1101, as well as other specific function adhesives manufactured by Bostik, Henkel or Fuller.

Ultra-thin high-loaded fluid-absorbent layers can be formed by immobilization of water-absorbent polymer particles on a non-woven sheet using hotmelt adhesives. Preferably the water-absorbent polymer particles form longitudinal strips, discrete spots or target zones. Other patterns of the water-absorbent polymer particles are also possible.

In a preferred embodiment the ultra-thin high-loaded fluid-absorbent layers comprise at least two sheets comprising immobilized water-absorbent polymer particles.

Examples of ultra-thin high-loaded fluid-absorbent layers are described in EP 1 293 187 A1, US 6,972,011, EP 1 447 066 A1, EP 1 447 067 A1, EP 1 609 448 A1, JP 2004/313580, US 2005/0137085, US 2006/0004336, US 2007/0135785 WO 2008/155699 A1, WO 2008/155701 A2, WO 2008/155702 A1, WO 2008/155710 A1, WO 2008/155711 A1, WO 2004/071363 A1, US 2003/0181115, WO 2005/097025, US 2007/156108, US 2008/0125735, and WO 2008/155722 A2, which explicitly forms part of the present disclosure.

Construction Example of Embodiment 11

The fluid-absorbent core consists of a double-layered high-loaded single core system each layer having a uniform rectangular size. The layered fluid-absorbent core between (A) and (B) comprises a double-layered system of hydrophilic fibers (synthetic fibers), each layer having a rectangular size. The fluid-absorbent core is encapsulated by wrapping with a spunbond material having a basis weight of 10 gsm. The density of the fluid-absorbent core is for the front overall average 0.20 g/cm$^3$, for the insult zone 0.20 g/cm$^3$, for the back overall average 0.21 g/cm$^3$. The basis weight of the fluid-absorbent core is for the front overall average 551.51 gsm, for the insult zone 585.71 gsm, for the back overall average 569.63 gsm. The thickness of the fluid-absorbent core has an average of 2.9 mm.

The fluid-absorbent core holds 81.6 % by weight distributed water-absorbent polymer particles, the quantity of water-absorbent polymer particles within the fluid-absorbent core is 12.9 g.

The water-absorbent polymer particles derived from dropletization polymerization as described in production example 24, exhibiting the following features and absorption profile:

CRC of 32.4g/g
SFC of 65 x $10^{-7}$cm$^3$s/g
AUHL of 24 g/g
AUL of 30 g/g
Extractables of 5 wt.%
Residual monomers of 594 ppm
Moisture content of 0.6 wt.%
FSR of 0.29 g/gs
Mean Sphericity of 0.89
PSD of 200 to 600 $\mu$m
Anticaking of 3

The water-absorbent polymer particles can be remoisturized to a moisture content of 13% by weight.

Dimension of the fluid-absorbent core: length: 40.8 cm; front width: 14.2 cm; crotch width: 14.5 cm; rear width: 14.1 cm.

An air through bonded acquisition-distribution layer between (A) and (C) having a basis weight of 35.7 gsm is rectangular shaped and smaller than the fluid-absorbent core having a size of 24.0 cm x 9.2 cm.

The fluid-absorbent article further comprises:

- flat rubber elastics; elastics from spandex type fibers: 5 cuff elastics
- leg cuffs from synthetic fibers showing the layer combination SMS and having a basis weight of between 13 to 17 gsm and a height of 4.7 cm
- wetness indicator at the lower side of the liquid-impervious layer (B)

Dimension of the fluid-absorbent article: length: 47.9 cm; front width: 31.3 cm; crotch width: 15.4 cm; rear width: 31.3 cm

Embodiment 12

A further preferred embodiment of the present invention is described in Embodiment 12 hereinafter. Thus, a preferred fluid-absorbent article comprising

(A) an upper liquid-pervious layer comprising a thermobond layer (coverstock);
(B) a lower liquid-impervious layer comprising a composite of breathable polyethylene film and spunbond nonwoven;
(C) a high-loaded single fluid-absorbent core between (A) and (B) comprising between 55 to 95 % by weight water-absorbent polymer particles based on the total absorbent core weight and including a fluid-storage section comprising a high-loaded mixed fluid-absorbent layer wrapped with a homogeneous layer of hydrophilic synthetic fibers; said high-loaded fluid-absorbent layer comprises water-absorbent polymer particles; suitable water-absorbent polymer particles for such construction having a saline flow conductivity (SFC) from about 50 to 150 x $10^{-7}$cm$^3$s/g; said homogeneous wrapping of hydrophilic synthetic fibers contains about 95 % of the total fluff amount; and
(D) an air through bonded acquisition-distribution layer between (A) and (C) having a basis weight of 40 to 80 gsm; the acquisition-distribution layer is rectangular shaped and smaller than the primary fluid-absorbent core having a size of about 150 to about 250 cm$^2$.

The construction of the products chassis and the components contained therein is made and controlled by the discrete application of hotmelt adhesives as known to people skilled in the art. Examples would be e.g. Dispomelt 505B, Dispomelt Cool 1101, as well as other specific function adhesives manufactured by Bostik, Henkel or Fuller.

Ultra-thin high-loaded fluid-absorbent layers can be formed by immobilization of water-absorbent polymer particles on a non-woven sheet using hotmelt adhesives. Preferably the water-absorbent polymer particles form longitudinal strips, discrete spots or target zones. Other patterns of the water-absorbent polymer particles are also possible.

In a preferred embodiment the ultra-thin high-loaded fluid-absorbent layers comprise at least two sheets comprising immobilized water-absorbent polymer particles.

Examples of ultra-thin high-loaded fluid-absorbent layers are described in EP 1 293 187 A1, US 6,972,011, EP 1 447 066 A1, EP 1 447 067 A1, EP 1 609 448 A1, JP 2004/313580, US 2005/0137085, US 2006/0004336, US 2007/0135785 WO 2008/155699 A1, WO 2008/155701 A2, WO 2008/155702 A1, WO 2008/155710 A1, WO 2008/155711 A1, WO 2004/071363 A1, US 2003/0181115, WO 2005/097025, US 2007/156108, US 2008/0125735, and WO 2008/155722 A2, which explicitly forms part of the present disclosure.

Construction Example of Embodiment 12

The fluid-absorbent core consists of a high-loaded single core system having a uniform rectangular size. The fluid-absorbent core between (A) and (B) comprises a fluid-storage section comprising a high-loaded fluid-absorbent layer wrapped in a homogeneous layer of hydrophilic synthetic fibers. The fluid-absorbent core is encapsulated by wrapping it both in a C-wrap and a full wrap configuration with a spunbond material having a basis weight of 10 gsm. The density of the fluid-absorbent core is for the front overall average 0.16 g/cm$^3$, for the insult zone 0.25 g/cm$^3$, for the back overall average 0.19 g/cm$^3$. The basis weight of the fluid-absorbent core is for the front overall average 436.86 gsm, for the insult zone 707.74 gsm, for the back overall average 555.73 gsm. The thickness of the fluid-absorbent core has an average of 3.0 mm.

The fluid-absorbent core holds 80.3 % by weight distributed water-absorbent polymer particles, the quantity of water-absorbent polymer particles within the fluid-absorbent core is 11.8 g.

The water-absorbent polymer particles derived from dropletization polymerization as described in production example 18, exhibiting the following features and absorption profile:

CRC of 27.6 g/g
SFC of 125 x 10$^{-7}$cm$^3$s/g
AUHL of 24 g/g
AUL of 28 g/g
Extractables of 3 wt.%
Residual monomers of 565 ppm
Moisture content of 0.8 wt.%
FSR 0.22 g/gs
Mean Sphericity of 0.89
PSD of 200 to 600 $\mu$m
Anticaking of 3

Dimension of the fluid-absorbent core: length: 40.8 cm; front width: 14.2 cm; crotch width: 14.5 cm; rear width: 14.1 cm.

An air through bonded acquisition-distribution layer between (A) and (C) having a basis weight of 35.7 gsm is rectangular shaped and smaller than the fluid-absorbent core having a size of 24.0 cm x 9.2 cm.

The fluid-absorbent article further comprises:

- flat rubber elastics; elastics from spandex type fibers: 5 cuff elastics
- leg cuffs from synthetic fibers showing the layer combination SMS and having a basis weight of between 13 to 17 gsm and a height of 4.7 cm

For improving the fit of the fluid-absorbent article, the stretchable pant of embodiment 12 provides elastics from spandex type fibers.

Dimension of the fluid-absorbent article: length: 47.9 cm; front width: 31.3 cm; crotch width: 15.4 cm; rear width: 31.3 cm.

Embodiment 13

A further preferred embodiment of the present invention is described in Embodiment 13 hereinafter. Thus, a preferred fluid-absorbent article comprising

(A) an upper liquid-pervious layer comprising a spunbonded layer (coverstock);
(B) a lower liquid-impervious layer comprising a composite of breathable polyethylene film and spunbond nonwoven;
(C) a high-loaded single fluid-absorbent core between (A) and (B) comprising between 55 to 95 % by weight water-absorbent polymer particles based on the total absorbent core weight including a fluid-storage section comprising a high-loaded fluid-absorbent layer wrapped with a spunbond material; said high-loaded fluid-absorbent layer comprises water-absorbent polymer particles; suitable water-absorbent polymer particles for such construction having a saline flow conductivity (SFC) from about 50 to 150 x 10$^{-7}$cm$^3$s/g; said homogeneous wrapping of spunbond material contains about 100 % of the total fluff amount; and
(D) a system of two acquisition-distribution layers between (A) and (C), comprising an upper resinbonded layer having a basis weight of 40 to 80 gsm; the upper acquisition-distribution layer is rectangular shaped having a size of about 150 to about 250 cm$^2$; the lower acquisition-distribution layer comprising of modified cellulose fibres having a basis weight of 40 to 80 gsm and a size of about 100 to about 300 cm$^2$; the upper acquisition-distribution layer is smaller than the lower acquisition-distribution layer; both acquisition-distribution layers are smaller than the fluid-absorbent core.

The construction of the products chassis and the components contained therein is made and controlled by the discrete application of hotmelt adhesives as known to people skilled in the art. Examples would be e.g. Dispomelt 505B, Dispomelt Cool 1101, as well as other specific function adhesives manufactured by Bostik, Henkel or Fuller.

Ultra-thin high-loaded fluid-absorbent layers can be formed by immobilization of water-absorbent polymer particles on a non-woven sheet using hotmelt adhesives. Preferably the water-absorbent polymer particles form longitudinal strips, discrete spots or target zones. Other patterns of the water-absorbent polymer particles are also possible.

In a preferred embodiment the ultra-thin high-loaded fluid-absorbent layers comprise at least two sheets comprising immobilized water-absorbent polymer particles.

Examples of ultra-thin high-loaded fluid-absorbent layers are described in EP 1 293 187 A1, US 6,972,011, EP 1 447 066 A1, EP 1 447 067 A1, EP 1 609 448 A1, JP 2004/313580, US 2005/0137085, US 2006/0004336, US 2007/0135785 WO 2008/155699 A1, WO 2008/155701 A2, WO 2008/155702 A1, WO 2008/155710 A1, WO 2008/155711 A1, WO 2004/071363 A1, US 2003/0181115, WO 2005/097025, US 2007/156108, US 2008/0125735, and WO 2008/155722 A2, which explicitly forms part of the present disclosure.

Construction Example of Embodiment 13

The fluid-absorbent core consists of a high-loaded mixed single core system having an almost uniform rectangular size. The fluid-absorbent core between (A) and (B) comprises a fluid-storage section comprising a high-loaded fluid-absorbent layer wrapped in a homogeneous layer of hydrophilic spunbond fibers having a basis weight of 10 gsm. The density of the fluid-absorbent core is for the front overall average 0.20 g/cm$^3$, for the insult zone 0.19 g/cm$^3$, for the back overall average 0.19 g/cm$^3$. The basis weight of the fluid-absorbent core is for the front overall average 1114 gsm, for the insult zone 1007 gsm, for the back overall average 658 gsm. The thickness of the fluid-absorbent core has an average of 4.5 mm.

The fluid-absorbent layer holds 67.2 % by weight distributed water-absorbent polymer particles, the quantity of water-absorbent polymer particles within the fluid-absorbent core is 14.1 g.

The water-absorbent polymer particles derived from dropletization polymerization as described in production example 20, exhibiting the following features and absorption profile:

CRC of 31.7 g/g
SFC of 41 x 10$^{-7}$cm$^3$s/g
AUHL of 22 g/g
AUL of 29 g/g
Extractables of 4 wt.%
Residual monomers of 421 ppm
Moisture content of 1.2 wt.%
FSR 0.30 g/gs
Mean Sphericity of 0.89
PSD of 200 to 600 $\mu$m
Anticaking of 3

The water-absorbent polymer particles can be remoisturized to a moisture content of 13% by weight.

Dimension of the fluid-absorbent core: length: 43.0 cm; front width: 11.5 cm; crotch width: 7.2 cm; rear width: 12.1 cm.

The upper resin bonded acquisition-distribution layer between (A) and the lower acquisition-distribution layer having a basis weight of 65.7 gsm is rectangular shaped with dimensions of 24.9 cm x 7 cm. The lower cellulose-based acquisition-distribution layer between the upper acquisition-distribution layer and (C) is rectangular shaped with dimensions of 24.9 cm x 7.5 cm. Both acquisition-distribution layers are smaller than the fluid-absorbent core.

The fluid-absorbent article further comprises:

- flat rubber elastics; elastics from spandex type fibers: 3 leg elastics and 2 cuff elastics
- leg cuffs from synthetic fibers showing the layer combination SMS and having a basis weight of between 13 to 17 gsm and a height of 3.4 cm

- mechanical closure system with landing zone of dimension 14.9 cm x 3.8 cm and adhesive closure tapes of 3.0 cm x 1.3 cm; attached to hook fastening tape of 3.0 cm x 1.3 cm

Dimension of the fluid-absorbent article: length: 50.9 cm; front width: 24.5 cm; crotch width: 24.3 cm; rear width: 24.5 cm.

Embodiment 14

A further preferred embodiment of the present invention is described in Embodiment 14 hereinafter. Thus, a preferred fluid-absorbent article comprising

(A) an upper liquid-pervious layer comprising a spunbonded layer (coverstock);
(B) a lower liquid-impervious layer comprising a composite of breathable polyethylene film and spunbond nonwoven;
(C) a high-loaded single fluid-absorbent core between (A) and (B) comprising between 55 to 95 % by weight water-absorbent polymer particles based on the total absorbent core weight including a fluid-storage section comprising a high-loaded fluid-absorbent layer wrapped with a spunbond material; said high-loaded fluid-absorbent layer comprises water-absorbent polymer particles; suitable water-absorbent polymer particles for such construction having a saline flow conductivity (SFC) from about 50 to 150 x $10^{-7}cm^3s/g$; said homogeneous wrapping of spunbond material contains about 100 % of the total fluff amount; and
(D) a system of two acquisition-distribution layers between (A) and (C), comprising an upper resinbonded layer having a basis weight of 40 to 80 gsm; the upper acquisition-distribution layer is rectangular shaped having a size of about 150 to about 250 cm$^2$; the lower acquisition-distribution layer comprising of modified cellulose fibres having a basis weight of 40 to 80 gsm and a size of about 100 to about 300 cm$^2$; the upper acquisition-distribution layer is smaller than the lower acquisition-distribution layer; both acquisition-distribution layers are smaller than the fluid-absorbent core;

The construction of the products chassis and the components contained therein is made and controlled by the discrete application of hotmelt adhesives as known to people skilled in the art. Examples would be e.g. Dispomelt 505B, Dispomelt Cool 1101, as well as other specific function adhesives manufactured by Bostik, Henkel or Fuller.

Ultra-thin high-loaded fluid-absorbent layers can be formed by immobilization of water-absorbent polymer particles on a non-woven sheet using hotmelt adhesives. Preferably the water-absorbent polymer particles form longitudinal strips, discrete spots or target zones. Other patterns of the water-absorbent polymer particles are also possible.

In a preferred embodiment the ultra-thin high-loaded fluid-absorbent layers comprise at least two sheets comprising immobilized water-absorbent polymer particles.

Examples of ultra-thin high-loaded fluid-absorbent layers are described in EP 1 293 187 A1, US 6,972,011, EP 1 447 066 A1, EP 1 447 067 A1, EP 1 609 448 A1, JP 2004/313580, US 2005/0137085, US 2006/0004336, US 2007/0135785 WO 2008/155699 A1, WO 2008/155701 A2, WO 2008/155702 A1, WO 2008/155710 A1, WO 2008/155711 A1, WO 2004/071363 A1, US 2003/0181115, WO 2005/097025, US 2007/156108, US 2008/0125735, and WO 2008/155722 A2, which explicitly forms part of the present disclosure.

Construction Example of Embodiment 14

The fluid-absorbent core consists of a high-loaded mixed single core system having an almost uniform rectangular size. The fluid-absorbent core between (A) and (B) comprises a fluid-storage section comprising a high-loaded fluid-absorbent layer wrapped in a homogeneous layer of hydrophilic spunbond fibers having a basis weight of 10 gsm. The density of the fluid-absorbent core is for the front overall average 0.25 g/cm$^3$, for the insult zone 0.25 g/cm$^3$, for the back overall average 0.26 g/cm$^3$. The basis weight of the fluid-absorbent core is for the front overall average 878.70 gsm, for the insult zone 1237.56 gsm, for the back overall average 495.60 gsm. The thickness of the fluid-absorbent core has an average of 3.1 mm.

The fluid-absorbent layer holds 100 % by weight distributed water-absorbent polymer particles, the quantity of water-absorbent polymer particles within the fluid-absorbent core is 14.14 g.

The water-absorbent polymer particles derived from dropletization polymerization as described in production example 24, exhibiting the following features and absorption profile:

CRC of 32.4g/g
SFC of 65 x $10^{-7}cm^3s/g$
AUHL of 24 g/g
AUL of 30 g/g

Extractables of 5 wt.%
Residual monomers of 594 ppm
Moisture content of 0.6 wt.%
FSR of 0.29 g/gs
Mean Sphericity of 0.89
PSD of 200 to 600 $\mu$m
Anticaking of 3

The water-absorbent polymer particles can be remoisturized to a moisture content of 13% by weight.

Dimension of the fluid-absorbent core: length: 42.4 cm; front width: 10.6 cm; crotch width: 10.2 cm; rear width: 10.5 cm.

The upper resin bonded acquisition-distribution layer between (A) and the lower acquisition-distribution layer having a basis weight of 58.8 gsm is rectangular shaped with dimensions of 24.7 cm x 7.3 cm. The lower cellulose-based acquisition-distribution layer between the upper acquisition-distribution layer and (C) is rectangular shaped with dimensions of 20.3 cm x 8.2 cm. Both acquisition-distribution layers are smaller than the fluid-absorbent core.

The fluid-absorbent article further comprises:

- flat rubber elastics; elastics from spandex type fibers: 2 leg elastics and 2 cuff elastics
- leg cuffs from synthetic fibers showing the layer combination SMS and having a basis weight of between 13 to 17 gsm and a height of 4.4 cm
- waistband: front: 13.7 cm x 2.1 cm; rear: 14.8 cm x 2.2 cm

Dimension of the fluid-absorbent article: length: 46.7 cm; front width: 33.5 cm; crotch width: 16.0 cm; rear width: 33.5 cm.

Embodiment 15

A further preferred embodiment of the present invention is described in Embodiment 15 hereinafter. Thus, a preferred fluid-absorbent article comprising

(A) an upper liquid-pervious layer comprising a spunbonded layer (three piece coverstock);
(B) a lower liquid-impervious layer comprising a composite of breathable polyethylene film and spunbond nonwoven;
(C) a high-loaded single fluid-absorbent core between (A) and (B) comprising between 95 to 100 percent by weight water-absorbent polymer particles based on the total absorbent core weight consisting of a fluid-storage section comprising two layers of homogeneous nonwoven (SMS) sandwiching water-absorbent polymer particles; said water-absorbent polymer particles are located in pockets and fixed by ultrasonic welding with varying bond strength allowing controlled increase of superabsorbent pocket volume biased to fluid movement in the distal direction; suitable water-absorbent polymer particles for such construction having a saline flow conductivity (SFC) from about 5 to 50 x 10"$^7$cm$^3$s/g; and
(D) a system of one acquisition-distribution layer between (A) and (C), comprising air through bonded layer having a basis weight of 40 to 180 gsm; the acquisition-distribution layer is rectangular shaped having a size of about 150 to about 250 cm$^2$; the acquisition-distribution layer is smaller than the fluid-absorbent core.

The construction of the products chassis and the components contained therein is made and controlled by the discrete application of hotmelt adhesives as known to people skilled in the art. Examples would be e.g. Dispomelt 505B, Dispomelt Cool 1 101, as well as other specific function adhesives manufactured by Bostik, Henkel or Fuller.

Ultra-thin high-loaded fluid-absorbent layers can be formed by immobilization of fluid- absorbent polymer particles on a non-woven sheet using ultra-sonic bonding. Preferably the water-absorbent polymer particles form longitudinal stripes or discrete spots, target zones or combinations of superabsorbent immobilization patterns. Other patterns of the water-absorbent polymer particles are also possible.

In a preferred embodiment the ultra-thin high-loaded fluid-absorbent layers comprise at least two sheets comprising immobilized water-absorbent polymer particles.

Examples of ultra-thin high-loaded fluid-absorbent layers are described in EP 1 293 187 A1, US 6,972,011, EP 1 447 066 A1, EP 1 447 067 A1, EP 1 609 448 A1, JP 2004/313580, US 2005/0137085, US 2006/0004336, US 2007/0135785 WO2008/155699 A1, WO 2008/155701 A2, WO 2008/155702 A1, WO 2008/155710 A1, WO 2008/155711 A1, WO 2004/071363 A1, US 2003/0181115, WO 2005/097025, US 2007/156108, US 2008/0125735, and WO 2008/155722 A2, which explicitly forms part of the present disclosure.

Construction Example of Embodiment 15

The upper liquid-pervious layer (A) comprising a hydrophilic spunbonded layer joined with hydrophobic barrier leg cuffs (three piece coverstock) with a basis weight of 15.2 gsm. The fluid-absorbent core consists of a high-loaded single core system having an almost uniform rectangular size.
The fluid-absorbent core between (A) and (B) comprises a fluid-storage section comprising two layers of homogeneous nonwoven (SMS) sandwiching water-absorbent polymer particles; the upper of said non-woven layers has a basis weight of 12.8 gsm, the lower of said non-woven layers has a basis weight of 11.8 gsm. Said water-absorbent polymer particles are located in pockets of 1 cm x 1 cm size in between said nonwoven layers and fixed by ultrasonic welding of 2 mm longitudinal and transverse discontinuous bond width with variable bond strength in both lateral and distal directions. The density of the fluid-absorbent core is for the front overall average 0.24 g/cm$^3$, for the insult zone 0.24g/cm$^3$, for the back overall average 0.24 g/cm$^3$. The basis weight of the fluid-absorbent core is for the front overall average 563.67 gsm, for the insult zone 703 gsm, for the back overall average 504.33 gsm. The thickness of the fluid- absorbent core has an average of 2.4 mm.

The fluid-absorbent layer holds 100 percent by weight distributed water-absorbent polymer particles, the quantity of water-absorbent polymer particles within the fluid-absorbent core is 16.9 g.
The water-absorbent polymer particles derived from dropletization polymerization as described in production example 25, exhibiting the following features and absorption profile:

CRC of 35.0g/g
SFC of 24 x 10$^{-7}$cm$^3$s/g
AULof 32 g/g
AUHL of 23 g/g
Extractables of 7 wt.%
Residual monomers of 684 ppm
Moisture content of 0.7 wt.%
FSR 0.30 g/gs
PSD of 150 to 600 $\mu$m
Sphericity 0.89
Hunter-Color 60 of 72.0 (L = 92.5, a = -0.72, b = 6.84)

The water-absorbent polymer particles can be re-moisturized to a moisture content of 13 percent by weight. Dimension of the fluid-absorbent core: length: 38.5 cm; width: 12.5 cm;
The fluid-absorbent article holds an air through bonded system of one acquisition-distribution layer ADL between (A) and (C), comprising 100% synthetic fibers. The acquisition-distribution layer ADL between (A) and (C) having a basis weight of 160.4 gsm is rectangular shaped with dimensions of 30.0 cm x 11.5 cm. The user facing surface of the acquisition-distribution layer ADL has a lofty open higher denier fibre structure for rapid fluid acquisition, whereas the garment facing surface (of the user) of the acquisition-distribution layer ADL shows a denser network of lower denier fibre for improved fluid wicking. The acquisition-distribution layer is smaller than the fluid-absorbent core.
The fluid-absorbent article further comprises:

* flat rubber elastics; elastics from spandex type fibers: 3 leg elastics and 1 cuff elastics
* leg cuffs from synthetic fibers showing the layer combination SMS and having a basis weight of between 10 to 13 gsm and a height of 4.2 cm

Dimension of the fluid-absorbent article: length: 47.2 cm; front width: 32.6 cm; crotch width: 22.5 cm; rear width: 34.0 cm.
The water-absorbent polymer particles and the fluid-absorbent articles are tested by means of the test methods described below.

Embodiment 16

A further preferred embodiment of the present invention is described in Embodiment 16 hereinafter. Thus, a preferred fluid-absorbent article comprising

(A) an upper liquid-pervious layer comprising a spunbonded layer (three piece coverstock);
(B) a lower liquid-impervious layer comprising a composite of breathable polyethylene film and spunbond nonwoven;
(C) a high-loaded single fluid-absorbent core between (A) and (B) comprising between 95 to 100 percent by weight water-absorbent polymer particles based on the total absorbent core weight consisting of a fluid-storage section comprising two layers of homogeneous nonwoven (SMS) sandwiching water-absorbent polymer particles; said water-absorbent polymer particles are located in pockets and fixed by ultrasonic welding with varying bond strength allowing controlled increase of superabsorbent pocket volume biased to fluid movement in the distal direction; suitable water-absorbent polymer particles for such construction having a saline flow conductivity (SFC) from about 0 to 50 x $10^{"7}$cm$^3$s/g; ; and;

(D) a system of one acquisition-distribution layer between (A) and (C), comprising air through bonded layer having a basis weight of 40 to 180 gsm; the acquisition-distribution layer is rectangular shaped having a size of about 150 to about 250 cm$^2$;; the acquisition-distribution layer is smaller than the fluid-absorbent core;

The construction of the products chassis and the components contained therein is made and controlled by the discrete application of hotmelt adhesives as known to people skilled in the art. Examples would be e.g. Dispomelt 505B, Dispomelt Cool 1 101, as well as other specific function adhesives manufactured by Bostik, Henkel or Fuller.

Ultra-thin high-loaded fluid-absorbent layers can be formed by immobilization of fluid- absorbent polymer particles on a non-woven sheet using ultra-sonic bonding. Preferably the water-absorbent polymer particles form longitudinal stripes or discrete spots, target zones or combinations of superabsorbent immobilization patterns. Other patterns of the water-absorbent polymer particles are also possible.

In a preferred embodiment the ultra-thin high-loaded fluid-absorbent layers comprise at least two sheets comprising immobilized water-absorbent polymer particles.

Examples of ultra-thin high-loaded fluid-absorbent layers are described in EP 1 293 187 A1, US 6,972,011, EP 1 447 066 A1, EP 1 447 067 A1, EP 1 609 448 A1, JP 2004/313580, US 2005/0137085, US 2006/0004336, US 2007/0135785 WO2008/155699 A1, WO 2008/155701 A2, WO 2008/155702 A1, WO 2008/155710 A1, WO 2008/15571 1 A1, WO 2004/071363 A1, US 2003/01811 15, WO 2005/097025, US 2007/156108, US 2008/0125735, and WO 2008/155722 A2, which explicitly forms part of the present disclosure.

Construction Example of Embodiment 16

The upper liquid-pervious layer (A) comprising a hydrophilic spunbonded layer joined with hydrophobic barrier leg cuffs (three piece coverstock) with a basis weight of 15.2 gsm. The fluid-absorbent core consists of a high-loaded single core system having an almost uniform rectangular size.

The fluid-absorbent core between (A) and (B) comprises a fluid-storage section comprising two layers of homogeneous nonwoven (SMS) sandwiching water-absorbent polymer particles; the upper of said non-woven layers has a basis weight of 12.8 gsm, the lower of said non-woven layers has a basis weight of 11.8 gsm. Said water-absorbent polymer particles are located in pockets of 1 cm x 1 cm size in between said nonwoven layers and fixed by ultrasonic welding of 2 mm longitudinal and transverse discontinuous bond width with variable bond strength in both lateral and distal directions. The density of the fluid-absorbent core is for the front overall average 0.24 g/cm$^3$, for the insult zone 0.24g/cm$^3$, for the back overall average 0.24 g/cm$^3$. The basis weight of the fluid-absorbent core is for the front overall average 563.67 gsm, for the insult zone 703 gsm, for the back overall average 504.33 gsm. The thickness of the fluid- absorbent core has an average of 2.4 mm.

The fluid-absorbent layer holds 100 percent by weight distributed water-absorbent polymer particles, the quantity of water-absorbent polymer particles within the fluid-absorbent core is 13.4 g.

The water-absorbent polymer particles derived from dropletization polymerization as described in production example 11, exhibiting the following features and absorption profile:

CRC of 40.8g/g
SFC of 1.0 x $10^{-7}$cm$^3$s/g
AUL of 33 g/g
AUHL of 21 g/g
Extractables of 4 wt.%
Residual monomers of 282 ppm
Moisture content of 5.1 wt.%
FSR 0.39 g/gs
PSD of 150 to 600 $\mu$m
Sphericity 0.89
Hunter-Color 60 of 72.0 (L = 92.5, a = -0.72), b = 6.84)

The water-absorbent polymer particles were re-moisturized to a moisture content of 13 percent by weight.
Dimension of the fluid-absorbent core: length: 38.5 cm; width: 12.5 cm;

The fluid-absorbent article holds an air through bonded system of one acquisition-distribution layer ADL between (A) and (C), comprising 100% synthetic fibers. The acquisition-distribution layer ADL between (A) having a basis weight of 160.4 gsm is rectangular shaped with dimensions of 30.0 cm x 11.5 cm. The user facing surface of the acquisition-distribution layer ADL has a lofty open higher denier fibre structure for rapid fluid acquisition, whereas the garment facing surface (of the user) of the acquisition-distribution layer ADL shows a denser network of lower denier fibre for improved fluid wicking. The acquisition-distribution layer is smaller than the fluid-absorbent core.

The fluid-absorbent article further comprises:

- flat rubber elastics; elastics from spandex type fibers: 3 leg elastics and 1 cuff elastics
- leg cuffs from synthetic fibers showing the layer combination SMS and having a basis weight of between 10 to 13 gsm and a height of 4.2 cm

Dimension of the fluid-absorbent article: length: 47.2 cm; front width: 32.6 cm; crotch width: 22.5 cm; rear width: 34.0 cm.

The water-absorbent polymer particles and the fluid-absorbent articles are tested by means of the test methods described below.

Embodiment 17

A further preferred embodiment of the present invention is described in Embodiment 17 hereinafter. Thus, a preferred fluid-absorbent article comprising

(A) an upper liquid-pervious layer comprising a spunbonded layer (three piece coverstock);
(B) a lower liquid-impervious layer comprising a composite of breathable polyethylene film and spunbond nonwoven;
(C) a high-loaded single fluid-absorbent core between (A) and (B) comprising between 95 to 100 percent by weight water-absorbent polymer particles based on the total absorbent core weight consisting of a fluid-storage section comprising two layers of homogeneous nonwoven (SMS) sandwiching water-absorbent polymer particles; said water-absorbent polymer particles are located in pockets and fixed by ultrasonic welding with varying bond strength allowing controlled increase of superabsorbent pocket volume biased to fluid movement in the distal direction; suitable water-absorbent polymer particles for such construction having a saline flow conductivity (SFC) from about 5 to 50 x 10"$^7$cm$^3$s/g; and;
(D) a system of one acquisition-distribution layer between (A) and (C), comprising air through bonded layer having a basis weight of 40 to 180 gsm; the acquisition-distribution layer is rectangular shaped having a size of about 150 to about 250 cm$^2$; the acquisition-distribution layer is smaller than the fluid-absorbent core;

The construction of the products chassis and the components contained therein is made and controlled by the discrete application of hotmelt adhesives as known to people skilled in the art. Examples would be e.g. Dispomelt 505B, Dispomelt Cool 1 101, as well as other specific function adhesives manufactured by Bostik, Henkel or Fuller.

Ultra-thin high-loaded fluid-absorbent layers can be formed by immobilization of fluid- absorbent polymer particles on a non-woven sheet using ultra-sonic bonding. Preferably the water-absorbent polymer particles form longitudinal stripes or discrete spots, target zones or combinations of superabsorbent immobilization patterns. Other patterns of the water-absorbent polymer particles are also possible.

In a preferred embodiment the ultra-thin high-loaded fluid-absorbent layers comprise at least two sheets comprising immobilized water-absorbent polymer particles.

Examples of ultra-thin high-loaded fluid-absorbent layers are described in EP 1 293 187 A1, US 6,972,011, EP 1 447 066 A1, EP 1 447 067 A1, EP 1 609 448 A1, JP 2004/313580, US 2005/0137085, US 2006/0004336, US 2007/0135785 WO2008/155699 A1, WO 2008/155701 A2, WO 2008/155702 A1, WO 2008/155710 A1, WO 2008/15571 1 A1, WO 2004/071363 A1, US 2003/01811 15, WO 2005/097025, US 2007/156108, US 2008/0125735, and WO 2008/155722 A2, which explicitly forms part of the present disclosure.

Construction Example of Embodiment 17

The upper liquid-pervious layer (A) comprising a hydrophilic spunbonded layer joined with hydrophobic barrier leg cuffs (three piece coverstock) with a basis weight of 15.2 gsm.

The fluid-absorbent core consists of a high-loaded single core system having an almost uniform rectangular size.

The fluid-absorbent core between (A) and (B) comprises a fluid-storage section comprising two layers of homogeneous nonwoven (SMS) sandwiching water-absorbent polymer particles; the upper of said non-woven layers has a basis weight of 12.8 gsm, the lower of said non-woven layers has a basis weight of 11.8 gsm. Said water-absorbent polymer particles are located in pockets of 1 cm x 1 cm size in between said nonwoven layers and fixed by ultrasonic welding of 2 mm longitudinal and transverse discontinuous bond width with variable bond strength in both lateral and distal directions. The density of the fluid-absorbent core is for the front overall average 0.24 g/cm$^3$, for the insult zone 0.24g/cm$^3$, for the back overall average 0.24 g/cm$^3$. The basis weight of the fluid-absorbent core is for the front overall average 563.67 gsm, for the insult zone 703 gsm, for the back overall average 504.33 gsm. The thickness of the fluid- absorbent core has an average of 2.4 mm.

The fluid-absorbent layer holds 100 percent by weight distributed water-absorbent polymer particles; the quantity of water-absorbent polymer particles within the fluid-absorbent core is 16.9 g.

The water-absorbent polymer particles are market available products:

> CRC of 32.3g/g
> SFC of 9.4 x 10$^{-7}$cm$^3$s/g
> AUL of 31 g/g
> AUHL of 24.2 g/g
> Extractables of 13.7 wt. %
> Residual monomers of 408 ppm
> Moisture content of 2.9 wt. %
> FSR 0.19 g/gs
> PSD of 150 to 600 $\mu$m
> Sphericity 0.89
> Hunter-Color 60 of 72.0 (L = 92.5, a = -0.72), b = 6.84)

The water-absorbent polymer particles were re-moisturized to a moisture content of 13 percent by weight.
Dimension of the fluid-absorbent core: length: 38.5 cm; width: 12.5 cm;

The fluid-absorbent article holds an air through bonded system of one acquisition-distribution layer ADL between (A) and (C), comprising 100% synthetic fibers. The acquisition-distribution layer ADL between (A) having a basis weight of 160.4 gsm is rectangular shaped with dimensions of 30.0 cm x 11.5 cm. The user facing surface of the acquisition-distribution layer ADL has a lofty open higher denier fibre structure for rapid fluid acquisition, whereas the garment facing surface (of the user) of the acquisition-distribution layer ADL shows a denser network of lower denier fibre for improved fluid wicking. The acquisition-distribution layer is smaller than the fluid-absorbent core.

The fluid-absorbent article further comprises:

- flat rubber elastics; elastics from spandex type fibers: 3 leg elastics and 1 cuff elastics
- leg cuffs from synthetic fibers showing the layer combination SMS and having a basis weight of between 10 to 13 gsm and a height of 4.2 cm.

Dimension of the fluid-absorbent article: length: 47.2 cm; front width: 32.6 cm; crotch width: 22.5 cm; rear width: 34.0 cm.

The water-absorbent polymer particles and the fluid-absorbent articles are tested by means of the test methods described below.

Embodiment 18

A further preferred embodiment of the present invention is described in Embodiment 16 hereinafter. Thus, a preferred fluid-absorbent article comprising

(A) an upper liquid-pervious layer comprising a spunbond web (three piece coverstock);
(B) a lower liquid-impervious layer comprising a composite of breathable poly- ethylene film and spunbond nonwoven;
(C) a single fluid-absorbent core between (A) and (B) comprising a mixture of wood pulp fibers (cellulose fibers) and between 10 to 80 percent by weight water-absorbent polymer particles within the fluid- absorbent core (C) showing a profiled geometrical distribution located asymmetrically with majority of the profile towards the front distal edge as worn by the user;
suitable water-absorbent polymer particles for such construction having a centrifuge retention capacity (CRC) from about 32 to 60 g/g.
The content of water-absorbent polymer particles can be calculated according to the following equation (I):

$$(A_{total} - A_{profiled}) \times Basisweight + Loading\text{-}Factor \times A_{profiled} \times Basisweight = W_{SAP}$$

(I)

With $A_{total}$ = Area of the whole fluid-absorbent core (C) in $m^2$,
$A_{profiled}$ = Area with different loading $m^2$,
Basisweight = the basis weight of the fluid-absorbent core in $g/m^2$
L = Loading-Factor (e.g. 2 means double loading, 3 means threefold loading) and
$W_{SAP}$ = total content of water-absorbent polymer particles within the fluid-absorbent core

(D) an air-through bonded acquisition-distribution layer between (A) and (C) having a basis weight of 30 to 100 gsm; the acquisition-distribution layer is rectangular shaped and smaller than the fluid-absorbent core having a size of about 150 to about 250 $cm^2$.

The construction of the products chassis and the components contained therein is made and controlled by the discrete application of hotmelt adhesives as known to people skilled in the art. Examples would be e.g. Dispomelt 505B, Dispomelt Cool 1 101, as well as other specific function adhesives manufactured by Bostik, Henkel or Fuller.
Ultra-thin high-loaded fluid-absorbent layers can be formed by immobilization of fluid- absorbent polymer particles on a non-woven sheet using hot-melt adhesives. Preferably the water-absorbent polymer particles form homogeneously with a discrete target zone. Other patterns of the water-absorbent polymer particles are also possible.
In a preferred embodiment the ultra-thin high-loaded fluid-absorbent layers comprise at least two distinct superabsorbent basis weight of immobilized water-absorbent polymer particles.
Examples of ultra-thin high-loaded fluid-absorbent layers are described in EP 1 293 187 A1, US 6,972,01 1, EP 1 447 066 A1, EP 1 447 067 A1, EP 1 609 448 A1, JP 2004/313580, US 2005/0137085, US 2006/0004336, US 2007/0135785 WO 2008/155699 A1, WO 2008/155701 A2, WO 2008/155702 A1, WO 2008/155710 A1, WO 2008/15571 1 A1, WO 2004/071363 A1, US 2003/0181 115, WO 2005/097025, US 2007/156108, US 2008/0125735, and WO 2008/155722 A2, which explicitly forms part of the present disclosure.

Construction Example of Embodiment 18

The fluid-absorbent core consists of a single fluid-absorbent Core between (A) and (B) comprising a mixture of wood pulp fibers (cellulose fibers) and 37.1 percent by weight water-absorbent polymer particles profiled distributed within the fluid-absorbent core (C) having a uniform rectangular size.
The profiled structure of the distribution of the water-absorbent polymer particles results from using a technology which is written in detail in WO2010103453, US 2013/0174959A1 and US 8,123,147B2 which is incorporated herein by reference. Using the described units for making the absorbent core, it is possible to apply discrete quantities of water-absorbent polymer particles to a circumscribed area of precise geometrical shape. Thus, in the case of Embodiment 16, there results a profiled content of water-absorbent polymer particles between the insult zone of defined geometrical shape and the outer regions using this technique. The insult zone has the double loading with water-absorbent polymer particles compared to the outer region. The geometrical shape is rectangular with dimensions of 25.0 cm x 8.0 cm to a factor of 2 of superabsorbent particles.
Calculated from the equation (I) above, there results with the parameters: basis weight = 180.0 gsm, $A_{total}$ = 0.04 m2, $A_{profiled}$ = 0.02 m2, L = 2, a quantity of water-absorbent polymer particles within the fluid-absorbent core of 10.8 g.
The total fluff pulp weight is 11 g. The density of the fluid-absorbent core is for the front overall average 0.22 $g/cm^3$, for the back overall average 0.19 $g/cm^3$. The basis weight of the fluid-absorbent core is for the front overall average 940 gsm, for the insult zone 1027 gsm, for the back overall average 610 gsm.
The water-absorbent polymer particles derived from dropletization polymerization as described in production example 24, exhibiting the following features and absorption profile:

CRC of 32.4 g/g
SFC of 65 x $10^{-7} cm^3 s/g$
AUHL of 24 g/g
AUL of 30 g/g
Extractables of 5 wt.%
Residual monomers of 594 ppm
Moisture content of 0.6 weight percent
FSR of 0.29 g/gs
PSD of 200 to 600 $\mu$m
Sphericity 0.89
Anticaking of 3

The water-absorbent polymer particles can be remoisturized to a moisture content of 13 percent by weight.

Dimension of the fluid-absorbent core: length: 40.2 cm; width: 11.3 cm with crotch width narrowed to 8.5cm. The thickness of the fluid-absorbent core has an average of 3.9 mm.

An air through bonded acquisition-distribution layer between (A) and (C) having a basis weight of 90 gsm is rectangular shaped with dimensions of 24.4 cm x 8.6 cm and smaller than the fluid-absorbent core.

The fluid-absorbent article further comprises:

- flat rubber elastics; elastics from spandex type fibers: 3 leg elastics and 1 cuff elastics • leg cuffs from synthetic fibers showing the layer combination SMS and having a basis weight of between 8 to 13 gsm and a height of 3.3 cm
- mechanical closure system with landing zone of dimension 13.9 cm x 3.8 cm and flexi-band closure ear of 6.5 x 6.5 cm; attached to hook fastening tape of 1.3 cm x 3.1 cm • Dimension of the fluid-absorbent article: length: 47.5 cm; front width: 23.3 cm; crotch width: 23.3 cm; rear width: 23.3 cm.

Embodiment 19

A further preferred embodiment of the present invention is described in Embodiment 19 hereinafter. Thus, a preferred fluid-absorbent article comprising

(A) an upper liquid-pervious layer comprising a spunbond web (three piece coverstock);
(B) a lower liquid-impervious layer comprising a composite of breathable polyethylene film and spunbond nonwoven;
(C) a high-loaded single fluid-absorbent core between (A) and (B) comprising between 55 to 95 percent by weight water-absorbent polymer particles based on the total absorbent core weight including a fluid-storage section comprising a high-loaded fluid-absorbent layer wrapped with a spunbond material; said high-loaded fluid-absorbent layer comprises water-absorbent polymer particles; suitable water-absorbent polymer particles for such construction having a saline flow conductivity (SFC) from about 10 to 150 x 10"$^7$cm$^3$s/g; said homogeneous wrapping of spunbond material contains about 100 percent of the total fluff amount; and
(D) an liquid-pervious layer comprising a synthetic resin film between (A) and (C), acting as an distribution layer and quickly transporting the supplied urine along the surface to the upper lateral portion of the fluid-absorbent core (C). Preferably, the upper liquid-pervious layer (D) is smaller than the under-laying fluid-absorbent core (C). Preferably, the upper liquid-impervious layer (D) comprises a porous polyethylene film for both quick acquisition and distribution of liquid.

Said distribution layer is rectangular shaped having a size of about 150 to about 250 cm$^2$ and preferably smaller than the fluid-absorbent core.

The construction of the products chassis and the components contained therein is made and controlled by the discrete application of hotmelt adhesives as known to people skilled in the art. Examples would be e.g. Dispomelt 505B, Dispomelt Cool 1 101, as well as other specific function adhesives manufactured by Bostik, Henkel or Fuller.

Ultra-thin high-loaded fluid-absorbent layers can be formed by immobilization of fluid- absorbent polymer particles on a non-woven sheet using hot-melt adhesives. Preferably the water-absorbent polymer particles form longitudinal strips, discrete spots and/or target zones. Other patterns of the water-absorbent polymer particles are also possible.

In a preferred embodiment the ultra-thin high-loaded fluid-absorbent layers comprise at least two sheets comprising immobilized water-absorbent polymer particles.

Examples of ultra-thin high-loaded fluid-absorbent layers are described in EP 1 293 187 A1, US 6,972,011 , EP 1 447 066 A1, EP 1 447 067 A1, EP 1 609 448 A1, JP 2004/313580, US 2005/0137085, US 2006/0004336, US 2007/0135785 WO 2008/155699 A1, WO 2008/155701 A2, WO 2008/155702 A1, WO 2008/155710 A1, WO 2008/15571 1 A1, WO 2004/071363 A1, US 2003/0181 115, WO 2005/097025, US 2007/156108, US 2008/0125735, and WO 2008/155722 A2, which explicitly forms part of the present disclosure.

Construction Example of Embodiment 19

The fluid-absorbent core consists of a high-loaded mixed single core system having an almost uniform rectangular size. The fluid-absorbent core between (A) and (B) comprises a fluid-storage section comprising a high-loaded fluid-absorbent layer wrapped in a homogeneous layer of hydrophilic spun-bond fibers having a basis weight of 10 gsm. The density of the fluid-absorbent core is for the front overall average 0.20 g/cm$^3$, for the insult zone 0.19 g/cm$^3$, for the back overall average 0.19 g/cm$^3$. The basis weight of the fluid-absorbent core is for the front overall average 1114 gsm, for the insult zone 1007 gsm, for the back overall average 658 gsm. The thickness of the fluid-absorbent core has an average of 4.5 mm.

The fluid-absorbent layer consists of 67.2 percent by weight distributed water-absorbent polymer particles, the quantity of water-absorbent polymer particles within the fluid-absorbent core is 14.1 g.

The water-absorbent polymer particles derived from dropletization polymerization as described production example 24, exhibiting the following features and absorption profile:

CRC of 32.4 g/g
SFC of 65 x $10^{-7}$ cm$^3$s/g
AUHL of 24 g/g
AUL of 30 g/g
Extractables of 5 wt.%
Residual monomers of 594 ppm
Moisture content of 0.6 weight percent
FSR 0.29 g/gs
PSD of 200 to 600 $\mu$m
Sphericity 0.89
Anticaking of 3

The water-absorbent polymer particles can be re-moisturized to a moisture content of 13 percent by weight. Dimension of the fluid-absorbent core: length: 43.0 cm; front width: 1 1.5 cm; crotch width: 7.2 cm; rear width: 12.1 cm.

(D) The liquid-impervious layer comprising a single-layer synthetic resin film between (A) and (C), comprises a porous polyethylene film manufactured by Tredegar Co and marketed as T10650-1 TEC.

Examples for polyethylene films are disclosed in US 6,953,510, US 2001/0008676 A1, WO 96/14038 A and EP 1 570 824 B1 which explicitly forms part of the present disclosure.

The distribution layer between (A) and the fluid-absorbent core is rectangular shaped with dimensions of 24.9 cm x 7 cm and thus smaller than the fluid-absorbent core.

The fluid-absorbent article further comprises:

- flat rubber elastics; elastics from spandex type fibers: 3 leg elastics and 2 cuff elastics
- leg cuffs from synthetic fibers showing the layer combination SMS and having a basis weight of between 13 to 17 gsm and a height of 3.4 cm
- mechanical closure system with landing zone of dimension 14.9 cm x 3.8 cm and adhesive closure tapes of 3.0 cm x 1.3 cm; attached to hook fastening tape of 3.0 cm x 1.3 cm Dimension of the fluid-absorbent article: length: 50.9 cm; front width: 24.5 cm; crotch width: 24.3 cm; rear width: 24.5 cm.

Embodiment 20

A further preferred embodiment of the present invention is described in Embodiment 20 hereinafter.

Thus, a preferred fluid-absorbent article comprising

(A) an upper liquid-pervious layer comprising a spunbond web (three piece coverstock);
(B) a lower liquid-impervious layer comprising a composite of breathable polyethylene film and spunbond nonwoven;
(C) a high-loaded single fluid-absorbent core between (A) and (B) comprising between 55 to 95 percent by weight water-absorbent polymer particles based on the total absorbent core weight including a fluid-storage section comprising a high-loaded fluid-absorbent layer wrapped with a spunbond material; said high-loaded fluid-absorbent layer comprises water-absorbent polymer particles; suitable water-absorbent polymer particles for such construction having a saline flow conductivity (SFC) from about 0 to 80 x $10^{-7}$ cm$^3$s/g; said homogeneous wrapping of spunbond material contains about 100 percent of the total fluff amount; and
(D) an liquid-pervious layer comprising a synthetic resin film between (A) and (C), acting as an distribution layer and quickly transporting the supplied urine along the surface to the upper lateral portion of the fluid-absorbent core (C). Preferably, the upper liquid-pervious layer (D) comprises a spunbond polypropylene nonwoven for both quick acquisition and distribution of liquid.

Said distribution layer is rectangular shaped having a size of about 150 to about 250 cm$^2$ and preferably smaller than the fluid-absorbent core.

The construction of the products chassis and the components contained therein is made and controlled by the discrete application of hotmelt adhesives as known to people skilled in the art. Examples would be e.g. Dispomelt 505B, Dispomelt Cool 1 101, as well as other specific function adhesives manufactured by Bostik or Henkel.

Ultra-thin high-loaded fluid-absorbent layers can be formed by immobilization of fluid- absorbent polymer particles on a non-woven sheet using hotmelt adhesives. Preferably the water-absorbent polymer particles form longitudinal strips or discrete spots. Other patterns of the water-absorbent polymer particles are also possible.

In a preferred embodiment the ultra-thin high-loaded fluid-absorbent layers comprise at least two sheets comprising immobilized water-absorbent polymer particles.

Examples of ultra-thin high-loaded fluid-absorbent layers are described in EP 1 293 187 A1, US 6,972,01 1, EP 1 447 066 A1, EP 1 447 067 A1, EP 1 609 448 A1, JP 2004/313580, US 2005/0137085, US 2006/0004336, US 2007/0135785 WO 2008/155699 A1, WO 2008/155701 A2, WO 2008/155702 A1, WO 2008/155710 A1, WO 2008/15571 1 A1, WO 2004/071363 A1, US 2003/0181 115, WO 2005/097025, US 2007/156108, US 2008/0125735, and WO 2008/155722 A2, which explicitly forms part of the present disclosure.

Construction Example of Embodiment 20

The fluid-absorbent core consists of a medium-loaded mixed single core system having an almost uniform rectangular size. The fluid-absorbent core between (A) and (B) comprises a fluid-storage section comprising a fluid-absorbent layer wrapped in a homogeneous layer of hydrophilic spunbond fibers having a basis weight of 10 gsm. The density of the fluid-absorbent core is for the front overall average 0.20 g/cm$^3$, for the insult zone 0.19 g/cm$^3$, for the back overall average 0.19 g/cm$^3$. The basis weight of the fluid-absorbent core is for the front overall average 920 gsm, for the insult zone 942 gsm, for the back overall average 658 gsm. The thickness of the fluid-absorbent core has an average of 4.5 mm.

The fluid-absorbent layer holds 41.3 percent by weight distributed water-absorbent polymer particles; the quantity of water-absorbent polymer particles within the fluid-absorbent core is 11.2 g.

The water-absorbent polymer particles derived from dropletization polymerization as described in production example 11, exhibiting the following features and absorption profile:

CRC of 40.8 g/g
SFC of 1 x 10$^{-7}$cm$^3$s/g
AUHL of 21 g/g
AUL of 33 g/g
Extractables of 4 wt.%
Residual monomers of 282 ppm
Moisture content of 5.1 wt.%
FSR 0.39 g/gs
PSD of 200 to 600 $\mu$m
Sphericity 0.89
Anticaking of 3

The water-absorbent polymer particles can be remoisturized to a moisture content of 13 percent by weight. Dimension of the fluid-absorbent core: length: 43.0 cm; front width: 11.5 cm; crotch width: 7.2 cm; rear width: 12.1 cm.

The liquid-impervious layer material (D) between (A) and (C) is a spun-bond polypropylene ("SBPP") nonwoven commercially available from Polymer Group Inc ("PGI") of Charlotte, N.C. (USA).

Examples of spun-bond nonwovens are disclosed in US 2011/0144610, US 2013/165886, US 7,642,398 B2, US 7,919,169 B2, US 7,105,716 B, which explicitly forms part of the present disclosure.

The distribution layer between (A) and the fluid-absorbent core is rectangular shaped with dimensions of 24.9 cm x 7 cm and thus smaller than the fluid-absorbent core.

The fluid-absorbent article further comprises:

- flat rubber elastics; elastics from spandex type fibers: 3 leg elastics and 2 cuff elastics
- leg cuffs from synthetic fibers showing the layer combination SMS and having a basis weight of between 7 to 17 gsm and a height of 3.4 cm
- mechanical closure system with landing zone of dimension 14.9 cm x 3.8 cm and adhesive closure tapes of 3.0 cm x 1.3 cm; attached to hook fasten ing tape of 3.0 cm x 1.3 cm Dimension of the fluid-absorbent article: length: 50.9 cm; front width: 24.5 cm; crotch width: 24.3 cm; rear width: 24.5 cm.

Embodiment 21

A further preferred embodiment of the present invention is described in Embodiment 21 hereinafter. Thus, a preferred fluid-absorbent article comprising

(A) an upper liquid-pervious layer comprising a spunbonded layer (coverstock);
(B) a lower liquid-impervious layer comprising a composite of breathable polyethylene film and spunbond nonwoven;

(C) a high-loaded single fluid-absorbent core between (A) and (B) comprising between 25 to 65 percent by weight water-absorbent polymer particles based on the total absorbent core weight including a fluid-storage section comprising a high-loaded fluid-absorbent layer wrapped with a spunbond material; said high-loaded fluid-absorbent layer comprises water-absorbent polymer particles; suitable water-absorbent polymer particles for such construction having a saline flow conductivity (SFC) from about 50 to 150 x 10"[7]$cm^3$s/g; said homogeneous wrapping of spunbond material contains about 100 percent of the total fluff amount; and

(D) an liquid-pervious layer between (A) and (C), acting as an acquisition-distribution layer and quickly transporting the supplied urine to the fluid-absorbent core, comprising a bundle of continuous fibers, having the desired fiber distribution and being bonded to the fluid-absorbent core in a bonding pattern, but without having bondings between the fibers. Preferably, the liquid-pervious layer (D) comprises polyethylene fibers or viscose or rayon or mixtures thereof.

Examples for such layers are disclosed in US 2006/0258999 A1 and US 2005/101929, which explicitly forms part of the present disclosure.

The construction of the products chassis and the components contained therein is made and controlled by the discrete application of hotmelt adhesives as known to people skilled in the art. Examples would be e.g. Dispomelt 505B, Dispomelt Cool 1 101, as well as other specific function adhesives manufactured by Bostik, Henkel or Fuller.

Ultra-thin high-loaded fluid-absorbent layers can be formed by immobilization of fluid- absorbent polymer particles on a non-woven sheet using hotmelt adhesives. Preferably the water-absorbent polymer particles form longitudinal strips, discrete spots and/or target zones. Other patterns of the water-absorbent polymer particles are also possible.

In a preferred embodiment the ultra-thin high-loaded fluid-absorbent layers comprise at least two sheets comprising immobilized water-absorbent polymer particles.

Examples of ultra-thin high-loaded fluid-absorbent layers are described in EP 1 293 187 A1, US 6,972,01 1, EP 1 447 066 A1, EP 1 447 067 A1, EP 1 609 448 A1, JP 2004/313580, US 2005/0137085, US 2006/0004336, US 2007/0135785 WO 2008/155699 A1, WO 2008/155701 A2, WO 2008/155702 A1, WO 2008/155710 A1, WO 2008/15571 1 A1, WO 2004/071363 A1, US 2003/0181 115, WO 2005/097025, US 2007/156108, US 2008/0125735, and WO 2008/155722 A2, which explicitly forms part of the present disclosure.

Construction Example of Embodiment 21

The fluid-absorbent core consists of a high-loaded mixed single core system having an almost uniform rectangular size. The fluid-absorbent core between (A) and (B) comprises a fluid-storage section comprising a high-loaded fluid-absorbent layer wrapped in a homogeneous layer of hydrophilic spunbond fibers having a basis weight of 10 gsm. The density of the fluid-absorbent core is for the front overall average 0.20 $g/cm^3$, for the insult zone 0.19 $g/cm^3$, for the back overall average 0.19 $g/cm^3$. The basis weight of the fluid-absorbent core is for the front overall average 1114 gsm, for the insult zone 1007 gsm, for the back overall average 658 gsm. The thickness of the fluid-absorbent core has an average of 4.5 mm. The fluid-absorbent layer holds 67.2 percent by weight distributed water-absorbent polymer particles, the quantity of water-absorbent polymer particles within the fluid-absorbent core is 14.1 g.

The water-absorbent polymer particles derived from dropletization polymerization as described in production example 24, exhibiting the following features and absorption profile:

CRC of 32.4 g/g
SFC of 65 x 10[-7]$cm^3$s/g
AUHL of 24 g/g
AUL of 30 g/g
Extractables of 5 wt.%
Residual monomers of 594 ppm
Moisture content of 0.6 weight percent
FSR 0.29 g/gs
PSD of 200 to 600 $\mu$m
Sphericity 0.89
Anticaking of 3

The water-absorbent polymer particles can be remoisturized to a moisture content of 13 percent by weight.

Dimension of the fluid-absorbent core: length: 43.0 cm; front width: 11.5 cm; crotch width: 7.2 cm; rear width: 12.1 cm.

The liquid-pervious layer (D) comprises polyethylene fibers. A bundle of continuous fibers is separated and evened to a layer having a homogeneous distribution. The desired bonding to the underlaying fluid-absorbent core takes place by thermobonding at temperatures of about 140 degree. The fibers are substantially unbonded to each other.

The fluid-absorbent article further comprises:

- flat rubber elastics; elastics from spandex type fibers: 3 leg elastics and 2 cuff elastics
- leg cuffs from synthetic fibers showing the layer combination SMS and having a basis weight of between 13 to 17 gsm and a height of 3.4 cm
- mechanical closure system with landing zone of dimension 14.9 cm x 3.8 cm and adhesive closure tapes of 3.0 cm x 1.3 cm; attached to hook fasten- ing tape of 3.0 cm x 1.3 cm Dimension of the fluid-absorbent article: length: 50.9 cm; front width: 24.5 cm; crotch width: 24.3 cm; rear width: 24.5 cm.

Embodiment 22

A further preferred embodiment of the present invention is described in Embodiment 22 hereinafter. Thus, a preferred fluid-absorbent article comprising

(A) an upper liquid-pervious layer comprising a spunbonded layer (coverstock);
(B) a lower liquid-impervious layer comprising a composite of breathable polyethylene film and spunbond nonwoven;
(C) a high-loaded single fluid-absorbent core between (A) and (B) comprising between 55 to 95 percent by weight water-absorbent polymer particles based on the total absorbent core weight including a fluid-storage section comprising a high-loaded fluid-absorbent layer wrapped with a spunbond material; said high-loaded fluid-absorbent layer comprises water-absorbent polymer particles; suitable water-absorbent polymer particles for such construction having a saline flow conductivity (SFC) from about 50 to 150 x 10"$^7$cm$^3$s/g; and
(D) a system of two acquisition-distribution layers between (A) and (C), comprising an upper resinbonded layer having a basis weight of 40 to 80 gsm; the upper acquisition-distribution layer is rectangular shaped having a size of about 150 to about 250 cm$^2$; the lower acquisition-distribution layer comprising synthetic fibers having a basis weight of 40 to 120 gsm and 0 to 10 percent by weight homogeneously distributed water-absorbent polymer particles within the acquisition-distribution layer; said acquisition-distribution layer having a size of about 150 to about 250 cm$^2$; both acquisition- distribution layers are smaller than the fluid-absorbent core;

The construction of the products chassis and the components contained therein is made and controlled by the discrete application of hotmelt adhesives as known to people skilled in the art. Examples would be e.g. Dispomelt 505B, Dispomelt Cool 1101, as well as other specific function adhesives manufactured by Bostik, Henkel or Fuller.

Ultra-thin high-loaded fluid-absorbent layers can be formed by immobilization of fluid- absorbent polymer particles on a non-woven sheet using hotmelt adhesives. Preferably the water-absorbent polymer particles form longitudinal strips, discrete spots and/or target zones. Other patterns of the water-absorbent polymer particles are also possible. In a preferred embodiment the ultra-thin high-loaded fluid-absorbent layers comprise at least two sheets comprising immobilized water-absorbent polymer particles.

Examples of ultra-thin high-loaded fluid-absorbent layers are described in EP 1 293 187 A1, US 6,972,011, EP 1 447 066 A1, EP 1 447 067 A1, EP 1 609 448 A1, JP 2004/313580, US 2005/0137085, US 2006/0004336, US 2007/0135785 WO 2008/155699 A1, WO 2008/155701 A2, WO 2008/155702 A1, WO 2008/155710 A1, WO 2008/15571 1 A1, WO 2004/071363 A1, US 2003/01811 15, WO 2005/097025, US 2007/156108, US 2008/0125735, and WO 2008/155722 A2, which explicitly forms part of the present disclosure.

Construction Example of Embodiment 22

The fluid-absorbent core consists of a high-loaded mixed single core system having an almost uniform rectangular size. The fluid-absorbent core between (A) and (B) comprises a fluid-storage section comprising a high-loaded fluid-absorbent layer wrapped in a homogeneous layer of hydrophilic spunbond fibers having a basis weight of 10 gsm. The density of the fluid-absorbent core is for the front overall average 0.25 g/cm$^3$, for the insult zone 0.25 g/cm$^3$, for the back overall average 0.26 g/cm$^3$. The basis weight of the fluid-absorbent core is for the front overall average 878.70 gsm, for the insult zone 1237.56 gsm, for the back overall average 495.60 gsm. The thickness of the fluid- absorbent core has an average of 3.1 mm.

The fluid-absorbent layer holds 100 percent by weight distributed water-absorbent polymer particles, the quantity of water-absorbent polymer particles within the fluid-absorbent core is 14.14 g.

The water-absorbent polymer particles derived from dropletization polymerization as described in production example 18, exhibiting the following features and absorption profile:

CRC of 27.6g/g

SFC of 125 x $10^{-7}cm^3s/g$

AUHL of 24 g/g

AUL of 28 g/g

Extractables of 3 wt.%

Residual monomers of 565 ppm

Moisture content of 0.8 weight percent

FSR 0.22 g/gs

PSD of 200 to 600 $\mu$m

Sphericity 0.89

Anticaking of 3

The water-absorbent polymer particles can be remoisturized to a moisture content of 13 percent by weight. Dimension of the fluid-absorbent core: length: 42.4 cm; front width: 10.6 cm; crotch width: 10.2 cm; rear width: 10.5 cm. The upper air through bonded acquisition-distribution layer between (A) and the lower acquisition-distribution layer having a basis weight of 58.8 gsm is rectangular shaped with dimensions of 24.7 cm x 7.3 cm. The lower air through bonded acquisition- distribution layer between the upper acquisition-distribution layer and (C) having a basis weight of 104.8 gsm and holding 11.5 percent by weight homogeneously distributed water-absorbent polymer particles. The quantity of water-absorbent polymer particles within the lower acquisition-distribution layer is 0.12 g; this can be any kind of superabsorbent particles. The lower acquisition- distribution layer is rectangular shaped with dimensions of 20.3 cm x 8.2 cm. Both acquisition-distribution layers are smaller than the fluid-absorbent core.

The fluid-absorbent article further comprises:

- flat rubber elastics; elastics from spandex type fibers: 2 leg elastics and 2 cuff elastics
- leg cuffs from synthetic fibers showing the layer combination SMS and having a basis weight of between 13 to 17 gsm and a height of 4.4 cm
- waistband: front: 13.7 cm x 2.1 cm; rear: 14.8 cm x 2.2 cm

Dimension of the fluid-absorbent article: length: 46.7 cm; front width: 33.5 cm; crotch width: 16.0 cm; rear width: 33.5 cm. The water-absorbent polymer particles and the fluid-absorbent articles are tested by means of the test methods described below.

Embodiment 23

A further preferred embodiment of the present invention is described in Embodiment 23 (pantiliner) hereinafter. Thus, a preferred fluid-absorbent article comprising

(A) an upper liquid-pervious layer comprising a spunbond layer (three piece coverstock);
(B) a lower liquid-impervious layer comprising a composite of breathable polyethylene film and spunbond nonwoven;
(C) a single fluid-absorbent core between (A) and (B) comprising between 40 to 90 percent by weight water-absorbent polymer particles based on the total absorbent core weight and including an absorbent laminate comprising:

1. a tissue layer of 20gsm comprising 50 percent cellulose fibres and 50% viscose wherein the lower surface is coated with hot-melt with a basis weight of 5 gsm allowing bonding to water-absorbent polymer particles having a saline flow conductivity (SFC) of less than 50 x $10^{"7}cm^3s/g$. The loading with said water-absorbent polymer particles is 150 to 500 gsm.

2. A bi-layer air through bonded layer of 40gsm + 40gsm with fibrous side of bi-layer air through bond web marrying with superabsorbent particles uniformly distributed on surface of fibrous side air-through bonded layer subsequently married with five grams per square metre hot-melt coated tissue layer via bonding methods know to persons skilled in the art. The basis weight of primary laminated structure is 200gsm

3. Manipulating primary bonded layer of tissue, hot-melt, superabsorbent and bi-layer airthrough bond layer to present second virgin fibrous layer of bi-layer airthrough bonded layer allows distribution of superabsorbent particles uniformly distributed on surface of air-through bonded layer to marry with second layer of tissue paper coated with hot-melt adhesive of 5gsm and processed through compression assembly known to persons skilled in the art. The basis weight of the secondary layer structure is 200gsm

4. The final absorbent laminate consists of tissue, hot-melt, superabsorbent particles, airthrough bonded layer, superabsorbent particles, hot-melt and tissue with basis weight of 400gsm.

(D) an air through bonded acquisition-distribution layer between (A) and (C) having a basis weight of 40 to 80 gsm; the acquisition-distribution layer is rectangular shaped and smaller than the primary fluid-absorbent core having a size of about 150 to about 250 cm$^2$.

The construction of the products chassis and the components contained therein is made and controlled by the discrete application of hotmelt adhesives as known to people skilled in the art. Examples would be e.g. Dispomelt 505B, Dispomelt Cool 1101, as well as other specific function adhesives manufactured by Bostik, Henkel or Fuller.

First Construction Example of Embodiment 23

The fluid-absorbent core consists of a single core absorbent paper system comprising a fluid-absorbent laminate having a rectangular size. The fluid-absorbent core (C) between (A) and (B) comprises a lower part with water-absorbent polymer particles having a saline flow conductivity (SFC) of $1 \times 10^{"7} cm^3 s/g$.

The fluid-absorbent core is encapsulated by wrapping with a spunbond material having a basis weight of 10 gsm. The density of the fluid-absorbent core is for the font overall average 0.14 g/cm$^3$, for the insult zone 0.14 g/cm$^3$, for the back overall average 0.14 g/cm$^3$. The basis weight of the fluid-absorbent core is for the front overall average 580 gsm, for the insult zone 575 gsm, for the back overall average 555 gsm. The thickness of the fluid-absorbent core has an average of 3.9mm.

The fluid-absorbent core holds 63.18 percent by weight distributed water-absorbent polymer particles, the quantity of water-absorbent polymer particles within the fluid-absorbent core is 9.59 g.

The water-absorbent polymer particles derived from dropletization polymerization as described in production example 11, exhibiting the following features and absorption profile:

CRC of 40.8 g/g
SFC of $1 \times 10^{-7} cm^3 s/g$
AUHL of 21 g/g
AUL of 33 g/g
Extractables of 4 wt.%
Residual monomers of 282 ppm
Moisture content of 5.1 wt.%
FSR 0.39 g/gs
PSD of 200 to 600 $\mu$m
Sphericity 0.89
Anticaking of 3

Dimension of the fluid-absorbent core: length: 40.8 cm; front width: 11.2 cm; crotch width: 8.5 cm; rear width: 11.1 cm.

An air through bonded acquisition-distribution layer between (A) and (C) having a basis weight of 33.5 gsm is rectangular shaped and smaller than the fluid-absorbent core having a size of 24.0 cm x 8.0 cm.

The fluid-absorbent article further comprises:

- flat rubber leg elastics; elastics from spandex type fibers: 2 cuff elastics; 2 leg cuffs from synthetic fibers showing the layer combination SMS and having a basis weight of between 8 to 12 gsm and a height of 3.3 cm

Dimension of the fluid-absorbent article: length: 47.9 cm; front width: 23.3 cm; crotch width: 23.3 cm; rear width: 23.3 cm.

Second Construction Example of Embodiment 23

The fluid-absorbent core consists of a single core absorbent paper system comprising a fluid-absorbent laminate having a rectangular size. The fluid-absorbent core (C) between (A) and (B) comprises a lower part with water-absorbent polymer particles having a saline flow conductivity (SFC) of $1 \times 10^{"7} cm^3 s/g$. and an upper part with water-absorbent polymer particles having a saline flow conductivity (SFC) of $65 \times 10^{"7} cm^3 s/g$.

The fluid-absorbent core is encapsulated by wrapping with a spunbond material having a basis weight of 10 gsm. The density of the fluid-absorbent core is for the font overall average 0.14 g/cm$^3$, for the insult zone 0.14 g/cm$^3$, for the back overall average 0.14 g/cm$^3$. The basis weight of the fluid-absorbent core is for the front overall average 580 gsm, for the insult zone 575 gsm, for the back overall average 555 gsm. The thickness of the fluid-absorbent core has an average of 3.9mm.

The fluid-absorbent core holds 63.18 percent by weight distributed water-absorbent polymer particles, the quantity of water-absorbent polymer particles within the fluid-absorbent core is 9.59 g which is divided between the upper and lower superabsorbent containing strata. The water-absorbent polymer particles derived from dropletization polymerization as described in production example 24 (upper part) and example 11 (lower part), exhibiting the following features and absorption profile:

Upper superabsorbent containing strata:

CRC of 32.4 g/g
SFC of 65 x $10^{-7}$ cm$^3$s/g
AUHL of 24.0 g/g
AUL of 30 g/g
Extractables of 5 wt.%
Residual monomers of 594 ppm
Moisture content of 0.6 wt.%
FSR 0.29 g/gs
PSD of 200 to 600 $\mu$m
Sphericity 0.89
Anticaking of 3

Lower superabsorbent containing lower strata:

CRC of 40.8 g/g
SFC of 1 x $10^{-7}$ cm$^3$s/g
AUHL of 21 g/g
AUL of 33 g/g
Extractables of 4 wt.%
Residual monomers of 282 ppm
Moisture content of 5.1 wt.%
FSR 0.39 g/gs
PSD of 200 to 600 $\mu$m
Sphericity 0.89
Anticaking of 3

Dimension of the fluid-absorbent core: length: 40.8 cm; front width: 11.2 cm; crotch width: 8.5 cm; rear width: 11.1 cm.

An air through bonded acquisition-distribution layer between (A) and (C) having a basis weight of 33.5 gsm is rectangular shaped and smaller than the fluid-absorbent core having a size of 24.0 cm x 8.0 cm.

The fluid-absorbent article further comprises:

- flat rubber leg elastics; elastics from spandex type fibers: 2 cuff elastics; 2 leg cuffs from synthetic fibers showing the layer combination SMS and having a basis weight of between 8 to 12 gsm and a height of 3.3 cm

Dimension of the fluid-absorbent article: length: 47.9 cm; front width: 23.3 cm; crotch width: 23.3 cm; rear width: 23.3 cm.

Embodiment 24

A further preferred embodiment of the present invention is described in Embodiment 24 hereinafter. Thus, a preferred fluid-absorbent article comprising

(A) an upper liquid-pervious layer comprising a spunbond layer (three piece coverstock);
(B) a lower liquid-impervious layer comprising a composite of breathable polyethylene film and spunbond nonwoven;
(C) a double fluid-absorbent core between (A) and (B) comprising a homogeneous mixture of wood pulp fibers (cellulose fibers) and water-absorbent polymer particles as primary core and a layered secondary fluid-absorbent core; the total double fluid-absorbent core comprising the following sequence:

1. a homogeneous primary core of hydrophilic fibrous matrix of wood pulp fibers (cellulose fibers) comprising between 40 to 80 percent by weight fluid- absorbent polymer particles based on the primary absorbent core weight; the primary core contains about 50 percent of the total fluff amount; suitable water-absorbent polymer particles for such construction having a saline flow conductivity (SFC) from about 35 to 100 x $10^{"7}$cm$^3$s/g;

2. a secondary core upper fluff layer of hydrophilic fibrous matrix of wood pulp fibers (cellulose fibers); the secondary core upper layer contains about 25 percent of the total fluff amount;

3. a fluid-absorbent layer comprising between 40 to 80 percent by weight fluid- absorbent polymer particles based on the secondary absorbent core weight; suitable water-absorbent polymer particles for such construction having a saline flow conductivity (SFC) of at least 25 to 100 x 10" 7cm$^3$s/g;

4. a secondary core lower fluff layer of hydrophilic fibrous matrix of wood pulp fibers (cellulose fibers) acting as a dusting layer; the lower core contains about 25 percent of the total fluff amount; and

(D) an air through bonded acquisition-distribution layer between (A) and (C) having a basis weight of 40 to 80 gsm; the acquisition-distribution layer is rectangular shaped and smaller than the primary fluid-absorbent core having a size of about 150 to about 250 cm$^2$.

The construction of the products chassis and the components contained therein is made and controlled by the discrete application of hotmelt adhesives as known to people skilled in the art. Examples would be e.g. Dispomelt 505B, Dispomelt Cool 1 101, as well as other specific function adhesives manufactured by Bostik, Henkel or Fuller.

Ultra-thin high-loaded fluid-absorbent layers can be formed by immobilization of fluid- absorbent polymer particles on a non-woven sheet using hotmelt adhesives. Preferably the water-absorbent polymer particles form longitudinal strips, discrete spots or target zones. Other patterns of the water-absorbent polymer particles are also possible.

In a preferred embodiment the ultra-thin high-loaded fluid-absorbent layers comprise at least two sheets comprising immobilized water-absorbent polymer particles.

Examples of ultra-thin high-loaded fluid-absorbent layers are described in EP 1 293 187 A1, US 6,972,01 1, EP 1 447 066 A1, EP 1 447 067 A1, EP 1 609 448 A1, JP 2004/313580, US 2005/0137085, US 2006/0004336, US 2007/0135785 WO 2008/155699 A1, WO 2008/155701 A2, WO 2008/155702 A1, WO 2008/155710 A1, WO 2008/15571 1 A1, WO 2004/071363 A1, US 2003/0181 115, WO 2005/097025, US 2007/156108, US 2008/0125735, and WO 2008/155722 A2, which explicitly forms part of the present disclosure.

Construction Example of Embodiment 24

The total fluid-absorbent core includes a double-core system, the primary and secondary cores each having an almost uniform rectangular size. The primary core is smaller than the secondary core and is positioned 6 cm from the front distal edge of the secondary core and 10 cm from the rear distal edge of the secondary core and is 9 cm in width. The primary fluid-absorbent core between (A) and (B) comprising a homogeneous mixture of hydrophilic fibrous matrix of wood pulp fibers and 50 percent by weight of water absorbent polymer particles. The primary core has a total weight of 8 g. The secondary core is a multi-layered system of hydrophilic fibrous matrix of wood pulp fibers (cellu lose fibers) and 50 percent by weight water-absorbent polymer particles. The quantity of fluid- absorbent polymer particles within the secondary fluid-absorbent core is 10.0 g. The density of the fluid-absorbent core is for the front overall average 0.19 g/cm$^3$, for the insult zone 0.19 g/cm$^3$, for the back overall average 0.18 g/cm$^3$. The basis weight of the fluid-absorbent core is for the front overall average 813.46 gsm, for the insult zone 1209.15 gsm, for the back overall average 986.27 gsm.

The water-absorbent polymer particles derived from dropletization polymerization as described in WO 2008/009580 A1, table 2, example 24, exhibiting the following features and absorption profile:

CRC of 32.4g/g
SFC of 65 x $10^{-7}$cm$^3$s/g
AUHL of 24 g/g
AUL of 30 g/g
Extractables of 5 wt.%
Residual monomers of 594 ppm
Moisture content of 0.6 weight percent
FSR of 0.29 g/gs
PSD of 200 to 600 $\mu$m
Sphericity 0.89
Anticaking of 3

The water-absorbent polymer particles can be remoisturized to a moisture content of 13 percent by weight.

Dimension of the secondary fluid-absorbent core: length: 40.8 cm; front width: 10.0 cm; crotch width: 9.0 cm;

rear width: 10.0 cm.

The total thickness of both fluid-absorbent cores has an average of 3.9 mm.

An air through bonded acquisition-distribution layer between (A) and (C) having a basis weight of 60 gsm is rectangular shaped and smaller than the primary fluid-absorbent core having a size of 19.0 cm x 7.6 cm.

The fluid-absorbent article further comprises:

- flat rubber elastics; elastics from spandex type fibers: 3 leg elastics and 2 cuff elastics
- leg cuffs from synthetic fibers showing the layer combination SMS and having a basis weight of between 13 to 17 gsm and a height of 3.8 cm
- elastic ears or side panels attached with one side of the ear to the I lateralrear (as worn by user) edge of the chassis of the fluid-absorbent article; the elastic ears are cut from an elastic laminate, e.g. as claimed by US2010262102: a laminate comprising

    a.a first nonwoven web comprising

    - a first layer of spunbond fibers,
    - a second layer of meltblown fibers of basis weight 0.25 to 5 gsm,
    - at least a third layer of meltblown fibers of basis weight 0.25 to 5 gsm,
    - a fourth layer of spunbond fibers, wherein said spunbond fibers are multicomponent fibers and said fourth layer has a basis weight of 1 to 25 gsm,
    - at least a fifth layer of spunbond fibers, wherein said spunbond fibers are multi-component fibers and said fourth layer has a basis weight of 1 to 25 gsm

    b.a web of elastomeric material having top and bottom surfaces, wherein the bottom surface of said fifth layer comprising spunbond fibers of said first nonwoven web is bonded to said top surface of said elastomeric web to form the laminate.

- mechanical closure system with landing zone of dimension 22.0 cm x 4.0 cm and closure tapes of 3.4 cm x 1.5 cm; attached to hook fastening tape of 3.4 cm x 1.4 cm Dimension of the fluid-absorbent article: length: 48.0 cm; front width: 29.7 cm; crotch width: 20.0 cm; rear width: 31.6 cm.

Embodiment 25

A further preferred embodiment of the present invention is described in Embodiment 25 hereinafter. Thus, a preferred fluid-absorbent article comprising

(A) an upper liquid-pervious layer comprising a spunbond web (three piece coverstock);
(B) a lower liquid-impervious layer comprising a composite of breathable poly- ethylene film and spunbond nonwoven;
(C) a single fluid-absorbent core between (A) and (B) comprising a mixture of wood pulp fibers (cellulose fibers) and thermobonding fibers as well as between 10 to 50 percent by weight homogeneously distributed water-absorbent polymer particles within the fluid- absorbent core (C) The fibres mixture preferably comprises 30 to 100 mass percent of wood pulp fibres and 0 to 70 mass % of thermobonding fibres, more preferably it comprises a mixture of wood pulp fibers (cellulose fibers) and thermobonding fibers 50 to 100 mass percent of wood pulp fibres and 0 to 50 mass percent of thermobonding fibres. Said core holds compressed channels derived from patterns inserted by calendaring or thermobonding and showing an improved fluid transport towards extended regions; suitable water-absorbent polymer particles for such construction having a centrifuge retention capacity (CRC) from about 28 to 60 g/g; suitable thermobonding fibers having a component with low melting point as e.g. bi-component fibers of core/shell type, where the core is made of polyethylene terephthalate, preferably polyethylene. Further examples of thermobonding fibers are ethylene-vinyl acetate copolymer fibers containing a mixture of 30-100 weight% ethylene-vinyl acetate copolymer (A) comprising 5-40 weight% vinyl acetate and 60-95 weight % ethylene or its saponified compound, and 0-70 weight % polyethylene (B). A fiber assembly results by mixing cellulose fibers with thermobonding composite fibers and water-absorbent polymer particles, heat treating this assembly by calendaring through heated rolls at a temperature from 90 to 150 degrees. Thereby said fibrous mixture is connected by a plurality of channel regions formed from compressive forces by heat-treatment between two heated calendar rolls. The density in the low density regions is preferably 0.01 to 0.1 $g/cm^3$ and more preferably 0.01 to 0.05 $g/cm^3$ and the density in the high density region is preferably 0.1 to 0.3 $g/cm^3$
(D) an air-through bonded acquisition-distribution layer between (A) and (C) having a basis weight of 30 to 80 gsm; the acquisition-distribution layer is rectangular shaped and smaller than the fluid-absorbent core having a size of about 150 to about 250 cm$^2$.

The construction of the products chassis and the components contained therein is made and controlled by the discrete application of hotmelt adhesives as known to people skilled in the art. Examples would be e.g. Dispomelt 505B, Dispomelt Cool 1 101, as well as other specific function adhesives manufactured by Bostik, Henkel or Fuller.

Ultra-thin high-loaded fluid-absorbent layers can be formed by immobilization of fluid- absorbent polymer particles on a non-woven sheet using hotmelt adhesives. Preferably the water-absorbent polymer particles form longitudinal strips or discrete spots. Other patterns of the water-absorbent polymer particles are also possible.

In a preferred embodiment the ultra-thin high-loaded fluid-absorbent layers comprise at least two sheets comprising immobilized water-absorbent polymer particles.

Examples of ultra-thin high-loaded fluid-absorbent layers are described in EP 1 293 187 A1, US 6,972,01 1, EP 1 447 066 A1, EP 1 447 067 A1, EP 1 609 448 A1, JP 2004/313580, US 2005/0137085, US 2006/0004336, US 2007/0135785 WO 2008/155699 A1, WO 2008/155701 A2, WO 2008/155702 A1, WO 2008/155710 A1, WO 2008/15571 1 A1, WO 2004/071363 A1, US 2003/0181 115, WO 2005/097025, US 2007/156108, US 2008/0125735, and WO 2008/155722 A2, which explicitly forms part of the present disclosure.

An example for diapers with compressed channels are e.g. disclosed in EP 2 484 321, which also forms part of the present disclosure.

Construction Example of Embodiment 25

The fluid-absorbent core consists of a single fluid-absorbent Core between (A) and (B) comprising a mixture of 73 mass % of cellulose fibers and 27 mass % ethylene-vinyl acetate copolymer fibers as well as 37.11 percent by weight homogeneously distributed water-absorbent polymer particles within the fluid-absorbent core (C) having a uniform rectangular size. Said fibrous mixture is heat treated by calendaring through heated rolls at a temperature of 143 degrees, thereby forming a plurality of channel regions for improved canalisation of insulted urine.

The quantity of water-absorbent polymer particles within the fluid-absorbent core is 11.38 g. The total fluff pulp weight is 19.25 g. The density of the fluid-absorbent core is in the compressed high density region for the front overall average 0.22 g/cm$^3$, for the insult zone 0.18 g/cm$^3$, for the back overall average 0.18 g/cm$^3$. The basis weight of the fluid-absorbent core is for the front overall average 914.18 gsm, for the insult zone 925.47 gsm, for the back overall average 886.32 gsm.

The water-absorbent polymer particles derived from dropletization polymerization as described in preparation example 11, exhibiting the following features and absorption profile:

CRC of 40,8 g/g
SFC of 1 x 10$^{-7}$cm$^3$s/g
AUHL of 21 g/g
AUL of 33 g/g
Extractables of 4 wt. %
Residual monomers of 282 ppm
Moisture content of 5.1 wt.%
FSR of 0.39 g/gs
PSD of 200 to 600 $\mu$m
Sphericity 0.89
Anticaking of 3

The water-absorbent polymer particles can be remoisturized to a moisture content of 13 percent by weight.

Dimension of the fluid-absorbent core: length: 39.2 cm; width: 10.0 cm.

The thickness of the fluid-absorbent core has an average of 4.7 mm.

An air through bonded acquisition-distribution layer between (A) and (C) having a basis weight of 40 gsm is rectangular shaped with dimensions of 24.4 cm x 8.6 cm and smaller than the fluid-absorbent core.

The fluid-absorbent article further comprises:

- flat rubber elastics; elastics from spandex type fibers: 3 leg elastics and 1 cuff elastics
- leg cuffs from synthetic fibers showing the layer combination SMS and having a basis weight of between 13 to 17 gsm and a height of 3.0 cm
- mechanical closure system with landing zone of dimension 18.9 cm x 3.8 cm and flexi-band closure tapes of 1.6 cm x 3.4 cm; attached to hook fastening tape of 1.3 cm x 3.4 cm
- also incorporated is elasticated waistband located to the rear of the product with dimensions of 10.8 cm x 2.8 cm

Dimension of the fluid-absorbent article: length: 47.8 cm; front width: 31.5 cm; crotch width: 20.6 cm; rear width: 31.1 cm.

Embodiment 26

A further preferred embodiment of the present invention is described in Embodiment 26 hereinafter. Thus, a preferred fluid-absorbent article comprising

(A) an upper liquid-pervious layer comprising a spunbond web ;
(B) a lower liquid-impervious layer comprising a composite of breathable polyethylene film and spunbond nonwoven;
(C) a single fluid-absorbent core between (A) and (B) comprising a mixture of wood pulp fibers (cellulose fibers) and between 10 to 60 % by weight homogeneously distributed water-absorbent polymer particles within the fluid-absorbent core (C); suitable water-absorbent polymer particles for such construction having a centrifuge retention capacity (CRC) from about 32 to 60 g/g; the fluid-absorbent core is further comprising a dusting layer adjacent to the liquid-impervious layer (B) and underlying the fluid-absorbent core above; the dusting layer is a fibrous layer comprising fluff only (cellulose fibers); and
(D) an air-through bonded acquisition-distribution layer between (A) and (C) having a basis weight of 30 to 80 gsm; the acquisition-distribution layer is rectangular shaped and smaller than the fluid-absorbent core having a size of about 150 to about 250 cm$^2$.

Located below said acquisition layer; an essentially super absorbent particle free fibrous layer consisting of post de-fibration cross linked cellulose fibres having a basis weight of 85 to 160 gsm, dimensions of secondary acquisition layer having a size of about 150 to 250 cm 2

The construction of the products chassis and the components contained therein is made and controlled by the discrete application of hotmelt adhesives as known to people skilled in the art. Examples would be e.g. Dispomelt 505B, Dispomelt Cool 1101, as well as other specific function adhesives manufactured by Bostik, Henkel or Fuller.

The final construction assembly technique of the fluid absorbent article is achieved using ultrasonic bonding methods know to persons skilled in the art to form a unitary article with user tearable side panels for ease of removal and disposal.

Ultra-thin high-loaded fluid-absorbent layers can be formed by immobilization of water-absorbent polymer particles on a non-woven sheet using hotmelt adhesives. Preferably the water-absorbent polymer particles form longitudinal strips, discrete spots or discrete target zones. Other patterns of the water-absorbent polymer particles are also possible.

In a preferred embodiment the thin high-loaded fluid-absorbent layers comprise at least two distinct strata with upper majorative proportion comprising immobilized water-absorbent polymer particles. Lower minor strata essentially free of fluid absorbent polymer particles forming a dusting layer of cellulose fibres.

Examples of ultra-thin high-loaded fluid-absorbent layers are described in EP 1 293 187 A1, US 6,972,011, EP 1 447 066 A1, EP 1 447 067 A1, EP 1 609 448 A1, JP 2004/313580, US 2005/0137085, US 2006/0004336, US 2007/0135785 WO 2008/155699 A1, WO 2008/155701 A2, WO 2008/155702 A1, WO 2008/155710 A1, WO 2008/155711 A1, WO 2004/071363 A1, US 2003/0181115, WO 2005/097025, US 2007/156108, US 2008/0125735, and WO 2008/155722 A2, which explicitly forms part of the present disclosure.

Construction Example of Embodiment 26

The fluid-absorbent core consists of a single fluid-absorbent Core between (A) and (B) comprising a mixture of wood pulp fibers (cellulose fibers) and 56.56% by weight homogeneously distributed water-absorbent polymer particles within the fluid-absorbent core (C) having a uniform rectangular size. The quantity of water-absorbent polymer particles within the fluid-absorbent core is 10.59 g. The total fluff pulp weight is 8.13 g. The density of the fluid-absorbent core is for the front overall average 0.12 g/cm$^3$, for the insult zone 0.11 g/cm$^3$, for the back overall average 0.12 g/cm$^3$. The basis weight of the fluid-absorbent core is for the front overall average 917.50 gsm, for the insult zone 907 gsm, for the back overall average 801.66 gsm.

The water-absorbent polymer particles derived from dropletization polymerization as described in preparation example 11, exhibiting the following features and absorption profile:

CRC of 40.8 g/g
SFC of 1 x 10$^{-7}$cm$^3$s/g
AUHL of 21 g/g
AUL of 33 g/g
Extractables of 4 wt.%
Residual monomers of 282 ppm

70

Moisture content of 5.1 wt.%

FSR of 0.39 g/gs

Sphericity 0.89

PSD of 200 to 600 $\mu$m

Anticaking of 3

The water-absorbent polymer particles can be remoisturized to a moisture content of 13% by weight.

Dimension of the fluid-absorbent core: length: 38.5 cm; width at front and rear distal edge: 10.7 cm. Crotch width 8.5cm. The distance of the front of the core to front chassis material: 4.8cm and distance of rear of the core to the rear chassis material: 3.5cm.

The thickness of the fluid-absorbent core has an average of 7.81 mm.

The total weight of the complete constructed absorbent article average: 34.58g with core weight of 18.72g.

An air through bonded acquisition-distribution layer between (A) and (C) having a basis weight of 58.0 gsm is rectangular shaped with dimensions of 23.8 cm x 7.2 cm and smaller than the fluid-absorbent core.

Located below said acquisition layer; an essentially super absorbent particle free fibrous layer consisting of post de-fibration cross linked cellulose fibre having a basis weight of 85gsm, dimensions of secondary acquisition layer: 23.8 cm x 7.2 cm and smaller than the fluid absorbent core.

The fluid-absorbent article further comprises:

- elastics from spandex type fibers: 2 cuff elastics enclosed in SMMS material folded over and bonded using hot-melt adhesive and ultra-sonic bonding, wrapped SMMS containing equidistant elastic location to top of cuff assembly and bond point to (A); bonding to upper liquid pervious layer (coverstock) by means of ultra-sonic bonding.
- leg cuffs from SMMS synthetic fibers showing the construction combination above and a height of 4.0 cm
- two elastics from spandex fibres located and adhered to liquid pervious layer (A) and liquid impervious layer (B) with longitudinal orientation on the right side of the absorbent core location.
- Two elastics from spandex fibres located and adhered to liquid pervious layer (A) and liquid impervious layer (B) with longitudinal orientation on the left side of the absorbent core location.
- also incorporated is elasticated waistband located to the rear of the product with dimensions of 25.5 cm in the machine direction (MD) x 19.0 cm in the cross direction.
- further incorporated is elasticated waistband located to the front of the product with dimensions of 10.0cm in the machine direction (MD) x 19.0cm in the cross direction (CD).

Dimension of the fluid-absorbent article: length: 46.5 cm; front width: 19.0 cm; crotch width: 17.0 cm; rear width: 19.0 cm.

Embodiment 27

A further preferred embodiment of the present invention is described in Embodiment 27 hereinafter. Thus, a preferred fluid-absorbent article comprising

(A) an upper liquid-pervious layer comprising a spunbond web (three piece coverstock);

(B) a lower liquid-impervious layer comprising a composite of breathable polyethylene film and spunbond nonwoven;

(C) a single fluid-absorbent core between (A) and (B) comprising a mixture of wood pulp fibers (cellulose fibers) and between 10 to 60 % by weight homogeneously distributed water-absorbent polymer particles within the fluid-absorbent core (C); suitable water-absorbent polymer particles for such construction having a centrifuge retention capacity (CRC) from about 32 to 60 g/g; the fluid-absorbent core is further comprising a wicking layer adjacent to the liquid pervious layer (A) and overlying the fluid-absorbent core below; the wicking layer is a fibrous layer comprising fluff only (cellulose fibers); and

(D) an air-through bonded acquisition-distribution layer between (A) and (C) having a basis weight of 30 to 80 gsm; the acquisition-distribution layer is rectangular shaped and smaller than the fluid-absorbent core having a size of about 150 to about 250 cm$^2$.

The construction of the products chassis and the components contained therein is made and controlled by the discrete application of hotmelt adhesives as known to people skilled in the art. Examples would be e.g. Dispomelt 505B, Dispomelt Cool 1101, as well as other specific function adhesives manufactured by Bostik, Henkel or Fuller.

The final construction assembly technique of the fluid absorbent article is achieved using ultrasonic bonding methods know to persons skilled in the art to form a unitary article with user tearable side panels for ease of removal and disposal.

Ultra-thin high-loaded fluid-absorbent layers can be formed by immobilization of water-absorbent polymer particles on a non-woven sheet using hotmelt adhesives. Preferably the water-absorbent polymer particles form longitudinal strips, discrete spots or discrete target zones. Other patterns of the water-absorbent polymer particles are also possible.

In a preferred embodiment the ultra-thin high-loaded fluid-absorbent layers comprise at least two sheets comprising immobilized water-absorbent polymer particles.

Examples of ultra-thin high-loaded fluid-absorbent layers are described in EP 1 293 187 A1, US 6,972,011, EP 1 447 066 A1, EP 1 447 067 A1, EP 1 609 448 A1, JP 2004/313580, US 2005/0137085, US 2006/0004336, US 2007/0135785 WO 2008/155699 A1,

WO 2008/155701 A2, WO 2008/155702 A1, WO 2008/155710 A1, WO 2008/155711 A1, WO 2004/071363 A1, US 2003/0181115, WO 2005/097025, US 2007/156108,

US 2008/0125735, and WO 2008/155722 A2, which explicitly forms part of the present disclosure.

Construction Example of Embodiment 27

The fluid-absorbent core consists of a single fluid-absorbent Core between (A) and (B) comprising a mixture of wood pulp fibers (cellulose fibers) and 47.74% by weight homogeneously distributed water-absorbent polymer particles within the fluid-absorbent core (C) having a uniform rectangular size. The quantity of water-absorbent polymer particles within the fluid-absorbent core is 8.7 g. The total fluff pulp weight is 9.53 g. The density of the fluid-absorbent core is for the front overall average 0.11 g/cm$^3$, for the insult zone 0.10 g/cm$^3$, for the back overall average 0.10 g/cm$^3$. The basis weight of the fluid-absorbent core is for the front overall average 1030.5 gsm, for the insult zone 892 gsm, for the back overall average 911.5 gsm.

The water-absorbent polymer particles derived from dropletization polymerization as described in preparation example 11, exhibiting the following features and absorption profile:

CRC of 40.8 g/g
SFC of 1 x 10$^{-7}$cm$^3$s/g
AUHL of 21 g/g
AUL of 33 g/g
Extractables of 4 wt.%
Residual monomers of 282 ppm
Moisture content of 5.1 wt.%
FSR of 0.39 g/gs
Sphericity 0.89
PSD of 200 to 600 $\mu$m
Anticaking of 3

The water-absorbent polymer particles can be remoisturized to a moisture content of 13% by weight.

Dimension of the fluid-absorbent core: length: 33.0 cm; width at front and rear distal edge: 12.2 cm. Crotch width 8.2cm. The distance of the front of the core to front distal edge of chassis material: 4.0cm and distance of rear distal edge of the core to the rear chassis material: 9.5cm.

The thickness of the fluid-absorbent core has an average of 9.04 mm.

The total weight of the complete constructed absorbent article average: 32.74g with core weight of 18.23g.

An air through bonded acquisition-distribution layer between (A) and (C) having a basis weight of 46.0 gsm is rectangular shaped with dimensions of 23.8 cm x 7.2 cm and smaller than the fluid-absorbent core.

The fluid-absorbent article further comprises:

- elastics from spandex type fibers: 2 cuff elastics enclosed in SMS material folded over and bonded using hot-melt adhesive and ultra-sonic bonding of leg cuff; cuff component bonding to upper liquid pervious layer (A) by means of hot-melt adhesive.

- leg cuffs from synthetic fibers showing the layer combination SMMS and a height of 4.0 cm

- distance between cuffs measured in the lateral direction (width of absorbent article) is 11.7cm.

- three elastics from spandex fibres located and adhered to liquid pervious layer (A) and liquid impervious layer (B) with longitudinal orientation on the right side of the absorbent core location when viewed with product rear facing observer.

- three elastics from spandex fibres located and adhered to liquid pervious layer (A) and liquid impervious layer (B) with longitudinal orientation on the left side of the absorbent core location when viewed with the product rear facing observer.

- elastomeric waistband panel with dimensions of 25.5 cm in the machine direction (MD) x 19.0 cm in the cross direction (CD) located at rear distal edge of the fluid absorbent article.

- further incorporated is elastomeric waistband panel with dimensions of 10.0cm in the machine direction (MD) x 19.0 cm in the cross direction (CD) located to the front distal edge with dimensions of 10.0cm in the machine direction (MD) x 19.0 cm in the cross direction (CD) located at the front distal edge of the fluid absorbent article.

Dimension of the fluid-absorbent article: length: 46.5 cm; front width: 19.0 cm; crotch width: 17.0 cm; rear width: 19.0 cm.

Embodiment 28

A further preferred embodiment of the present invention is described in Embodiment 28 hereinafter. Thus, a preferred fluid-absorbent article comprising

(A) an upper liquid-pervious layer comprising a spunbond web (three piece coverstock);
(B) a lower liquid-impervious layer comprising a composite of breathable polyethylene film and spunbond nonwoven;
(C) a single fluid-absorbent core between (A) and (B) comprising a mixture of wood pulp fibers (cellulose fibers) and between 10 to 60 % by weight homogeneously distributed water-absorbent polymer particles within the fluid-absorbent core (C); suitable water-absorbent polymer particles for such construction having a centrifuge retention capacity (CRC) from about 32 to 60 g/g; and
(D) an air-through bonded acquisition-distribution layer between (A) and (C) having a basis weight of 30 to 80 gsm; the acquisition-distribution layer is rectangular shaped and smaller than the fluid-absorbent core having a size of about 150 to about 250 cm$^2$.

Located below said acquisition layer; an essentially super absorbent particle free fibrous layer consisting of post de-fibration cross linked cellulose fibres having a basis weight of 85 to 160 gsm, dimensions of secondary acquisition layer having a size of about 150 to 250 cm 2

The construction of the products chassis and the components contained therein is made and controlled by the discrete application of hotmelt adhesives as known to people skilled in the art. Examples would be e.g. Dispomelt 505B, Dispomelt Cool 1101, as well as other specific function adhesives manufactured by Bostik, Henkel or Fuller.

The final construction assembly technique of the fluid absorbent article is achieved using ultrasonic bonding methods known to persons skilled in the art to form a unitary article with user tearable side panels for ease of removal and disposal.
Examples of articles with user tearable side panels attached by ultrasonic bonding are described in US2012157953, US2012157954, US2012157955, US2011313381.

Ultra-thin high-loaded fluid-absorbent layers can be formed by immobilization of water-absorbent polymer particles on a non-woven sheet using hotmelt adhesives. Preferably the water-absorbent polymer particles form longitudinal strips, discrete spots or discrete target zones. Other patterns of the water-absorbent polymer particles are also possible.

In a preferred embodiment the ultra-thin high-loaded fluid-absorbent layers comprise at least two sheets comprising immobilized water-absorbent polymer particles.

Examples of ultra-thin high-loaded fluid-absorbent layers are described in EP 1 293 187 A1, US 6,972,011, EP 1 447 066 A1, EP 1 447 067 A1, EP 1 609 448 A1, JP 2004/313580, US 2005/0137085, US 2006/0004336, US 2007/0135785 WO 2008/155699 A1,
WO 2008/155701 A2, WO 2008/155702 A1, WO 2008/155710 A1, WO 2008/155711 A1, WO 2004/071363 A1, US 2003/0181115, WO 2005/097025, US 2007/156108,
US 2008/0125735, and WO 2008/155722 A2, which explicitly forms part of the present disclosure.

Construction Example of Embodiment 28

The fluid-absorbent core consists of a single fluid-absorbent Core between (A) and (B) comprising a mixture of wood pulp fibers (cellulose fibers) and 53.13% by weight homogeneously distributed water-absorbent polymer particles within the fluid-absorbent core (C) having a uniform rectangular size. The quantity of water-absorbent polymer particles within the fluid-absorbent core is 10.0 g. The total fluff pulp weight is 8.82 g. The density of the fluid-absorbent core is for the front overall average 0.12 g/cm$^3$, for the insult zone 0.12 g/cm$^3$, for the back overall average 0.11 g/cm$^3$. The basis weight of the fluid-absorbent core is for the front overall average 887.0 gsm, for the insult zone 828 gsm, for the back overall average 644.3 gsm.

The water-absorbent polymer particles derived from dropletization polymerization as described in preparation example 11, exhibiting the following features and absorption profile:

CRC of 40.8 g/g
SFC of $1 \times 10^{-7} cm^3 s/g$
AUHL of 21 g/g
AUL of 33 g/g
Extractables of 4 wt.%
Residual monomers of 282 ppm
Moisture content of 5.1 wt.%
FSR of 0.39 g/gs
Sphericity 0.89
PSD of 200 to 600 $\mu$m
Anticaking of 3

The water-absorbent polymer particles can be remoisturized to a moisture content of 13% by weight.

Dimension of the fluid-absorbent core: length: 38.5 cm; width at front and rear distal edge: 11.0 cm. Crotch width 9.0cm. The distance of the front of the core to front abdominal distal edge of chassis material: 5.8cm and distance of rear dorsal distal edge of the core to the rear chassis material: 3.7cm.

The thickness of the fluid-absorbent core has an average of 6.6 mm.

The total weight of the complete constructed absorbent article average: 34.48g with core weight of 18.82g.

An air through bonded acquisition-distribution layer between (A) and (C) having a basis weight of 44.1 gsm is rectangular shaped with dimensions of 23.6 cm x 7.2 cm and smaller than the fluid-absorbent core.

Located below said acquisition layer; an essentially super absorbent particle free fibrous layer consisting of post de-fibration cross linked cellulose fibre having a basis weight of 85gsm, dimensions of secondary acquisition layer: 23.6 cm x 7.2 cm and smaller than the fluid absorbent core.

The fluid-absorbent article further comprises:

- elastics from spandex type fibers: 1 cuff elastics enclosed in SMS material folded over and bonded using hot-melt adhesive and ultra-sonic bonding of leg cuff; cuff component bonding to upper liquid pervious layer (A) by means of hot-melt adhesive.
- symmetrical leg cuffs from synthetic fibers showing the layer combination SMMS and a height of 4.3 cm
- distance between cuffs measured in the lateral direction (width of absorbent article) is 12.0cm.
- two elastics from spandex fibres located and adhered to liquid pervious layer (A) and liquid impervious layer (B) with longitudinal orientation on the right side of the absorbent core location when viewed with product rear facing observer.
- two elastics from spandex fibres located and adhered to liquid pervious layer (A) and liquid impervious layer (B) with longitudinal orientation on the left side of the absorbent core location when viewed with the product rear facing observer.
- elastomeric waistband panel with dimensions of 14.5 cm in the machine direction(MD) x 20.0 cm in the cross direction (CD) located at rear dorsal distal edge of the fluid absorbent article.
- further incorporated is elastomeric waistband panel with dimensions of 10.0cm in the machine direction (MD) x 20.0 cm in the cross direction (CD) located to the front abdominal.
- further incorporated re-fastening element in the form of rectangular strip zone of mechanical hook material adhered to elasticated bonded side panels liquid impervious layer (B).

Dimension of the fluid-absorbent article: length: 48.5 cm; front width: 36.0 cm; crotch width: 18.0 cm; rear width: 36.0 cm.

Embodiment 29

A further preferred embodiment of the present invention is described in Embodiment 29 hereinafter. Thus, a preferred fluid-absorbent article comprising

(A) an upper liquid-pervious layer comprising a spunbond web (three piece coverstock);
(B) a lower liquid-impervious layer comprising a composite of breathable polyethylene film and spunbond nonwoven;
(C) a single fluid-absorbent core between (A) and (B) comprising a mixture of wood pulp fibers (cellulose fibers) and between 10 to 50 % by weight homogeneously distributed water-absorbent polymer particles within the fluid-absorbent core (C); suitable water-absorbent polymer particles for such construction having a centrifuge retention capacity (CRC) from about 32 to 60 g/g; the fluid-absorbent core is further comprising a dusting layer adjacent to the liquid-impervious layer (B) and underlying the fluid-absorbent core above; the dusting layer is a fibrous layer comprising fluff only (cellulose fibers); and

(D) an air-through bonded acquisition-distribution layer between (A) and (C) having a basis weight of 30 to 80 gsm; the acquisition-distribution layer is rectangular shaped and smaller than the fluid-absorbent core having a size of about 150 to about 250 cm$^2$.

The construction of the products chassis and the components contained therein is made and controlled by the discrete application of hotmelt adhesives as known to people skilled in the art. Examples would be e.g. Dispomelt 505B, Dispomelt Cool 1101, as well as other specific function adhesives manufactured by Bostik, Henkel or Fuller.

The final construction assembly technique of the fluid absorbent article is achieved using ultrasonic bonding methods know to persons skilled in the art to form a unitary article with user tearable side panels for ease of removal and disposal.

Ultra-thin high-loaded fluid-absorbent layers can be formed by immobilization of water-absorbent polymer particles on a non-woven sheet using hotmelt adhesives. Preferably the water-absorbent polymer particles form longitudinal strips, discrete spots or discrete target zones. Other patterns of the water-absorbent polymer particles are also possible.

In a preferred embodiment the ultra-thin high-loaded fluid-absorbent layers comprise at least two sheets comprising immobilized water-absorbent polymer particles.

Examples of ultra-thin high-loaded fluid-absorbent layers are described in EP 1 293 187 A1, US 6,972,011, EP 1 447 066 A1, EP 1 447 067 A1, EP 1 609 448 A1, JP 2004/313580, US 2005/0137085, US 2006/0004336, US 2007/0135785 WO 2008/155699 A1, WO 2008/155701 A2, WO 2008/155702 A1, WO 2008/155710 A1, WO 2008/155711 A1, WO 2004/071363 A1, US 2003/0181115, WO 2005/097025, US 2007/156108, US 2008/0125735, and WO 2008/155722 A2, which explicitly forms part of the present disclosure.

Construction Example of Embodiment 29

The fluid-absorbent core consists of a single fluid-absorbent Core between (A) and (B) comprising a mixture of wood pulp fibers (cellulose fibers) and 64.42% by weight homogeneously distributed water-absorbent polymer particles within the fluid-absorbent core (C) having a uniform rectangular size. The quantity of water-absorbent polymer particles within the fluid-absorbent core is 11.47 g. The total fluff pulp weight is 6.33 g. The density of the fluid-absorbent core is for the front overall average 0.13 g/cm$^3$, for the insult zone 0.13 g/cm$^3$, for the back overall average 0.13 g/cm$^3$. The basis weight of the fluid-absorbent core is for the front overall average 720 gsm, for the insult zone 672 gsm, for the back overall average 687 gsm.

The water-absorbent polymer particles derived from dropletization polymerization as described in preparation example 24, exhibiting the following features and absorption profile:

CRC of 32.4 g/g
SFC of 65 x 10$^{-7}$cm$^3$s/g
AUHL of 24 g/g
AUL of 30 g/g
Extractables of 5 wt.%
Residual monomers of 594 ppm
Moisture content of 0.6 wt.%
FSR of 0.29 g/gs
Sphericity 0.89
PSD of 200 to 600 $\mu$m
Anticaking of 3

The water-absorbent polymer particles can be remoisturized to a moisture content of 13% by weight.

Dimension of the fluid-absorbent core: length: 33.5 cm; width at front and rear distal edge: 12.0 cm. Crotch width 9.0 cm. The distance of the front of the core to front distal edge of chassis material: 6.0cm and distance of rear distal edge of the core to the rear chassis material: 8.5cm.

The thickness of the fluid-absorbent core has an average of 5.2 mm.

The total weight of the complete constructed absorbent article average: 34.6g with core weight of 17.8g.

An air through bonded acquisition-distribution layer between (A) and (C) having a basis weight of 28.4 gsm is rectangular shaped with dimensions of 16.5 cm x 6.4 cm and smaller than the fluid-absorbent core.

The fluid-absorbent article further comprises:

- elastics from spandex type fibers: 2 cuff elastics enclosed in SMS material folded over and bonded using hot-melt adhesive and ultra-sonic bonding of leg cuff; cuff component bonding to upper liquid pervious layer (A) by means of hot-melt adhesive.

- leg cuffs from synthetic fibers showing the layer combination SMMS and a height of 4.8 cm
- distance between cuffs measured in the lateral direction (width of absorbent article) is 12.0 cm.
- two elastics from spandex fibres located and adhered to liquid pervious layer (A) and liquid impervious layer (B) with longitudinal orientation on the right side of the absorbent core location when viewed with product rear facing observer.
- two elastics from spandex fibres located and adhered to liquid pervious layer (A) and liquid impervious layer (B) with longitudinal oriental on the left side of the absorbent core location when viewed with the product rear facing observer.
- elastomeric waistband panel with dimensions of 25.5 cm in the machine direction (MD) x 19.0 cm in the cross direction (CD) located at rear distal edge of the fluid absorbent article.
- further incorporated is elastomeric waistband panel with dimensions of 10.0cm in the machine direction (MD) x 19.0 cm in the cross direction (CD) located to the front distal edge with dimensions of 10.0cm in the machine direction (MD) x 19.0cm in the cross direction (CD) located at the front distal edge of the fluid absorbent article.
- further incorporated re-fastening element in the form of rectangular strip zone of mechanical hook material adhered to elasticated bonded side panels liquid impervious layer (B).

Dimension of the fluid-absorbent article: length: 49.0 cm; front width: 30.0 cm; crotch width: 13.0 cm; rear width: 30.0cm.

The water-absorbent polymer particles and the fluid-absorbent articles are tested by means of the test methods described below.

Methods:

The measurements should, unless stated otherwise, be carried out at an ambient temperature of $23 \pm 2°C$ and a relative atmospheric humidity of $50 \pm 10\%$. The water-absorbent polymers are mixed thoroughly before the measurement.

The "WSP" standard test methods are described in: "Standard Test Methods for the Nonwovens Industry", jointly issued by the "Worldwide Strategic Partners" EDANA (European Disposables and Nonwovens Association, Avenue Eugene Plasky, 157, 1030 Brussels, Belgium, www.edana.org) and INDA (Association of the Nonwoven Fabrics Industry, 1100 Crescent Green, Suite 115, Cary, N.C. 27518, U.S.A., www.inda.org). This publication is available both from EDANA and INDA.

Vortex

$50.0 \pm 1.0$ml of 0.9% NaCl solution are added into a 100ml beaker. A cylindrical stirrer bar (30 x 6mm) is added and the saline solution is stirred on a stir plate at 60 rpm. $2.000 \pm 0.010$g of water-absorbent polymer particles are added to the beaker as quickly as possible, starting a stop watch as addition begins. The stopwatch is stopped when the surface of the mixture becomes "still" that means the surface has no turbulence, and while the mixture may still turn, the entire surface of particles turns as a unit. The displayed time of the stopwatch is recorded as Vortex time.

Rewet Value

This test consists of multiple insults of 0.9 wt.% NaCl solution in deionized water. The rewet is measured by the amount of fluid the article released under pressure.The rewet is measured after each insult.

The fluid-absorbent article is clamped nonwoven side upward onto the inspection table. The insult point is marked accordingly with regard to the type and gender of the diaper to be tested (i.e. in the centre of the core for girl, 2.5 cm towards the front for unisex and 5cm towards the front for boy). A separatory funnel is positioned above the fluid-absorbent article so that the spout is directly above the marked insult point.

For the primary insult 100 g of aqueous saline solution (0.9% by weight) is poured into the fluid-absorbent article via the funnel in one shot. The liquid is allowed to be absorbed for 10 minutes, and after that time the stack of 10 filter papers (Whatman®) having 9 cm diameter and known dry weight (D1) is placed over the insult point on the fluid-absorbent article. On top of the filter paper, the 2.5 kg weight with 8 cm diameter is added. After 2 minutes have elapsed the weight is removed and filter paper reweighed giving the wet weight value (D2).

The rewet value is calculated as follows:

$$RV [g] = D2 - D1$$

For the rewet of the secondary insult the procedure for the primary insult is repeated. 50 g of aqueous saline solution (0.9% by weight) and 20 filter papers are used.

For the rewet of the tertiary and following insults the procedure for the primary insult is repeated. For each of the following insults 3rd, 4th and 5th 50 g of aqueous saline solution (0.9% by weight) and 30, 40 and 50 filter papers respectively are used.

Rewet Under Load (RUL)

The test determines the amount of fluid a fluid-absorbent article will release after being maintained at a pressure of 0.7 psi (49.2 g/cm$^2$) for 10 min following multiple separate insults. The rewet under load is measured by the amount of fluid the fluid-absorbent article releases under pressure. The rewet under load is measured after each insult.

The fluid-absorbent article is clamped nonwoven side upward onto the inspection table. The insult point is marked accordingly with regard to the type and gender of the diaper to be tested (i.e. in the centre of the core for girl, 2.5 cm towards the front for unisex and 5cm towards the front for boy). A 3.64 kg circular weight (10 cm diameter) having a central opening (2.3 cm diameter) with perspex tube is placed with on the previously marked insult point.

For the primary insult 100 g of aqueous saline solution (0.9% by weight) is poured into the perspex tube in one shot. Amount of time needed for the fluid to be fully absorbed into the fluid-absorbent article is recorded. After 10 minutes have elapsed, the load is removed and the stack of 10 filter papers (Whatman®) having 9 cm diameter and known dry weight (W1) is placed over the insult point on the fluid-absorbent article. On top of the filter paper, the 2.5 kg weight with 8 cm diameter is added. After 2 minutes have elapsed the weight is removed and filter paper reweighed giving the wet weight value (W2).

The rewet under load is calculated as follows:

$$RUL\ [g] = W2 - W1$$

For the rewet under load of the secondary insult the procedure for the primary insult is repeated. 50 g of aqueous saline solution (0.9% by weight) and 20 filter papers are used.

For the rewet under load of the tertiary and following insults the procedure for the primary insult is repeated. For each of the following insults 3rd, 4th and 5th 50 g of aqueous saline solution (0.9% by weight) and 30, 40 and 50 filter papers respectively are used.

Basis weight

The basis weight is determined at discrete regions of the fluid-absorbent core: the front overall average; the insult zone and the back overall average.

The article nonwoven face is pinned upwards onto the inspection table. Then an insult point is marked on the fluid-absorbent article. The insult point is marked on the article accordingly with regard to the type and gender of the diaper to be tested (i.e. in the centre of the fluid-absorbent core for girl, 2.5cm towards the front for unisex and 5cm towards the front for boy).

Then lines for the following zones are marked on the fluid-absorbent article in dependence of the diaper to be tested, e. g. for boy diapers:

- for the front overall average zone 5.5 cm forward of the center of the core to the front distal edge of the core;
- for the insult zone 5.5 cm forward and 0.5 cm backwards of the center of the core;
- for the back overall average zone 0.5 cm backward of the center of the core to the rear distal edge of the core

The length (ZL) and width (ZW) of each zone is recorded. Then the previously marked zones are cut out and the record weight (ZWT) of each zone is taken.

Before calculating the basis weight, the area of each zone must first be calculated as follows:

$$Zonal\ Area\ (ZA) = (ZW \times ZL)\ [cm^2]$$

The Zonal Basis Weight (ZBW) is then calculated as follows:

$$Zonal\ Basis\ Weight\ (ZBW) = ZWT\ /\ (ZW * ZL) * 10000\ [g/m^2]$$

For example, if ZW is 6 cm, ZL is 10 cm and ZWT is 4,5 g the Zonal Basis Weight (ZBW) is:

$$ZBW = 4.5g / (6cm \times 10cm) * 10000 = 750 \text{ gsm}$$

Conversion of Gram per Square Centimeter $g/cm^2$ to Gram per Square Meter $g/m^2$:

$$10\ 000 \times g/cm^2 = g/m^2$$

Conversion of Gram per Square Meter $g/m^2$ to Gram per Square Centimeter $g/cm^2$:

$$0.0001 \times g/m^2 = g/cm^2$$

Density of a fluid-absorbent core

This test determines the density of the fluid-absorbent core in the point of interest.

The fluid-absorbent article is clamped nonwoven side up onto the inspection table. The insult point is marked on the article accordingly with regard to the type and gender of the diaper to be tested (i.e. in the centre of the fluid-absorbent core for girl, 2.5cm towards the front for unisex and 5cm towards the front for boy).

Next, a 6cm x core width section is marked on the fluid-absorbent core with the point of interest in the centre of the section. Three readings of thickness of the section are taken using a Portable Thickness Gauge Model J100 (SDL Atlas, Inc.; Stockport; UK) and the average is recorded (T). The section of the fluid-absorbent core is cut out of and the weight of the cut out section is recorded (WT).

The density of the fluid-absorbent core is calculated as follows:

$$\text{Density } [g/cm^3] = WT/(36cm^2 \times T)$$

Zonal Density of a fluid-absorbent article

This test will determine absorbent core density of individual zones of a hygiene article.

1. Preparation of the article(s):

a. The article is pinned nonwoven face upwards onto an inspection table.

b. The width of the absorbent core in the crotch area is measured, and a longitudinal panel the same width as crotch area, from front to back of the hygiene article is marked.

c. The insult point is marked on the article. The point is gender specific i.e. in the centre of the core for girl product, 2.5cm from centre towards front for unisex and 5cm from centre towards front for boy products.

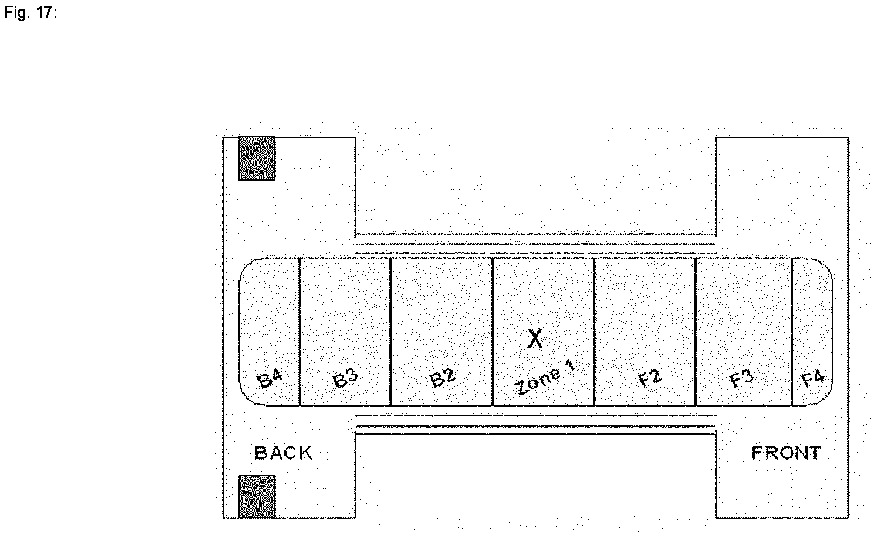

d. A line 3cm either side of insult point is marked, this zone is to be labelled insult zone.
The remainder of the article both front and back is devided into 6cm zones, if the end zones are smaller than 2.0cm then it should be included in the previous zone.
The zones should be marked as F2, F3, F4 etc to the front and B2, B3, B4, etc to the back of the hygiene article. See fig. 17

e. The length (ZL) and width (ZW) of each zone is recorded.

2. Determination of the zonal thickness.

a. Each zone is taken in turn; three readings of thickness of each are taken and the average (ZT) recorded. The average thickness is recorded for all zones

b. Longitudinal panel are cut out and panel is cut into previously marked zones.

c. Each zone is taken in turn weight as (ZWT) recorded. The weight is recorded for all zones

Before calculating the zonal density, the volume of each zone is first calculated.

$$\textit{Zonal Volume } ZV = ZW * ZL * ZT \textit{ [cm³]}$$

$$\text{Zonal Density } ZD = \frac{ZWT}{ZV} \text{ [g/cm³]}$$

Saline Flow Conductivity (SFC)

The saline flow conductivity is, as described in EP 0 640 330 A1, determined as the gel layer permeability of a swollen gel layer of water-absorbent polymer particles, although the apparatus described on page 19 and in figure 8 in the aforementioned patent application was modified to the effect that the glass frit (40) is no longer used, the plunger (39) consists of the same polymer material as the cylinder (37) and now comprises 21 bores having a diameter of 9.65mm each distributed uniformly over the entire contact surface. The procedure and the evaluation of the measurement remains unchanged from EP 0 640 330 A1. The flow rate is recorded automatically.

The saline flow conductivity (SFC) is calculated as follows:

$$\text{SFC [cm³s/g] = (Fg(t=0)xL0)/(dxAxWP),}$$

where $Fg(t = 0)$ is the flow rate of NaCl solution in g/s, which is obtained by means of a linear regression analysis of the $Fg(t)$ data of the flow determinations by extrapolation to $t = 0$, L0 is the thickness of the gel layer in cm, d is the density of the NaCl solution in $g/cm^3$, A is the surface area of the gel layer in $cm^2$ and WP is the hydrostatic pressure over the gel layer in $dyn/cm^2$.

Free Swell Rate (FSR)

1.00 g (= W1) of the dry water-absorbent polymer particles is weighed into a 25 ml glass beaker and is uniformly distributed on the base of the glass beaker. 20 ml of a 0.9% by weight sodium chloride solution are then dispensed into a second glass beaker, the content of this beaker is rapidly added to the first beaker and a stopwatch is started. As soon as the last drop of salt solution is absorbed, confirmed by the disappearance of the reflection on the liquid surface, the stopwatch is stopped. The exact amount of liquid poured from the second beaker and absorbed by the polymer in the first beaker is accurately determined by weighing back the second beaker (=W2). The time needed for the absorption, which was measured with the stopwatch, is denoted t. The disappearance of the last drop of liquid on the surface is defined as time t.

The free swell rate (FSR) is calculated as follows:

$$\text{FSR [g/gs] = W2/(W1xt)}$$

When the moisture content of the hydrogel-forming polymer is more than 3% by weight, however, the weight W1 must be corrected for this moisture content.

Water Vapor Transmission Rate (WVTR)

The water vapor transmission rate (WVTR) is determined according to the test method written in US 6,217,890, column 32, lines 15 to 56.

Anticaking

The anticaking is determined according to the test method written in WO 2005/097881 A1, page 19, lines 14 to 24. For quantitative ranking grades are given between 1 and 5, whereby grade 1 does not leave any residue in the beaker and at grade 5 no material can be poured out of the beaker.

Residual Monomers

The level of residual monomers in the water-absorbent polymer particles is determined by the EDANA recommended test method No. WSP 210.3- (11) "Residual Monomers".

Particle Size Distribution

The particle size distribution of the water-absorbent polymer particles is determined with the Camziser® image analysis sytem (Retsch Technology GmbH; Haan; Germany).

For determination of the average particle diameter and the particle diameter distribution the proportions of the particle fractions by volume are plotted in cumulated form and the average particle diameter is determined graphically.

The average particle diameter (APD) here is the value of the mesh size which gives rise to a cumulative 50% by weight.

The particle diameter distribution (PDD) is calculated as follows:

$$PDD = \frac{x_2 - x_1}{APD},$$

wherein $x_1$ is the value of the mesh size which gives rise to a cumulative 90% by weight and $x_2$ is the value of the mesh size which gives rise to a cumulative 10% by weight.

Mean Sphericity

The mean sphericity is determined with the Camziser® image analysis system (Retsch Technology GmbH; Haan; Germany) using the particle diameter fraction from 100 to 1,000 $\mu$m.

Moisture Content

The moisture content of the water-absorbent polymer particles is determined by the EDANA recommended test method No. WSP 230.3 (11) "Mass Loss Upon Heating".

Centrifuge Retention Capacity (CRC)

The centrifuge retention capacity of the water-absorbent polymer particles is determined by the EDANA recommended test method No. WSP 241.3 (11) "Free Swell Capacity in Saline, After Centrifugation", wherein for higher values of the centrifuge retention capacity larger tea bags have to be used.

Absorbency Under No Load (AUNL)

The absorbency under no load of the water-absorbent polymer particles is determined analogously to the EDANA recommended test method No. WSP 242.3 (11) "Gravimetric Determination of Absorption Under Pressure", except using a weight of 0.0 g/cm$^2$ instead of a weight of 21.0 g/cm$^2$.

Absorbency Under Load (AUL)

The absorbency under load of the water-absorbent polymer particles is determined by the EDANA recommended test method No. WSP 242.3 (11) "Gravimetric Determination of Absorption Under Pressure"

Absorbency Under High Load (AUHL)

The absorbency under high load of the water-absorbent polymer particles is determined analogously to the EDANA recommended test method No. WSP 242.3 (11) "Gravimetric Determination of Absorption Under Pressure", except using a weight of 49.2 g/cm$^2$ instead of a weight of 21.0 g/cm$^2$.

Volumetric Absorbency Under Load (VAUL)

The volumetric absorbency under a load is used in order to measure the swelling kinetics, i.e. the characteristic swelling time, of water-absorbent polymer particles under different applied pressures. The height of swelling is recorded as a function of time.

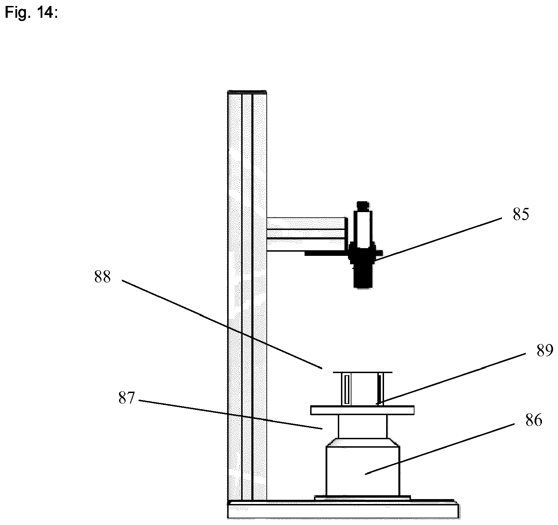

The set up is shown in Figure 14 and consists of

- An ultrasonic distance sensor (85) type BUS M18K0-XBFX-030-S04K (Balluff GmbH, Neuhausen a.d.F.; Germany) is placed above the cell. The sensor receives ultrasound reflected by the metal plate. The sensor is connected to an electronic recorder.
- A PTFE cell (86) having a diameter of 75 mm, a height of 73 mm and an internal diameter of 52 mm
- A cylinder (87) made of metal or plastic having a diameter of 50 mm, a height of 71 mm and a mesh bottom)
- A metal reflector (88) having a diameter of 57 mm and a height of 45 mm
- Metal ring weights (89) having a diameter of 100mm and weights calibrated to 278.0 g or 554.0 g

It is possible to adjust the pressure applied to the sample by changing the combination of cylinder (86) and metal ring (88) weight as summarized in the following tables:

| Available Equipment | Weight | psi |
| --- | --- | --- |
| Metal reflector | 13.0 g | 0.009 |
| Plastic cylinder | 28.0 g | 0.020 |
| Metal cylinder | 126,0 g | 0.091 |
| Small ring weight | 278.0 g | 0.201 |
| Large ring weight | 554.0 g | 0.401 |

| Possible Combinations | psi |
| --- | --- |
| Metal reflector + plastic cylinder | 0.03 |
| Metal reflector + metal cylinder | 0.10 |
| Metal reflector + metal cylinder + small ring weight | 0.30 |
| Metal reflector + metal cylinder + large ring weight | 0.50 |
| Metal reflector + metal cylinder + small ring weight + large ring weight | 0.70 |

A sample of 2.0 g of water-absorbent polymer particles is placed in the PTFE cell (86). The cylinder (equipped with mesh bottom) and the metal reflector (88) on top of it are placed into the PTFE cell (86). In order to apply higher pressure, metal rings weights (89) can be placed on the cylinder.

60.0 g of aqueous saline solution (0.9% by weight) are added into the PTFE cell (86) with a syringe and the recording is started. During the swelling, the water-absorbent polymer particles push the cylinder (87) up and the changes in the distance between the metal reflector (88) and the sensor (85) are recorded.

After 120 minutes, the experiment is stopped and the recorded data are transferred from the recorder to a PC using a USB stick. The characteristic swelling time is calculated according to the equation $Q(t)=Q_{max} \cdot (1-e^{-t/\tau})$ as described by "Modern Superabsorbent Polymer Technology" (page 155, equation 4.13) wherein Q(t) is the swelling of the superabsorbent which is monitored during the experiment, $Q_{max}$ corresponds to the maximum swelling reached after 120 minutes (end of the experiment) and $\tau$ is the characteristic swelling time ($\tau$ is the inverse rate constant k).

Using the add-in functionality "Solver" of Microsoft Excel software, a theoretical curve can be fitted to the measured data and the characteristic time for 0.03 psi is calculated.

The measurements are repeated for different pressures (0.1 psi, 0.3 psi, 0.5 psi and 0.7 psi) using combinations of cylinder and ring weights. The characteristic swelling times for the different pressures can be calculated using the equation $Q(t)=Q_{max} \cdot (1-e^{-t/\tau})$.

Wicking absorption

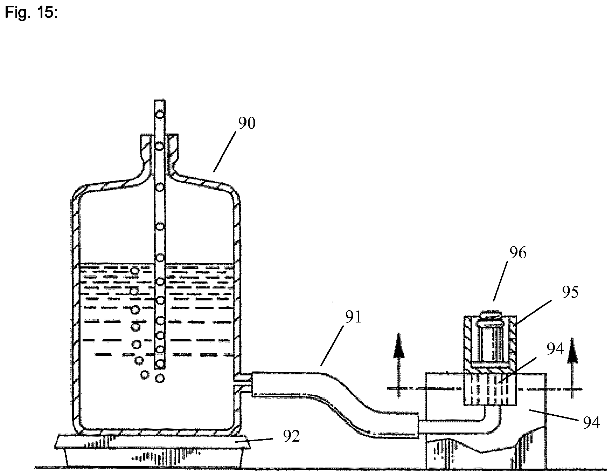

The wicking absorption is used in order to measure the total liquid uptake of water-absorbent polymer particles under applied pressure. The set-up is show in Figure 15.

A 500 ml glass bottle (90) (scale division 100 mL, height 26.5 cm) equipped with an exit tube of Duran® glass, is filled with 500 mL of aqueous saline solution (0.9% by weight). The bottle has an opening at the bottom end which can be connected to the Plexiglas plate through a flexible hose (91).

A balance (92) connected to a computer is placed on Plexiglas block (area 20 x 26 cm$^2$, height 6 cm). The glass bottle is then placed on the balance.

A Plexiglas plate (93) (area: 11 x 11 cm$^2$, height: 3.5 cm) is placed on a lifting platform. A porosity P1 glass frit of 7cm in diameter and 0.45 cm high (94) has been liquid-tightly embedded in the Plexiglas plate, i.e. the fluid exits through the pores of the frit and not via the edge between Plexiglas plate and frit. A Plexiglas tube leads through the outer shell of Plexiglas plate into the center of the Plexiglas plate up to the frit to ensure fluid transportation. The fluid tube is then connected with the flexible hose (35 cm in length, 1.0 cm external diameter, 0.7 cm internal diameter) to the glass bottle (90).

The lifting platform is used to adjust the upper side of the frit to the level of the bottom end of the glass bottle, so that an always atmospheric flux of fluid from the bottle to the measuring apparatus is ensured during measurement. The upper side of the frit is adjusted such that its surface is moist but there is no supernatant film of water on the frit.

The fluid in the glass bottle (90) is made up to 500 mL before every run.

In a Plexiglas cylinder (95) (7 cm in external diameter, 6 cm in internal diameter, 16 cm in height) and equipped with a 400 mesh (36μm) at the bottom are placed 26 g of water-absorbent polymer particles. The surface of the water-absorbent polymer particles is smoothed. The fill level is about 1.5 cm. Then a weight (96) of 0.3 psi (21.0 g/cm$^2$) is placed on top of the water-absorbent polymer particles.

The Plexiglas cylinder is placed on the (moist) frit and the electronic data recording started. A decrease in the weight of the balance is registered as a function of time. This then indicates how much aqueous saline solution has been absorbed by the swelling gel of water-absorbent polymer particles at a certain time. The data are automatically captured every 10 seconds. The measurement is carried out at 0.3 psi (21.0 g/cm$^2$) for a period of 120 minutes per sample. The total liquid uptake is the total amount of aqueous saline solution absorbed by each 26 g sample.

Porosity

The porosity of the water-absorbent polymer particles is calculated as follows:

$$\text{Porosity} = \frac{\text{AUNL} - \text{CRC}}{\text{AUNL}}$$

Bulk Density/Flow Rate

The bulk density (BD) and the flow rate (FR) of the water-absorbent polymer particles is determined by the EDANA recommended test method No. WSP 250.3 (11) "Gravimetric Determination of flow rate, Gravimetric Determination of Density".

Extractables

The level of extractable constituents in the water-absorbent polymer particles is determined by the EDANA recommended test method No. WSP 470.2-05 "Extractables".

Hunter Color (HC60) and b-Value

This test procedure is a method of measuring the perceived color of polymer related to its spectral characteristics. Spectral characteristics are specified by reflectance (or transmittance) as a function of wavelength. The measurement is performed on the polymer powder using a MACBETH Color-Eye 2180 Spectrophotometer according to the manufacturer's instructions, using a reflection cuvette or 35×10 mm petri dish with lid as a sample cell.

In this system, "L" is a measure of the lightness of a sample, and ranges from 0 (black) to 100 (white), "b" is a measure of yellowness (positive b-values) or blueness (negative b-values), Hunter Color HC60 is defined as: HC60=L-3b.

Softness

The softness of the absorbent structure is an important factor contributing to the overall conformability of the structure. As used herein, "softness" is the inverse of the amount of energy necessary to compress a sheet, in this case the sheet being the absorbent structure. The greater the amount of energy necessary to compress a sheet, the less soft it is.

To measure softness of the core, the following procedure (a modified compression test) according to WO 2000/041882 is used:

1. Prepare samples by cutting three 4 inch.times.8 inch pieces (if sample is a diaper, cut from the thicker section of diaper (if thickness is not uniform). For samples with obvious machine direction and cross direction, cut 8-inch dimension in machine direction.

2. Allow plastic backsheet and coverstock material to remain on sample (applies to commercial diaper samples). If testing prototype core samples, apply plastic backsheet, Exxon EMB-685 polyethylene film, to bottom of sample and coverstock, 15 gsm Avgol spunbond polypropylene, to top of sample (same size as sample, adhered with a small amount of spray adhesive).

3. Program modified compression test (for example, a Thwing-Albert LT-150 Universal Materials Tester): Compression test using following non-default settings: Break Detection Method= percent Drop/Displacement, Break Value= percent Drop=50, Distance Traps=0.3 in./0.5 in./0.7 in., Units: Distance/Displacement=inches; Force=grams, Test speed=1 in./min. All other settings left at defaults.

4. Insert sample into Tensile Tester using custom clamps as depicted in Fig. 3. Sample is inserted on its edge, such that it will be compressed in the y-direction (4-inch direction), having 1 inch on both edges within the custom clamps, thus leaving a 2-inch gap.

5. Start test.

6. When deflection exceeds 0.7 inch, push down on top pressurized clamp to simulate a sample break and stop the test (does not affect test results). Record results displayed. These results include Force at Peak, Deflection at Peak, Maximum Deflection, Energy at Peak, and Energy at Maximum Deflection, and Force at Distance Traps.

The value, which is used to calculate the softness, is Energy at Maximum Deflection, which is expressed in Joules. Energy of Maximum Deflection, E d max, is calculated according to the following formula:

$$E_{dmax} = \int_{dmin}^{dmax} F \, d_d$$

where $E_{d\,max}$ is Energy at Maximum Deflection, F is force at given deflection, d and d min and d max are the deflections at the start of the test and at the end of the test, respectively.

Softness, S, is defined here according to the following formula: S=1/(Energy at Maximum Deflection). The result, S, is expressed here in 1 per Joule, 1/J.

Preparation of the base polymer

Example 1

The process was performed in a concurrent spray drying plant with an integrated fluidized bed (27) and an external fluidized bed (29) as shown in Fig. 1. The cylindrical part of the spray dryer (5) had a height of 22m and a diameter of 3.4m. The internal fluidized bed (IFB) had a diameter of 3m and a weir height of 0.25m. The external fluidized bed (EFB) had a length of 3.0 m, a width of 0.65m and a weir height of 0.5m.

The drying gas was fed via a gas distributor (3) at the top of the spray dryer. The drying gas was partly recycled (drying gas loop) via a baghouse filter (9) and a condenser column (12). The drying gas was nitrogen that comprises from 1% to 4% by volume of residual oxygen: Before start of polymerization the drying gas loop was filled with nitrogen until the residual oxygen was below 4% by volume. The gas velocity of the drying gas in the cylindrical part of the spray dryer (5) was 0.8m/s. The pressure inside the spray dryer was 4mbar below ambient pressure.

The spray dryer outlet temperature was measured at three points around the circumference at the end of the cyclindrical part as shown in Fig.3. Three single measurements (47) were used to calculate the average cylindrical spray dryer outlet temperature. The drying gas loop was heated up and the dosage of monomer solution is started up. From this time the spray dryer outlet temperature was controlled to 117°C by adjusting the gas inlet temperature via the heat exchanger (20).

The product accumulated in the internal fluidized bed (27) until the weir height was reached. Conditioned internal fluidized bed gas having a temperature of 122°C and a relative humidity of 4% was fed to the internal fluidized bed (27) via line (25). The gas velocity of the internal fluidized bed gas in the internal fluidized bed (27) was 0.80m/s. The residence time of the product was 120min.

The spray dryer offgas was filtered in baghouse filter (9) and sent to a condenser column (12) for quenching/cooling. Excess water was pumped out of the condenser column (12) by controlling the (constant) filling level inside the condenser column (12). The water inside the condenser column (12) was cooled by a heat exchanger (13) and pumped counter-current to the gas via quench nozzles (11) so that the temperature inside the condenser column (12) was 45 °C. The water inside the condenser column (12) was set to an alkaline pH by dosing sodium hydroxide solution to wash out acrylic acid vapors.

The condenser column offgas was split to the drying gas inlet pipe (1) and the conditioned internal fluidized bed gas (25). The gas temperatures were controlled via heat exchangers (20) and (22). The hot drying gas was fed to the concurrent spray dryer via gas distributor (3). The gas distributor (3) consists of a set of plates providing a pressure drop of 2 to 4mbar depending on the drying gas amount.

The product was discharged from the internal fluidized bed (27) via rotary valve (28) into external fluidized bed (29). Conditioned external fluidized bed gas having a temperature of 60°C was fed to the external fluidized bed (29) via line (40). The external fluidized bed gas was air. The gas velocity of the external fluidized bed gas in the external fluidized bed (29) was 0.8m/s. The residence time of the product was 1min.

The product was discharged from the external fluidized bed (29) via rotary valve (32) into sieve (32). The sieve (33) was used for sieving off overs/lumps having a particle diameter of more than 800 µm.

The monomer solution was prepared by mixing first acrylic acid with 3-tuply ethoxylated glycerol triacrylate (internal crosslinker) and secondly with 37.3% by weight sodium acrylate solution. The temperature of the resulting monomer solution was controlled to 10 °C by using a heat exchanger and pumping in a loop. A filter unit having a mesh size of 250µm was used in the loop after the pump. The initiators were metered into the monomer solution upstream of the dropletizer by means of static mixers (41) and (42) via lines (43) and (44) as shown in Fig.1. Sodium peroxodisulfate solution having a temperature of 20°C was added via line (43) and [2,2'-azobis[2-(2-imidazolin-2-yl)propane]dihydrochloride solution together with Bruggolite FF7 having a temperature of 5°C was added via line (44). Each initiator was pumped in a loop and dosed via control valves to each dropletizer unit. A second filter unit having a mesh size of 140 µm was used after the static mixer (42). For dosing the monomer solution into the top of the spray dryer three dropletizer units were used as shown in Fig.4.

A dropletizer unit consisted of an outer pipe (51) having an opening for the dropletizer cassette (53) as shown in Fig.5. The dropletizer cassette (53) was connected with an inner pipe (52). The inner pipe (53) having a PTFE block (54) at the end as sealing can be pushed in and out of the outer pipe (51) during operation of the process for maintenance purposes.

The temperature of the dropletizer cassette (61) was controlled to 8°C by water in flow channels (59) as shown in Fig.6. The dropletizer cassette (61) had 256 bores having a diameter of 170µm and a bore separation of 15mm. The dropletizer cassette (61) consisted of a flow channel (60) having essential no stagnant volume for homogeneous distribution of the premixed monomer and initiator solutions and one droplet plate (57). The droplet plate (57) had an angled configuration with an angle of 3°. The droplet plate (57) was made of stainless steel and had a length of 630mm, a width of 128mm and a thickness of 1mm.

The feed to the spray dryer consisted of 10.45% by weight of acrylic acid, 33.40% by weight of sodium acrylate, 0.018% by weight of 3-tuply ethoxylated glycerol triacrylate, 0.072% by weight of [2,2'-azobis[2-(2-imidazolin-2-yl)propane]dihydrochloride, 0.0029% by weight of Bruggolite FF7 (5% by weight in water), 0.054% by weight of sodiumperoxodisulfate solution (15% by weight in water) and water. The degree of neutralization was 71%. The feed per bore was 1.6 kg/h.

The polymer particles (base polymer A1) exhibit the following features and absorption profile:

CRC of 40.2 g/g
AUNL of 51.8 g/g
AUL of 22.4 g/g
AUHL of 8.2 g/g
Porosity of 22.3%
Extractables of 4.3 wt.%
Residual monomers of 12161 ppm
Moisture content of 6.1 wt.%
Vortex time of 67 s

The resulting polymer particles had a bulk density of 68g/100ml and an average particle diameter of 407µm.

Example 2

Example 1 was repeated, except that the resulting polymer particles having a content of the residual monomer of 12161 ppm were demonomerized in a plastic bottle in the lab oven at 90°C for 60 minutes after spraying 15% by weight of water onto the polymer particles in a laboratory ploughshare mixer. Therefore the content of the residual monomer was decreased to 256 ppm and the moisture content was increased to 17.5% by weight.

The polymer particles (base polymer B1) exhibit the following features and absorption profile:

CRC of 33.1 g/g
AUNL of 42.3 g/g
AUL of 17.0 g/g
AUHL of 8.1 g/g
Porosity of 21.7%
Extractables of 8.2 wt.%
Residual monomers of 256 ppm
Moisture content of 17.5 wt.%
Vortex time of 54 s

Example 3

Example 1 was repeated, except that the feed to the spray dryer consisted of 0.036% by weight of [2,2'-azobis[2-(2-imidazolin-2-yl)propane]dihydrochloride and that the conditioned internal fluidized bed gas had a temperature of 122°C and a relative humidity of 4% and that the residence time of the product in the internal fluidized bed was 120min.

The polymer particles (base polymer C1) exhibit the following features and absorption profile:

CRC of 39.5 g/g
AUNL of 51.4 g/g
AUHL of 9.0 g/g
Porosity of 23.2%
Residual monomers of 1581 ppm
Moisture content of 10.9 wt.%

Surface-postcrosslinking of the base polymer

Example 4

1200 g of the water-absorbent polymer particles prepared in Example 1 (base polymer A1) having a content of residual monomers of 12161 ppm were put into a laboratory ploughshare mixer (model MR5, manufactured by Gebrüder Lödige Maschinenbau GmbH; Paderborn; Germany). A surface-postcrosslinker solution was prepared by mixing 60g of surface-postcrosslinker as described in Table 1 and 60g of deionized water, into a beaker. At a mixer speed of 200 rpm, the aqueous solution was sprayed onto the polymer particles within one minute by means of a spray nozzle. The mixing was continued for additional 5 minutes. The product was removed and transferred into another ploughshare mixer (model MR5, manufactured by Gebrüder Lödige Maschinenbau GmbH; Paderborn; Germany) which was heated to 140°C before. After mixing for further 80 minutes at 140°C with sample taking every 10 minutes, the product was removed from the mixer and sifted from 150 to 850 μm. The samples were analyzed. The results are summarized in Table 1.

The resulting polymer particles that were surface-postcrosslinked with ethylene carbonate had a bulk density of 69.0g/100ml, an average particle diameter (APD) of 481μm, a particle diameter distribution (PDD) of 0.28, and a mean sphericity of 0.82.

Tab. 1: Effect of the postcrosslinking agent

| Postcrosslinker | Curing Time [min] | Residual Monomers [ppm] | Moisture [wt.%] | CRC [g/g] | AUNL [g/g] | AUL [g/g] | AUHL [g/g] | Porosity [%] | Extractables [wt.%] | Vortex [s] |
|---|---|---|---|---|---|---|---|---|---|---|
| EC | 10 | 533 | 10.1 | 49.2 | 58.1 | 18.8 | 8.2 | 15.3 | 5.3 | 78 |
| | 20 | 384 | 7.0 | 51.4 | 60.8 | 20.6 | 8.0 | 15.5 | 4.5 | 77 |
| | 30 | 376 | 5.1 | 51.6 | 61.0 | 28.0 | 9.5 | 15.4 | 4.8 | 76 |
| | 40 | 386 | 3.6 | 50.5 | 62.1 | 31.9 | 12.9 | 18.7 | 3.8 | 74 |
| | 50 | 432 | 1.9 | 49.1 | 61.8 | 36.2 | 18.2 | 20.6 | 3.4 | 70 |
| | 60 | 416 | 1.1 | 47.3 | 61.2 | 37.9 | 22.2 | 22.7 | 3.1 | 75 |
| | 70 | 417 | 0.6 | 46.7 | 62.5 | 38.6 | 24.3 | 25.3 | 3.1 | 75 |
| | 80 | 429 | 0.4 | 46.2 | 62.6 | 39.5 | 24.2 | 26.2 | 3.1 | 75 |
| HEONON | 10 | 1538 | 7.6 | 50.2 | 58.4 | 16.6 | 7.7 | 14.0 | 7.0 | 86 |
| | 20 | 1377 | 5.3 | 51.3 | 59.7 | 16.3 | 7.8 | 14.1 | 6.8 | 85 |
| | 30 | 1153 | 3.7 | 52.5 | 61.3 | 17.7 | 7.9 | 14.4 | 7.1 | 84 |
| | 40 | 1061 | 2.8 | 51.5 | 61.4 | 19.8 | 8.0 | 16.1 | 6.6 | 86 |
| | 50 | 1002 | 2.5 | 50.9 | 60.3 | 25.2 | 8.7 | 15.6 | 6.3 | 85 |
| | 60 | 997 | 2.4 | 49.1 | 59.9 | 27.7 | 10.3 | 18.0 | 6.0 | 82 |
| | 70 | 939 | 2.2 | 48.1 | 59.8 | 30.0 | 13.1 | 19.6 | 5.8 | 81 |
| | 80 | 913 | 2.0 | 47.3 | 59.3 | 32.6 | 16.2 | 20.2 | 5.1 | 81 |
| EGDGE | 10 | 714 | 8.0 | 34.2 | 43.7 | 23.1 | 17.1 | 21.7 | 22.1 | 95 |
| | 20 | 572 | 5.4 | 35.2 | 44.7 | 23.6 | 17.2 | 21.3 | 23.3 | 93 |
| | 30 | 554 | 3.9 | 35.9 | 45.0 | 24.2 | 17.8 | 20.2 | 23.8 | 91 |
| | 40 | 549 | 2.8 | 36.3 | 45.5 | 24.4 | 18.0 | 20.2 | 24.2 | 94 |
| | 50 | 544 | 2.2 | 36.6 | 46.2 | 24.5 | 18.3 | 20.8 | 23.5 | 96 |
| | 60 | 550 | 1.9 | 36.6 | 45.9 | 23.8 | 18.4 | 20.3 | 23.8 | 93 |
| | 70 | 542 | 1.6 | 36.5 | 46.3 | 23.8 | 18.4 | 21.2 | 23.9 | 94 |
| | 80 | 562 | 1.4 | 37.1 | 46.4 | 24.0 | 18.5 | 20.0 | 24.1 | 92 |
| EC: Ethylene carbonate; HEONON: N-(2-hydroxy ethyl)-2-oxazolidinone; EGDGE: Ethylene glycol diglycidyl ether | | | | | | | | | | |

Example 5

1200 g of the water-absorbent polymer particles as described in Table 2 having different contents of residual monomers were put into a laboratory ploughshare mixer (model MR5, manufactured by Gebrüder Lödige Maschinenbau GmbH; Paderborn; Germany). A surface-postcrosslinker solution was prepared by mixing 30g ethylene carbonate and 30g of deionized water, into a beaker. At a mixer speed of 200 rpm, the aqueous solution was sprayed onto the polymer particles within one minute by means of a spray nozzle. The mixing was continued for additional 5 minutes. The product was removed and transferred into another ploughshare mixer (model MR5, manufactured by Gebrüder Lödige Maschinenbau GmbH; Paderborn; Germany) which was heated to 150°C before. After mixing for further 80 minutes at 150°C with sample taking every 10 minutes, the product was removed from the mixer and sifted from 150 to 850 $\mu$m. The samples were analyzed. The results are summarized in Table 2.

The resulting polymer particles based on base polymer A1 had a bulk density of 68.0g/100ml, an average particle diameter (APD) of 397$\mu$m, a particle diameter distribution (PDD) of 0.38, and a mean sphericity of 0.87.

The resulting polymer particles based on base polymer B1 had a bulk density of 64.7g/100ml, an average particle diameter (APD) of 553$\mu$m, a particle diameter distribution (PDD) of 0.25, and a mean sphericity of 0.74.

The resulting polymer particles based on base polymer C1 had a bulk density of 69.8g/100ml, an average particle diameter (APD) of 377$\mu$m, a particle diameter distribution (PDD) of 0.42, and a mean sphericity of 0.86.

Example 6

1200 g of the water-absorbent polymer particles as described in Table 3 having different contents of residual monomers were put into a laboratory ploughshare mixer (model MR5, manufactured by Gebrüder Lödige Maschinenbau GmbH; Paderborn; Germany). A surface-postcrosslinker solution was prepared by mixing 30g ethylene carbonate and 60g of deionized water, into a beaker. At a mixer speed of 200 rpm, the aqueous solution was sprayed onto the polymer particles within one minute by means of a spray nozzle. The mixing was continued for additional 5 minutes. The product was removed and transferred into another ploughshare mixer (model MR5, manufactured by Gebrüder Lödige Maschinenbau GmbH; Paderborn; Germany) which was heated to 140°C before. After mixing for further 80 minutes at 140°C with sample taking every 10 minutes, the product was removed from the mixer and sifted from 150 to 850 $\mu$m. The samples were analyzed. The results are summarized in Table 3.

The resulting polymer particles based on base polymer A1 had a bulk density of 65.6g/100ml, an average particle diameter (APD) of 450$\mu$m, a particle diameter distribution (PDD) of 0.32, and a mean sphericity of 0.82.

The resulting polymer particles based on base polymer B1 had a bulk density of 64.7g/100ml, an average particle diameter (APD) of 564$\mu$m, a particle diameter distribution (PDD) of 0.22, and a mean sphericity of 0.75.

The resulting polymer particles based on base polymer C1 had a bulk density of 70.3g/100ml, an average particle diameter (APD) of 399$\mu$m, a particle diameter distribution (PDD) of 0.36, and a mean sphericity of 0.84.

Tab. 2: Effect of the residual monomers

| Base Polymer | Curing Time [min] | Residual Monomers [ppm] | Moisture [wt.%] | CRC [g/g] | AUNL [g/g] | AUL [g/g] | AUHL [g/g] | Porosity [%] | Extractables [wt.%] | Vortex [s] |
|---|---|---|---|---|---|---|---|---|---|---|
| Base Polymer A1 | 10 | 2187 | 5.2 | 57.5 | 61.4 | 11.0 | 7.6 | 6.4 | 8.4 | |
| | 20 | 1646 | 3.0 | 57.7 | 63.8 | 12.6 | 8.2 | 9.6 | 7.2 | |
| | 30 | 1273 | 1.5 | 53.5 | 65.4 | 33.5 | 11.6 | 18.2 | 5.3 | |
| | 40 | 1229 | 0.5 | 51.5 | 63.9 | 38.6 | 18.8 | 19.4 | 4.7 | |
| | 50 | 1225 | 0.5 | 49.7 | 63.5 | 40.6 | 24.5 | 21.7 | 5.0 | |
| | 60 | 1214 | 0.3 | 47.6 | 63.5 | 40.8 | 27.6 | 25.0 | 6.1 | 70 |
| | 70 | 1243 | 0.3 | 48.1 | 62.7 | 40.3 | 30.2 | 23.3 | 6.2 | 69 |
| | 80 | 1225 | 0.1 | 46.4 | 60.1 | 38.2 | 30.7 | 22.8 | 6.3 | 67 |
| Base Polymer B1 | 10 | 133 | 13.7 | 35.8 | | | | | | |
| | 20 | 133 | 8.4 | 36.9 | | | | | | |
| | 30 | 136 | 3.3 | 35.5 | | | | | | 89 |
| | 40 | 157 | 1.9 | 33.2 | | | | | 7,5 | 93 |
| | 50 | 160 | 1.3 | 30.9 | 43.0 | 27.7 | 18.9 | 28.1 | | 92 |
| | 60 | 166 | 0.9 | 28.8 | 40.9 | 27.3 | 20.3 | 29.6 | | 95 |
| | 70 | 172 | 0.7 | 26.7 | 38.2 | 26.6 | 21.2 | 30.1 | | 94 |
| | 80 | 178 | 0.7 | 25.9 | 36.9 | 26.4 | 21.2 | 29.8 | 3,7 | 94 |
| Base Polymer C1 | 10 | 399 | 8.0 | 42.2 | 53.8 | 28.1 | 10.7 | 21.6 | | 70 |
| | 20 | 398 | 3.8 | 43.3 | 56.6 | 32.1 | 13.6 | 23.5 | | 70 |
| | 30 | 417 | 1.8 | 44.1 | 56.9 | 36.4 | 21.7 | 22.5 | | 71 |
| | 40 | 446 | 1.3 | 43.0 | 55.9 | 38.4 | 25.4 | 23.1 | 3.1 | 73 |
| | 50 | 419 | 1.0 | 39.5 | 54.3 | 36.9 | 29.0 | 27.3 | | 73 |
| | 60 | 413 | 0.8 | 38.7 | 52.1 | 37.0 | 29.5 | 25.7 | | 75 |
| | 70 | 403 | 0.6 | 37.6 | 51.6 | 36.2 | 29.4 | 27.1 | | 76 |
| | 80 | 402 | 0.6 | 36.4 | 51.3 | 35.0 | 29.7 | 29.0 | 3.3 | 76 |

Tab. 3: Effect of the residual monomers

| Base Polymer | Curing Time [min] | Residual Monomers [ppm] | Moisture [wt.%] | CRC [g/g] | AUNL [g/g] | AUL [g/g] | AUHL [g/g] | Porosity [%] | Extractables [wt.%] | Vortex [s] |
|---|---|---|---|---|---|---|---|---|---|---|
| Base Polymer A1 | 10 | 506 | 7.7 | 51.7 | 59.3 | 17.4 | 7.8 | 12.8 | 5.9 | |
| | 20 | 417 | 4.9 | 53.0 | 61.2 | 22.1 | 8.0 | 13.4 | 5.5 | |
| | 30 | 312 | 2.7 | 51.6 | 61.9 | 29.8 | 10.8 | 16.6 | 4.8 | |
| | 40 | 328 | 2.0 | 52.2 | 62.3 | 35.5 | 14.6 | 16.2 | 4.1 | |
| | 50 | 359 | 1.1 | 50.1 | 62.1 | 37.3 | 19.0 | 19.3 | 4.3 | |
| | 60 | 370 | 0.8 | 49.5 | 62.9 | 38.4 | 22.6 | 21.3 | 4.3 | |
| | 70 | 385 | 0.7 | 47.1 | 61.9 | 39.0 | 25.4 | 23.9 | 4.2 | 73 |
| | 80 | 340 | 0.5 | 45.3 | 60.3 | 37.9 | 27.5 | 24.9 | 4.0 | 72 |
| Base Polymer B1 | 10 | | | 33.7 | | | | | | |
| | 20 | | 0.8 | 36.1 | | | 8.1 | | | |
| | 30 | | | 36.1 | | | 8.6 | | | |
| | 40 | 123 | 4.1 | 35.4 | 48.1 | 22.9 | 10.2 | 26.4 | | |
| | 50 | 124 | 2.8 | 33.8 | 46.5 | 25.8 | 11.7 | 27.3 | | 87 |
| | 60 | 125 | 2.3 | 32.6 | 45.0 | 25.4 | 13.1 | 27.6 | | 88 |
| | 70 | 132 | 0.9 | 31.9 | 44.8 | 26.1 | 14.4 | 28.8 | | 92 |
| | 80 | 138 | 0.8 | 31.2 | 43.8 | 26.4 | 13.7 | 28.8 | | 90 |
| Base Polymer C1 | 10 | 373 | 9.6 | 41.1 | 52.9 | 28.8 | 13.1 | 22.0 | | 70 |
| | 20 | 356 | 5.6 | 42.6 | 55.1 | 31.5 | 14.4 | 23.0 | | 71 |
| | 30 | 362 | 3.0 | 42.0 | 55.6 | 34.1 | 19.6 | 24.0 | | 72 |
| | 40 | 378 | 1.3 | 42.2 | 55.8 | 34.8 | 23.2 | 24.0 | 4.2 | 73 |
| | 50 | 381 | 1.1 | 41.4 | 55.2 | 34.7 | 24.9 | 25.0 | | 73 |
| | 60 | 389 | 0.7 | 40.2 | 55.4 | 35.2 | 26.0 | 27.0 | | 72 |
| | 70 | 396 | 0.6 | 40.9 | 53.9 | 34.4 | 26.8 | 24.0 | | 70 |
| | 80 | 395 | 0.4 | 40.6 | 53.5 | 35.2 | 26.6 | 24.0 | 4.2 | 72 |

Example 7

The process was performed in a concurrent spray drying plant with an integrated fluidized bed (27) and an external fluidized bed (29) as shown in Fig. 1. The cylindrical part of the spray dryer (5) had a height of 22m and a diameter of 3.4m. The internal fluidized bed (IFB) had a diameter of 3m and a weir height of 0.25m.

The drying gas was fed via a gas distributor (3) at the top of the spray dryer. The drying gas was partly recycled (drying gas loop) via a baghouse filter (9) and a condenser column (12). Instead of the baghouse filter (9) any other filter and/or cyclone can be used. The drying gas was nitrogen that comprises from 1% to 4% by volume of residual oxygen: Before start of polymerization the drying gas loop was filled with nitrogen until the residual oxygen was below 4% by volume. The gas velocity of the drying gas in the cylindrical part of the spray dryer (5) was 0.82 m/s. The pressure inside the spray dryer was 4mbar below ambient pressure.

The spray dryer outlet temperature was measured at three points around the circumference at the end of the cyclindrical part as shown in Fig. 3. Three single measurements (47) were used to calculate the average cylindrical spray dryer outlet temperature. The drying gas loop was heated up and the dosage of monomer solution is started up. From this time the spray dryer outlet temperature was controlled to 118°C by adjusting the gas inlet temperature via the heat exchanger (20). The gas inlet temperature was 167°C and the steam content of the drying gas was 0.058 kg steam per kg dry gas.

The product accumulated in the internal fluidized bed (27) until the weir height was reached. Conditioned internal fluidized bed gas having a temperature of 104°C and a steam content of 0.058 or 0.130 kg steam per kg dry gas was fed to the internal fluidized bed (27) via line (25). The gas velocity of the internal fluidized bed gas in the internal fluidized bed (27) was 0.65 m/s. The residence time of the product was 150 min. The temperature of the water-absorbent polymer particles in the internal fluidized bed was 82°C.

The spray dryer offgas was filtered in baghouse filter (9) and sent to a condenser column (12) for quenching/cooling. Excess water was pumped out of the condenser column (12) by controlling the (constant) filling level inside the condenser column (12). The water inside the condenser column (12) was cooled by a heat exchanger (13) and pumped counter-current to the gas via quench nozzles (11) so that the temperature inside the condenser column (12) was 45°C. The water inside the condenser column (12) was set to an alkaline pH by dosing sodium hydroxide solution to wash out acrylic acid vapors.

The condenser column offgas was split to the drying gas inlet pipe (1) and the conditioned internal fluidized bed gas (25). The gas temperatures were controlled via heat exchangers (20) and (22). The hot drying gas was fed to the concurrent spray dryer via gas distributor (3). The gas distributor (3) consists of a set of plates providing a pressure drop of 2 to 4mbar depending on the drying gas amount.

The product was discharged from the internal fluidized bed (27) via rotary valve (28) into sieve (29). The sieve (29) was used for sieving off overs/lumps having a particle diameter of more than 800 $\mu$m.

The monomer solution was prepared by mixing first acrylic acid with 3-tuply ethoxylated glycerol triacrylate (internal crosslinker) and secondly with 37.3% by weight sodium acrylate solution. The temperature of the resulting monomer solution was controlled to 10°C by using a heat exchanger and pumping in a loop. A filter unit having a mesh size of 250$\mu$m was used in the loop after the pump. The initiators were metered into the monomer solution upstream of the dropletizer by means of static mixers (41) and (42) via lines (43) and (44) as shown in Fig.1. Sodium peroxodisulfate solution having a temperature of 20°C was added via line (43) and [2,2'-azobis[2-(2-imidazolin-2-yl)propane]dihydrochloride solution together with Bruggolite FF7 having a temperature of 5°C was added via line (44). Each initiator was pumped in a loop and dosed via control valves to each dropletizer unit. A second filter unit having a mesh size of 140 $\mu$m was used after the static mixer (42). For dosing the monomer solution into the top of the spray dryer three dropletizer units were used as shown in Fig.4.

A dropletizer unit consisted of an outer pipe (51) having an opening for the dropletizer cassette (53) as shown in Fig. 5. The dropletizer cassette (53) was connected with an inner pipe (52). The inner pipe (53) having a PTFE block (54) at the end as sealing can be pushed in and out of the outer pipe (51) during operation of the process for maintenance purposes.

The temperature of the dropletizer cassette (61) was controlled to 8°C by water in flow channels (59) as shown in Fig.6. The dropletizer cassette (61) had 256 bores having a diameter of 170 $\mu$m and a bore separation of 15 mm. The dropletizer cassette (61) consisted of a flow channel (60) having essential no stagnant volume for homogeneous distribution of the premixed monomer and initiator solutions and one droplet plate (57). The droplet plate (57) had an angled configuration with an angle of 3°. The droplet plate (57) was made of stainless steel and had a length of 630mm, a width of 128mm and a thickness of 1mm.

The feed to the spray dryer consisted of 10.45% by weight of acrylic acid, 33.40% by weight of sodium acrylate, 0.018% by weight of 3-tuply ethoxylated glycerol triacrylate, 0.036% by weight of [2,2'-azobis[2-(2-imidazolin-2-yl)propane]dihydrochloride, 0.0029% by weight of Bruggolite FF7, 0.054% by weight of sodium peroxodisulfate and water. The degree of neutralization was 71%. The feed per bore was 1.4 kg/h.

The resulting water-absorbent polymer particles were analyzed. The results are summarized in Table 4.

Tab. 4: Base polymers, used for the surface-postcrosslinking reactions

| Example | Steam Content [kg/kg] | Bulk Density [g/cm$^3$] | CRC [g/g] | Residual Monomers [ppm] | Extractables [wt.%] | Moisture [wt.%] | FSR [g/gs] |
|---|---|---|---|---|---|---|---|
| 7a | 0.058 | 0.74 | 47.2 | 9300 | 4.5 | 5.7 | 0,20 |
| 7b | 0.130 | 0.71 | 49.1 | 4000 | 5.4 | 7.5 | 0,07 |

Production Examples 8 to 26

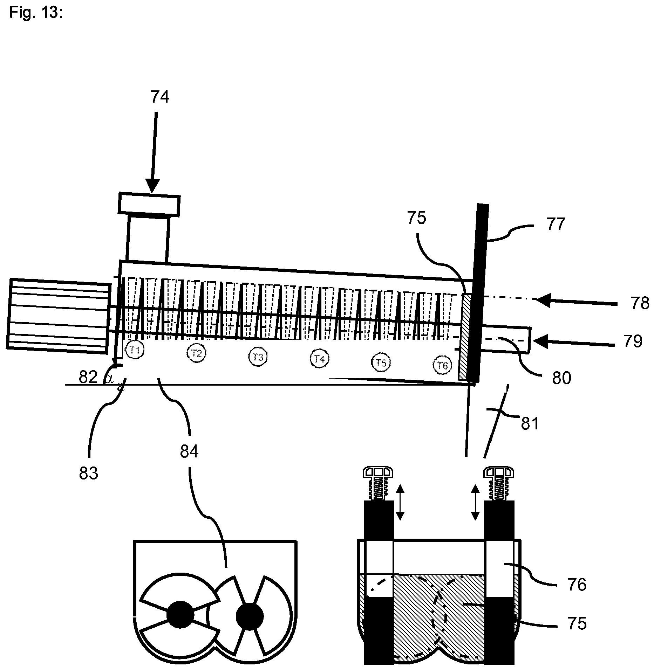

In a Schugi Flexomix® (model Flexomix-160, manufactured by Hosokawa Micron B.V., Doetinchem, the Netherlands) with a speed of 2000 rpm, the base polymer 7a or 7b was coated with a surface-postcrosslinker solution by using 2 or 3 round spray nozzle systems (model Gravity-Fed Spray Set-ups, External Mix Typ SU4, Fluid Cap 60100 and Air Cap SS-120, manufactured by Spraying Systems Co, Wheaton, Illinois, USA) and then filled via inlet (74) and dried in a NARA heater (model NPD 5W-18, manufactured by GMF Gouda, Waddinxveen, the Netherlands) with a speed of the shaft (80) of 6 rpm. The NARA heater has two paddles with a shaft offset of 90° (84) and a fixed discharge zone (75) with two flexible weir plates (77). Each weir has a weir opening with a minimal weir height at 50% (79) and a maximal weir opening at 100% (78) as shown in Fig. 13.

The inclination angle $\alpha$ (82) between the floor plate and the NARA paddle dryer is approx. 3°. The weir height of the NARA heater is between 50 to 100% corresponding to a residence time of approx. 40 to 150 min, by a product density of approx. 700 to 750 kg/m$^3$. The product temperature in the NARA heater is in a range of 120 to 165°C. After drying, the surface-postcrosslinked base polymer was transported over discharge cone (81) in the NARA cooler (GMF Gouda, Waddinxveen, the Netherlands), to cool down the surface postcrosslinked base polymer to approx. 60°C with a speed of 11 rpm and a weir height of 145 mm. After cooling, the material was sieved with a minimum cut size of 150 $\mu$m and a maximum size cut of 710 $\mu$m.

Ethylene carbonate, water, Plantacare® UP 818 (BASF SE, Ludwigshafen, Germany) and aqueous aluminum lactate (26% by weight) was premixed and spray coated as summarized in Tab 6. Aqueous aluminum sulfate (26% by weight) was separate spray coated (position of the nozzle = 180°). As aluminum lactate, Lohtragon® Al 220 (manufactured by Dr. Paul Lohmann GmbH, Emmerthal, Germany) was used.

The metered amounts and conditions of the coating into the Schugi Flexomix®, the conditions, the formulation and values of the drying and cooling step are summarized in Table 5 to 6:

All physical properties of the resulting polymers are summarized in Table 7 and 8:

Tab. 5: Process parameters of the thermal treatment in the heater

| Production Example | Product Temp. Set Value | Steam Pressure valve | Steam Pressure Jacket | Heater T1 | Heater T2 | Heater T3 | Heater T4 | Heater T5 | Heater T6 | Through-put | Heater Weir | No. of Nozzles | Pos. of Nozzles |
|---|---|---|---|---|---|---|---|---|---|---|---|---|---|
| | °C | Bar | bar | °C | °C | °C | °C | °C | °C | kg/h | % | | |
| Polymer particles without aluminum salt | | | | | | | | | | | | | |
| 8 | 140 | 4.6 | 4.3 | 84 | 81 | 111 | 123 | 130 | 140 | 400 | 56 | 2 | 90/270° |
| 9 | 150 | 6.2 | 6.2 | 90 | 86 | 115 | 129 | 137 | 150 | 400 | 56 | 2 | 90/270° |
| 10 | 150 | 7.4 | 7.4 | 79 | 81 | 110 | 121 | 133 | 159 | 500 | 67 | 3 | 90/180/270 |
| Polymer particles with aluminum lactate | | | | | | | | | | | | | |
| 11 | 120 | 2.5 | 2.3 | 69 | 72 | 103 | 111 | 114 | 120 | 500 | 57 | 3 | 90/180/270 |
| 12 | 130 | 2.3 | 3.5 | 75 | 84 | 110 | 117 | 121 | 130 | 500 | 57 | 3 | 90/180/270 |
| 13 | 140 | 6.7 | 6.6 | 82 | 93 | 118 | 127 | 135 | 150 | 500 | 67 | 3 | 90/180/270 |
| 14 | 150 | 5.5 | 5.0 | 84 | 107 | 117 | 131 | 138 | 150 | 400 | 56 | 2 | 90/270° |
| 15 | 150 | 5.2 | 6.2 | 71 | 100 | 118 | 132 | 140 | 150 | 400 | 56 | 2 | 90/270° |
| 16 | 150 | 5.9 | 5.9 | 91 | 109 | 120 | 131 | 140 | 150 | 500 | 68 | 3 | 90/180/270 |
| 17 | 150 | 6.1 | 6.1 | 83 | 110 | 120 | 131 | 139 | 150 | 500 | 68 | 3 | 90/180/270 |
| 18 | 160 | 6.5 | 6.5 | 89 | 114 | 123 | 138 | 151 | 160 | 400 | 82 | 3 | 90/270° |
| 19 | 170 | 8.1 | 8.1 | 91 | 113 | 129 | 151 | 163 | 170 | 400 | 82 | 2 | 90/270° |
| Polymer particles with aluminum sulfate | | | | | | | | | | | | | |
| 20 | 150 | 5.6 | 5.5 | 66 | 99 | 119 | 137 | 145 | 150 | 500 | 87 | 3 | 90/180/270° |
| 21 | 150 | 5.0 | 5.0 | 95 | 96 | 121 | 135 | 144 | 150 | 400 | 75 | 3 | 90/180/270° |
| 22 | 150 | 5,6 | 5,6 | 74 | 104 | 117 | 127 | 136 | 150 | 500 | 87 | 3 | 90/180/270° |
| 23 | 155 | 6.1 | 6.0 | 79 | 100 | 119 | 130 | 142 | 155 | 500 | 87 | 3 | 90/180/270° |

EP 3 039 044 B1

| Polymer particles with aluminum sulfate | | | | | | | | | | | | |
|---|---|---|---|---|---|---|---|---|---|---|---|---|
| 24 | 160 | 6.6 | 6.6 | 109 | 115 | 124 | 143 | 154 | 160 | 400 | 75 | 3 | 90/180/270° |
| 25 | 160 | 6.5 | 6.5 | 96 | 105 | 125 | 144 | 154 | 160 | 400 | 75 | 3 | 90/180/270° |
| 26 | 165 | 7.8 | 7.8 | 79 | 109 | 122 | 138 | 152 | 165 | 500 | 87 | 3 | 90/180/270° |

Tab. 6: Surface-postcrosslinker formulation of the thermal treatment in the heater - EC: Ethylene carbonate; bop: based on polymer

| Production Example | Base polymer | EC | Water | Plantacare 818 UP | Al-lactate (dry) | Al-sulfate (dry) |
|---|---|---|---|---|---|---|
| | | bop% | bop% | bop ppm | bop% | bop% |
| Polymer particles without aluminum salt | | | | | | |
| 8 | 7b | 2.5 | 5.0 | 50 | | |
| 9 | 7b | 2.5 | 5.0 | 50 | | |
| 10 | 7b | 2.5 | 5.0 | 25 | | |
| Polymer particles with aluminum lactate | | | | | | |
| 11 | 7b | 2.5 | 5.0 | 25 | 0.5 | |
| 12 | 7b | 2.5 | 5.0 | 25 | 0.5 | |
| 13 | 7b | 1.5 | 5.0 | 50 | 0.5 | |
| 14 | 7a | 2.5 | 5.0 | | 0.5 | |
| 15 | 7a | 2.5 | 5.0 | 50 | 0.5 | |
| 16 | 7a | 2.5 | 5.0 | 25 | 0.5 | |
| 17 | 7b | 2.5 | 5.0 | 25 | 0.5 | |
| 18 | 7b | 2.5 | 5.0 | 25 | 0.5 | |
| 19 | 7a | 2.5 | 5.0 | 25 | 0.5 | |
| Polymer particles with aluminum sulfate | | | | | | |
| 20 | 7b | 2.5 | 5.0 | 25 | | 0.50 |
| 21 | 7a | 2.5 | 5.0 | 25 | | 0.36 |
| 22 | 7b | 2.5 | 5.0 | 25 | | 0.75 |
| 23 | 7b | 2.5 | 5.0 | 25 | | 0.50 |
| 24 | 7b | 2.5 | 5.0 | 50 | | 0.36 |
| 25 | 7a | 2.5 | 5.0 | 25 | | 0.36 |
| 26 | 7b | 2.5 | 5.0 | 25 | | 0.50 |

Tab. 7: Physical properties of the polymer particles after surface-postcrosslinking

| Production Example | CR C | AUNL | AUL | AUHL | SFC | GBP | Vortex | FSR | Moisture | Residual Mono no-mers | Extrac-ta-bles | Bulk Den-sity | FR | Fines <150pm | Overs >710 μm |
|---|---|---|---|---|---|---|---|---|---|---|---|---|---|---|---|
| | g/g | g/g | g/g | g/g | $10^{-7}cm^3 \cdot s/g$ | Da | S | g/g·s | % | ppm | % | g/100 ml | g/s | % | % |
| Polymer particles without aluminum salt | | | | | | | | | | | | | | | |
| 8 | 47 | 61 | 37 | 21 | 0 | 1 | 73 | 0.29 | 1.7 | 373 | 5 | 78 | 15 | 0.0 | 2.0 |
| 9 | 42 | 55 | 36 | 26 | 3 | 2 | 69 | 0.27 | 1.2 | 557 | 6 | 74 | 15 | 0.0 | 2.4 |
| 10 | 37 | 48 | 33 | 26 | 6 | 5 | 91 | 0.22 | 1.2 | 170 | 3 | 80 | 14 | 0.1 | 0.5 |
| Polymer particles with aluminum lactate | | | | | | | | | | | | | | | |
| 11 | 41 | 54 | 33 | 21 | 1 | 5 | 60 | 0.39 | 5.1 | 282 | 4 | 77 | 14 | 0.2 | 0.9 |
| 12 | 39 | 53 | 34 | 25 | 1 | 6 | 65 | 0.31 | 2.6 | 367 | 4 | 79 | 14 | 0.5 | 1.1 |
| 13 | 36 | 51 | 33 | 25 | 6 | 9 | 74 | 0.19 | 1.0 | 351 | 4 | 77 | 14 | 0.1 | 0.6 |
| 14 | 46 | 60 | 37 | 20 | 0 | 1 | 73 | 0.30 | 1.2 | 510 | 6 | 75 | 13 | 0.3 | 0.5 |
| 15 | 45 | 60 | 36 | 22 | 9 | 1 | 65 | 0.32 | 1.1 | 515 | 8 | 73 | 13 | 1.0 | 2.5 |
| 16 | 42 | 55 | 36 | 26 | 3 | 2 | 69 | 0.27 | 1.2 | 557 | 6 | 72 | 12 | 0.3 | 0.2 |
| 17 | 39 | 54 | 35 | 27 | 8 | 5 | 68 | 0.32 | 1.2 | 510 | 5 | 77 | 14 | 0.3 | 1.0 |
| 18 | 28 | 41 | 28 | 24 | 125 | 32 | 86 | 0.22 | 0.8 | 565 | 3 | 75 | 14 | 0.4 | 0.9 |
| 19 | 25 | 36 | 26 | 23 | 153 | 31 | 111 | 0.20 | 0.6 | 629 | 5 | 74 | 13 | 1.0 | 2.0 |
| Polymer particles with aluminum sulfate | | | | | | | | | | | | | | | |
| 20 | 32 | 49 | 29 | 22 | 41 | 36 | 70 | 0.30 | 1.2 | 421 | 4 | 79 | 14 | 0.4 | 0.5 |
| 21 | 37 | 53 | 33 | 25 | 23 | 17 | 74 | 0.31 | 1.1 | 373 | 6 | 78 | 13 | 1.0 | 2.6 |
| 22 | 28 | 43 | 25 | 20 | 93 | 78 | 75 | 0.25 | 1.2 | 296 | 3 | 80 | 15 | 0.3 | 0.7 |
| 23 | 28 | 43 | 27 | 22 | 106 | 59 | 89 | 0.22 | 0.9 | 375 | 3 | 81 | 14 | 0.1 | 0.0 |
| 24 | 32 | 48 | 30 | 24 | 65 | 35 | 80 | 0.29 | 0.6 | 594 | 5 | 75 | 13 | 0.3 | 1.0 |
| 25 | 35 | 52 | 32 | 23 | 24 | 25 | 66 | 0.30 | 0.7 | 684 | 7 | 75 | 13 | 1.1 | 2.0 |
| 26 | 24 | 36 | 24 | 20 | 275 | 100 | 100 | 0.21 | 0.6 | 360 | 3 | 80 | 15 | 0.2 | 0.1 |

Tab. 8: Physical properties of the polymer particles after surface-postcrosslinking

| Production Example | CRC | τ 0.03psi | τ 0.1psi | τ 0.3psi | τ 0.5psi | τ 0.7psi | Total liquid uptake |
|---|---|---|---|---|---|---|---|
| | g/g | s | s | s | s | s | g |
| Polymer particles without aluminum salt | | | | | | | |
| 8 | 47.1 | 464 | 525 | 659 | 832 | 924 | 61.5 |
| 9 | 41.7 | 418 | 501 | 546 | 678 | 716 | 108.9 |
| 10 | 36.5 | 324 | 387 | 437 | 571 | 568 | 149.5 |
| Polymer particles with aluminum lactate | | | | | | | |
| 11 | 40.8 | 490 | 611 | 493 | 439 | 383 | 73.5 |
| 12 | 38.7 | 463 | 563 | 538 | 489 | 465 | 86.2 |
| 13 | | | | | | | 136.0 |
| 14 | 46.4 | 467 | 551 | 598 | 599 | 596 | 54.7 |
| 15 | 46.3 | 466 | 551 | 535 | 535 | 497 | 62.9 |
| 16 | 42.6 | | | | | | 93.3 |
| 17 | 26.9 | | | | | | 261.1 |
| 18 | 27.6 | 235 | 281 | 407 | 393 | 391 | 261.0 |
| 19 | 25.3 | | | | | | 324.6 |
| Polymer particles with aluminum sulfate | | | | | | | |
| 20 | 31.7 | | | | | | 134.0 |
| 21 | 37.7 | | | | | | 105.8 |
| 22 | 27.5 | | | | | | 171.3 |
| 23 | 28.2 | 272 | 323 | 382 | 436 | 494 | 167.8 |
| 24 | 32.4 | 295 | 358 | 418 | 404 | 363 | 158.3 |
| 25 | 35.0 | 309 | 376 | 401 | 389 | 385 | 125.8 |
| 26 | 23.6 | 210 | 258 | 278 | 358 | 338 | 218.3 |

Example 27

1200 g of the water-absorbent polymer particles prepared in Example 7b (base polymer) having a content of residual monomers of 4000 ppm were put into a laboratory ploughshare mixer (model MR5, manufactured by Gebrüder Lödige Maschinenbau GmbH, Paderborn, Germany). A surface-postcrosslinker solution was prepared by mixing 12 g of 3-methyl-2-oxazolidinone as described in Table 1 and 60 g of deionized water, into a beaker. At a mixer speed of 200 rpm, the aqueous solution was sprayed onto the polymer particles within one minute by means of a spray nozzle. The mixing was continued for additional 5 minutes. The product was removed and transferred into another ploughshare mixer (model MR5, manufactured by Gebrüder Lödige Maschinenbau GmbH; Paderborn; Germany) which was heated to 150°C before. After mixing for further 80 minutes at 150°C with sample taking every 10 minutes, the product was removed from the mixer and sifted from 150 to 850 $\mu$m. The samples were analyzed. The results are summarized in Table 10.

The resulting polymer particles that were surface-postcrosslinked with 3-methyl-1,3-oxazolidin-2-one had a bulk density of 70.4 g/100ml and a flow rate of 11.5 g/s.

Example 28

1200 g of the water-absorbent polymer particles prepared in Example 7b (base polymer) having a content of residual monomers of 4000 ppm were put into a laboratory ploughshare mixer (model MR5, manufactured by Gebrüder Lödige Maschinenbau GmbH; Paderborn; Germany). A surface-postcrosslinker solution was prepared by mixing 6 g of 3-Methyl-3-oxethanmethanol as described in Table 9 and 60 g of deionized water, into a beaker. At a mixer speed of 200 rpm, the aqueous solution was sprayed onto the polymer particles within one minute by means of a spray nozzle. The mixing was continued for additional 5 minutes. The product was removed and transferred into another ploughshare mixer (model MR5, manufactured by Gebrüder Lödige Maschinenbau GmbH, Paderborn, Germany) which was heated to 150°C before. After mixing for further 80 minutes at 150°C with sample taking every 10 minutes, the product was removed from the mixer and sifted from 150 to 850 $\mu$m. The samples were analyzed. The results are summarized in Table 10.

The resulting polymer particles that were surface-postcrosslinked with 3-methyl-3-oxethanmethanol had a bulk density of 72.2/100ml and a flow rate of 12.0 g/s.

Example 29

1200 g of the water-absorbent polymer particles prepared in Example 7b (base polymer) having a content of residual monomers of 4000 ppm were put into a laboratory ploughshare mixer (model MR5, manufactured by Gebrüder Lödige Maschinenbau GmbH, Paderborn, Germany). A surface-postcrosslinker solution was prepared by mixing 6 g of 2-oxazolidinone as described in Table 9 and 60 g of deionized water, into a beaker. At a mixer speed of 200 rpm, the aqueous solution was sprayed onto the polymer particles within one minute by means of a spray nozzle. The mixing was continued for additional 5 minutes. The product was removed and transferred into another ploughshare mixer (model MR5, manufactured by Gebrüder Lödige Maschinenbau GmbH, Paderborn, Germany) which was heated to 150°C before. After mixing for further 80 minutes at 150°C with sample taking every 10 minutes, the product was removed from the mixer and sifted from 150 to 850 $\mu$m. The samples were analyzed. The results are summarized in Table 10.

The resulting polymer particles that were surface-postcrosslinked with 1,3-oxazolidin-2-one had a bulk density of 69.7 g/100ml and a flow rate of 10.8 g/s.

Example 30

1200 g of the water-absorbent polymer particles prepared in Example 7b (base polymer) having a content of residual monomers of 4000 ppm were put into a laboratory ploughshare mixer (model MR5, manufactured by Gebrüder Lödige Maschinenbau GmbH, Paderborn, Germany). A surface-postcrosslinker solution was prepared by mixing 6 g of Solution of 3-(2-hydroxyethyl)-2- oxazolidinon and 6 g propandiol as described in Table 9 and 60 g of deionized water, into a beaker. At a mixer speed of 200 rpm, the aqueous solution was sprayed onto the polymer particles within one minute by means of a spray nozzle. The mixing was continued for additional 5 minutes. The product was removed and transferred into another ploughshare mixer (model MR5, manufactured by Gebrüder Lödige Maschinenbau GmbH,Paderborn, Germany) which was heated to 165°C before. After mixing for further 80 minutes at 165°C with sample taking every 10 minutes, the product was removed from the mixer and sifted from 150 to 850 $\mu$m. The samples were analyzed. The results are summarized in Table 10.

The resulting polymer particles that were surface-postcrosslinked with of 3-(2-hydroxyethyl)-1,3-oxazolidin-2-one and 6 g propandiol had a bulk density of 67.4g/100ml and a flow rate of 10.1 g/s.

Example 31

1200 g of the water-absorbent polymer particles prepared in Example 7b (base polymer) having a content of residual monomers of 4000 ppm were put into a laboratory ploughshare mixer (model MR5, manufactured by Gebrüder Lödige Maschinenbau GmbH, Paderborn, Germany). A surface-postcrosslinker solution was prepared by mixing 3 g of N,N,N',N'-Tetrakis(2-hydroxyethyl)adipamide (Primid® XL 552, manufactured by Ems Chemie AG; Domat; Switzerland) as described in Table 9 and 60 g of deionized water, into a beaker. At a mixer speed of 200 rpm, the aqueous solution was sprayed onto the polymer particles within one minute by means of a spray nozzle. The mixing was continued for additional 5 minutes. The product was removed and transferred into another ploughshare mixer (model MR5, manufactured by Gebrüder Lödige Maschinenbau GmbH,Paderborn, Germany) which was heated to 160°C before. After mixing for further 80 minutes at 160°C with sample taking every 10 minutes, the product was removed from the mixer and sifted from 150 to 850 $\mu$m. The samples were analyzed. The results are summarized in Table 10.

The resulting polymer particles that were surface-postcrosslinked with N,N,N',N'-Tetrakis(2-hydroxyethyl)adipamide had a bulk density of 65.8g/100ml and a flow rate of 10.2 g/s.

Example 32

1200 g of the water-absorbent polymer particles prepared in Example 7b (base polymer) having a content of residual monomers of 4000 ppm were put into a laboratory ploughshare mixer (model MR5, manufactured by Gebrüder Lödige Maschinenbau GmbH, Paderborn, Germany). A surface-postcrosslinker solution was prepared by mixing 24 g of 1.3-Dioxan-2-on as described in Table 9 and 60 g of deionized water, into a beaker. At a mixer speed of 200 rpm, the aqueous solution was sprayed onto the polymer particles within one minute by means of a spray nozzle. The mixing was continued for additional 5 minutes. The product was removed and transferred into another ploughshare mixer (model MR5, manufactured by Gebrüder Lödige Maschinenbau GmbH, Paderborn, Germany) which was heated to 160°C before. After mixing for further 80 minutes at 160°C with sample taking every 10 minutes, the product was removed from the mixer and sifted from 150 to 850 $\mu$m. The samples were analyzed. The results are summarized in Table 10.

The resulting polymer particles that were surface-postcrosslinked with 1.3-Dioxan-2-on had a bulk density of 68.4 g/100ml and a flow rate of 10.5 g/s.

Tab. 9: Formulation of the polymer particles after surface-postcrosslinking by using different surface-postcrosslinkers

| Example | Surface-postcrosslinker - Typ | Surface-postcrosslinker bop% | Water bop% | Temperature °C | Time min | Base polymer |
|---|---|---|---|---|---|---|
| 27 | 3-Methyl-2-oxazolidinone | 1.00 | 5.0 | 150 | 80 | 7b |
| 28 | 3-Methyl-3-oxethanmethanol | 0.50 | 5.0 | 150 | 80 | 7b |
| 29 | 2-oxazolidinone | 0.50 | 5.0 | 150 | 80 | 7b |
| 30 | 50 wt% solution of 3-(2-hydroxyethyl)-2-oxazolidinone in 1,3-propandiol | 0.50 | 5.0 | 165 | 80 | 7b |
| 31 | N,N,N',N'-Tetrakis(2-hydroxyethyl)adipamide | 0.25 | 5.0 | 160 | 80 | 7b |
| 32 | 1.3-Dioxan-2-on | 2.0 | 5.0 | 160 | 80 | 7b |
| Bop: based on polymer | | | | | | |

Tab. 10: Physical properties of the polymer particles after surface-postcrosslinking by using different surface-postcrosslinkers

| Example | CRC | AUNL | AUL | AUHL | SFC | GBP | Vortex | Total liquid uptake |
|---|---|---|---|---|---|---|---|---|
|  | g/g | g/g | g/g | g/g | $10\text{-}7\,cm^3 \cdot s/g$ | Da | s | g |
| 27 | 41.9 | 56.4 | 36.1 | 24.8 | 0 | 2 | 83 | 114.8 |
| 28 | 41.3 | 55.0 | 33.9 | 22.8 | 0 | 0 | 61.5 | 92.5 |
| 29 | 39.3 | 53.1 | 33.0 | 22.1 | 9 | 13 | 54.5 | 102.2 |
| 30 | 30.9 | 43.1 | 29.2 | 23.6 | 0 | 2 | 83 | 208.5 |
| 31 | 34.3 | 46.3 | 30.5 | 24.1 | 6 | 8 | 104 | 91.9 |
| 32 | 39.2 | 53.6 | 36.6 | 30.1 | 12 | 25 | 87 | 110.5 |

Comparative Examples

AQUA KEEP® SA60SII, AQUA KEEP® SA55XSII, AQUA KEEP® SA60SXII are water-absorbent polymer particles from SUMITOMO SEIKA CHEMICALS CO.,LTD, produced by a suspension polymerization process.

ASAP® 535, Hysorb® B7075, Hysorb® T9700, Hysorb® B7055, Hysorb® T8760, Hysorb® M7055N, Hysorb® B7015, Hysorb® M7015N, Hysorb® M7015 and Hysorb® 7400 are water-absorbent polymer particles from BASF SE, produced by a kneader polymerization process.

CE corresponds to water-absorbent polymer particles that are prepared in accordance to Example 25 of WO 2013/007819 A1.

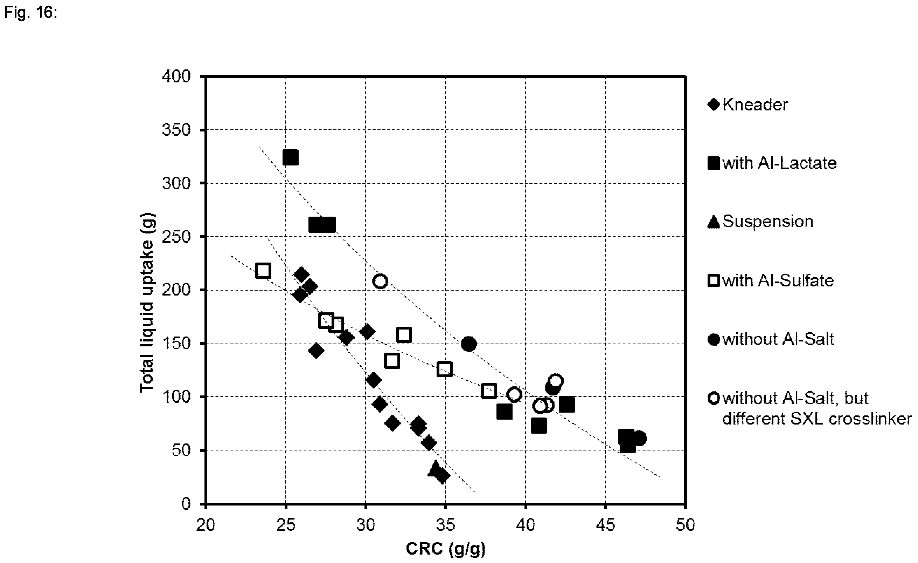

Fig. 16 is a diagram that shows that the water-absorbent polymer particles preferred for the inventive fluid-absorbent article have an improved total quid uptake compared to conventional water-absorbent polymer particles having the same centrifuge retention capacity (CRC).

Tab. 11: Physical Properties of Comparison Example

| Comparison Example | CRC | AUNL | AUL | AUHL | SFC | GBP | Vortex | Extractables | FSR | $\tau$ 0.03psi | $\tau$ 0.1psi | $\tau$ 0.3psi | $\tau$ 0.5psi | $\tau$ 0.7psi | Total liquid uptake |
|---|---|---|---|---|---|---|---|---|---|---|---|---|---|---|---|
| | g/g | g/g | g/g | g/g | $10^{-7}$ cm³·s/g | Da | s | % | g/g·s | s | s | s | s | s | g |
| Comparison Polymer Particles - Suspension Polymerization | | | | | | | | | | | | | | | |
| AQUA KEEP® SA60SII | 34.4 | 56 | 27 | 14 | 0 | 2 | 38 | 3,1 | | 108 | 134 | 1033 | 1667 | 1534 | 32.9 |
| AQUA KEEP® SA55XSII | 28.9 | | | 22 | 7 | 6 | 42 | 4.4 | 0.50 | 82 | 98 | 190 | 549 | 892 | |
| AQUA KEEP® SA60SXII | 33.2 | | | 15 | 0 | 2 | 38 | 4.2 | 0.37 | 74 | 138 | 1215 | 2103 | 2154 | |
| Comparison Polymer Particles - Kneader Polymerization | | | | | | | | | | | | | | | |
| CE | 27.6 | 34.9 | 26.7 | 22.8 | 98 | 15 | 120 | 12.7 | 0.38 | 275 | 218 | 271 | 248 | | 214.2 |
| Hysorb® B7075 | 28.8 | 40.9 | 29.4 | 24.3 | 45 | 7 | 92 | 8.2 | 0.25 | 319 | 389 | 467 | 442 | 372 | 155.6 |
| ASAP® 535 | 30.1 | 45.6 | 29.7 | 23.5 | 50 | 18 | | 13.0 | 0.18 | 317 | 395 | 425 | 456 | 432 | 161.0 |
| Hysorb® T9700 | 30.5 | 46.9 | 26.5 | 19.4 | 33 | 55 | | 11.5 | 0.26 | 376 | 406 | 400 | 374 | 372 | 115.1 |
| Hysorb® B7055 | 29.4 | 43.3 | 29.8 | 22.3 | 9 | 4 | 93 | 10.0 | 0.19 | 291 | 363 | 353 | 462 | | 106.4 |
| Hysorb® T8760 | 30.9 | 49.2 | 28.8 | 19.3 | 18 | 33 | 69 | 13.7 | 0.26 | 300 | 333 | 367 | 483 | 451 | 93.1 |
| Hysorb® M7055N | 32.0 | 40.9 | 29.1 | 24.5 | 9 | 4 | 97 | 12.2 | 0.23 | 429 | 469 | 538 | 566 | 702 | 70.6 |
| Hysorb® B7015 | 33.5 | 45.2 | 30.3 | 22.2 | 4 | 1 | 84 | 9.5 | 0.21 | 343 | 391 | 365 | 409 | 509 | 75.5 |

(continued)

| Comparison Polymer Particles - Kneader Polymerization | | | | | | | | | | | | | | |
|---|---|---|---|---|---|---|---|---|---|---|---|---|---|---|
| Hysorb® M7015N | 34.0 | 46.3 | 30.2 | 22.0 | 3 | 2 | 81 | 11.9 | 0.23 | 260 | 379 | 427 | 434 | 1184 | 57.0 |
| Hysorb® M7015 | 34.0 | 47.2 | 29.5 | 21.7 | 2 | 7 | 59 | 12.5 | 0.29 | 227 | 229 | 356 | 500 | | 74.9 |
| Hysorb® 7400 | 34.8 | 50.0 | 28.8 | 13.2 | 0 | 3 | 35 | 16.7 | 0.33 | 238 | 276 | 374 | 738 | 841 | 26.0 |

Example 33

A fluid-absorbent article - the baby diaper of size L - consisting 53% by weight of surface-postcrosslinked polymer of Example 12, was manufactured in a standard diaper production process:
The fluid-absorbent article comprises

(A) an upper liquid-pervious layer comprising a spunbond nonwoven (three piece coverstock) having a basis weight of 12 gsm
(B) a lower liquid-impervious layer comprising a composite of breathable polyethylene film and spunbond nonwoven;
(C) an absorbent core between (A) and (B) comprising

1) lower fluff layer of hydrophilic fibrous matrix of wood pulp fibers (cellulose fibers) acting as a dusting layer;
2) a homogeneous mixture of wood pulp fibers (cellulose fibers) and surface-postcrosslinked polymer. The fluid-absorbent core holds 53% by weight distributed surface-postcrosslinked polymer, the quantity of surface-postcrosslinked polymer within the fluid-absorbent core is 14.5 g. Dimensions of the fluid-absorbent core: length: 42 cm; front width: 12.8 cm; crotch width: 8,4 cm; rear width: 11.8 cm. The density of the fluid-absorbent core is for the font overall average 0.23 $g/cm^3$, for the insult zone 0.29 $g/cm^3$, for the back overall average 0.19 $g/cm^3$. The average thickness of fluid-absorbent core is 0.36 cm. The fluid-absorbent core is wrapped with a spunbond - meltblown - spunbond (SMS) nonwoven material having a basis weight of 10 gsm. The basis weight of fluid-absorbent core is for the font overall average 990 $g/cm^3$, for the insult zone 1130 $g/cm^3$, for the back overall average 585 $g/cm^3$.

(D) an air through bonded acquisition-distribution layer between (A) and (C) having a basis weight of 60 $g/m^2$; the acquisition-distribution layer is rectangular shaped and smaller than the fluid-absorbent core having a size of about 212 $cm^2$.

Dimension of the fluid-absorbent article: length: 51 cm; front width: 31.8 cm; crotch width: 22.4 cm; rear width: 31.8 cm. The fluid-absorbent article has average weight of 38.1 g.
The fluid-absorbent article further comprises:

a.flat rubber elastics; elastics from spandex type fibers: 2 leg elastics and 1 cuff elastic
b.leg cuffs from synthetic fibers, nonwoven material showing the layer combination SMS and having a basis weight of between 13 to 15 $g/m^2$ and a height of 3.0 cm
c. mechanical closure system with landing zone of dimension 16.9 cm x 3.4 cm and flexiband closure tapes of 3.1 cm x 5.4 cm; attached to hook fastening tape of 1.9 cm x 2.7 cm

The rewet under load and rewet value of the fluid-absorbent article were determined. The results are summarized in Table 12 and 13.

Example 34

A fluid-absorbent article - the baby diaper of size L - consisting 49% by weight of surface-postcrosslinked polymer of Example 12 was manufactured in a standard diaper production process:
The fluid-absorbent article comprises the same components (A), (B) and (D) as in Example 33.
The absorbent core (C) between (A) and (B) comprises

1)lower fluff layer of hydrophilic fibrous matrix of wood pulp fibers (cellulose fibers) acting as a dusting layer;
2)a homogeneous mixture of wood pulp fibers (cellulose fibers) and surface-postcrosslinked polymer. The fluid-absorbent core holds 49% by weight distributed surface-postcrosslinked polymer, the quantity of surface-postcrosslinked polymer within the fluid-absorbent core is 12.5 g. Dimensions of the fluid-absorbent core are the same as in Example 32. The density of the fluid-absorbent core is for the font overall average 0.26 $g/cm^3$, for the insult zone 0.27 $g/cm^3$, for the back overall average 0.19 $g/cm^3$. The average thickness of fluid-absorbent core is 0.31 cm. The fluid-absorbent core is wrapped with a spunbond - meltblown - spunbond (SMS) nonwoven material having a basis weight of 10 gsm. The basis weight of fluid-absorbent core is for the font overall average 971 $g/cm^3$, for the insult zone 979 $g/cm^3$, for the back overall average 515 $g/cm^3$.

Dimensions of the fluid-absorbent article are the same as in Example 33. The fluid-absorbent article has average weight of 35.7 g.

The fluid-absorbent article further comprises:

a.flat rubber elastics as in Example 33
b.leg cuffs, as in Example 33
c. mechanical closure system, as in Example 33

The rewet under load and rewet value of the fluid-absorbent article were determined. The results are summarized in Table 12 and 13.

Example 35

A fluid-absorbent article - the baby diaper of size L - consisting 47.5% by weight of surface-postcrosslinked polymer of Example 12 was manufactured in a standard diaper production process:
The fluid-absorbent article comprises the same components (A), (B) and (D) as in Example 33.

The absorbent core (C) between (A) and (B) comprises

1)lower fluff layer of hydrophilic fibrous matrix of wood pulp fibers (cellulose fibers) acting as a dusting layer;
2)a homogeneous mixture of wood pulp fibers (cellulose fibers) and surface-postcrosslinked base polymer. The fluid-absorbent core holds 47.7 % by weight distributed surface-postcrosslinked polymer, the quantity of surface-postcrosslinked polymer within the fluid-absorbent core is 11.5 g. Dimensions of the fluid-absorbent core are the same as in Example 32. The density of the fluid-absorbent core is for the font overall average 0.24 g/cm$^3$, for the insult zone 0.26 g/cm$^3$, for the back overall average 0.18 g/cm$^3$. The average thickness of fluid-absorbent core is 0.32 cm. The fluid-absorbent core is wrapped with a spunbond - meltblown - spunbond (SMS) nonwoven material having a basis weight of 10 gsm. The basis weight of fluid- absorbent core is for the font overall average 928 g/cm$^3$, for the insult zone 967 g/cm$^3$, for the back overall average 495 g/cm$^3$.

Dimensions of the fluid-absorbent article are the same as in Example 33. The fluid-absorbent article has average weight of 34.8 g.

The fluid-absorbent article further comprises:

a.flat rubber elastics as in Example 33
b.leg cuffs, as in Example 33
c. mechanical closure system, as in Example 33

The rewet under load and rewet value of the fluid-absorbent article were determined. The results are summarized in Table 12 and 13.

Comparative Example

A fluid-absorbent article - the baby diaper of size L - consisting 52% by weight of surface-postcrosslinked polymer HySorb®B7075 (BASF SE, Ludwigshafen, Germany) was manufactured in a standard diaper production process:
The fluid-absorbent article comprises the same components (A), (B) and (D) as in Example 33.

The absorbent core (C) between (A) and (B) comprises

1)lower fluff layer of hydrophilic fibrous matrix of wood pulp fibers (cellulose fibers) acting as a dusting layer;
2)a homogeneous mixture of wood pulp fibers (cellulose fibers) and surface-postcrosslinked base polymer (HySorb®B7075). The fluid-absorbent core holds 52 % by weight distributed surface-postcrosslinked polymer, the quantity of surface-postcrosslinked polymer within the fluid-absorbent core is 14.5 g. Dimensions of the fluid-absorbent core are the same as in Example 32. The density of the fluid-absorbent core is for the font overall average 0.24 g/cm$^3$, for the insult zone 0.25 g/cm$^3$, for the back overall average 0.19 g/cm$^3$. The average thickness of fluid-absorbent core is 0.34 cm. The fluid-absorbent core is wrapped with a spunbond - meltblown - spunbond (SMS) nonwoven material having a basis weight of 10 gsm. The basis weight of fluid- absorbent core is for the font overall average 1013 g/cm$^3$, for the insult zone 971 g/cm$^3$, for the back overall average 548 g/cm$^3$.

Dimensions of the fluid-absorbent article are the same as in Example 33. The fluid-absorbent article has average weight of 38 g.

The fluid-absorbent article further comprises:

a.flat rubber elastics as in Example 33
b.leg cuffs, as in Example 33
c. mechanical closure system, as in Example 33

The rewet under load and rewet value of the fluid-absorbent article were determined. The results are summarized in Table 12 and 13.

Table 12. Rewet Under Load

| Example | Rewet Under Load | | | | |
|---|---|---|---|---|---|
| | 1st insult | 2nd insult | 3rd in-sult | 4th in-sult | 5th in-sult |
| 33 | 0,10 g | 0,12 g | 0,10 g | 0,09 g | 0,16 g |
| 34 | 0,10 g | 0,08 g | 0,07 g | 0,10 g | 0,27 g |
| 35 | 0,10 g | 0,06 g | 0,07 g | 0,14 g | 0,30 g |
| Comparative Example | 0,08 g | 0,08 g | 0,08 g | 0,29 g | 0,52 g |

Table 13. Rewet value

| Example | Rewet value | | | | |
|---|---|---|---|---|---|
| | 1st insult | 2nd insult | 3rd insult | 4th insult | 5th insult |
| 33 | 0,14 g | 0,10 g | 0,09 g | 0,10 g | 0,75 g |
| 34 | 0,15 g | 0,11 g | 0,11 g | 0,40 g | 1,61 g |
| 35 | 0,14 g | 0,10 g | 0,09 g | 0,87 g | 3,84 g |
| Comparative Example | 0,12 g | 0,22 g | 0,11 g | 0,82 g | 4,30 g |

The examples demonstrate that the fluid-absorbent article comprising spherical shaped surface-postcrosslinked polymer particles shows better rewet performance, even when the loading of spherical shaped surface-postcrosslinked polymer particles in the absorbent core is reduced up to 20%, in comparison to the fluid-absorbent article containing irregular shaped surface-postcrosslinked polymer particles.

**Claims**

**1.** Fluid-absorbent article, comprising

(A) an upper liquid-pervious layer,
(B) a lower liquid-impervious layer,
(C) a fluid-absorbent core between the layer (A) and the layer (B), comprising from 0 to 90% by weight fibrous material and from 10 to 100% by weight water-absorbent polymer particles, based on the sum of water-absorbent polymer particles and fibrous material; wherein the basis weight of the fluid-absorbent core at the insult zone is of at least 500 gsm,
(D) an optional acquisition-distribution layer between (A) and (C),
(E) an optional tissue layer disposed immediately above and/or below (C); and
(F) other optional components,
wherein the water-absorbent polymer particles having a mean sphericity (SPHT) from 0.8 to 0.95 and satisfying the following conditions:

(a) the sum of centrifuge retention capacity (CRC) and absorbency under high load (AUHL) being at least 60 g/g, and/or
(b) the $\log_{10}(SFC) > 5.7 - 0.138 \times CRC$, with SFC being not less than
5 , wherein CRC is given in g/g and
SFC in $10^{-7} cm^3 \cdot s/g$.

2. Fluid-absorbent article according to claim 1, wherein the sum of CRC and AUHL being at least 60 g/g, and the amount of the basis weight of the acquisition distribution layer (D) in gsm being not less than the amount of water-absorbent polymer particles contained in the fluid absorbent core (C) in % by weight, based on the sum of water-absorbent polymer particles and fibrous material.

3. Fluid-absorbent article according to claim 1 or 2, wherein the fluid-absorbent core comprises at least 12 % by weight of water-absorbent polymer particles.

4. Fluid-absorbent article according to claim 1 or 3, wherein the fluid-absorbent core comprises at least 50% by weight of water-absorbent polymer particles.

5. Fluid-absorbent article according to claim 1 to 4, wherein the fluid-absorbent core comprises at least 85% by weight of water-absorbent polymer particles.

6. Fluid-absorbent article according to any of claims 1 to 5, wherein the fluid-absorbent core comprises not more than 10% by weight of an adhesive.

7. Fluid-absorbent article according to any of claims 1 to 6, wherein the water-absorbent polymer particles are placed within the core in discrete regions.

8. Fluid-absorbent article according to any of claims 1 to 7, wherein the fluid-absorbent core comprises at least two layers of water-absorbent polymer particles.

9. Fluid-absorbent article according to any of claims 1 to 8, wherein the water-absorbent polymer particles have a centrifuge retention capacity of at least 25 g/g and an absorbency under high load of at least 20 g/g.

10. Fluid-absorbent article according to any of claims 1 to 9, wherein the water-absorbent polymer particles have a level of extractable constituents of less than 10% by weight

11. Fluid-absorbent article according to any of claims 1 to 10, wherein the fluid-absorbent core comprises less than 20% by weight fibrous material.

12. Fluid-absorbent article according to any of claims 1 to 11, wherein the water-absorbent polymer particles are profiled in the fluid-absorbent core according to the equation (I):

$$(A_{total} - A_{profiled}) \times Basisweight + Loading\text{-}Factor \times A_{profiled} \times Basisweight = W_{SAP} \ (I)$$

With $A_{total}$ = Area of the total fluid-absorbent core (C) in m$^2$,
$A_{profiled}$ = Area with different loading in m$^2$,
L = Loading-Factor
Basisweight = basis weight of the fluid-absorbent core in g/m$^2$, and
$W_{SAP}$ = total content of water-absorbent polymer particles within the fluid-absorbent core in g.

**Patentansprüche**

1. Flüssigkeitsabsorbierender Artikel, umfassend

(A) eine obere flüssigkeitsdurchlässige Schicht,
(B) eine untere flüssigkeitsunddurchlässige Schicht,
(C) einen flüssigkeitsabsorbierendes Kern zwischen der Schicht (A) und der Schicht (B), umfassend 0 bis 90 Gew.-% Fasermaterial und 10 bis 100 Gew.-% wasserabsorbierende Polymerteilchen, bezogen auf die Summe von wasserabsorbierenden Polymerteilchen und Fasermaterial; wobei das Flächengewicht des flüssigkeitsabsorbierenden Kerns in der Beaufschlagungszone mindestens 500 gsm beträgt,
(D) eine fakultative Akquisitions- und Verteilungsschicht zwischen (A) und (C),
(E) eine fakultative Tissueschicht, die direkt über und/oder unter (C) angeordnet ist, und

(F) andere fakultative Komponenten,

wobei die wasserabsorbierenden Polymerteilchen eine mittlere Sphärizität (SPHT) von 0,8 bis 0,95 aufweisen und die folgenden Bedingungen erfüllen:

(a) die Summe von Zentrifugenretentionskapazität (CRC) und Absorption unter hoher Last (AUHL) beträgt mindestens 60 g/g, und/oder

(b) der $\log_{10}$(SFC) > 5,7 - 0,138 x CRC, wobei SFC nicht weniger als 5 beträgt, wobei CRC in g/g und SFC in $10^{-7}$ cm$^3$·s/g angegeben ist.

2. Flüssigkeitsabsorbierender Artikel nach Anspruch 1, wobei die Summe von CRC und AUHL mindestens 60 g/g beträgt und die Menge des Flächengewichts der Akquisitions- und Verteilungsschicht (D) in gsm nicht kleiner ist als die in dem flüssigkeitsabsorbierenden Kern (C) enthaltene Menge von wasserabsorbierenden Polymerteilchen in Gew.-%, bezogen auf die Summe von wasserabsorbierenden Polymerteilchen und Fasermaterial.

3. Flüssigkeitsabsorbierender Artikel nach Anspruch 1 oder 2, wobei der flüssigkeitsabsorbierende Kern mindestens 12 Gew.-% wasserabsorbierende Polymerteilchen umfasst.

4. Flüssigkeitsabsorbierender Artikel nach Anspruch 1 oder 3, wobei der flüssigkeitsabsorbierende Kern mindestens 50 Gew.-% wasserabsorbierende Polymerteilchen umfasst.

5. Flüssigkeitsabsorbierender Artikel nach Anspruch 1 bis 4, wobei der flüssigkeitsabsorbierende Kern mindestens 85 Gew.-% wasserabsorbierende Polymerteilchen umfasst.

6. Flüssigkeitsabsorbierender Kern nach einem der Ansprüche 1 bis 5, wobei der flüssigkeitsabsorbierende Kern nicht mehr als 10 Gew.-% eines Klebstoffs umfasst.

7. Flüssigkeitsabsorbierender Kern nach einem der Ansprüche 1 bis 6, wobei die wasserabsorbierenden Polymerteilchen in dem Kern in diskreten Bereichen angeordnet sind.

8. Flüssigkeitsabsorbierender Artikel nach einem der Ansprüche 1 bis 7, wobei der flüssigkeitsabsorbierende Kern mindestens zwei Schichten von wasserabsorbierenden Polymerteilchen umfasst.

9. Flüssigkeitsabsorbierender Artikel nach einem der Ansprüche 1 bis 8, wobei die wasserabsorbierenden Polymerteilchen eine Zentrifugenretentionskapazität von mindestens 25 g/g und eine Absorption unter hoher Last von mindestens 20 g/g aufweisen.

10. Flüssigkeitsabsorbierender Artikel nach einem der Ansprüche 1 bis 9, wobei die wasserabsorbierenden Polymerteilchen einen Gehalt an extrahierbaren Bestandteilen von weniger als 10 Gew.-% aufweisen.

11. Flüssigkeitsabsorbierender Artikel nach einem der Ansprüche 1 bis 10, wobei der flüssigkeitsabsorbierende Kern weniger als 20 Gew.-% Fasermaterial umfasst.

12. Flüssigkeitsabsorbierender Artikel nach einem der Ansprüche 1 bis 11, wobei die wasserabsorbierenden Polymerteilchen in dem flüssigkeitsabsorbierenden Kern gemäß der Gleichung (I) profiliert sind:

$$(A_{gesamt} - A_{profiliert}) \times \text{Flächengewicht} + \text{Beladungsfaktor} \times A_{profiliert} \times \text{Flächengewicht} = W_{SAP} \quad (I)$$

mit $A_{gesamt}$ = Fläche des gesamten flüssigkeitsabsorbierenden Kerns (C) in m$^2$,
$A_{profiliert}$ = Fläche mit anderer Beladung in m$^2$,
L = Beladungsfaktor
Flächengewicht = Flächengewicht des flüssigkeitsabsorbierenden Kerns in g/m$^2$ und
$W_{SAP}$ = Gesamtgewicht von wasserabsorbierenden Polymerteilchen in dem flüssigkeitsabsorbierenden Kern in g.

**Revendications**

1. Article absorbant les fluides, comprenant :

   (A) une couche supérieure perméable aux liquides,
   (B) une couche inférieure imperméable aux liquides,
   (C) une partie centrale absorbant les fluides entre la couche (A) et la couche (B), comprenant de 0 à 90 % en poids de matériau fibreux et de 10 à 100 % en poids de particules de polymère absorbant l'eau, relativement à la somme des particules de polymère absorbant l'eau et du matériau fibreux ; le grammage de la partie centrale absorbant les fluides dans la zone d'exposition aux fluides étant d'au moins 500 g/m$^2$,
   (D) une couche optionnelle d'acquisition-distribution entre (A) et (C),
   (E) une couche de papier mince optionnelle disposée juste au-dessus et/ou en dessous de (C) ; et
   (F) d'autres éléments constituants optionnels,

   dans lequel les particules de polymère absorbant l'eau ont une sphéricité moyenne (SPHT) de 0,8 à 0,95 et satisfont les conditions suivantes :

   (a) la somme de la capacité de rétention centrifuge (CRC) et du pouvoir absorbant sous charge élevée (AUHL) est d'au moins 60 g/g, et/ou
   (b) $\log_{10}(SFC) > 5,7 - 0,138 \times CRC$, la conductivité de flux salin (SFC) n'étant pas inférieure à 5, la CRC étant exprimée en g/g et la SFC étant exprimée en $10^{-7} cm^3 \cdot s/g$.

2. Article absorbant les fluides selon la revendication 1, dans lequel la somme de la CRC et de l'AUHL est d'au moins 60 g/g, et la valeur du grammage de la couche d'acquisition-distribution (D) en g/m$^2$ n'est pas inférieure à la quantité de particules de polymère absorbant l'eau présentes dans la partie centrale absorbant les fluides (C) exprimée en pourcentage pondéral relativement à la somme des particules de polymère absorbant l'eau et du matériau fibreux.

3. Article absorbant les fluides selon la revendication 1 ou 2, dans lequel la partie centrale absorbant les fluides comprend au moins 12 % en poids de particules de polymère absorbant l'eau.

4. Article absorbant les fluides selon la revendication 1 ou 3, dans lequel la partie centrale absorbant les fluides comprend au moins 50 % en poids de particules de polymère absorbant l'eau.

5. Article absorbant les fluides selon la revendication 1 à 4, dans lequel la partie centrale absorbant les fluides comprend au moins 85 % en poids de particules de polymère absorbant l'eau.

6. Article absorbant les fluides selon l'une quelconque des revendications 1 à 5, dans lequel la partie centrale absorbant les fluides ne comprend pas plus de 10 % en poids d'un adhésif.

7. Article absorbant les fluides selon l'une quelconque des revendications 1 à 6, dans lequel les particules de polymère absorbant l'eau sont disposées à l'intérieur de la partie centrale dans des régions discontinues.

8. Article absorbant les fluides selon l'une quelconque des revendications 1 à 7, dans lequel la partie centrale absorbant les fluides comprend au moins deux couches de particules de polymère absorbant l'eau.

9. Article absorbant les fluides selon l'une quelconque des revendications 1 à 8, dans lequel les particules de polymère absorbant l'eau ont une capacité de rétention centrifuge d'au moins 25 g/g et un pouvoir absorbant sous charge élevée d'au moins 20 g/g.

10. Article absorbant les fluides selon l'une quelconque des revendications 1 à 9, dans lequel les particules de polymère absorbant l'eau ont un niveau de constituants extractibles inférieur à 10 % en poids.

11. Article absorbant les fluides selon l'une quelconque des revendications 1 à 10, dans lequel la partie centrale absorbant les fluides comprend moins de 20 % en poids de matériau fibreux.

12. Article absorbant les fluides selon l'une quelconque des revendications 1 à 11, dans lequel les particules de polymère absorbant l'eau sont profilées dans la partie centrale absorbant les fluides selon l'équation (I) :

$$(A_{totale} - A_{profilée}) \times Grammage + Facteur\ de\ chargement$$
$$\times A_{profilée} \times Grammage = W_{SAP}\ (I)$$

où $A_{totale}$ = aire de la partie centrale absorbant les fluides totale (C) en $m^2$,
$A_{profilée}$ = aire ayant un chargement différent en $m^2$,
L = Facteur de chargement,
Grammage = grammage de la partie centrale absorbant les fluides en $g/m^2$, et
$W_{SAP}$ = teneur totale en particules de polymère absorbant l'eau dans la partie centrale absorbant les fluides en g.

$$(A_{totale} - A_{profilée}) \times Grammage + Facteur\ de\ chargement$$
$$\times A_{profilée} \times Grammage = W_{SAP}\ (I)$$

Fig 1.

**Fig 2:**

Fig 3:

Fig 4:

Fig 5:

Fig 6:

Fig. 7:

Fig. 8:

Fig. 9:

Fig. 10:

**Fig 11:**

**Fig 12:**

Fig. 13:

Fig. 14:

Fig. 15:

Fig. 16:

EP 3 039 044 B1

Fig. 17:

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 0348180 A1
- WO 9640427 A1
- US 5269980 A
- WO 2008009580 A1
- WO 2008052971 A1
- WO 2011026876 A1
- WO 2011117263 A1
- EP 1562747 B1
- DE 10107709 A1
- US 7176149 B
- WO 2009094506 A1
- WO 2002055469 A1
- WO 2003078378 A1
- WO 2004035514 A1
- EP 0530438 A1
- EP 0547847 A1
- EP 0559476 A1
- EP 0632068 A1
- WO 9321237 A1
- WO 2003104299 A1
- WO 2003104300 A1
- WO 2003104301 A1
- DE 10331450 A1
- DE 10331456 A1
- DE 10355401 A1
- DE 19543368 A1
- DE 19646484 A1
- WO 9015830 A1
- WO 200232962 A2
- WO 2001038402 A1
- DE 3825366 A1
- US 6241928 B
- WO 2008040715 A2
- WO 2008069639 A1
- WO 2008086976 A1
- US 3243321 A
- EP 0083022 A2
- EP 0543303 A1
- EP 0937736 A2
- DE 3314019 A1
- DE 3523617 A1
- EP 0450922 A2
- DE 10204938 A1
- US 6239230 B
- DE 4020780 C1
- DE 19807502 A1
- DE 19807992 C1
- EP 0999238 A1

- DE 19854573 A1
- DE 19854574 A1
- DE 10204937 A1
- DE 10334584 A1
- EP 1199327 A2
- WO 200331482 A1
- DE 3713601 A1
- WO 2012045705 A1
- WO 2004024816 A1
- WO 2007031441 A1
- EP 2301499 A1
- EP 2314264 A1
- EP 2387981 A1
- EP 2486901 A1
- EP 2524679 A1
- EP 2524680 A1
- EP 2565031 A1
- US 6972011 B
- US 20110162989 A
- US 20110270204 A
- WO 2010004894 A1
- WO 2010004895 A1
- WO 2010076857 A1
- WO 2010082373 A1
- WO 2010118409 A1
- WO 2010133529 A2
- WO 2010143635 A1
- WO 2011084981 A1
- WO 2011086841 A1
- WO 2011086842 A1
- WO 2011086843 A1
- WO 2011086844 A1
- WO 2011117997 A1
- WO 2011136087 A1
- WO 2012048879 A1
- WO 2012052173 A1
- WO 2012052172 A1
- WO 2010103453 A
- EP 1293187 A1

- EP 1447066 A1
- EP 1447067 A1
- EP 1609448 A1
- JP 2004313580 A
- US 20050137085 A
- US 20060004336 A
- US 20070135785 A
- WO 2008155699 A1
- WO 2008155701 A2
- WO 2008155702 A1
- WO 2008155710 A1
- WO 2008155711 A1
- WO 2004071363 A1
- US 20030181115 A
- WO 2005097025 A
- US 2007156108 A
- US 20080125735 A
- WO 2008155722 A2
- WO 2008155701 A
- WO A2 A
- WO 2004071363 A
- WO A1 A
- US 20130174959 A1
- US 8123147 B2
- US 6953510 B
- US 20010008676 A1
- WO 9614038 A
- EP 1570824 B1
- US 20110144610 A
- US 2013165886 A
- US 7642398 B2
- US 7919169 B2
- US 7105716 B
- US 20060258999 A1
- US 2005101929 A
- US 2010262102 A
- EP 2484321 A
- US 2012157953 A
- US 2012157954 A
- US 2012157955 A
- US 2011313381 A

- EP 0640330 A1
- US 6217890 B
- WO 2005097881 A1

- WO 2000041882 A
- WO 2013007819 A1


**Non-patent literature cited in the description**

- **F.L. BUCHHOLZ ; A.T. GRAHAM.** Modern Super-absorbent Polymer Technology. Wiley-VCH, 1998, 252-258
- **F.L. BUCHHOLZ ; A.T. GRAHAM.** Modern Super-absorbent Polymer Technology. Wiley-VCH, 1998, 71-103
- Ullmann's Encyclopedia of Industrial Chemistry. vol. 35, 84

- **F.L. BUCHHOLZ ; A.T. GRAHAM.** Modern Super-absorbent Polymer Technology. Wiley-VCH, 1998, 98
- **ROBERT F. GOULD.** Contact angle, wettability and adhesion. American Chemical Society, 1964
- Electronic Release. Ullmann's Encyclopedia of Industrial Chemistry. 2000
- Standard Test Methods for the Nonwovens Industry. Worldwide Strategic Partners. EDANA (European Disposables and Nonwovens Association